# EUROPEAN PATENT APPLICATION

(11) **EP 4 442 685 A1**
(43) Date of publication of application: **09.10.2024**
(21) Application number: 22903465.7
(22) Date of filing: 06.12.2022
(51) Int. Cl.: C07D 403/12, C07D 471/04, C07D 498/14, A61K 31/496, A61K 31/437, A61K 31/499, A61P 35/00

(54) **ANTI-APOPTOTIC PROTEIN BCL-2 INHIBITOR, PHARMACEUTICAL COMPOSITION AND USES THEREOF**

(30) Priority: 06.12.2021 CN 202111476265; 22.08.2022 CN 202211009107
(71) Applicant: Hangzhou HealZen Therapeutics Co., Ltd., Hangzhou, Zhejiang 310018 (CN); Guangdong Hongye Pharmaceutical Co., Ltd., Zhongshan, Guangdong 528449 (CN)
(72) Inventor: LIU, Xingguo, Hangzhou, Zhejiang 310018 (CN); SU, Mingbo, Zhongshan, Guangdong 528449 (CN); WU, Yizhe, Hangzhou, Zhejiang 310018 (CN); GAO, Anhui, Zhongshan, Guangdong 528449 (CN); ZHOU, Xinglu, Hangzhou, Zhejiang 310018 (CN); ZHONG, Li, Zhongshan, Guangdong 528449 (CN); HU, Miao, Hangzhou, Zhejiang 310018 (CN); HUANG, Jinglai, Hangzhou, Zhejiang 310018 (CN); JING, Hanghui, Hangzhou, Zhejiang 310018 (CN); ZHU, Jianrong, Hangzhou, Zhejiang 310018 (CN)
(74) Representative: Gong, Jinping
(86) International application number: PCT/CN2022/136989
(87) International publication number: WO 2023/104043

(57) **Abstract**

Disclosed is a BCL-2 protein apoptosis inducer, and further disclosed are the uses of the compound and a pharmaceutical composition comprising the compound in the preparation of a drug for treating diseases related to anti-apoptotic protein BCL-2. The compound has potent BCL-2BAK blocking activity, and can be used for treating diseases such as infectious diseases, immune diseases, inflammatory diseases or cell proliferation abnormalities that benefit from the inhibition of anti-apoptotic protein BCL-2.

## Description

### FIELD OF TECHNOLOGY

The present invention belongs to the technical field of pharmaceutical synthesis, and specifically relates to a class of anti-apoptotic protein BCL-2 inhibitors that can inhibit anti-apoptotic B cell lymphoma-2 (Bcl-2), a pharmaceutical composition and uses thereof.

### BACKGROUND

Apoptosis, also known as programmed cell death, is regulated by both external and internal pathways. Apoptosis is closely related to the occurrence and development of many human diseases, and inhibiting apoptosis is related to the production of diseases such as tumors.

Bcl-2 family proteins include anti-apoptotic proteins such as BCL-2, BCL-XL and MCL-1, as well as pro-apoptotic proteins such as Bid, Bim, Bad, Bak, and Bax. In some malignant tumors, members of the anti-apoptotic Bcl-2 family have been found to be upregulated and associated with disease staging and prognosis. Therefore, Bcl-2 protein has been studied as a potential drug therapeutic target and has become a hot topic in anti-tumor drug research in recent years.

It is known that Bcl-2 protein expression can serve as an independent indicator of poor prognosis in tumors such as chronic lymphocytic leukemia (CLL), prostate cancer, and small cell lung cancer (SCLC). In other tumors, such as colon cancer, the Bcl-2 inhibitor Venetoclax (ABT-199) has been approved for marketing by the FDA, and there are also many literatures or patents reporting Bcl-2 inhibitors such as Nature Medicine (2013), 19 (2), 202-210; WO2021208963A/WO2021173523A/WO2020140005A2/WO2019210828A1, etc. But with the use of BCL-2 inhibitors, some patients develop resistance after receiving treatment with Bcl-2 inhibitors. The G101V and D103Y mutations in the BCL-2 gene reduce the efficacy of BCL-2 inhibitors (Cancer Discov. 2019, 9, 342-353). There is an urgent need to find more suitable treatments for BCL-2 inhibitor-resistant patients.

Therefore, there is an urgent need to develop new Bcl-2 inhibitors with better efficacy, higher stability, superior safety, and more effectiveness against mutant cell lines.

### SUMMARY

The purpose of the present invention is to provide a novel and unreported compound that can inhibit anti-apoptotic B cell lymphoma-2 (Bcl-2) family proteins, or an optical isomer, stereoisomer or stereoisomer mixture or pharmaceutical combination of pharmaceutically acceptable salts, and to provide a use in preparation of a drug for treating diseases, disorders or conditions that benefit from inhibiting anti-apoptotic B cell lymphoma-2 (Bcl-2), such as hyperproliferative diseases such as cancer and inflammation, as well as immune and autoimmune diseases.

The first aspect of the present invention provides a compound as shown in formula I, or a stereoisomer or stereoisomer mixture or pharmaceutically acceptable salt thereof, 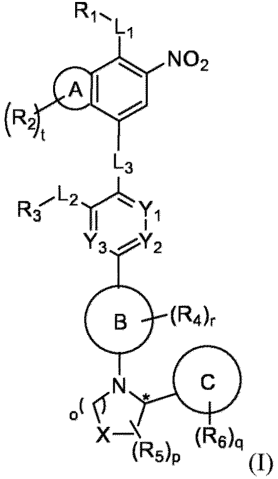
where a C marked with * is of an R configuration or S configuration;
a ring A is selected from absent, 3-10 membered cycloalkyl, 4-10 membered cycloalkenyl, 5-10 membered cycloalkynyl, 4-10 membered heterocyclyl, a 6-10 membered aromatic ring, or a 5-10 membered heteroaromatic ring; and the heterocyclyl and heteroaromatic ring contain 1 to 4 heteroatoms selected from N, O and S;
R₂ is selected from hydrogen, deuterium, -C₁-C₈ alkyl, -C₂-C₈ alkenyl, -C₂-C₈ alkynyl, -C₁-C₈ alkoxy, cycloalkyl, 4-10 membered heterocyclyl, aryl, heteroaryl, halogen, hydroxyl, amino, oxo, cyano, nitro, -OR_{g}, -SR_{g}, -S(O)R_{g}, -SO₂R_{g}, -C(O)R_{g}, -C(O)OR_{g}, - C(O)NRₕRᵢ, -OC(O)R_{g}, -NRₕRᵢ, -NRₕC(O)Rᵢ, -NRₕC(O)NRᵢRₕ, -NRₕC(O)OR_{g}, -NRₕS(O)NRᵢRₕ, -NRₕSO₂NRᵢRₕ, -P(O)RₕRᵢ, halogenated C₁-C₈ alkyl, halogenated C₁-C₈ alkoxy, halogenated C₁-C₈ cycloalkyl, hydroxyl-substituted C₁-C₈ alkyl, or hydroxyl-substituted C₁-C₈ alkoxy; and 2 R₂ can form a 3-6 membered spiral ring when on the same atom and can form a 3-8 membered parallel ring when on adjacent atoms;
Y₁, Y₂ and Y₃ are each independently selected from CR₇ or N;
R₇ is selected from H, halogen (preferably F), C₁-C₈ alkyl, halogenated C₁-C₈ alkyl, cycloalkyl, or alkoxy;
L₂ is selected from a chemical bond, -NRₐ-, -O-, -S-, -C(O)-, -C(O)NRₐ-, or - NRₐC(O)-;
L₃ is selected from -C(O)NR₈SO₂-;
R₈ is selected from H, C₁-C₈ alkyl, -R_{P}OC(O)R_{q}, -RₚOC(O)OR_{q}, -RₚOC(O)NR_{q}Rₛ, or -RₚCOOR_{q};
Rₚ, R_{q} and Rₛ are each independently selected from H, C₁-C₈ alkyl, 3-10 membered cycloalkyl, 4-10 membered heterocyclyl, aryl, or 5-10 membered heteroaryl; and the alkyl, cycloalkyl, heterocyclyl, aryl, and heteroaryl can be further substituted by C₁-C₈ alkyl, 3-10 membered cycloalkyl, 4-10 membered heterocyclyl, aryl, and 5-10 membered heteroaryl;
R₃ is selected from 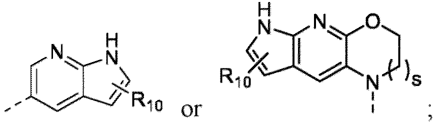
R₁₀ is selected from hydrogen, deuterium, alkyl, alkenyl, alkynyl, cycloalkyl, cycloalkenyl, heterocyclyl, aryl, heteroaryl, halogen, nitro, oxo, cyano, or haloalkyl, where R₁₀ can be substituted on a carbon or nitrogen atom;
a ring B is selected from 5-12 membered spiroheterocyclyl;
a ring C is selected from cycloalkyl, cycloalkenyl, a bridged ring group, heterocyclyl, or aryl or heteroaryl;
X is selected from CR_{b}R_{c} or NR_{b};
o is selected from 0, 1, 2, 3, or 4;
p is selected from 0, 1, 2, or 3;
q is selected from 0, 1, 2, or 3;
r is selected from 0, 1, 2, or 3;
s is selected from 0, 1, 2, or 3;
t is selected from 0, 1, 2, or 3;
Rₐ, R_{b} and R_{c} are each independently selected from H, deuterium, halogen, C₁-C₈ alkyl, alkoxy, cycloalkyl, heterocyclyl, heteroaryl, or aryl; and the alkyl, cycloalkyl, heteroaryl, and aryl can be further substituted by one or more R_{d} substituents;
R_{d} is selected from H, deuterium, halogen, C₁-C₈ alkyl, alkoxy, oxo, amino, hydroxyl, cyano, alkenyl, alkynyl, cycloalkyl, heterocyclyl, aryl, heteroaryl, or a fused bicyclic group; and the alkyl, alkenyl, alkynyl, cycloalkyl, heterocyclyl, heteroaryl, aryl, and fused bicyclic group can be further substituted by one or more Rₑ substituents, and 2 Rₑ can form a 3-6 membered spiral ring when on the same atom and can form a 3-8 membered parallel ring when on adjacent atoms;
Rₑ is selected from H, deuterium, halogen, C₁-C₈ alkyl, C₁-C₈ alkoxy, oxo, amino, hydroxyl, cyano, alkenyl, alkynyl, cycloalkyl, heterocyclyl, haloalkyl, haloalkoxy, methylsulfonyl, aryl, or heteroaryl; the alkyl, alkoxy, alkenyl, alkynyl, cycloalkyl, heterocyclyl, heteroaryl, and aryl can be further substituted by one or more Rₘ; and 2 Rₘ can form a 3-6 membered spiral ring when on the same atom and can form a 3-8 membered parallel ring when on adjacent atoms;
R₄ is selected from H, halogen, C₁-C₈ alkyl, halogenated C₁-C₈ alkyl, cycloalkyl, or alkoxy;
R₅ is selected from H, deuterium, halogen, C₁-C₈ alkyl, haloalkyl, alkoxy, cycloalkyl, heterocyclyl, heteroaryl, or aryl;
R₆ is selected from H, deuterium, halogen, C₁-C₈ alkyl, haloalkyl, alkoxy, cycloalkyl, heterocyclyl, heteroaryl, or aryl;
L₁ is selected from a chemical bond, NR_{f} or O;
R_{f} is selected from H, C₁-C₈ alkyl, haloalkyl, cycloalkyl, heterocyclyl, heteroaryl, or aryl;
R₁ is selected from hydrogen, deuterium, alkyl, a bridged ring group, spirocyclyl, alkenyl, alkynyl, cycloalkyl, cycloalkenyl, heterocyclyl, aryl or heteroaryl, halogen, nitro, oxo, cyano, -OR_{g}, -SR_{g}, alkyl-R_{g}, NH(CH)R_{g}, -C(O)R_{g}, -S(O)R_{g}, -SO₂R_{g}, -C(O)OR_{g}, -OC(O)R_{g}, - NRₕRᵢ, C(O)N(Rₕ)Rᵢ, -N(Rₕ)C(O)Rᵢ, -NRₕC(O)NRᵢRₕ, -NRₕC(O)OR_{g}, -NRₕS(O)NRᵢRₕ, - NRₕSCₕNRᵢRₕ, or -P(O)RₕRᵢ, and the alkyl, bridged ring group, spirocyclyl, alkenyl, alkynyl, cycloalkyl, cycloalkenyl, heterocyclyl, and aryl or heteroaryl can be further substituted by one or more Rⱼ;
R_{g}, Rₕ, Rᵢ, and Rⱼ are selected from hydrogen, deuterium, C₁-C₈ alkyl, spirocyclyl, alkenyl, alkynyl, halogen, cyano, amino, nitro, hydroxyl, oxo, carboxyl, amide, alkoxy, haloalkyl, hydroxyalkyl, aminoalkyl, alkylcarbonyl, alkoxycarbonyl, alkylamino, halohydroxyalkyl, haloalkylamino, cycloalkyl, cycloalkenyl, a bridged ring group, heterocyclyl, or aryl or heteroaryl, and the alkyl, spirocyclyl, alkenyl, alkynyl, alkoxy, hydroxyalkyl, aminoalkyl, alkylcarbonyl, alkoxycarbonyl, alkylamino, cycloalkyl, cycloalkenyl, bridged ring group, heterocyclyl, and aryl or heteroaryl can be further substituted by one or more Rₘ;
Rₘ is selected from hydrogen, deuterium, C₁-C₈ alkyl, halogen, cyano, amino, nitro, hydroxyl, oxo, alkoxy, haloalkyl, hydroxyalkyl, aminoalkyl, alkylcarbonyl, alkoxycarbonyl, alkylamino, halohydroxyalkyl, haloalkylamino, cycloalkyl, or heterocyclyl, and the alkyl, cycloalkyl and heterocyclyl can be further substituted by one or more Rᵣ;
Rᵣ is selected from hydrogen, deuterium, C₁-C₈ alkyl, halogen, cyano, amino, hydroxyl, oxo, alkoxy, hydroxyalkyl, aminoalkyl, heterocyclyl, alkylamino, alkylcarbonyl, alkoxycarbonyl, halohydroxyalkyl, haloalkylamino, haloalkyl, cycloalkyl, spirocyclyl, alkenyl, alkynyl, nitro, carboxyl, amide, cycloalkenyl, a bridged ring, or aryl or heteroaryl; and
when the ring A is selected from absent,
X is selected from NR_{b}, and at least one of Y₁/Y₂/Y₃ is selected from N or CR₇, and R₇ is not H. In some implementations, the ring A is preferably
absent, or

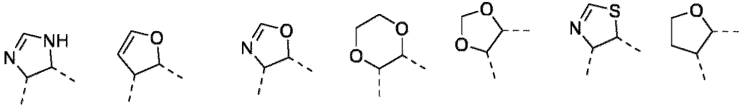

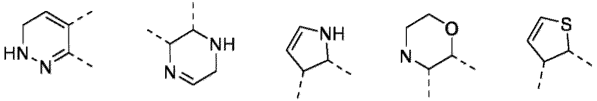

Further, in some implementations, the 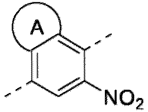 is preferably selected from

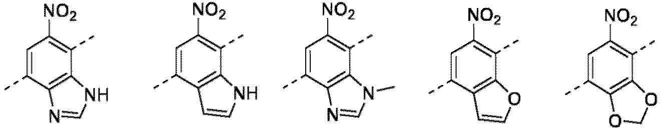

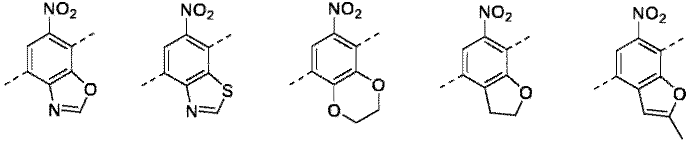

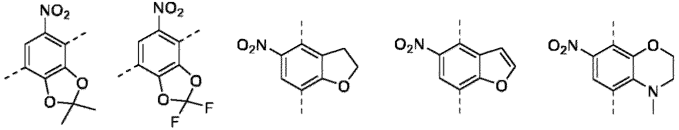

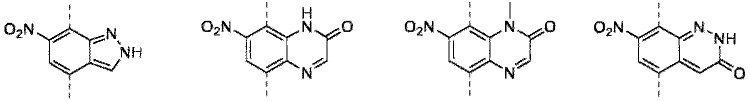

In some implementations, the ring B is preferably

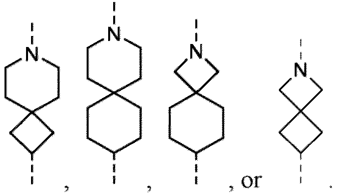

In some implementations, the ring C is preferably an aromatic ring or heteroaromatic ring.

In some implementations, when the ring A is selected from 4-10 membered heterocyclyl or a 5-10 membered heteroaromatic ring, Y₂ is N or CH, CF; and when the ring A is selected from absent, Y₂ is N or CF;
X is selected from NR_{b}, and at least one of Y₁/Y₂/Y₃ is selected from N or CR₇, and R₇ is not H.

Further, the preferred compound of the present invention has a structure of general formula I' : 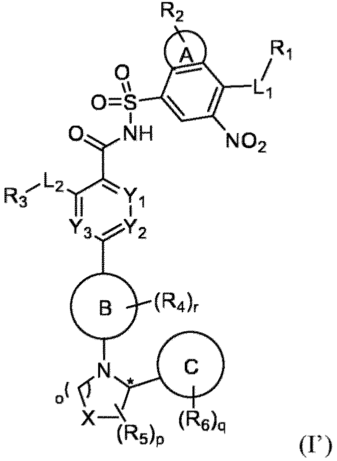
or a stereoisomer or stereoisomer mixture or pharmaceutically acceptable salt thereof,
where a C marked with * is of an R configuration or S configuration;
the ring A is selected from absent, 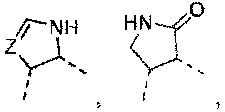 a 6-10 membered heterocyclic ring, a 6-10 membered aromatic ring, or a 6-10 membered heteroaromatic ring;
Z is selected from N or CH;
R₂ is selected from hydrogen, deuterium, C₁-C₈ alkyl, halogen, or halogenated C₁-Cs alkyl;
Y₁, Y₂ and Y₃ are each independently selected from CR₇ or N;
R₇ is selected from H, halogen, C₁-C₈ alkyl, halogenated C₁-C₈ alkyl, cycloalkyl, or alkoxy;
when the ring A is selected from absent, and when two of Y₁, Y₂ and Y₃ are CH,
the other Y₁/Y₂/Y₃ is N or CR₇, and R₇ is not H;
R₃ is selected from 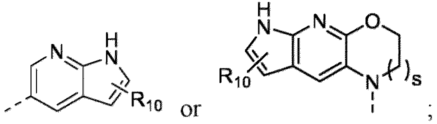
R₁₀ is selected from hydrogen, deuterium, alkyl, alkenyl, alkynyl, cycloalkyl, cycloalkenyl, heterocyclyl, aryl, heteroaryl, halogen, nitro, oxo, cyano, or haloalkyl; and R₁₀ can be substituted on a carbon or nitrogen atom;
the ring B is selected from 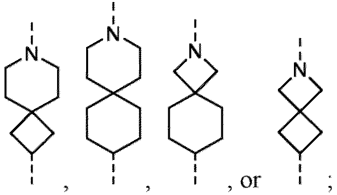
o is selected from 0, 1, 2, 3, or 4;
p is selected from 0, 1, 2, or 3;
q is selected from 0, 1, 2, or 3;
r is selected from 0, 1, 2, or 3;
s is selected from 0, 1, 2, or 3;
L₂ is selected from a chemical bond, NRₐ or O;
X is selected from CR_{b}R_{c} or NR_{b};
Rₐ, R_{b} and R_{c} are each independently selected from H, deuterium, halogen, C₁-C₈ alkyl, alkoxy, cycloalkyl, heterocyclyl, heteroaryl, or aryl; and the alkyl, cycloalkyl, heteroaryl, and aryl can be further substituted by one or more R_{d} substituents;
R_{d} is selected from H, deuterium, halogen, C₁-C₈ alkyl, alkoxy, oxo, amino, hydroxyl, cyano, alkenyl, alkynyl, cycloalkyl, heterocyclyl, aryl, heteroaryl, or a fused bicyclic group; and the alkyl, alkenyl, alkynyl, cycloalkyl, heterocyclyl, heteroaryl, aryl, and fused bicyclic group can be further substituted by one or more Rₑ substituents, and 2 Rₑ can form a 3-6 membered spiral ring when on the same carbon and can form a 3-8 membered parallel ring when on adjacent carbons;
Rₑ is selected from H, deuterium, halogen, C₁-C₈ alkyl, C₁-C₈ alkoxy, oxo, amino, hydroxyl, cyano, alkenyl, alkynyl, cycloalkyl, heterocyclyl, haloalkyl, haloalkoxy, or methyl sulfonyl;
R₄ is selected from H, halogen, C₁-C₈ alkyl, halogenated C₁-C₈ alkyl, cycloalkyl, or alkoxy;
R₅ is selected from H, deuterium, halogen, C₁-C₈ alkyl, haloalkyl, alkoxy, cycloalkyl, heterocyclyl, heteroaryl, or aryl;
R₆ is selected from H, deuterium, halogen, C₁-C₈ alkyl, haloalkyl, alkoxy, cycloalkyl, heterocyclyl, heteroaryl, or aryl;
the ring C is selected from cycloalkyl, cycloalkenyl, a bridged ring group, heterocyclyl, or aryl or heteroaryl;
L₁ is selected from NR_{f} or O;
R_{f} is selected from H, C₁-C₈ alkyl, haloalkyl, cycloalkyl, heterocyclyl, heteroaryl, or aryl;
R₁ is selected from hydrogen, deuterium, alkyl, a bridged ring group, spirocyclyl, alkenyl, alkynyl, cycloalkyl, cycloalkenyl, heterocyclyl, aryl or heteroaryl, halogen, nitro, oxo, cyano, OR_{g}, SR_{g}, alkyl-R_{g}, NH(CH) R_{g}, C(O) R_{g}, S(O) R_{g}, SO₂R_{g}, C(O)OR_{g}, OC(O)R_{g}, NRₕRᵢ, C(O)N(Rₕ)Rᵢ, N(Rₕ)C(O)Rᵢ, or -P(O)RₕRᵢ, and the alkyl, bridged ring group, spirocyclyl, alkenyl, alkynyl, cycloalkyl, cycloalkenyl, heterocyclyl, and aryl or heteroaryl can be further substituted by one or more Rⱼ;
R_{g}, Rₕ, Rᵢ and Rⱼ are selected from hydrogen, deuterium, C₁-C₈ alkyl, spirocyclyl, alkenyl, alkynyl, halogen, cyano, amino, nitro, hydroxyl, oxo, carboxyl, amide, alkoxy, haloalkyl, hydroxyalkyl, aminoalkyl, alkylcarbonyl, alkoxycarbonyl, alkylamino, halohydroxyalkyl, haloalkylamino, cycloalkyl, cycloalkenyl, a bridged ring group, heterocyclyl, or aryl or heteroaryl, and the alkyl, spirocyclyl, alkenyl, alkynyl, alkoxy, hydroxyalkyl, aminoalkyl, alkylcarbonyl, alkoxycarbonyl, alkylamino, cycloalkyl, cycloalkenyl, bridged ring group, heterocyclyl, and aryl or heteroaryl can be further substituted by one or more Rₘ;
Rₘ is selected from hydrogen, deuterium, C₁-C₈ alkyl, halogen, cyano, amino, nitro, hydroxyl, oxo, alkoxy, haloalkyl, hydroxyalkyl, aminoalkyl, alkylcarbonyl, alkoxycarbonyl, alkylamino, halohydroxyalkyl, haloalkylamino, cycloalkyl, or heterocyclyl, and the alkyl, cycloalkyl and heterocyclyl can be further substituted by one or more Rᵣ;
Rᵣ is selected from hydrogen, deuterium, C₁-C₈ alkyl, halogen, cyano, amino, hydroxyl, oxo, alkoxy, hydroxyalkyl, aminoalkyl, alkylcarbonyl, heterocyclyl, alkylamino, alkoxycarbonyl, halohydroxyalkyl, haloalkylamino, haloalkyl, cycloalkyl, spirocyclyl, alkenyl, alkynyl, nitro, carboxyl, amide, cycloalkenyl, a bridged ring, or aryl or heteroaryl; and
further, the preferred compound of the present invention has a structure of general formulas IIa and IIb: 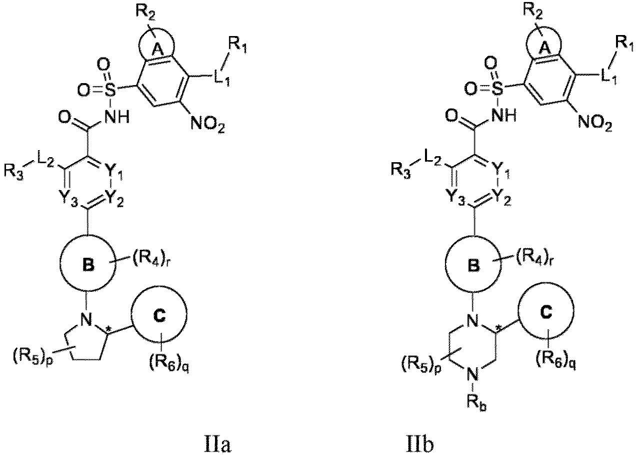
or a stereoisomer or stereoisomer mixture or pharmaceutically acceptable salt thereof;
further, the preferred compound of the present invention has a structure of general formulas IIIa and IIIb: 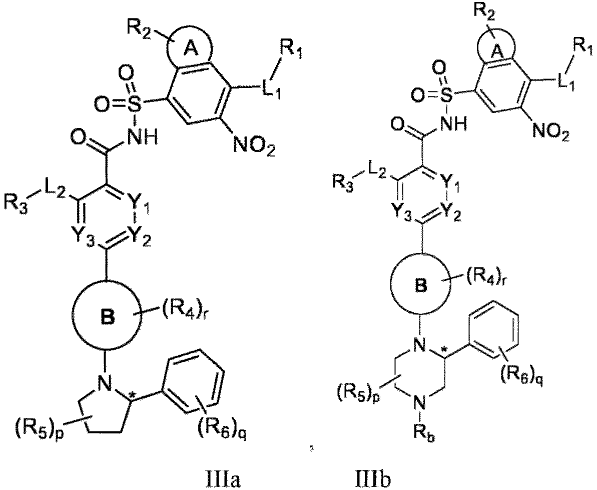
or a stereoisomer or stereoisomer mixture or pharmaceutically acceptable salt thereof;
further, the preferred compound of the present invention has a structure of general formulas IVa, IVb and IVc: 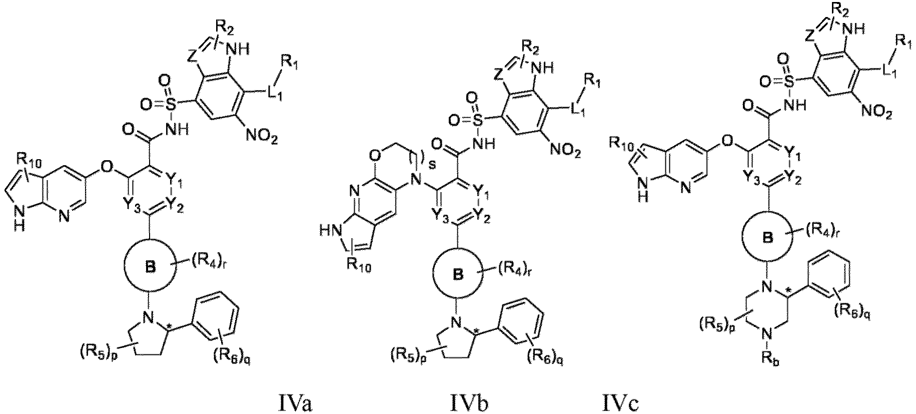
or a stereoisomer or stereoisomer mixture or pharmaceutically acceptable salt thereof,
where R₂ is selected from hydrogen, deuterium, C₁-C₈ alkyl, halogen, or halogenated C₁-C₈ alkyl; and R₂ can be substituted on carbon or nitrogen.

Further, the preferred compound of the present invention has a structure of general formulas Va-Vf: 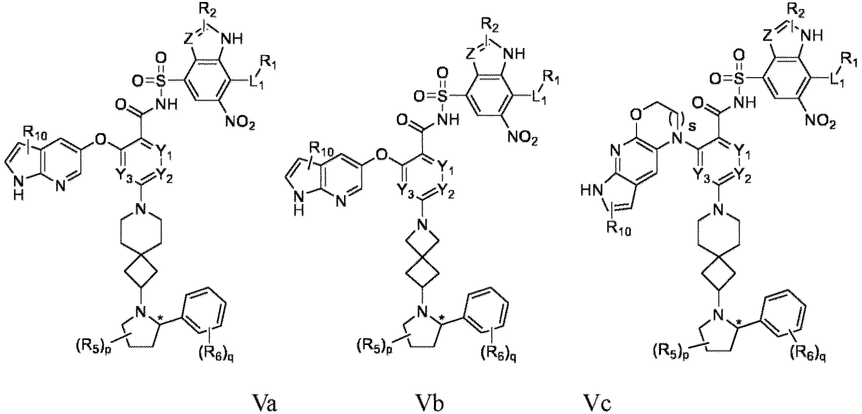 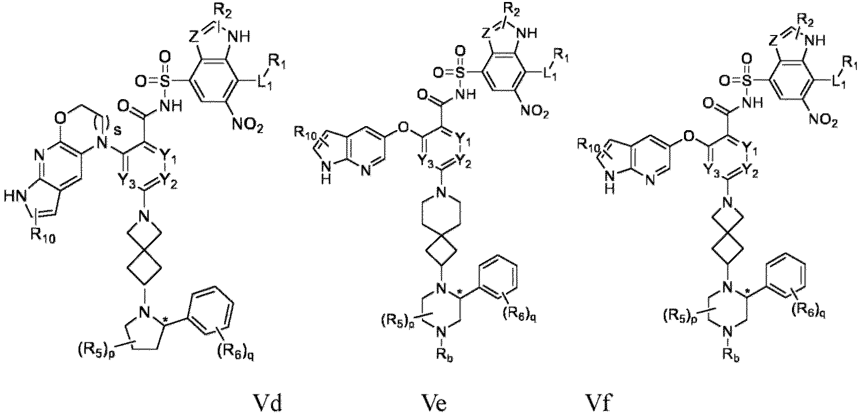
or a stereoisomer or stereoisomer mixture or pharmaceutically acceptable salt thereof,
where R₁₀ is selected from hydrogen, deuterium, alkyl, cycloalkyl, halogen, nitro, cyano, or haloalkyl; and
R₂ is selected from hydrogen, deuterium, C₁-C₈ alkyl, halogen, or halogenated C₁-C₈ alkyl; and R₂ can be substituted on carbon or nitrogen.

Further, the preferred compound of the present invention has a structure of general formulas VIa and VIb: 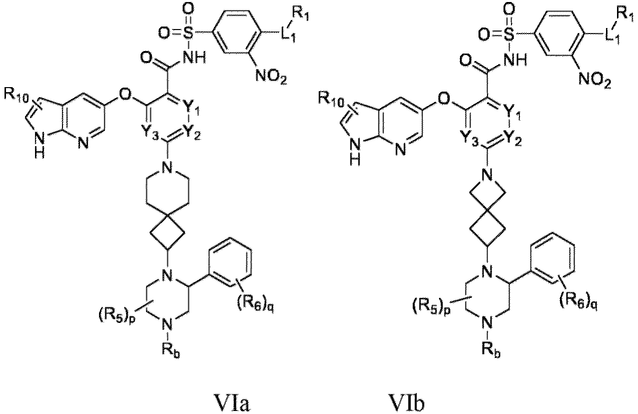
or a stereoisomer or stereoisomer mixture or pharmaceutically acceptable salt thereof,
where when two of Y₁, Y₂ and Y₃ are CH, the other Y₁/Y₂/Y₃ is N or CR₇; and
R₇ is selected from halogen, C₁-C₈ alkyl, halogenated C₁-C₈ alkyl, cycloalkyl, or alkoxy.

Further, for the preferred compound of the present invention:
the ring A is selected from a 4-membered heterocyclic ring, a 5-membered heterocyclic ring, and 5-membered heteroaryl;
L₃ is selected from -C(O)NHSO₂-;
the ring C is selected from aryl or heteroaryl; further preferably, the ring C is selected from a benzene ring; and
the ring B is selected from 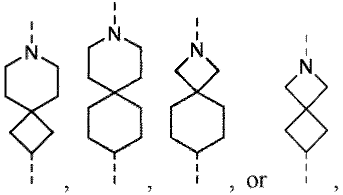 where nitrogen is connected to a ring containing Y₁/Y₂/Y₃.

Further, for the preferred compound and stereoisomer or stereoisomer mixture or pharmaceutically acceptable salt thereof of the present invention:
X is selected from CR_{b}R_{c};
R_{b} and R_{c} are each independently selected from H, deuterium, halogen, C₁-C₈ alkyl, C₁-C₈ alkoxy, cycloalkyl, heterocyclyl, heteroaryl, or aryl;
o is selected from 0, 1 or 2; and
L₂ is selected from a chemical bond, -NH- or -O-;

Further, the preferred compound of the present invention has a structure of general formula (VII): 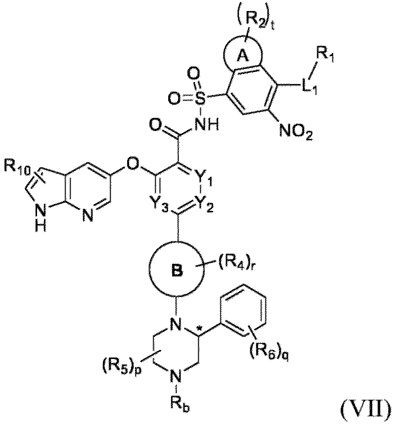
or a stereoisomer or stereoisomer mixture or pharmaceutically acceptable salt thereof,
where
Y₁, Y₂ and Y₃ are each independently selected from CR₇ or N; and
R₇ is selected from H, halogen, C₁-C₈ alkyl, halogenated C₁-C₈ alkyl, cycloalkyl, or alkoxy.

The ring B is selected from

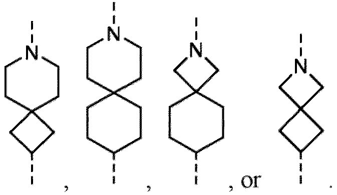

In some implementations, for fragment 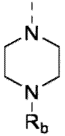 in general formulas (I), (I'), II(b), III(b), IV(c), Ve-Vf, VIa-VIb, and VII, where R_{b} is preferably selected from 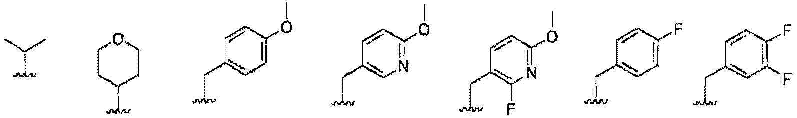 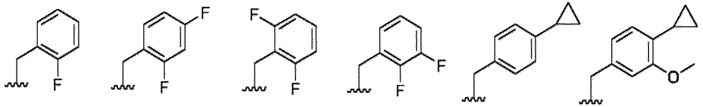 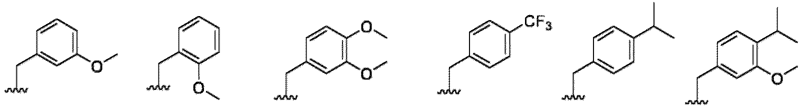 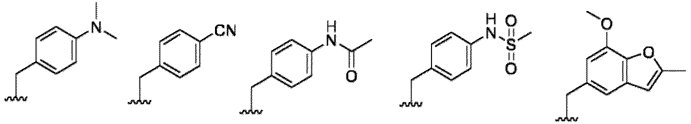 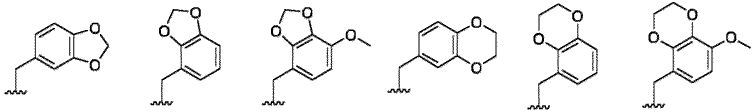 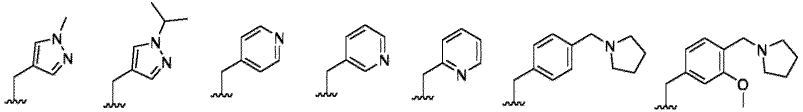 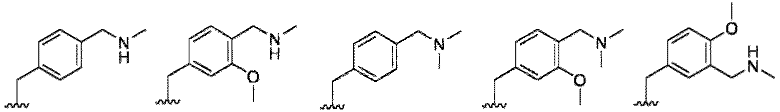 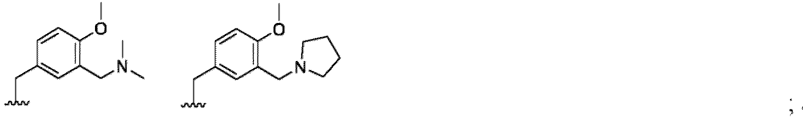 and

in some implementations, R₁ is selected from 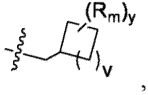 and any carbon atom on its ring can be substituted by one or more Nor O;
y is selected from 0, 1, 2, 3, or 4; and
v is selected from 0, 1, 2, or 3.

In some implementations, fragment -L₁-R₁ of general formulas I, I', IIa-IIb, IIIa-IIIb, IVa-IVc, Va-Vf, VIa, VIb, and VII are selected from 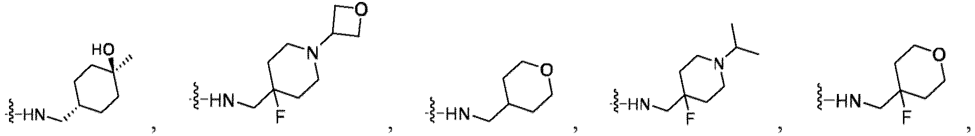 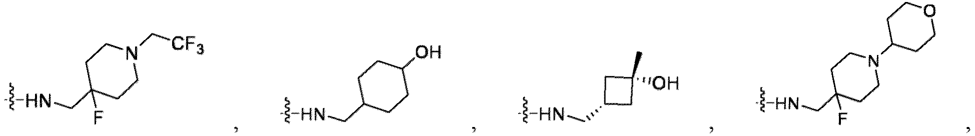 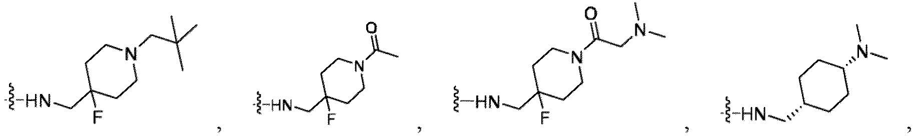 and

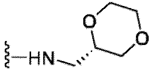

Preferably, the compound or stereoisomer or stereoisomer mixture or pharmaceutically acceptable salt thereof is selected from following compounds:
2-((1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-4-(2-((*S*)-2-(2-isopropylphenyl)pyrrolidin-1-yl)-7-azaspiro[3.5]non-7-yl)benzamide 001
(*S*)-2-((1*H*-pyrrolo[2,3-b]pyrid-5-yl)oxy)-N-((7-((((4-fluoro-1-(oxocyclobut-3-yl)piperid-4-yl)methyl)amino)-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **002**
(*S*)-2-((1*H*-pyrrolo[2,3-b]pyrid-5-yl)oxy)-N-((7-(((4-fluoro-1-(oxocyclobut-3-yl)piperid-4-yl)methyl)amino)-6-nitro-1H-benzo[d]imidazol-4-yl)sulfonyl)-4-(2-(2-(2-isopropylphenyl)pyrrolidin-1-yl)-7-azaspiro[3.5]non-7-yl)benzamide **003**
(*S*)-2-((1*H*-pyrrolo[2,3-b]pyrid-5-yl)oxy)-N-((7-(((4-fluoro-1-isopropylpiperid-4-yl)methyl)amino)-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **004**
(*S*)-2-((1*H*-pyrrolo[2,3-b]pyrid-5-yl)oxy)-N-(7-(((4-fluorotetrahydro-2H-pyran-4-yl)methyl)amino)-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **005**
(*S*)-2-((1*H*-pyrrolo[2,3-b]pyrid-5-yl)oxy)-N-((7-(((4-fluoro-1-(2,2,2-trifluoroethyl)piperid-4-yl)methyl)amino)-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **006**
2-((1*H*-pyrrolo[2,3-b]pyrid-5-yl)oxy)-N-((7-((((1*r*,4*r*)-4-hydroxy-4-methylcyclohexyl)methyl)amino)-1-methyl-6-nitro-1*H*-benzo[*d*]imidazol-4-yl)sulfonyl)-4-(2-((*S*)-2-(2-isopropylphenyl)pyrrolidin-1-yl)-7-azaspiro[3.5]non-7-yl)benzamide **007**
(*S*)-2-((1*H*-pyrrolo[2,3-b]pyrid-5-yl)oxy)-N-((7-(((4-fluoro-1-isopropylpiperid-4-yl)methyl)amino)-1-methyl-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **008**
2-((1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-indol-4-yl)sulfonyl)-4-(2-((*S*)-2-(2-isopropylphenyl)pyrrolidin-1-yl)-7-azaspiro[3.5]non-7-yl)benzamide **009**
(*S*)-2-((1*H*-pyrrolo[2,3-b]pyrid-5-yl)oxy)-N-((7-(((4-fluoro-1-isopropylpiperid-4-yl)methyl)amino)-6-nitro-1*H*-indol-4-yl)sulfonyl)-4-(2-(2-(2-isopropylphenyl)pyrrolidin-1-yl)-7-azaspiro[3.5]non-7-yl)benzamide **010**
2-((3-fluoro-1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-4-(2-((*S*)-2-(2-isopropylphenyl)pyrrolidin-1-yl)-7-azaspiro[3.5]non-7-yl)benzamide **011**
(*S*)-N-((7-(((4-fluoro-1-(oxocyclobut-3-yl)piperid-4-yl)methyl)amino)-6-nitro-1*H*-benzo[*d*]imidazol-4-yl)sulfonyl)-2-((3-fluoro-1*H*-pyrrolo[2,3-b]pyrid-5-yl)oxy)-4-(2-(2-(2-isopropylphenyl)pyrrolidin-1-yl)-7-azaspiro[3.5]non-7-yl)benzamide **012**
4-((1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-6-(2-((*S*)-2-(2-isopropylphenyl)pyrrolidin-1-yl)-7-azaspiro[3.5]non-7-yl)nicotinamide **013**
2-((1*H*-pyrrolo[2,3-b]pyrid-5-yl)oxy)-5-fluoro-N-((7-((((1*r*,4*r*)-4-hydroxy-4-methylcyclohexyl)methyl)amino)-6-nitro-1*H*-benzo[*d*]imidazol-4-yl)sulfonyl)-4-(2-((*S*)-2-(2-isopropylphenyl)pyrrolidin-1-yl)-7-azaspiro[3.5]non-7-yl)benzamide **014**
(*S*)-4-((1*H*-pyrrolo[2,3-b]pyrid-5-yl)oxy)-N-((7-(((4-hydroxycyclohexyl)methyl)amino)-6-nitro-1*H*-benzo[*d*]imidazol-4-yl)sulfonyl)-6-(2-(2-(2-isopropylphenyl)pyrrolidin-1-yl)-7-azaspiro[3.5]non-7-yl)nicotinamide **015**
(*S*)-2-((1*H*-pyrrolo[2,3-b]pyrid-5-yl)oxy)-5-fluoro-N-(7-(((4-hydroxycyclohexyl)methyl)amino)-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **016**
4-((1*H*-pyrrolo[2,3-b]pyrid-5-yl)oxy)-N-((7-((((1*S*,3*S*)-3-hydroxy-3-methylcyclobutyl)methyl)amino)-6-nitro-1*H*-benzo[*d*]imidazol-4-yl)sulfonyl)-6-(2-((*S*)-2-(2-isopropylphenyl)pyrrolidin-1-yl)-7-azaspiro[3.5]non-7-yl)nicotinamide **017**
2-((1*H*-pyrrolo[2,3-b]pyrid-5-yl)oxy)-5-fluoro-N-((7-((((1S,3S)-3-hydroxy-3-methylcyclobutyl)methyl)amino)-6-nitro-1*H*-benzo[*d*]imidazol-4-yl)sulfonyl)-4-(2-((S)-2-(2-isopropylphenyl)pyrrolidin-1-yl)-7-azaspiro[3.5]non-7-yl)benzamide **018**
(*S*)-4-((1H-pyrrolo[2,3-*b*]pyrid-5-yl)oxy)-N-((7-(((4-fluoro-1-(oxocyclobut-3-yl)piperid-4-yl)methyl)amino)-6-nitro-1*H*-benzo[*d*]imidazol-4-yl)sulfonyl)-6-(2-(2-(2-isopropylphenyl)pyrrolidin-1-yl)-7-azaspiro[3.5]non-7-yl)nicotinamide **019**
(*S*)-2-((1H-pyrrolo[2,3-*b*]pyrid-5-yl)oxy)-5-fluoro-N-((7-(((4-fluoro-1-(oxocyclobut-3-yl)piperid-4-yl)methyl)amino)-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **020**
(*S*)-4-((1*H*-pyrrolo[2,3-b]pyrid-5-yl)oxy)-N-((7-(((4-fluoro-1-isopropylpiperid-4-yl)methyl)amino)-6-nitro-1*H*-benzo[*d*]imidazol-4-yl)sulfonyl)-6-(2-(2-(2-isopropylphenyl)pyrrolidin-1-yl)-7-azaspiro[3.5]non-7-yl)nicotinamide **021**
(*S*)-2-((1*H*-pyrrolo[2,3-b]pyrid-5-yl)oxy)-5-fluoro-N-((7-(((4-fluoro-1-isopropylpiperid-4-yl)methyl)amino)-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **022**
(*S*)-4-((1*H*-pyrrolo[2,3-b]pyrid-5-yl)oxy)-N-((7-(((4-fluoro-1-(tetrahydro-2*H-*pyran-4-yl))piperid-4-yl)methyl)amino)-6-nitro-1*H*-benzo[*d*]imidazol-4-yl)sulfonyl)-6-(2-(2-(2-isopropylphenyl)pyrrolidin-1-yl)-7-azaspiro[3.5]non-7-yl)nicotinamide **023**
(*S*)-2-((1*H*-pyrrolo[2,3-b]pyrid-5-yl)oxy)-5-fluoro-N-((7-(((4-fluoro-1-(tetrahydro-2*H*-pyran-4-yl)piperid-4-yl)methyl)amino)-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **024**
(*S*)-4-((1*H*-pyrrolo[2,3-b]pyrid-5-yl)oxy)-N-((7-(((4-fluoro-l-neopentylpiperid-4-yl)methyl)amino)-6-nitro-1*H*-benzo[*d*]imidazol-4-yl)sulfonyl)-6-(2-(2-(2-isopropylphenyl)pyrrolidin-1-yl)-7-azaspiro[3.5]non-7-yl)nicotinamide **025**
(*S*)-2-((1*H*-pyrrolo[2,3-b]pyrid-5-yl)oxy)-5-fluoro-N-((7-(((4-fluoro-1-neopentylpiperid-4-yl)methyl))amino)-6-nitro-1*H-*benzo[*d*]imidazol-4-yl)sulfonyl)-4-(2-(2-(2-isopropylphenyl)pyrrolidin-1-yl)-7-azaspiro[3.5]non-7-yl)benzamide **026**
(*S*)-4-((1*H*-pyrrolo[2,3-b]pyrid-5-yl)oxy)-N-((7-(((1-acetyl-4-fluoropiperid-4-yl)methyl)amino)-6-nitro-1*H*-benzo[*d*]imidazol-4-yl)sulfonyl)-6-(2-(2-(2-isopropylphenyl)pyrrolidin-1-yl)-7-azaspiro[3.5]non-7-yl)nicotinamide **027**
(*S*)-2-((1*H*-pyrrolo[2,3-b]pyrid-5-yl)oxy)-N-((7-(((1-acetyl-4-fluoropiperid-4-yl)methyl)amino)-6-nitro-1*H*-benzo[*d*]imidazol-4-yl)sulfonyl)-5-fluoro-4-(2-(2-(2-isopropylphenyl)pyrrolidin-1-yl)-7-azaspiro[3.5]non-7-yl)benzamide **028**
(*S*)-4-((1*H*-pyrrolo[2,3-b]pyrid-5-yl)oxy)-N-((7-(((1-(dimethylglycyl)-4-fluoropiperid-4-yl)methyl)amino)-6-nitro-1*H*-benzo[*d*]imidazol-4-yl)sulfonyl)-6-(2-(2-(2-isopropylphenyl)pyrrolidin-1-yl)-7-azaspiro[3.5]non-7-yl)nicotinamide **029**
(*S*)-2-((1*H*-pyrrolo[2,3-b]pyrid-5-yl)oxy)-N-((7-(((1-(dimethylglycyl)-4-fluoropiperid-4-yl)methyl)amino)-6-nitro-1*H*-benzo[*d*]imidazol-4-yl)sulfonyl)-5-fluoro-4-(2-(2-(2-isopropylphenyl)pyrrolidin-1-yl)-7-azaspiro[3.5]non-7-yl)benzamide **030**
(*S*)-4-((1*H*-pyrrolo[2,3-b]pyrid-5-yl)oxy)-N-((7-(((4-fluoro-1-(2,2,2-trifluoroethyl)piperid-4-yl)methyl)amino)-6-nitro-1*H*-benzo[*d*]imidazol-4-yl)sulfonyl)-6-(2-(2-(2-isopropylphenyl)pyrrolidin-1-yl)-7-azaspiro[3.5]non-7-yl)nicotinamide **031**
(*S*)-2-((1*H*-pyrrolo[2,3-b]pyrid-5-yl)oxy)-5-fluoro-N-((7-(((4-fluoro-1-(2,2,2-trifluoroethyl)piperid-4-yl)methyl)amino)-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **032**
4-((1*H*-pyrrolo[2,3-b]pyrid-5-yl)oxy)-N-((7-((((1*S*,4*S*)-4-(dimethylamino)cyclohexyl)methyl)amino)-6-nitro-1*H*-benzo[*d*]imidazol-4-yl)sulfonyl)-6-(2-((*S*)-2-(2-isopropylphenyl)pyrrolidin-1-yl)-7-azaspiro[3.5]non-7-yl)nicotinamide **033**
2-((1*H*-pyrrolo[2,3-b]pyrid-5-yl)oxy)-N-((7-((((1*S*,4*S*)-4-(dimethylamino)cyclohexyl)methyl)amino)-6-nitro-1*H*-benzo[*d*]imidazol-4-yl)sulfonyl)-5-fluoro-4-(2-((*S*)-2-(2-isopropylphenyl)pyrrolidin-1-yl)-7-azaspiro[3.5]non-7-yl)benzamide **034**
4-((1*H*-pyrrolo[2,3-b]pyrid-5-yl)oxy)-N-((7-((((1*r*,4*r*)-4-hydroxy-4-methylcyclohexyl)methyl)amino)-1-methyl-6-nitro-1*H*-benzo[*d*]imidazol-4-yl)sulfonyl)-6-(2-((*S*)-2-(2-isopropylphenyl)pyrrolidin-1-yl)-7-azaspiro[3.5]non-7-yl)nicotinamide **035**
(*S*) -2-((1*H*-pyrrolo[2,3-b]pyrid-5-yl)oxy)-5-fluoro-N-((7-(((4-fluoro-1-(oxocyclobut-3-yl)piperid-4-yl)methyl)amino)-1-methyl-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **036**
4-((3-fluoro-1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-6-(2-((*S*)-2-(2-isopropylphenyl)pyrrolidin-1-yl)-7-azaspiro[3.5]non-7-yl)nicotinamide **037**
(*S*)-5-fluoro-N-((7-(((4-fluoro-1-(oxocyclobut-3-yl)piperid-4-yl)methyl)amino)-6-nitro-1*H*-benzo[*d*]imidazol-4-yl)sulfonyl)-2-((3-fluoro-1*H*-pyrrolo[2,3-b]pyrid-5-yl)oxy)-4-(2-(2-(2-isopropylphenyl)pyrrolidin-1-yl)-7-azaspiro[3.5]non-7-yl)benzamide **038**
2-((1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-4-(2-(2-(2-isopropylphenyl)-4-(4-methoxybenzyl)piperazin-1-yl)-7-azaspiro[3.5]non-7-yl)benzamide **039**
2-((1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-4-(6-(2-(2-isopropylphenyl)-4-(4-methoxybenzyl)piperazin-1-yl)-2-azaspiro[3.3]hept-2-yl)benzamide **040**
2-((1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-4-(2-(4-isopropyl-2-(2-isopropylphenyl)piperazin-1-yl)-7-azaspiro[3.5]non-7-yl)benzamide **041**
2-((1*H*-pyrrolo[2,3-b]pyrid-5-yl)oxy)-N-((7-(((4-fluoro-1-isopropylpiperid-4-yl)methyl)amino)-6-nitro-1*H*-benzo[*d*]imidazol-4-yl)sulfonyl)-4-(6-(4-isopropyl-2-(2-isopropylphenyl)piperazin-1-yl)-2-azaspiro[3.3]hept-2-yl)benzamide **042**
2-((1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-4-(2-(2-(2-isopropylphenyl)-4-(tetrahydro-2*H*-pyran-4-yl)piperazin-1-yl)-7-azaspiro[3 .5]non-7-yl)benzamide **043**
2-((1*H*-pyrrolo[2,3-b]pyrid-5-yl)oxy)-N-((7-(((4-fluoro-1-isopropylpiperid-4-yl)methyl)amino)-6-nitro-1*H*-benzo[*d*]imidazol-4-yl)sulfonyl)-4-(6-(2-(2-isopropylphenyl)-4-(tetrahydro-2*H*-pyran-4-yl)piperazin-1-yl)-2-azaspiro[3.3]hept-2-yl)benzamide **044**
2-((1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-4-(2-(2-(2-isopropylphenyl)-4-((6-methoxypyrid-3-yl)methyl)piperazin-1-yl)-7-azaspiro[3.5]non-7-yl)benzamide **045**
2-((1*H*-pyrrolo[2,3-b]pyrid-5-yl)oxy)-N-((7-(((4-fluoro-1-isopropylpiperid-4-yl)methyl)amino)-6-nitro-1*H*-benzo[*d*]imidazol-4-yl)sulfonyl)-4-(6-(2-(2-isopropylphenyl)-4-(4-methoxybenzyl)piperazin-1-yl)-2-azaspiro[3.3]hept-2-yl)benzamide **046**
2-((1*H*-pyrrolo[2,3-b]pyrid-5-yl)oxy)-N-((7-(((4-fluorotetrahydro-2*H*-pyran-4-yl)methyl)amino)-6-nitro-1*H*-benzo[*d*]imidazol-4-yl)sulfonyl)-4-(2-(2-(2-isopropylphenyl)-4-(4-methoxybenzyl)piperazin-1-yl)-7-azaspiro[3.5]non-7-yl)benzamide **047**
2-((1*H*-pyrrolo[2,3-b]pyrid-5-yl)oxy)-N-((7-(((4-fluoro-1-isopropylpiperid-4-yl)methyl)amino)-6-nitro-1*H*-benzo[*d*]imidazol-4-yl)sulfonyl)-4-(2-(2-(2-isopropylphenyl)-4-(4-methoxybenzyl)piperazin-1-yl)-7-azaspiro[3.5]non-7-yl)benzamide **048**
2-((3-fluoro-1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-4-(2-(2-(2-isopropylphenyl)-4-((6-methoxypyrid-3-yl)methyl)piperazin-1-yl)-7-azaspiro[3.5]non-7-yl)benzamide **049**
N-((7-(((4-fluoro-1-isopropylpyrid-4-yl)methyl)amino)-6-nitro-1*H-*benzo[*d*]imidazol-4-yl)sulfonyl)-2-((3-fluoro-1H-pyrrolo[2,3-b]pyrid-5-yl)oxy)-4-(2-(2-(2-isopropylphenyl)-4-(4-methoxybenzyl)piperazin-1-yl)-7-azaspiro[3.5]non-7-yl)benzamide **050**
4-((1*H*-pyrrolo[2,3-b]pyrid-5-yl)oxy)-N-((4-((((1*r*,4*r*)-4-hydroxy-4-methylcyclohexyl)methyl)amino)-3-nitrophenyl)sulfonyl)-6-(2-(2-(2-isopropylphenyl)-4-(tetrahydro-2*H*-pyran-4-yl)piperazin-1-yl)-7-azaspiro[3.5]non-7-yl)nicotinamide **051**
4-((1*H*-pyrrolo[2,3-b]pyrid-5-yl)oxy)-N-((4-((((1*r*,4*r*)-4-hydroxy-4-methylcyclohexyl)methyl)amino)-3-nitrophenyl)sulfonyl)-6-(6-(2-(2-isopropylphenyl)-4-(tetrahydro-2*H*-pyran-4-yl)piperazin-1-yl)-2-azaspiro[3.3]hept-2-yl)nicotinamide **052**
4-((1*H*-pyrrolo[2,3-b]pyrid-5-yl)oxy)-N-((4-(((4-fluoro-1-(oxocyclobut-3-yl)piperid-4-yl)methyl)amino)-3-nitrophenyl)sulfonyl)-6-(2-(2-(2-isopropylphenyl)-4-(4-methoxybenzyl)piperazin-1-yl)-7-azaspiro[3.5]non-7-yl)nicotinamide **053**
4-((1*H*-pyrrolo[2,3-b]pyrid-5-yl)oxy)-N-((4-(((4-fluoro-1-(oxocyclobut-3-yl)piperid-4-yl)methyl)amino)-3-nitrophenyl)sulfonyl)-6-(6-(2-(2-isopropylphenyl)-4-(4-methoxybenzyl)piperazin-1-yl)-2-azaspiro[3.3]hept-2-yl)nicotinamide **054**
2-((1*H*-pyrrolo[2,3-b]pyrid-5-yl)oxy)-5-fluoro-N-((4-((((1*r*,4*r*)-4-hydroxy-4-methylcyclohexyl)methyl)amino)-3-nitrophenyl)sulfonyl)-4-(2-(2-(2-isopropylphenyl)-4-(4-methoxybenzyl)piperazin-1-yl)-7-azaspiro[3.5]non-7-yl)benzamide **055**
2-((1*H*-pyrrolo[2,3-b]pyrid-5-yl)oxy)-5-fluoro-N-((4-((((1*r*,4*r*)-4-hydroxy-4-methylcyclohexyl)methyl)amino)-3-nitrophenyl)sulfonyl)-4-(6-(2-(2-isopropylphenyl)-4-(4-methoxybenzyl)piperazin-1-yl)-2-azaspiro[3.3]hept-2-yl)benzamide **056**
2-((1*H*-pyrrolo[2,3-b]pyrid-5-yl)oxy)-5-fluoro-N-((4-(((4-fluoro-1-(oxocyclobut-3-yl)piperid-4-yl)methyl)amino)-3-nitrophenyl)sulfonyl)-4-(2-(2-(2-isopropylphenyl)-4-(4-methoxybenzyl)piperazin-1-yl)-7-azaspiro[3.5]non-7-yl)benzamide **057**
2-((1*H*-pyrrolo[2,3-b]pyrid-5-yl)oxy)-5-fluoro-N-((4-(((4-fluoro-1-(oxocyclobut-3-yl)piperid-4-yl)methyl)amino)-3-nitrophenyl)sulfonyl)-4-(6-(2-(2-isopropylphenyl)-4-(4-methoxybenzyl)piperazin-1-yl)-2-azaspiro[3.3]hept-2-yl)benzamide **058**
4-((3-fluoro-1*H*-pyrrolo[2,3-b]pyrid-5-yl)oxy)-N-((4-((((1*r*,4*r*)-4-hydroxy-4-methylcyclohexyl)methyl)amino)-3-nitrophenyl)sulfonyl)-6-(2-(2-(2-isopropylphenyl)-4-(tetrahydro-2H-pyran-4-yl)piperazin-1-yl)-7-azaspiro[3.5]non-7-yl)nicotinamide **059**
5-fluoro-2-((3-fluoro-1*H*-pyrrolo[2,3-b]pyrid-5-yl)oxy)-N-((4-((((1*r*,4*r*)-4-hydroxy-4-methylcyclohexyl)methyl)amino)-3-nitrophenyl)sulfonyl)-4-(2-(2-(2-isopropylphenyl)-4-(4-methoxybenzyl)piperazin-1-yl)-7-azaspiro[3.5]non-7-yl)benzamide **060**
N-((4-(((4-fluoro-1-isopropylpiperid-4-yl)methyl)amino)-3-nitrophenyl)sulfonyl)-4-((3-fluoro-1*H*-pyrrolo[2,3-b]pyrid-5-yl)oxy)-6-(2-(2-(2-isopropylphenyl)-4-(4-methoxybenzyl)piperazin-1-yl)-7-azaspiro[3.5]non-7-yl)nicotinamide **061**
5-fluoro-N-(4-((((4-fluoro-1-isopropylpiperid-4-yl)methyl)amino)-3-nitrophenyl)sulfonyl)-2-(3-fluoro-1*H*-pyrrolo[2,3-b]pyrid-5-yl)oxy)-4-(2-(2-(2-isopropylphenyl)-4-(4-methoxybenzyl)piperazin-1-yl)-7-azaspiro[3.5]non-7-yl)benzamide **062**
4-((1*H*-pyrrolo[2,3-b]pyrid-5-yl)oxy)-6-(2-(2-(2-isopropylphenyl)-4-(4-methoxybenzyl)piperazin-1-yl)-7-azaspiro[3.5]non-7-yl)-N-((3-nitro-4-(((tetrahydro-2*H*-pyran-4-yl)methyl)amino) phenyl)sulfonyl)nicotinamide **063**
2-((1*H*-pyrrolo[2,3-b]pyrid-5-yl)oxy)-5-fluoro-4-(2-(2-(2-isopropylphenyl)-4-(4-methoxybenzyl)piperazin-1-yl)-7-azaspiro[3.5]non-7-yl)-N-((3-nitro-4-(((tetrahydro-2*H*-pyran-4-yl)methyl)amino) phenyl)sulfonyl)benzamide **064**
4-((1*H*-pyrrolo[2,3-b]pyrid-5-yl)oxy)-N-((4-(((4-fluorotetrahydro-2H-pyran-4-yl)methyl)amino)-3-nitrophenyl)sulfonyl)-6-(2-(2-(2-isopropylphenyl)-4-(4-methoxybenzyl)piperazin-1-yl)-7-azaspiro[3.5]non-7-yl)nicotinamide **065**
2-((1*H*-pyrrolo[2,3-b]pyiid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-methoxybenzyl)piperazin-1-yl)-7-azaspiro[3.5]non-7-yl)benzamide **066**
4-((1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-6-(2-(2-(2-isopropylphenyl)-4-(4-methoxybenzyl)piperazin-1-yl)-7-azaspiro[3.5]non-7-yl)nicotinamide **067**
4-((1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-6-(6-(2-(2-isopropylphenyl)-4-(4-methoxybenzyl)piperazin-1-yl)-2-azaspiro[3.3]hept-2-yl)nicotinamide 068
4-((1*H*-pyrrolo[2,3-b]pyrid-5-yl)oxy)-N-((7-(((4-fluoro-1-isopropylpiperid-4-yl)methyl)amino)-6-nitro-1*H*-benzo[*d*]imidazol-4-yl)sulfonyl)-6-(2-(2-(2-isopropylphenyl)-4-(4-methoxybenzyl)piperazin-1-yl)-7-azaspiro[3.5]non-7-yl)nicotinamide **069**
4-((1*H*-pyrrolo[2,3-b]pyrid-5-yl)oxy)-N-((7-(((4-fluoro-1-isopropylpiperid-4-yl)methyl)amino)-6-nitro-1*H*-benzo[*d*]imidazol-4-yl)sulfonyl)-6-(6-(2-(2-isopropylphenyl)-4-(4-methoxybenzyl)piperazin-1-yl)-2-azaspiro[3.3]hept-2-yl)nicotinamide **070**
2-((1*H*-pyrrolo[2,3-b]pyrid-5-yl)oxy)-5-fluoro-N-((7-((((1*r*,4*r*)-4-hydroxy-4-methylcyclohexyl)methyl)amino)-6-nitro-1*H*-benzo[*d*]imidazol-4-yl)sulfonyl)-4-(2-(2-(2-isopropylphenyl)-4-(4-methoxybenzyl)piperazin-1-yl)-7-azaspiro[3.5]non-7-yl)benzamide **071**
2-((1*H*-pyrrolo[2,3-b]pyrid-5-yl)oxy)-5-fluoro-N-((7-((((1*r*,4*r*)-4-hydroxy-4-methylcyclohexyl)methyl)amino)-6-nitro-1*H*-benzo[*d*]imidazol-4-yl)sulfonyl)-4-(6-(2-(2-isopropylphenyl)-4-(4-methoxybenzyl)piperazin-1-yl)-2-azaspiro[3.3]hept-2-yl)benzamide **072**
2-((1*H*-pyrrolo[2,3-b]pyrid-5-yl)oxy)-5-fluoro-N-((7-(((4-fluoro-1-isopropylpiperid-4-yl)methyl)amino)-6-nitro-1*H*-benzo[*d*]imidazol-4-yl)sulfonyl)-4-(2-(2-(2-isopropylphenyl)-4-(4-methoxybenzyl)piperazin-1-yl)-7-azaspiro[3.5]non-7-yl)benzamide **073**
2-((1*H*-pyrrolo[2,3-b]pyrid-5-yl)oxy)-5-fluoro-N-((7-(((4-fluoro-1-isopropylpiperid-4-yl)methyl)amino)-6-nitro-1H-benzo[d]imidazol-4-yl)sulfonyl)-4-(6-(2-(2-isopropylphenyl)-4-(4-methoxybenzyl)piperazin-1-yl)-2-azaspiro[3.3]hept-2-yl)benzamide **074**
4-((1*H*-pyrrolo[2,3-b]pyrid-5-yl)oxy)-6-(2-(2-(2-isopropylphenyl)-4-(4-methoxybenzyl)piperazin-1-yl)-7-azaspiro[3.5]non-7-yl)-N-((6-nitro-7-(((tetrahydro-2*H*-pyran-4-yl)methyl)amino)-1*H*-benzo[*d*]imidazol-4-yl)sulfonyl)nicotinamide **075**
2-((1*H*-pyrrolo[2,3-b]pyrid-5-yl)oxy)-5-fluoro-4-(2-(2-(2-isopropylphenyl)-4-(4-methoxybenzyl)piperazin-1-yl)-7-azaspiro[3.5]non-7-yl)-N-((6-nitro-7-(((tetrahydro-2H-pyran-4-yl)methyl)amino)-1*H*-benzo[d]imidazol-4-yl)sulfonyl)benzamide **076**
4-((1*H*-pyrrolo[2,3-b]pyrid-5-yl)oxy)-N-((7-(((4-fluorotetrahydro-2*H*-pyran-4-yl)methyl)amino)-6-nitro-1H-benzo[d]imidazol-4-yl)sulfonyl)-6-(2-(2-(2-isopropylphenyl)-4-(4-methoxybenzyl)piperazin-1-yl)-7-azaspiro[3.5]non-7-yl)nicotinamide **077**
2-((1*H*-pyrrolo[2,3-b]pyrid-5-yl)oxy)-5-fluoro-N-((7-(((4-fluorotetrahydro-2*H-*pyran-4-yl)methyl)amino)-6-nitro-1*H-*benzo[*d*]imidazol-4-yl)sulfonyl)-4-(2-(2-(2-isopropylphenyl)-4-(4-methoxybenzyl)piperazin-1-yl)-7-azaspiro[3.5]non-7-yl)benzamide **078**
2-(2,3-dihydropyrrolo[3',2':5,6]pyridino[2,3-b][1,4]oxazin-1(6*H*)-yl)-N-((7-((((1*r*,4*r*)-4-hydroxy-4-methylcyclohexyl)methyl)amino)-6-nitro-1*H*-benzo[*d*]imidazol-4-yl)sulfonyl)-4-(2-((*S*)-2-(2-isopropylphenyl)pyrrolidin-1-yl)-7-azaspiro[3.5]non-7-yl)benzamide 079
(*S*)-2-(2,3-dihydropyrrolo[3',2':5,6]pyridino[2,3-b][1,4]oxazin-1(6H)-yl)-N-((7-(((4-fluoro-1-(oxocyclobut-3-yl)piperid-4-yl)methyl)amino)-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **080**
4-(2,3-dihydropyrrolo[3',2':5,6]pyridino[2,3-b][1,4]oxazin-1(6H)-yl)-N-((7-((((1*r*,4*r*)-4-hydroxy-4-methylcyclohexyl)methyl)amino)-6-nitro-1*H*-benzo[*d*]imidazol-4-yl)sulfonyl)-6-(2-((*S*)-2-(2-isopropylphenyl)pyrrolidin-1-yl)-7-azaspiro[3.5]non-7-yl)nicotinamide **081**
2-(2,3-dihydropyrrolo[3',2':5,6]pyridino[2,3-b][1,4]oxazin-1(6*H*)-yl)-5-fluoro-N-((7-((((1*r*,4*r*)-4-hydroxy-4-methylcyclohexyl)methyl)amino)-6-nitro-1*H*-benzo[*d*]imidazol-4-yl)sulfonyl)-4-(2-((*S*)-2-(2-isopropylphenyl)pyrrolidin-1-yl)-7-azaspiro[3.5]non-7-yl)benzamide **082**
(*S*)-4-(2,3-dihydropyrrolo[3',2':5,6]pyridino[2,3-b][1,4]oxazin-1(6*H*)-yl)-N-((7-(((4-fluoro-1-isopropylpiperid-4-yl)methyl)amino)-6-nitro-1*H*-benzo[*d*]imidazol-4-yl)sulfonyl)-6-(2-(2-(2-isopropylphenyl)pyrrolidin-1-yl)-7-azaspiro[3.5]non-7-yl)nicotinamide **083**
(*S*)-2-(2,3-dihydropyrrolo[3',2':5,6]pyridino[2,3-b][1,4]oxazin-1(6*H*)-yl)-5-fluoro-N-((7-(((4-fluoro-1-isopropylpiperid-4-yl)methyl)amino)-6-nitro-1H-benzo[d]imidazol-4-yl)sulfonyl)-4-(2-(2-(2-isopropylphenyl)pyrrolidin-1-yl)-7-azaspiro[3.5]non-7-yl)benzamide **084**
2-(3,4-dihydro-2*H*-pyrrolo[3',2':5,6]pyridino[2,3-b][1,4]oxazacyclohept-1(7*H*)-yl)-N-((7-((((1*r*,4*r*)-4-hydroxy-4-methylcyclohexyl)methyl)amino)-6-nitro-1*H-*benzo[*d*]imidazol-4-yl)sulfonyl)-4-(2-((*S*)-2-(2-isopropylphenyl)pyrrolidin-1-yl)-7-azaspiro[3.5]non-7-yl)benzamide **085**
(*S*)-2-(3,4-dihydro-2*H*-pyrrolo[3',2':5,6]pyridino[2,3-b][1,4] oxazacyclohept-1(7*H*)-yl)-N-((7-(((4-fluoro-1-isopropylpiperid-4-yl)methyl)amino)-6-nitro-1*H-*benzo[*d*]imidazol-4-yl)sulfonyl)-4-(2-(2-(2-isopropylphenyl)pyrrolidin-1-yl)-7-azaspiro[3.5]non-7-yl)benzamide **086**
(*S*)-2-(2,3-dihydropyrrolo[3',2':5,6]pyridino[2,3-b][1,4]oxazin-1(6*H*)-yl)-N-((7-(((4-fluorotetrahydro-2*H*-pyran-4-yl)methyl)amino)-6-nitro-1*H*-benzo[*d*]imidazol-4-yl)sulfonyl)-4-(2-(2-(2-isopropylphenyl)pyrrolidin-1-yl)-7-azaspiro[3.5]non-7-yl)benzamide **087**
(*S*)-2-(3,4-dihydro-2*H*-pyrrolo[3',2':5,6]pyridino[2,3-b][1,4] oxazacyclohept-1(7*H*)-yl)-N-((7-(((4-fluorotetrahydro-2*H*-pyran-4-yl)methyl)amino)-6-nitro-1*H-*benzo[*d*]imidazol-4-yl)sulfonyl)-4-(2-(2-(2-isopropylphenyl)pyrrolidin-1-yl)-7-azaspiro[3.5]non-7-yl)benzamide **088**
2-(2,3-dihydropyrrolo[3',2':5,6]pyridino[2,3-b][1,4]oxazin-1(6*H*)-yl)-N-((7-((((1*r*,4*r*)-4-hydroxy-4-methylcyclohexyl)methyl)amino)-6-nitro-1*H*-benzo[*d*]imidazol-4-yl)sulfonyl)-4-(2-(2-(2-isopropylphenyl)-4-(4-methoxybenzyl)piperazin-1-yl)-7-azaspiro[3.5]non-7-yl)benzamide **089**
2-(3,4-dihydro-2*H*-pyrrolo[3',2':5,6]pyridino[2,3-b][1,4] oxazacyclohept-1(7*H*)-yl)-N-((7-((((1*r*,4*r*)-4-hydroxy-4-methylcyclohexyl)methyl)amino)-6-nitro-1*H-*benzo[*d*]imidazol-4-yl)sulfonyl)-4-(2-(2-(2-isopropylphenyl)-4-(4-methoxybenzyl)piperazin-1-yl)-7-azaspiro[3.5]non-7-yl)benzamide **090**
2-(2,3-dihydropyrrolo[3',2':5,6]pyridino[2,3-b][1,4]oxazin-1(6*H*)-yl)-4-(2-(2-(2-isopropylphenyl)-4-(4-methoxybenzyl)piperazin-1-yl)-7-azaspiro[3.5]non-7-yl)-N-((6-nitro-7-(((tetrahydro-2*H*-pyran-4-yl)methyl)amino)-1*H-*benzo[*d*]imidazol-4-yl)sulfonyl)benzamide **091**
2-(3,4-dihydro-2H-pyrrolo[3',2':5,6]pyridino[2,3-b][1,4]oxazacyclohept-1(7*H*)-yl)-4-(2-(2-(2-isopropylphenyl)-4-(4-methoxybenzyl)piperazin-1-yl)-7-azaspiro[3.5]non-7-yl)-N-((6-nitro-7-(((tetrahydro-2*H*-pyran-4-yl)methyl)amino)-1*H* benzo[d]imidazol-4-yl)sulfonyl)benzamide **092**
2-(2,3-dihydropyrrolo[3',2':5,6]pyridino[2,3-b][1,4]oxazin-1(6*H*)-yl)-N-((7-(((4-fluorotetrahydro-2*H*-pyran-4-yl)methyl)amino)-6-nitro-1*H-*benzo[*d*]imidazol-4-yl)sulfonyl)-4-(2-(2-(2-isopropylphenyl)-4-(4-methoxybenzyl)piperazin-1-yl)-7-azaspiro[3.5]non-7-yl)benzamide **093**
2-(3,4-dihydro-2*H*-pyrrolo[3',2':5,6]pyridino[2,3-b][1,4] oxazacycloheptane - 1(7*H*)-yl)-N-((7-(((4-fluorotetrahydro-2H-pyran-4-yl)methyl)amino)-6-nitro-1H-benzo[d]imidazol-4-yl)sulfonyl)-4-(2-(2-(2-isopropylphenyl)-4-(4-methoxybenzyl)piperazin-1-yl)-7-azaspiro[3.5]non-7-yl)benzamide **094**
2-((1*H*-pyrrolo[2,3-b]pyrid-5-yl)oxy)-5-fluoro-N-((7-((((1*r*, 4*r*)-4-hydroxy-4-methylcyclohexyl)methyl)amino)-6-nitrobenzofuran-4-yl)sulfonyl)-4-(2-((*S*)-2-(2-isopropylphenyl)pyrrolidin-1-yl)-7-azaspiro[3.5]non-7-yl)benzamide **095**
2-((1*H*-pyrrolo[2,3-b]pyrid-5-yl)oxy)-5-fluoro-N-((7-((((1*r*, 4*r*)-4-hydroxy-4-methylcyclohexyl)methyl)amino)-6-nitrobenzo[*d*][1,3]dioxocyclopent-4-yl)sulfonyl)-4-(2-((*S*)-2-(2-isopropylphenyl)pyrrolidin-1-yl)-7-azaspiro[3.5]non-7-yl)benzamide **096**
2-((1*H*-pyrrolo[2,3-b]pyrid-5-yl)oxy)-5-fluoro-N-((7-((((1*r*, 4*r*)-4-hydroxy-4-methylcyclohexyl)methyl)amino)-6-nitrobenzo[*d*] oxazol-4-yl)sulfonyl)-4-(2-((*S*)-2-(2-isopropylphenyl)pyrrolidin-1-yl)-7-azaspiro[3.5]non-7-yl)benzamide **097**
2-((1*H*-pyrrolo[2,3-b]pyrid-5-yl)oxy)-5-fluoro-N-((7-((((1*r*, 4*r*)-4-hydroxy-4-methylcyclohexyl)methyl)amino)-6-nitrobenzo[*d*] thiazol-4-yl)sulfonyl)-4-(2-((*S*)-2-(2-isopropylphenyl)pyrrolidin-1-yl)-7-azaspiro[3.5]non-7-yl)benzamide **098**
2-((1*H*-pyrrolo[2,3-b]pyrid-5-yl)oxy)-5-fluoro-N-((8-((((1*r*, 4*r*)-4-hydroxy-4-methylcyclohexyl)methyl)amino)-7-nitro-2,3-dihydrobenzo[b][1,4]dioxan-5-yl)sulfonyl)-4-(2-((*S*)-2-(2-isopropylphenyl)pyrrolidin-1-yl)-7-azaspiro[3.5]non-7-yl)benzamide **099**
2-((1*H*-pyrrolo[2,3-b]pyrid-5-yl)oxy)-5-fluoro-N-((7-((((1*r*, 4*r*)-4-hydroxy-4-methylcyclohexyl)methyl)amino)-6-nitro-2,3-dihydrobenzofuran-4-yl)sulfonyl)-4-(2-((*S*)-2-(2-isopropylphenyl)pyrrolidin-1-yl)-7-azaspiro[3.5]non-7-yl)benzamide **100**
(*S*)-2-((1*H*-pyrrolo[2,3-b]pyrid-5-yl)oxy)-5-fluoro-N-((7-(((4-fluorotetrahydro-2*H-*pyran-4-yl)methyl)amino)-6-nitrobenzo[*d*][1,3]dioxocyclopent-4-yl)sulfonyl)-4-(2-(2-(2-isopropylphenyl)pyrrolidin-1-yl)-7-azaspiro[3.5]non-7-yl)benzamide **101**
(*S*)-2-((1*H*-pyrrolo[2,3-b]pyrid-5-yl)oxy)-5-fluoro-N-((7-(((4-fluorotetrahydro-2H-pyran-4-yl)methyl)amino)-6-nitrobenzofuran-4-yl)sulfonyl)-4-(2-(2-(2-isopropylphenyl)pyrrolidin-1-yl)-7-azaspiro[3.5]non-7-yl)benzamide **102**
(*S*)-2-((1*H*-pyrrolo[2,3-b]pyrid-5-yl)oxy)-5-fluoro-N-((7-(((4-fluorotetrahydro-2H-pyran-4-yl)methyl)amino)-6-nitrobenzo[d] oxazol-4-yl)sulfonyl)-4-(2-(2-(2-isopropylphenyl)pyrrolidin-1-yl)-7-azaspiro[3.5]non-7-yl)benzamide **103**
(*S*)-2-((1*H*-pyrrolo[2,3-b]pyrid-5-yl)oxy)-5-fluoro-N-((7-(((4-fluorotetrahydro-2H-pyran-4-yl)methyl)amino)-6-nitrobenzo[*d*] thiazol-4-yl)sulfonyl)-4-(2-(2-(2-isopropylphenyl)pyrrolidin-1-yl)-7-azaspiro[3.5]non-7-yl)benzamide **104**
(*S*)-2-((1*H*-pyrrolo[2,3-b]pyrid-5-yl)oxy)-5-fluoro-N-((7-(((4-fluorotetrahydro-2H pyran-4-yl)methyl)amino)-6-nitro-2,3-dihydrobenzofuran-4-yl)sulfonyl)-4-(2-(2-(2-isopropylphenyl)pyrrolidin-1-yl)-7-azaspiro[3.5]non-7-yl)benzamide **105**
(*R*)-2-((1*H*-pyrrolo[2,3-b]pyrid-5-yl)oxy)-5-fluoro-N-((7-(((4-fluorotetrahydro-2*H*-pyran-4-yl)methyl)amino)-6-nitrobenzo[*d*][1,3]dioxocyclopent-4-yl)sulfonyl)-4-(2-(2-(2-isopropylphenyl)-4-(4-methoxybenzyl)piperazin-1-yl)-7-azaspiro[3.5]non-7-yl)benzamide **106**
(*R*)-2-((1*H-*pyrrolo[2,3-b]pyrid-5-yl)oxy)-5-fluoro-N-((7-(((4-fluorotetrahydro-2*H*-pyran-4-yl)methyl)amino)-6-nitrobenzo[*d*] oxazol-4-yl)sulfonyl)-4-(2-(2-(2-isopropylphenyl)-4-(4-methoxybenzyl)piperazin-1-yl)-7-azaspiro[3.5]non-7-yl)benzamide **107**
(*R*)-2-((1*H*-pyrrolo[2,3-b]pyrid-5-yl)oxy)-5-fluoro-N-((7-(((4-fluorotetrahydro-2*H*-pyran-4-yl)methyl))amino)-6-nitrobenzo[d] thiazol-4-yl)sulfonyl)-4-(2-(2-(2-isopropylphenyl)-4-(4-methoxybenzyl)piperazin-1-yl)-7-azaspiro[3.5]non-7-yl)benzamide **108**
(*R*)-2-((1*H*-pyrrolo[2,3-b]pyrid-5-yl)oxy)-5-fluoro-N-((7-(((4-fluorotetrahydro-2*H*-pyran-4-yl)methyl)amino)-6-nitrobenzofuran-4-yl)sulfonyl)-4-(2-(2-(2-isopropylphenyl)-4-(4-methoxybenzyl)piperazin-1-yl)-7-azaspiro[3.5]non-7-yl)benzamide **109**
(*R*)-2-((1*H*-pyrrolo[2,3-b]pyrid-5-yl)oxy)-5-fluoro-N-((8-(((4-fluorotetrahydro-2*H*-pyran-4-yl)methyl)amino)-7-nitro-2,3-dihydrobenzo[b][1,4]dioxane-5yl)sulfonyl)-4-(2-(2-(2-isopropylphenyl)-4-(4-methoxybenzyl)piperazin-1-yl)-7-azaspiro[3.5]non-7-yl)benzamide 110
(*R*)-2-((1*H*-pyrrolo[2,3-b]pyrid-5-yl)oxy)-5-fluoro-N-((7-(((4-fluorotetrahydro-2H-pyran-4-yl)methyl)amino)-2-methyl-6-nitrobenzofuran-4-yl)sulfonyl)-4-(2-(2-(2-isopropylphenyl)-4-(4-methoxybenzyl)piperazin-1-yl)-7-azaspiro[3.5]non-7-yl)benzamide **111**
(*R*)-2-((1*H*-pyrrolo[2,3-b]pyrid-5-yl)oxy)-5-fluoro-N-((7-(((4-fluorotetrahydro-2H-pyran-4-yl)methyl)amino)-6-nitro-2,3-dihydrobenzofuran-4-yl)sulfonyl)-4-(2-(2-(2-isopropylphenyl)-4-(4-methoxybenzyl)piperazin-1-yl)-7-azaspiro[3.5]non-7-yl)benzamide **112**
2-((1*H*-pyrrolo[2,3-b]pyrid-5-yl)oxy)-5-fluoro-N-((7-((((1*r*, 4*r*)-4-hydroxy-4-methylcyclohexyl)methyl)amino)-6-nitrobenzo[*d*][1,3]dioxocyclopent-4-yl)sulfonyl)-4-(2-((R)-2-(2-isopropylphenyl)-4-(4-methoxybenzyl)piperazin-1-yl)-7-azaspiro[3.5]non-7-yl)benzamide **113**
2-((1*H*-pyrrolo[2,3-b]pyrid-5-yl)oxy)-5-fluoro-N-((7-((((1*r*, 4*r*)-4-hydroxy-4-methylcyclohexyl)methyl)amino)-6-nitrobenzo[*d*] oxazol-4-yl)sulfonyl)-4-(2-((*R*)-2-(2-isopropylphenyl)-4-(4-methoxybenzyl)piperazin-1-yl)-7-azaspiro[3.5]non-7-yl)benzamide **114**
2-((1*H*-pyrrolo[2,3-b]pyrid-5-yl)oxy)-5-fluoro-N-((7-((((1*r*, 4*r*)-4-hydroxy-4-methylcyclohexyl)methyl)amino)-6-nitrobenzofuran-4-yl)sulfonyl)-4-(2-((*R*)-2-(2-isopropylphenyl)-4-(4-methoxybenzyl)piperazin-1-yl)-7-azaspiro[3.5]non-7-yl)benzamide **115**
2-((1*H*-pyrrolo[2,3-b]pyrid-5-yl)oxy)-5-fluoro-N-((7-((((1*r*, 4*r*)-4-hydroxy-4-methylcyclohexyl)methyl)amino)-6-nitrobenzo[*d*] thiazol-4-yl)sulfonyl)-4-(2-((*R*)-2-(2-isopropylphenyl)-4-(4-methoxybenzyl)piperazin-1-yl)-7-azaspiro[3.5]non-7-yl)benzamide **116**
2-((1*H*-pyrrolo[2,3-b]pyrid-5-yl)oxy)-5-fluoro-N-((7-((((1*r*, 4*r*)-4-hydroxy-4-methylcyclohexyl)methyl)amino)-2-methyl-6-nitrobenzofuran-4-yl)sulfonyl)-4-(2-((*R*)-2-(2-isopropylphenyl)-4-(4-methoxybenzyl)piperazin-1-yl)-7-azaspiro[3.5]non-7-yl)benzamide **117**
2-((1*H*-pyrrolo[2,3-b]pyrid-5-yl)oxy)-5-fluoro-N-((8-((((1*r*, 4*r*)-4-hydroxy-4-methylcyclohexyl)methyl)amino)-7-nitro-2,3-dihydrobenzo[b][1,4]dioxan-5-yl)sulfonyl)-4-(2-((*R*)-2-(2-isopropylphenyl)-4-(4-methoxybenzyl)piperazin-1-yl)-7-azaspiro[3.5]non-7-yl)benzamide **118**
2-((1*H*-pyrrolo[2,3-b]pyrid-5-yl)oxy)-5-fluoro-N-((7-((((1*r*, 4*r*)-4-hydroxy-4-methylcyclohexyl)methyl)amino)-6-nitro-2,3-dihydrobenzofuran-4-yl)sulfonyl)-4-(2-((*R*)-2-(2-isopropylphenyl)-4-(4-methoxybenzyl)piperazin-1-yl)-7-azaspiro[3.5]non-7-yl)benzamide **119**
(*R*)-2-((1*H-*pyrrolo[2,3-b]pyrid-5-yl)oxy)-5-fluoro-4-(2-(2-(2-isopropylphenyl)-4-(4-methoxybenzyl)piperazin-1-yl)-7-azaspiro[3.5]non-7-yl)-N-((6-nitro-7-(((tetrahydro-2*H-*pyran-4-yl)methyl)amino)benzo[*d*][1,3]dioxocyclopent-4-yl)sulfonyl)benzamide **120**
(*R*)-2-((1*H*-pyrrolo[2,3-b]pyrid-5-yl)oxy)-5-fluoro-4-(2-(2-(2-isopropylphenyl)-4-(4-methoxybenzyl)piperazin-1-yl)-7-azaspiro[3.5]non-7-yl)-N-((6-nitro-7-(((tetrahydro-2*H-*pyran-4-yl)methyl)amino)benzo[*d*] oxazol-4-yl)sulfonyl)benzamide **121**
(*R*)-2-((1*H*-pyrrolo[2,3-b]pyrid-5-yl)oxy)-5-fluoro-4-(2-(2-(2-isopropylphenyl)-4-(4-methoxybenzyl)piperazin-1-yl)-7-azaspiro[3.5]non-7-yl)-N-((6-nitro-7-(((tetrahydro-2*H-*pyran-4-yl)methyl)amino)benzo[*d*] thiazol-4-yl)sulfonyl)benzamide **122**
N-((7-((((*S*)-1,4-dioxan-2-yl)methyl)amino)-6-nitrobenzo[*d*][1,3]dioxocyclopent-4-yl)sulfonyl)-2-(1*H*pyrrolo[2,3-b]pyrid-5-yl)oxy)-5-fluoro-4-(2-((*R*)-2-(2-isopropylphenyl)-4-(4-methoxybenzyl)piperazin-1-yl)-7-azaspiro[3.5]non-7-yl)benzamide **123**
N-((7-((((*S*)-1,4-dioxan-2-yl)methyl)amino)-6-nitrobenzo[*d*] oxazol-4-yl)sulfonyl)-2-(1*H*-pyrrolo[2,3-b]pyrid-5-yl)oxy)-5-fluoro-4-(2-((*R*)-2-(2-isopropylphenyl)-4-(4-methoxybenzyl)piperazin-1-yl)-7-azaspiro[3.5]non-7-yl)benzamide **124**
N-((7-((((*S*)-1,4-dioxan-2-yl)methyl)amino)-6-nitrobenzo[*d*] thiazol-4-yl)sulfonyl)-2-(1*H*-pyrrolo[2,3-b]pyrid-5-yl)oxy)-5-fluoro-4-(2-((*R*)-2-(2-isopropylphenyl)-4-(4-methoxybenzyl)piperazin-1-yl)-7-azaspiro[3.5]non-7-yl)benzamide **125**
(*R*)-2-((1*H-*pyrrolo[2,3-b]pyrid-5-yl)oxy)-5-fluoro-N-((7-((((4-fluorotetrahydro-2*H*-pyran-4-yl)methyl)amino)-6-nitrobenzo[*d*][1,3]dioxocyclopent-4-yl)sulfonyl)-4-(2-(2-(2-isopropylphenyl)-4-((6-methoxypyrid-3-yl)methyl)piperazin-1-yl)-7-azaspiro[3.5]non-7-yl)benzamide **126**
(*R*)-2-((1*H*-pyrrolo[2,3-b]pyrid-5-yl)oxy)-5-fluoro-N-((7-(((4-fluorotetrahydro-2*H*-pyran-4-yl)methyl)amino)-6-nitrobenzo[*d*]oxazol-4-yl)sulfonyl)-4-(2-(2-(2-isopropylphenyl)-4-((6-methoxypyrid-3-yl)methyl)piperazin-1-yl)-7-azaspiro[3.5]non-7-yl)benzamide **127**
(*R*)-2-((1*H*-pyrrolo[2,3-b]pyrid-5-yl)oxy)-5-fluoro-N-((7-(((4-fluorotetrahydro-2*H*-pyran-4-yl)methyl)amino)-6-nitrobenzo[*d*]thiazol-4-yl)sulfonyl)-4-(2-(2-(2-isopropylphenyl)-4-((6-methoxypyrid-3-yl)methyl)piperazin-1-yl)-7-azaspiro[3.5]non-7-yl)benzamide **128**
(*R*)-2-((1*H*-pyrrolo[2,3-b]pyrid-5-yl)oxy)-5-fluoro-N-((7-(((4-fluorotetrahydro-2*H*-pyran-4-yl)methyl)amino)-6-nitrobenzofuran-4-yl)sulfonyl)-4-(2-(2-(2-isopropylphenyl)-4-((6-methoxypyrid-3-yl)methyl)piperazin-1-yl)-7-azaspiro[3.5]non-7-yl)benzamide **129**
(*R*)-2-((1*H*-pyrrolo[2,3-b]pyrid-5-yl)oxy)-5-fluoro-4-(2-(4-((2-fluoro-6-methoxypyrid-3-yl)methyl)-2-(2-isopropylphenyl)piperazin-1-yl)-7-azaspiro[3.5]non-7-yl)-N-((7-(((4-fluorotetrahydro-2*H*-pyran-4-yl)methyl)amino)-6-nitrobenzo[*d*][1,3]dioxocyclopent-4-yl)sulfonyl)benzamide **130**
(*R*)-2-((1*H*-pyrrolo[2,3-b]pyrid-5-yl)oxy)-5-fluoro-4-(2-(4-((2-fluoro-6-methoxypyrid-3-yl)methyl)-2-(2-isopropylphenyl)piperazin-1-yl)-7-azaspiro[3.5]non-7-yl)--N-((7-(((4-fluorotetrahydro-2*H*-pyran-4-yl)methyl)amino)-6-nitrobenzo[*d*]oxazo-4-yl)sulfonyl)benzamide **131**
(*R*)-4-((1*H*-pyrrolo[2,3-b]pyrid-5-yl)oxy)-N-((7-(((4-fluorotetrahydro-2*H*-pyran-4-yl)methyl)amino)-6-nitrobenzo[*d*][1,3]dioxocyclopent-4-yl)sulfonyl)-6-(2-(2-(2-isopropylphenyl)-4-(4-methoxybenzyl)piperazin-1-yl)-7-azaspiro[3.5]non-7-yl)nicotinamide **132**
(*R*)-4-((1*H*-pyrrolo[2,3-b]pyridine-5-yl)oxy)-N-((7-(((4-fluorotetrahydro-2*H-*pyran-4-yl)methyl)amino)-6-nitrobenzo[*d*]oxazol-4-yl)sulfonyl)-6-(2-(2-(2-isopropylphenyl)-4-(4-methoxybenzyl)piperazin-1-yl)-7-azaspiro[3.5]non-7-yl)nicotinamide **133**
(*R*)-4-((1*H*-pyrrolo[2,3-b]pyridine-5-yl)oxy)-N-((7-(((4-fluorotetrahydro-2*H-*pyran-4-yl)methyl)amino)-6-nitrobenzo[d]thiazol-4-yl)sulfonyl)-6-(2-(2-(2-isopropylphenyl)-4-(4-methoxybenzyl)piperazin-1-yl)-7-azaspiro[3.5]non-7-yl)nicotinamide **134**
(*R*)-4-((1*H*-pyrrolo[2,3-b]pyrid-5-yl)oxy)-N-((7-(((4-fluorotetrahydro-2*H*-pyran-4-yl)methyl)amino)-6-nitrobenzofuran-4-yl)sulfonyl)-6-(2-(2-(2-isopropylphenyl)-4-(4-methoxybenzyl)piperazin-1-yl)-7-azaspiro[3.5]non-7-yl)nicotinamide **135**
(*R*)-4-((1*H*-pyrrolo[2,3-b]pyrid-5-yl)oxy)-N-((8-(((4-fluorotetrahydro-2*H*-pyran-4-yl)methyl)amino)-7-nitro-2,3-dihydrobenzo[b][1,4]dioxan-5-yl)sulfonyl)-6-(2-(2-(2-isopropylphenyl)-4-(4-methoxybenzyl)piperazin-1-yl)-7-azaspiro[3.5]non-7-yl)nicotinamide **136**
4-((1*H*-pyrrolo[2,3-b]pyrid-5-yl)oxy)-N-((7-((((1*r*, 4*r*)-4-hydroxy-4-methylcyclohexyl)methyl)amino)-6-nitrobenzo[*d*][1,3]dioxocyclopent-4-yl)sulfonyl)-6-(2-((R)-2-(2-isopropylphenyl)-4-(4-methoxybenzyl)piperazin-1-yl)-7-azaspiro[3.5]non-7-yl)nicotinamide **137**
4-((1*H*-pyrrolo[2,3-b]pyrid-5-yl)oxy)-N-((7-((((1*r*, 4*r*)-4-hydroxy-4-methylcyclohexyl)methyl)amino)-6-nitrobenzo[*d*] oxazol-4-yl)sulfonyl)-6-(2-((*R*)-2-(2-isopropylphenyl)-4-(4-methoxybenzyl)piperazin-1-yl)-7-azaspiro[3.5]non-7-yl)nicotinamide **138**
4-((1*H*-pyrrolo[2,3-b]pyrid-5-yl)oxy)-N-((7-((((1*r*, 4*r*)-4-hydroxy-4-methylcyclohexyl)methyl)amino)-6-nitrobenzofuran-4-yl)sulfonyl)-6-(2-((*R*)-2-(2-isopropylphenyl)-4-(4-methoxybenzyl)piperazin-1-yl)-7-azaspiro[3.5]non-7-yl)nicotinamide **139**
4-((1*H*-pyrrolo[2,3-b]pyrid-5-yl)oxy)-N-((7-((((1*r*, 4*r*)-4-hydroxy-4-methylcyclohexyl)methyl)amino)-6-nitrobenzo[*d*] thiazol-4-yl)sulfonyl)-6-(2-((*R*)-2-(2-isopropylphenyl)-4-(4-methoxybenzyl)piperazin-1-yl)-7-azaspiro[3.5]non-7-yl)nicotinamide **140**
(*R*)-2-((1*H*-pyrrolo[2,3-b]pyrid-5-yl)oxy)-5-fluoro-N-((8-(((4-fluorotetrahydro-2*H*-pyran-4-yl)methyl)amino)-7-nitro-3-oxo-2,3-misoline-5-yl)sulfonyl)-4-(2-(2-(2-isopropylphenyl)-4-(4-methoxybenzyl)piperazin-1-yl)-7-azaspiro[3.5]non-7-yl)benzamide **141**
(*R*)-2-((1*H*-pyrrolo[2,3-b]pyrid-5-yl)oxy)-5-fluoro-N-((4-(((4-fluorotetrahydro-2*H*-pyran-4-yl)methyl)amino)-5-nitro-2,3-dihydrobenzofuran-7-yl)sulfonyl)-4-(2-(2-(2-isopropylphenyl)-4-(4-methoxybenzyl)piperazin-1-yl)-7-azaspiro[3.5]non-7-yl)benzamide **142**
(*R*)-2-((1*H*-pyrrolo[2,3-b]pyrid-5-yl)oxy)-5-fluoro-N-((4-(((4-fluorotetrahydro-2*H*-pyran-4-yl)methyl)amino)-5-nitrobenzofuran-7-yl)sulfonyl)-4-(2-(2-(2-isopropylphenyl)-4-(4-methoxybenzyl)piperazin-1-yl)-7-azaspiro[3.5]non-7-yl)benzamide **143**
(*R*)-2-((1*H-*pyrrolo[2,3-b]pyrid-5-yl)oxy)-5-fluoro-N-((8-(((4-fluorotetrahydro-2*H*-pyran-4-yl)methyl)amino)-1-methyl-7-nitro-2-oxo-1,2-dihydroquinoline-5-yl)sulfonyl)-4-(2-(2-(2-isopropylphenyl)-4-(4-methoxybenzyl)piperazin-1-yl)-7-azaspiro[3.5]non-7-yl)benzamide **144**
(R)-2-((1H-pyrrolo[2,3-b]pyrid-5-yl)oxy)-4-(2-(2-(2-isopropylphenyl)-4-(4-methoxybenzyl)piperazin-1 -yl)-7-azaspiro[3. 5]non-7-yl)-N-((6-nitro-7-(((tetrahydro-2H-pyran-4-yl)methyl)amino)benzo[d][1,3]dioxocyclopent-4-yl)sulfonyl)benzamide **145**
(R)-2-((1H-pyrrolo[2,3-b]pyrid-5-yl)oxy)-4-(2-(2-(2-isopropylphenyl)-4-(4-methoxybenzyl)piperazin-1-yl)-7-azaspiro[3.5]non-7-yl)-N-((7-nitro-8-(((tetrahydro-2H-pyran-4-yl)methyl)amino)-2,3-dihydrobenzo[b][1,4]dioxan-5-yl)sulfonyl)benzamide **146**
(R)-2-((1H-pyrrolo[2,3-b]pyrid-5-yl)oxy)-N-((7-(((4-fluorotetrahydro-2H-pyran-4-yl)methyl)amino)-6-nitrobenzo[d][1,3]dioxocyclopent-4-yl)sulfonyl)-4-(2-(2-(2-isopropylphenyl)-4-(4-methoxybenzyl)piperazin-1-yl)-7-azaspiro[3.5]non-7-yl)benzamide **147**
N-((7-((((S)-1,4-dioxan-2-yl)methyl)amino)-6-nitrobenzo[d][1,3]dioxocyclopent-4-yl)sulfonyl)-2-((1H-pyrrolo[2,3-b]pyrid-5-yl)oxy)-4-(2-((R)-2-(2-isopropylphenyl)-4-(4-methoxybenzyl)piperazin-1-yl)-7-azaspiro[3.5]non-7-yl)benzamide **148**
2-((1H-pyrrolo[2,3-b]pyrid-5-yl)oxy)-N-((7-((((1r, 4r)-4-hydroxy-4-methylcyclohexyl)methyl)amino)-6-nitrobenzo[d][1,3]dioxocyclopent-4-yl)sulfonyl)-4-(2-((R)-2-(2-isopropylphenyl)-4-(4-methoxybenzyl)piperazin-1-yl)-7-azaspiro[3.5]non-7-yl)benzamide 149
(R)-4-((1H-pyrrolo[2,3-b]pyrid-5-yl)oxy)-6-(2-(2-(2-isopropylphenyl)-4-(4-methoxybenzyl)piperazin-1-yl)-7-azaspiro[3.5]non-7-yl)-N-((6-nitro-7-(((tetrahydro-2H-pyran-4-yl)methyl)amino)benzo[d][1,3]dioxocyclopent-4-yl)sulfonyl)nicotinamide **150**
N-((7-((((S)-1,4-dioxan-2-yl)methyl)amino)-6-nitrobenzo[d][1,3]dioxocyclopent-4-yl)sulfonyl)-4-((1H-pyrrolo[2,3-b]pyrid-5-yl)ox)-6-(2-((R)-2-(2-isopropylphenyl)-4-(4-methoxybenzyl)piperazin-1-yl)-7-azaspiro[3.5]non-7-yl)nicotinamide **151**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-methoxybenzyl)piperazin-1-yl)-7-azaspiro[3.5]non-7-yl)benzamide **152**
(S)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-methoxybenzyl)piperazin-1-yl)-7-azaspiro[3.5]non-7-yl)benzamide **153**
2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-((2R,5S)-2-(2-isopropylphenyl)-4-(4-methoxybenzyl)-5-methylpiperazin-1-yl)-7-azaspiro[3.5]non-7-yl)benzamide **154**
2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-((2R,5R)-2-(2-isopropylphenyl)-4-(4-methoxybenzyl)-5-methylpiperazin-1-yl)-7-azaspiro[3.5]non-7-yl)benzamide **155**
(S)-2-((1H-pyrrolo[2,3-b]pyrid-5-yl)oxy)-5-fluoro-4-(2-(2-(2-isopropylphenyl)pyrrolidin-1-yl)-7-azaspiro[3.5]non-7-yl)-N-((6-nitro-7-(((tetrahydro-2H-pyran-4-yl)methyl)amino)benzo[d][1,3]dioxocyclopent-4-yl)sulfonyl)benzamide **156**
N-((7-((((S)-1,4-dioxan-2-yl)methyl)amino)-6-nitrobenzo[d][1,3]dioxocyclopent-4-yl)sulfonyl)-2-((1H-pyrrolo[2,3-b]pyrid-5-yl)oxy)-5-fluoro-4-(2-((S)-2-(2-isopropylphenyl)pyrrolidin-1-yl)-7-azaspiro[3.5]non-7-yl)benzamide **157**
2-((1H-pyrrolo[2,3-b]pyrid-5-yl)oxy)-N-((7-((((1r, 4r)-4-hydroxy-4-methylcyclohexyl)methyl)amino)-6-nitrobenzo[d][1,3]dioxocyclopent-4-yl)sulfonyl)-4-(2-((S)-2-(2-isopropylphenyl)pyrrolidin-1-yl)-7-azaspiro[3.5]non-7-yl)benzamide **158**
(S)-2-((1H-pyrrolo[2,3-b]pyrid-5-yl)oxy)-N-((7-(((4-fluorotetrahydro-2H-pyran-4-yl)methyl)amino)-6-nitrobenzo[d][1,3]dioxocyclopent-4-yl)sulfonyl)-4-(2-(2-(2-isopropylphenyl)pyrrolidin-1-yl)-7-azaspiro[3.5]non-7-yl)benzamide **159**
(S)-2-((1H-pyrrolo[2,3-b]pyrid-5-yl)oxy)-4-(2-(2-(2-isopropylphenyl)pyrrolidin-1-yl)-7-azaspiro[3.5]non-7-yl)-N-((6-nitro-7-(((tetrahydro-2H-pyran-4-yl)methyl)amino)benzo[d][1,3]dioxocyclopent-4-yl)sulfonyl)benzamide **160**
N-((7-((((S)-1,4-dioxan-2-yl)methyl)amino)-6-nitrobenzo[d][1,3]dioxocyclopent-4-yl)sulfonyl)-2-((1H-pyrrolo[2,3-b]pyrid-5-yl)oxy)-4-(2-((S)-2-(2-isopropylphenyl)pyrrolidin-1-yl)-7-azaspiro[3.5]non-7-yl)benzamide **161**
(R)-2-((1H-pyrrolo[2,3-b]pyrid-5-yl)oxy)-N-((7-(((4-fluorotetrahydro-2H-pyran-4-yl)methyl)amino)-2,2-dimethyl-6-nitrobenzo[d][1,3]dioxocyclopent-4-yl)sulfonyl)-4-(2-(2-(2-isopropylphenyl)-4-(4-methoxybenzyl)piperazin-1-yl)-7-azaspiro[3.5]non-7-yl)benzamide **162**
2-((1H-pyrrolo[2,3-b]pyrid-5-yl)oxy)-N-((7-((((1r, 4r)-4-hydroxy-4-methylcyclohexyl)methyl)amino)-2,2-dimethyl-6-nitrobenzo[d][1,3]dioxocyclopent-4-yl)sulfonyl)-4-(2-((R)-2-(2-isopropylphenyl)-4-(4-methoxybenzyl)piperazin-1-yl)-7-azaspiro[3.5]non-7-yl)benzamide **163**
(R)-2-((1H-pyrrolo[2,3-b]pyrid-5-yl)oxy)-N-((2,2-difluoro-7-(((4-fluorotetrahydro-2H-pyran-4-yl)methyl)amino)-6-nitrobenzo[d][1,3]dioxocyclopent-4-yl)sulfonyl)-4-(2-(2-(2-isopropylphenyl)-4-(4-methoxybenzyl)piperazin-1-yl)-7-azaspiro[3.5]non-7-yl)benzamide **164**
2-((1H-pyrrolo[2,3-b]pyrid-5-yl)oxy)-N-((2,2-difluoro-7-((((1r, 4r)-4-hydroxy-4-methylcyclohexyl)methyl)amino)-6-nitrobenzo[d][1,3]dioxocyclopent-4-yl)sulfonyl)-4-(2-((R)-2-(2-isopropylphenyl)-4-(4-methoxybenzyl)piperazin-1-yl)-7-azaspiro[3.5]non-7-yl)benzamide 165
(R)-2-((1H-pyrrolo[2,3-b]pyrid-5-yl)oxy)-N-((7-(((4-fluorotetrahydro-2H-pyran-4-yl)methyl)amino)-6-nitrobenzo[d][1,3]dioxocyclopent-4-yl)sulfonyl)-4-(2-(2-(2-isopropylphenyl)-4-((6-methoxypyrid-3-yl)methyl)piperazin-l-yl)-7-azaspiro[3.5]non-7-yl)benzamide **166**
(R)-2-((1H-pyrrolo[2,3-b]pyrid-5-yl)oxy)-4-(2-(4-(4-fluorobenzyl)-2-(2-isopropylphenyl)piperazin-1-yl)-7-azaspiro[3.5]non-7-yl)-N-((7-(((4-fluorotetrahydro-2H-pyran-4-yl)methyl)amino)-6-nitrobenzo[d][1,3]dioxocyclopent-4-yl)sulfonyl)benzamide **167**
(R)-2-((1H-pyrrolo[2,3-b]pyrid-5-yl)oxy)-4-(2-(4-(3,4-difluorobenzyl)-2-(2-isopropylphenyl)piperazin-1-yl)-7-azaspiro[3.5]non-7-yl)-N-((7-(((4-fluorotetrahydro-2H-pyran-4-yl)methyl)amino)-6-nitrobenzo[d][1,3]dioxocyclopent-4-yl)sulfonyl)benzamide **168**
(R)-2-((1H-pyrrolo[2,3-b]pyrid-5-yl)oxy)-4-(2-(4-(2,4-difluorobenzyl)-2-(2-isopropylphenyl)piperazin-1-yl)-7-azaspiro[3.5]non-7-yl)-N-((7-(((4-fluorotetrahydro-2H-pyran-4-yl)methyl)amino)-6-nitrobenzo[d][1,3]dioxocyclopent-4-yl)sulfonyl)benzamide **169**
(R)-2-((1H-pyrrolo[2,3-b]pyrid-5-yl)oxy)-4-(2-(4-(2,6-difluorobenzyl)-2-(2-isopropylphenyl)piperazin-1-yl)-7-azaspiro[3.5]non-7-yl)-N-((7-(((4-fluorotetrahydro-2H-pyran-4-yl)methyl)amino)-6-nitrobenzo[d][1,3]dioxocyclopent-4-yl)sulfonyl)benzamide **170**
(R)-2-((1H-pyrrolo[2,3-b]pyrid-5-yl)oxy)-N-((7-(((4-fluorotetrahydro-2H-pyran-4-yl)methyl)amino)-6-nitrobenzo[d][1,3]dioxocyclopent-4-yl)sulfonyl)-4-(2-(2-(2-isopropylphenyl)-4-((7-methoxy-2-methylbenzofuran-5-yl)methyl)piperazin-1-yl)-7-azaspiro[3.5]non-7-yl)benzamide **171**
(R)-2-((1H-pyrrolo[2,3-b]pyrid-5-yl)oxy)-4-(2-(4-(4-cyclopropylbenzyl)-2-(2-isopropylphenyl)piperazin-1-yl)-7-azaspiro[3.5]non-7-yl)-N-((7-(((4-fluorotetrahydro-2H-pyran-4-yl)methyl)amino)-6-nitrobenzo[d][1,3]dioxocyclopent-4-yl)sulfonyl)benzamide **172**
(R)-2-((1H-pyrrolo[2,3-b]pyrid-5-yl)oxy)-4-(2-(4-(4-cyclopropyl-3-methoxybenzyl)-2-(2-isopropylphenyl)piperazin-1-yl)-7-azaspiro[3.5]non-7-yl)-N-((7-(((4-fluorotetrahydro-2H-pyran-4-yl)methyl)amino)-6-nitrobenzo[d][1,3]dioxocyclopent-4-yl)sulfonyl)benzamide **173**
2-((1H-pyrrolo[2,3-b]pyrid-5-yl)oxy)-N-((7-((((1r, 4r)-4-hydroxy-4-methylcyclohexyl)methyl)amino)-6-nitrobenzo[d][1,3]dioxocyclopent-4-yl)sulfonyl)-4-(2-((R)-2-(2-isopropylphenyl)-4-((6-methoxypyrid-3-yl)methyl)piperazin-1-yl)-7-azaspiro[3.5]non-7-yl)benzamide **174**
2-((1H-pyrrolo[2,3-b]pyrid-5-yl)oxy)-4-(2-((R)-4-(4-fluorobenzyl)-2-(2-isopropylphenyl)piperazin-1-yl)-7-azaspiro[3.5]non-7-yl)-N-((7-((((1r, 4r)-4-hydroxy-4-methylcyclohexyl)methyl)amino)-6-nitrobenzo[d][1,3]dioxocyclopent-4-yl)sulfonyl)benzamide **175**
2-((1H-pyrrolo[2,3-b]pyrid-5-yl)oxy)-4-(2-((R)-4-(3,4-difluorobenzyl)-2-(2-isopropylphenyl)piperazin-1-yl)-7-azaspiro[3.5]non-7-yl)-N-((7-((((1r, 4r)-4-hydroxy-4-methylcyclohexyl)methyl)amino)-6-nitrobenzo[d][1,3]dioxocyclopent-4-yl)sulfonyl)benzamide **176**
2-((1H-pyrrolo[2,3-b]pyrid-5-yl)oxy)-4-(2-((R)-4-(2,6-difluorobenzyl)-2-(2-isopropylphenyl)piperazin-1-yl)-7-azaspiro[3.5]non-7-yl)-N-((7-((((1r, 4r)-4-hydroxy-4-methylcyclohexyl)methyl)amino)-6-nitrobenzo[d][1,3]dioxocyclopent-4-yl)sulfonyl)benzamide **177**
2-((1H-pyrrolo[2,3-b]pyrid-5-yl)oxy)-4-(2-((R)-4-(4-cyclopropylbenzyl)-2-(2-isopropylphenyl)piperazin-1-yl)-7-azaspiro[3.5]non-7-yl)-N-((7-((((1r, 4r)-4-hydroxy-4-methylcyclohexyl)methyl)amino)-6-nitrobenzo[d][1,3]dioxocyclopent-4-yl)sulfonyl)benzamide **178**
2-((1H-pyrrolo[2,3-b]pyrid-5-yl)oxy)-4-(2-((R)-4-(4-cyclopropyl-3-methoxybenzyl)-2-(2-isopropylphenyl)piperazin-1-yl)-7-azaspiro[3.5]non-7-yl)-N-((7-((((1r, 4r)-4-hydroxy-4-methylcyclohexyl)methyl)amino)-6-nitrobenzo[d][1,3]dioxocyclopent-4-yl)sulfonyl)benzamide **179**
2-((1H-pyrrolo[2,3-b]pyrid-5-yl)oxy)-N-((7-((((1r, 4r)-4-hydroxy-4-methylcyclohexyl)methyl)amino)-6-nitrobenzo[d][1,3]dioxocyclopent-4-yl)sulfonyl)-4-(2-((R)-2-(2-isopropylphenyl)-4-(7-methoxy-2-methylbenzofuran-5-yl)methyl)piperazin-1-yl)-7-azaspiro[3.5]non-7-yl)benzamide **180**
(R)-2-((1H-pyrrolo[2,3-b]pyrid-5-yl)oxy)-N-((8-((((4-fluorotetrahydro-2H-pyran-4-yl)methyl)amino)-7-nitro-2,3-dihydrobenzo[b][1,4]dioxane-5yl)sulfonyl)-4-(2-(2-(2-isopropylphenyl)-4-(4-methoxybenzyl)piperazin-1-yl)-7-azaspiro[3.5]non-7-yl)benzamide **181**
2-((1H-pyrrolo[2,3-b]pyrid-5-yl)oxy)-N-((8-((((1r, 4r)-4-hydroxy-4-methylcyclohexyl)methyl)amino)-7-nitro-2,3-dihydrobenzo[b][1,4]dioxan-5-yl)sulfonyl)-4-(2-((R)-2-(2-isopropylphenyl)-4-(4-methoxybenzyl)piperazin-1-yl)-7-azaspiro[3.5]non-7-yl)benzamide **182**
N-((8-((((S)-1,4-dioxan-2-yl)methyl)amino)-7-nitro-2,3-dihydrobenzo[b][1,4]dioxan-5-yl)sulfonyl)-2-((1H-pyrrolo[2,3-b]pyrid-5-yl)oxy)-4-(2-((R)-2-(2-isopropylphenyl)-4-(4-methoxybenzyl)piperazin-1-yl)-7-azaspiro[3.5]non-7-yl)benzamide **183**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(3-methoxybenzyl)piperazin-1-yl)-7-azaspiro[3.5]non-7-yl)benzamide **184**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(2-methoxybenzyl)piperazin-1-yl)-7-azaspiro[3.5]non-7-yl)benzamide **185**
(R)-2-((1H-pyrrolo[2,3-b]pyrid-5-yl)oxy)-5-fluoro-4-(2-(4-(4-fluorobenzyl)-2-(2-isopropylphenyl)piperazin-1-yl)-7-azaspiro[3.5]non-7-yl)-N-((4-(((4-fluorotetrahydro-2H-pyran-4-yl)methyl)amino)-3-nitrophenyl)sulfonyl)benzamide **186**
(R)-2-((1H-pyrrolo[2,3-b]pyrid-5-yl)oxy)-5-fluoro-4-(2-(4-(2-fluorobenzyl)-2-(2-isopropylphenyl)piperazin-1-yl)-7-azaspiro[3.5]non-7-yl)-N-((4-(((4-fluorotetrahydro-2H-pyran-4-yl)methyl)amino)-3-nitrophenyl)sulfonyl)benzamide **187**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-(trifluoromethyl)benzyl)piperazin-1-yl)-7-azaspiro[3.5]non-7-yl)benzamide **188**
(R)-2-((1H-pyrrolo[2,3-b]pyrid-5-yl)oxy)-4-(2-(4-(2,3-difluorobenzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **189**
(R)-2-((1H-pyrrolo[2,3-b]pyrid-5-yl)oxy)-4-(2-(4-(3,4-difluorobenzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **190**
(R)-2-((1H-pyrrolo[2,3-b]pyrid-5-yl)oxy)-4-(2-(4-(2,4-difluorobenzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **191**
(R)-2-((1H-pyrrolo[2,3-b]pyrid-5-yl)oxy)-4-(2-(4-(2,6-difluorobenzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **192**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(4-(4-isopropylbenzyl)-2-(2-isopropylphenyl)piperazin-1-yl)-7-azaspiro[3.5]non-7-yl)benzamide **193**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(4-(4-isopropyl-3-methoxybenzyl)-2-(2-isopropylphenyl)piperazin-1-yl)-7-azaspiro[3.5]non-7-yl)benzamide **194**
(R)-2-((1H-pyrrolo[2,3-b]pyrid-5-yl)oxy)-4-(2-(4-(4-cyclopropylbenzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **195**
(R)-2-((1H-pyrrolo[2,3-b]pyrid-5-yl)oxy)-4-(2-(4-(4-cyclopropyl-3-methoxybenzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **196**
(R)-2-((1H-pyrrolo[2,3-b]pyrid-5-yl)oxy)-4-(2-(4-(3,4-dimethoxybenzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **197**
(R)-2-((1H-pyrrolo[2,3-b]pyrid-5-yl)oxy)-4-(2-(4-(4-(dimethylamino)benzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **198**
(R)-2-((1H-pyrrolo[2,3-b]pyrid-5-yl)oxy)-4-(2-(4-(4-cyanobenzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **199**
(R)-2-((1H-pyrrolo[2,3-b]pyrid-5-yl)oxy)-4-(2-(4-(4-acetylaminobenzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **200**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-(methylsulfonylamino)benzyl)piperazin-1-yl)-7-azaspiro[3.5]non-7-yl)benzamide **201**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-((7-methoxy-2-methylbenzofuran-5-yl)methyl)piperazin-1-yl)-7-azaspiro[3.5]non-7-yl)benzamide 202
(R)-2-((1H-pyrrolo[2,3-b]pyrid-5-yl)oxy)-4-(2-(4-(benzo[d][1,3]dioxocyclopent-5-ylmethyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **203**
(R)-2-((1H-pyrrolo[2,3-b]pyrid-5-yl)oxy)-4-(2-(4-(benzo[d][1,3]dioxocyclopent-4-ylmethyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **204**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(7-methoxybenzo[d][1,3]dioxocyclopent-4-yl)methyl)piperazin-1-yl)-7-azaspiro[3.5]non-7-yl)benzamide **205**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(8-methoxy-2,3 -dihydrobenzo[b][1,4-dioxan-5-yl)methyl)piperazin-1-yl)-7-azaspiro[3.5]non-7-yl)benzamide **206**
(R)-2-((1H-pyrrolo[2,3-b]pyrid-5-yl)oxy)-4-(2-(4-((2,3-dihydrobenzo[b][1,4]dioxane-6-yl)methyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **207**
(R)-2-((1H-pyrrolo[2,3-b]pyrid-5-yl)oxy)-4-(2-(4-((2,3-dihydrobenzo[b][1,4]dioxan-5-yl)methyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **208**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-((1-methyl-1H-pyrazole-4-yl)methyl)piperazin-1-yl)-7-azaspiro[3.5]non-7-yl)benzamide **209**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(4-((1-isopropyl-1H-pyrazol-4-yl)methyl)-2-(2-isopropylphenyl)piperazin-1-yl)-7-azaspiro[3.5]non-7-yl)benzamide **210**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(pyrid-4-ylmethyl)piperazin-1-yl)-7-azaspiro[3.5]non-7-yl)benzamide **211**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(pyrid-2-ylmethyl)piperazin-1-yl)-7-azaspiro[3.5]non-7-yl)benzamide **212**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(pyrid-3-ylmethyl)piperazin-1-yl)-7-azaspiro[3.5]non-7-yl)benzamide **213**
2-((1H-pyrrolo[2,3-b]pyrid-5-yl)oxy)-4-(2-((R)-4-(4-cyanobenzyl)-2-(2-isopropylphenyl)piperazin-1-yl)-7-azaspiro[3.5]non-7-yl)-5-fluoro-N-((4-((((1r, 4r)-4-hydroxy-4-methylcyclohexyl)methyl)amino)-3-nitrophenyl)sulfonyl)benzamide **214**
2-((1H-pyrrolo[2,3-b]pyrid-5-yl)oxy)-5-fluoro-4-(2-((R)-4-(4-fluorobenzyl)-2-(2-isopropylphenyl)piperazin-1-yl)-7-azaspiro[3.5]non-7-yl)-N-((4-((((1r, 4r)-4-hydroxy-4-methylcyclohexyl)methyl)amino)-3-nitrophenyl)sulfonyl)benzamide **215**
2-((1H-pyrrolo[2,3-b]pyrid-5-yl)oxy)-5-fluoro-N-((4-((((1r, 4r)-4-hydroxy-4-methylcyclohexyl)methyl)amino)-3-nitrophenyl)sulfonyl)-4-(2-((R)-2-(2-isopropylphenyl)-4-(4-(trifluoromethyl)benzyl)piperazin-1-yl)-7-azaspiro[3.5]non-7-yl)benzamide **216**
2-((1H-pyrrolo[2,3-b]pyrid-5-yl)oxy)-4-(2-((R)-4-(2,6-difluorobenzyl)-2-(2-isopropylphenyl)piperazin-1-yl)-7-azaspiro[3.5]non-7-yl)-5-fluoro-N-((4-((((1r, 4r)-4-hydroxy-4-methylcyclohexyl)methyl)amino)-3-nitrophenyl)sulfonyl)benzamide **217**
2-((1H-pyrrolo[2,3-b]pyrid-5-yl)oxy)-4-(2-((R)-4-(2,3-difluorobenzyl)-2-(2-isopropylphenyl)piperazin-1-yl)-7-azaspiro[3.5]non-7-yl)-5-fluoro-N-((4-((((1r, 4r)-4-hydroxy-4-methylcyclohexyl)methyl)amino)-3-nitrophenyl)sulfonyl)benzamide **218**
2-((1H-pyrrolo[2,3-b]pyrid-5-yl)oxy)-4-(2-((R)-4-(4-cyclopropylbenzyl)-2-(2-isopropylphenyl)piperazin-1-yl)-7-azaspiro[3.5]non-7-yl)-5-fluoro-N-((4-((((1r, 4r)-4-hydroxy-4-methylcyclohexyl)methyl)amino)-3-nitrophenyl)sulfonyl)benzamide **219**
2-((1H-pyrrolo[2,3-b]pyrid-5-yl)oxy)-4-(2-((R)-4-(4-cyclopropyl-3-methoxybenzyl)-2-(2-isopropylphenyl)piperazin-1-yl)-7-azaspiro[3.5]non-7-yl)-5-fluoro-N-((4-((((1r, 4r)-4-hydroxy-4-methylcyclohexyl)methyl)amino)-3-nitrophenyl)sulfonyl)benzamide **220**
2-((1H-pyrrolo[2,3-b]pyrid-5-yl)oxy)-4-(2-((R)-4-(3,4-dimethoxybenzyl)-2-(2-isopropylphenyl)piperazin-1-yl)-7-azaspiro[3.5]non-7-yl)-5-fluoro-N-((4-((((1r, 4r)-4-hydroxy-4-methylcyclohexyl)methyl)amino)-3-nitrophenyl)sulfonyl)benzamide **221**
2-((1H-pyrrolo[2,3-b]pyrid-5-yl)oxy)-4-(2-((R)-4-(benzo[d][1,3]dioxocyclopentane-4-ylmethyl)-2-(2-isopropylphenyl)piperazin-1-yl)-7-azaspiro[3.5]non-7-yl)-5-fluoro-N-((4-((((1r, 4r)-4-hydroxy-4-methylcyclohexyl)methyl)amino)-3-nitrophenyl)sulfonyl)benzamide **222**
2-((1H-pyrrolo[2,3-b]pyrid-5-yl)oxy)-5-fluoro-N-((4-((((1r, 4r)-4-hydroxy-4-methylcyclohexyl)methyl)amino)-3-nitrophenyl)sulfonyl)-4-(2-((R)-2-(2-isopropylphenyl)-4-((7-methoxybenzo[d][1,3]dioxycyclopent-4-yl)methyl)piperazin-1-yl)-7-azaspiro[3.5]non-7-yl)benzamide **223**
2-((1H-pyrrolo[2,3-b]pyrid-5-yl)oxy)-4-(2-((R)-4-(benzo[d][1,3]dioxocyclopent-5-ylmethyl)-2-(2-isopropylphenyl)piperazin-1-yl)-7-azaspiro[3.5]non-7-yl) -5-fluoro-N-((4-((((1r, 4r)-4-hydroxy-4-methylcyclohexyl)methyl)amino)-3-nitrophenyl)sulfonyl)benzamide **224**
2-((1H-pyrrolo[2,3-b]pyrid-5-yl)oxy)-5-fluoro-N-((4-((((1r, 4r)-4-hydroxy-4-methylcyclohexyl)methyl)amino)-3-nitrophenyl)sulfonyl)-4-(2-((R)-2-(2-isopropylphenyl)-4-((7-methoxy-2-methylbenzofuran-5-yl)methyl)piperazin-1-yl)-7-azaspiro[3.5]non-7-yl)benzamide **225**
2-((1H-pyrrolo[2,3-b]pyrid-5-yl)oxy)-4-(2-((R)-4-((2,3-dihydrobenzo[b][1,4]dioxane-6-yl)methyl)-2-(2-isopropylphenyl)piperazin-1-yl)-7-azaspiro[3.5]non-7-yl)-5-fluoro-N-((4-((((1r, 4r)-4-hydroxy-4-methylcyclohexyl)methyl)amino)-3-nitrophenyl)sulfonyl)benzamide **226**
2-((1H-pyrrolo[2,3-b]pyrid-5-yl)oxy)-4-(2-((R)-4-((2,3-dihydrobenzo[b][1,4]dioxane-5-yl)methyl)-2-(2-isopropylphenyl)piperazin-1-yl)-7-azaspiro[3.5]non-7-yl)-5-fluoro-N-((4-((((1r, 4r)-4-hydroxy-4-methylcyclohexyl)methyl)amino)-3-nitrophenyl)sulfonyl)benzamide **227**
2-((1H-pyrrolo[2,3-b]pyrid-5-yl)oxy)-5-fluoro-N-((4-((((1r, 4r)-4-hydroxy-4-methylcyclohexyl)methyl)amino)-3-nitrophenyl)sulfonyl)-4-(2-((R)-2-(2-isopropylphenyl)-4-((8-methoxy-2,3-dihydrobenzo[b][1,4]dioxan-5-yl)methyl)piperazin-1-yl)-7-azaspiro[3.5]non-7-yl)benzamide **228**
2-((1H-pyrrolo[2,3-b]pyrid-5-yl)oxy)-5-fluoro-N-((4-((((1r, 4r)-4-hydroxy-4-methylcyclohexyl)methyl)amino)-3-nitrophenyl)sulfonyl)-4-(2-((R)-2-(2-isopropylphenyl)-4-((1-methyl-1H-pyrazole-4-yl)methyl)piperazin-1-yl)-7-azaspiro[3.5]non-7-yl)benzamide **229**
2-((1H-pyrrolo[2,3-b]pyrid-5-yl)oxy)-5-fluoro-N-((4-((((1r, 4r)-4-hydroxy-4-methylcyclohexyl)methyl)amino)-3-nitrophenyl)sulfonyl)-4-(2-((R)-4-((1-isopropyl-1H-pyrazole-4-yl)methyl)-2-(2-isopropylphenyl)piperazin-1-yl)-7-azaspiro[3.5]non-7-yl)benzamide **230**
2-((1H-pyrrolo[2,3-b]pyrid-5-yl)oxy)-5-fluoro-N-((4-((((1r, 4r)-4-hydroxy-4-methylcyclohexyl)methyl)amino)-3-nitrophenyl)sulfonyl)-4-(2-((R)-2-(2-isopropylphenyl)-4-(pyrid-4-ylmethyl)piperazin-1-yl)-7-azaspiro[3.5]non-7-yl)benzamide **231**
2-((1H-pyrrolo[2,3-b]pyrid-5-yl)oxy)-5-fluoro-N-((4-((((1r, 4r)-4-hydroxy-4-methylcyclohexyl)methyl)amino)-3-nitrophenyl)sulfonyl)-4-(2-((R)-2-(2-isopropylphenyl)-4-(pyrid-3-ylmethyl)piperazin-1-yl)-7-azaspiro[3.5]non-7-yl)benzamide **232**
2-((1H-pyrrolo[2,3-b]pyrid-5-yl)oxy)-5-fluoro-N-((4-((((1r, 4r)-4-hydroxy-4-methylcyclohexyl)methyl)amino)-3-nitrophenyl)sulfonyl)-4-(2-((R)-2-(2-isopropylphenyl)-4-(pyrid-2-ylmethyl)piperazin-1-yl)-7-azaspiro[3.5]non-7-yl)benzamide **233**
2-((1*H*-pyrrolo[2,3-b]pyrid-5-yl)oxy)-5-fluoro-N-(4-(((((1r, 4r)-4-hydroxy-4-methylcyclohexyl)methyl)amino)-3-nitrophenyl)sulfonyl)-4-(2-(R)-2-(2-isopropylphenyl)-4-(4-methoxybenzyl)piperazin-1-yl)-7-azaspiro[3.5]non-7-yl)benzamide **234**
5-fluoro-2-((3-fluoro-1H-pyrrolo[2,3-b]pyrid-5-yl)oxy)-N-((4-((((1r, 4r)-4-hydroxy-4-methylcyclohexyl)methyl)amino)-3-nitrophenyl)sulfonyl)-4-(2-((R)-2-(2-isopropylphenyl)-4-(4-methoxybenzyl)piperazin-1-yl)-7-azaspiro[3.5]non-7-yl)benzamide **235**
2-((1H-pyrrolo[2,3-b]pyrid-5-yl)oxy)-5-fluoro-N-((4-((((1r, 4r)-4-hydroxy-4-methylcyclohexyl)methyl)amino)-3-nitrophenyl)sulfonyl)-4-(2-((R)-2-(2-isopropylphenyl)-4-((6-methoxypyrid-3-yl)methyl)piperazin-1-yl)-7-azaspiro[3.5]non-7-yl)benzamide **236**
2-((1H-pyrrolo[2,3-b]pyrid-5-yl)oxy)-4-(2-((R)-4-(3,4-dimethoxybenzyl)-2-(2-isopropylphenyl)piperazin-1-yl)-7-azaspiro[3.5]non-7-yl)-5-fluoro-N-((4-((((1r, 4r)-4-hydroxy-4-methylcyclohexyl)methyl)amino)-3-nitrophenyl)sulfonyl)benzamide **237**
2-((1H-pyrrolo[2,3-b]pyrid-5-yl)oxy)-5-fluoro-N-((4-((((1r, 4r)-4-hydroxy-4-methylcyclohexyl)methyl)amino)-3-nitrophenyl)sulfonyl)-4-(2-((R)-2-(2-isopropylphenyl)-4-((1-methyl-1H-pyrazole-4-yl)methyl)piperazin-1-yl)-7-azaspiro[3.5]non-7-yl)benzamide **238**
(R)-2-((1*H-*pyrrolo[2,3-b]pyrid-5-yl)oxy)-5-fluoro-4-(2-(2-(2-isopropylphenyl)-4-(4-methoxybenzyl)piperazin-1-yl)-7-azaspiro[3.5]non-7-yl)-N-((3-nitro-4-(((tetrahydro-2*H-*pyran-4-yl)methyl)amino)phenyl)sulfonyl)benzamide **239**
(R)-5-fluoro-2-((3-fluoro-1H-pyrrolo[2,3-b]pyrid-5-yl)oxy)-4-(2-(2-(2-isopropylphenyl)-4-(4-methoxybenzyl)piperazin-1-yl)-7-azaspiro[3.5]non-7-yl)-N-((3-nitro-4-(((tetrahydro-2H-pyran-4-yl)methyl)amino)phenyl)sulfonyl)benzamide **240**
N-((4-((((S)-1,4-dioxan-2-yl)methyl)amino)-3-nitrophenyl)sulfonyl)-2-((1H-pyrrolo[2,3-b]pyrid-5-yl)oxy)-5-fluoro-4-(2-((R)-2-(2-isopropylphenyl)-4-(4-methoxybenzyl)piperazin-1-yl)-7-azaspiro[3.5]non-7-yl)benzamide **241**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-((6-methoxypyrid-3-yl)methyl)piperazin-1-yl)-7-azaspiro[3.5]non-7-yl)benzamide **242**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-((methylamino)methyl)benzyl)piperazin-1-yl)-7-azaspiro[3.5]non-7-yl)benzamide **243**
(R)-2-((1H-pyrrolo[2,3-b]pyrid-5-yl)oxy)-4-(2-(4-(4-((dimethylamino)methyl)benzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **244**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-(pyrrolidin-1-ylmethyl)benzyl)piperazin-1-yl)-7-azaspiro[3.5]non-7-yl)benzamide **245**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(3-methoxy-4-((methylamino)methyl)benzyl)piperazin-1-yl)-7-azaspiro[3.5]non-7-yl)benzamide **246**
(R)-2-((1H-pyrrolo[2,3-b]pyrid-5-yl)oxy)-4-(2-(4-(4-((dimethylamino)methyl)-3-methoxybenzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **247**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(3-methoxy-4-(pyrroli din-1-ylmethyl)benzyl)piperazin-1-yl)-7-azaspiro[3.5]non-7-yl)benzamide **248**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-methoxy-3-((methylamino)methyl)benzyl)piperazin-1-yl)-7-azaspiro[3.5]non-7-yl)benzamide **249**
(R)-2-((1H-pyrrolo[2,3-b]pyrid-5-yl)oxy)-4-(2-(4-(3-((dimethylamino)methyl)-4-methoxybenzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide **250**
(R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-methoxy-3 -(pyrroli din-1-ylmethyl)benzyl)piperazin-1-yl)-7-azaspiro[3.5]non-7-yl)benzamide **251**
(R)-5-fluoro-2-((3-fluoro-1H-pyrrolo[2,3-b]pyrid-5-yl)oxy)-N-((4-(((4-fluorotetrahydro-2H-pyran-4-yl)methyl)amino)-3-nitrophenyl)sulfonyl)-4-(2-(2-(2-isopropylphenyl)-4-(4-methoxybenzyl)piperazin-1-yl)-7-azaspiro[3.5]non-7-yl)benzamide 252
(R)-4-(2-(4-(3,4-dimethoxybenzyl)-2-(2-isopropylphenyl)piperazin-1-yl)-7-azaspiro[3.5]non-7-yl)-5-fluoro-2-((3-fluoro-1H-pyrrolo[2,3-b]pyrid-5-yl)oxy)-N-((4-(((4-fluorotetrahydro-2H-pyran-4-yl)methyl)amino)-3-nitrophenyl)sulfonyl)benzamide **253**
(R)-5-fluoro-2-((3-fluoro-1H-pyrrolo[2,3-b]pyrid-5-yl)oxy)-N-((4-(((4-fluorotetrahydro-2H-pyran-4-yl)methyl)amino)-3-nitrophenyl)sulfonyl)-4-(2-(2-(2-isopropylphenyl)-4-((6-methoxypyrid-3-yl)methyl)piperazin-1-yl)-7-azaspiro[3.5]non-7-yl)benzamide **254**
(R)-5-fluoro-2-((3-fluoro-1H-pyrrolo[2,3-b]pyrid-5-yl)oxy)-N-((4-(((4-fluorotetrahydro-2H-pyran-4-yl)methyl)amino)-3-nitrophenyl)sulfonyl)-4-(2-(2-(2-isopropylphenyl)-4-((1-methyl-1H-pyrazol-4-yl)methyl)piperazin-1-yl)-7-azaspiro[3.5]non-7-yl)benzamide **255**
4-((1H-pyrrolo[2,3-b]pyrid-5-yl)oxy)-N-((4-((((1r, 4r)-4-hydroxy-4-methylcyclohexyl)methyl)amino)-3-nitrophenyl)sulfonyl)-6-(2-((R)-2-(2-isopropylphenyl)-4-(4-methoxybenzyl)piperazin-1-yl)-7-azaspiro[3.5]non-7-yl)nicotinamide **256**
(R)-4-((1*H-*pyrrolo[2,3-b]pyrid-5-yl)oxy)-6-(2-(2-(2-isopropylphenyl)-4-(4-methoxybenzyl)piperazin-1-yl)-7-azaspiro[3.5]non-7-yl)-N-((3-nitro-4-(((tetrahydro-2*H*-pyran-4-yl)methyl)amino) phenyl)sulfonyl)nicotinamide **257**
(R)-4-((1*H*-pyrrolo[2,3-b]pyrid-5-yl)oxy)-N-((4-(((4-fluorotetrahydro-2*H*-pyran-4-yl)methyl)amino)-3-nitrophenyl)sulfonyl)-6-(2-(2-(2-isopropylphenyl)-4-(4-methoxybenzyl)piperazin-1-yl)-7-azaspiro[3.5]non-7-yl)nicotinamide **258**
2-((1*H*-pyrrolo[2,3-b]pyrid-5-yl)oxy)-N-((7-((((1*r*,4*r*)-4-hydroxy-4-methylcyclohexyl)methyl)amino)-6-nitro-1H-benzo[d]imidazol-4-yl)sulfonyl)-4-(2-((R)-2-(2-isopropylphenyl)-4-(4-methoxybenzyl)piperazin-1-yl)-7-azaspiro[3.5]non-7-yl)benzamide **259**
(R)-2-((1H-pyrrolo[2,3-b]pyrid-5-yl)oxy)-4-(2-(2-(2-isopropylphenyl)-4-(4-methoxybenzyl)piperazin-1-yl)-7-azaspiro[3.5]non-7-yl)-N-(6-nitro-7-(((tetrahydro-2H-pyran-4-yl)methyl)amino)-1H benzo[d]imidazol-4-yl)sulfonyl)benzamide **260**
(*R*)-2-((1*H-*pyrrolo[2,3-b]pyrid-5-yl)oxy)-N-((7-(((4-fluorotetrahydro-2*H*-pyran-4-yl)methyl)amino)-6-nitro-1*H*-benzo[*d*]imidazol-4-yl)sulfonyl)-4-(2-(2-(2-isopropylphenyl)-4-(4-methoxybenzyl)piperazin-1-yl)-7-azaspiro[3.5]non-7-yl)benzamide **261**
2-((1*H*-pyrrolo[2,3-b]pyrid-5-yl)oxy)-5-fluoro-N-((7-((((1*r*,4*r*)-4-hydroxy-4-methylcyclohexyl)methyl)amino)-6-nitro-1*H*-benzo[*d*]imidazol-4-yl)sulfonyl)-4-(2-((R)-2-(2-isopropylphenyl)-4-(4-methoxybenzyl)piperazin-1-yl)-7-azaspiro[3.5]non-7-yl)benzamide **262**
(R)-2-((1*H*-pyrrolo[2,3-b]pyrid-5-yl)oxy)-5-fluoro-4-(2-(2-(2-isopropylphenyl)-4-(4-methoxybenzyl)piperazin-1-yl)-7-azaspiro[3.5]non-7-yl)-N-((6-nitro-7-(((tetrahydro-2*H-*pyran-4-yl)methyl)amino)-1*H*-benzo[*d*]imidazol-4-yl)sulfonyl)benzamide **263**
(*R*)-2-((1*H*-pyrrolo[2,3-b]pyrid-5-yl)oxy)-5-fluoro-N-((7-(((4-fluorotetrahydro-27/-pyran-4-yl)methyl)amino)-6-nitro-1*H*-benzo[*d*]imidazol-4-yl)sulfonyl)-4-(2-(2-(2-isopropylphenyl)-4-(4-methoxybenzyl)piperazin-1-yl)-7-azaspiro[3.5]non-7-yl)benzamide **264**
(R)-2-((1H-pyrrolo[2,3-b]pyrid-5-yl)oxy)-5-fluoro-4-(2-(2-(2-isopropylphenyl)-4-(4-methoxybenzyl)piperazin-1-yl)-7-azaspiro[3.5]non-7-yl)-N-((7-nitro-8-(((tetrahydro-2H-pyran-4-yl)methyl)amino)-2,3-dihydrobenzo[b][1,4] dioxin-5-yl)sulfonyl)benzamide **265**
4-((1*H*-pyrrolo[2,3-b]pyrid-5-yl)oxy)-N-((7-((((1*r*,4*r*)-4-hydroxy-4-methylcyclohexyl)methyl)amino)-6-nitro-1*H*-benzo[*d*]imidazol-4-yl)sulfonyl)-6-(2-((R)-2-(2-isopropylphenyl)-4-(4-methoxybenzyl)piperazin-1-yl)-7-azaspiro[3.5]non-7-yl)nicotinamide **266**
(R)-4-((1*H-*pyrrolo[2,3-b]pyrid-5-yl)oxy)-6-(2-(2-(2-isopropylphenyl)-4-(4-methoxybenzyl)piperazin-1-yl)-7-azaspiro[3.5]non-7-yl)-N-((6-nitro-7-(((tetrahydro-2H-pyran-4-yl)methyl)amino)-1*H*-benzo[*d*]imidazol-4-yl)sulfonyl)nicotinamide **267**
(R)-4-((1*H*-pyrrolo[2,3-b]pyrid-5-yl)oxy)-N-((7-(((4-fluorotetrahydro-2*H*-pyran-4-yl)methyl)amino)-6-nitro-1*H*-benzo[*d*]imidazol-4-yl)sulfonyl)-6-(2-(2-(2-isopropylphenyl)-4-(4-methoxybenzyl)piperazin-1-yl)-7-azaspiro[3.5]non-7-yl)nicotinamide **268**

### Terminology description

Unless otherwise defined, all technical and scientific terms used in the present invention have the same meanings as those commonly understood by those skilled in the art. Unless otherwise specified, all patent literature, publicly disclosed materials, etc. referred to in the present invention are incorporated in their entirety in the references. If there are multiple definitions for the same term in the present invention, the definitions in this section shall prevail.

It should be understood that the general description in the previous text and the detailed description in the following text are only exemplary and explanatory, and have no limitation on any claims. It should be noted that in the specification and the accompanying claims, unless otherwise specified in the text, reference to singular forms such as "a", "one" and "this" comprises reference to plural forms. It should also be noted that unless otherwise specified, "or" represents "and/or". In addition, terms such as "comprising" and "including" are not restrictive.

"Substituted" refers to hydrogen atoms being substituted by substituents. It should be noted that substituents on specific atoms are limited by their valence states. In the definition section, "Cᵢ-Cⱼ" refers to the range that includes the starting and ending points, where i and j are integers representing the number of carbon atoms. For example, C₁-C₄, Ci-Cs, C₃-C₈, etc.

The C, H, O, S, N, F, Cl, Br, I, etc. involved in the described functional groups and compounds of the present invention all include their isotopic information. Meanwhile, the C, H, O, S, N, F, Cl, Br, and I involved in the described functional groups and compounds of the present invention can be optionally substituted by one or more corresponding isotopes, including but not limited to carbon isotopes ¹²C, ¹³C and ¹⁴C, hydrogen isotopes protium (H), deuterium (D) and tritium (T), oxygen isotopes ¹⁶0, ¹⁷O and ¹⁸O, sulfur isotopes ³²S, ³³S, ³⁴S, and ³⁶S, nitrogen isotopes ¹⁴N and ¹⁵N, fluorine isotopes ¹⁷F and ¹⁹F, chlorine isotopes ³⁵Cl and ³⁷Cl, and bromine isotopes ⁷⁹Br and ⁸¹Br, etc.

The term "alkyl" used in the present invention refers to a straight or branched saturated hydrocarbon group containing 1 to 8 carbon atoms, including but not limited to methyl, ethyl, propyl, isopropyl, n-butyl, isobutyl, sec-butyl, tert-butyl, n-pentyl, isopentyl, neopentyl, tertpentyl, n-hexyl, isohexyl, neohexyl, heptyl, isoheptyl, neoheptyl, octyl, isooctyl, etc. Alkyl can be substituted by one or more substituents, and when there are ploysubstitutions, the substituents can be the same or different; the substituents are independently D (deuterium), oxo, halogen, cyano, nitro, hydroxyl, amino, aminoalkyl, alkenyl, alkynyl, carboxyl, a carboxylate group, acyl, acylamino, methylsulfonyl, alkylacylamino, C₁-C₈ alkyl, C₁-C₈ alkoxy, C₁-C₈ hydroxyalkyl, C₁-Cs alkylamino, halogenated Ci-Cs alkyl, halogenated C₁-C₈ alkoxy, halogenated C₁-C₈ hydroxyalkyl, halogenated C₁-C₈ alkylamino, C₃-C₁₂ cycloalkyl, halogenated C₃-C₁₂ cycloalkyl, cycloalkenyl, cycloalkynyl, alkoxycarbonyl, alkylthio, alkylsulfonyl, hydroxyalkylacylamino, sulfonamido, spiroalkyl, C₆-C₁₂ aryl, C₅-C₁₄ heteroaryl, or C₃-C₁₂ heterocyclyl.

The term "alkenyl" used in the present invention refers to a straight or branched hydrocarbon group containing 1 to 8 carbon atoms and at least one C=C double bond, including but not limited to vinyl, 1-propenyl, 2-propenyl, 1-butenol, 2-butenol, 3-butenol, 1-pentenol, 2-pentenol, 3-pentenol, 4-pentenol, 1-methyl-1-butenol, 1-methyl-2-butenol, 1-hexenol, 2-hexenol, 3-hexenol, 4-hexenol. 5-hexenyl, 1-methyl-1-pentenyl, 2-methyl-1-pentenyl, 1-heptenyl, 2-heptenyl, 3-heptenyl, 1-methyl-2-hexenyl, 2-methyl-2-hexenyl, 2-methyl-3-hexenyl, 3,5-dimethyl-2-hexenyl, 3,3-dimethyl-1-pentenyl, 3-methyl-2-ethyl-1-butenyl, 1-octenyl, 2-octenyl, etc. Alkenyl can be substituted by one or more substituents, and when there are ploysubstitutions, the substituents can be the same or different; the substituents are independently D, alkyl, haloalkyl, alkoxy, haloalkoxy, hydroxyalkyl, halohydroxyalkyl, alkylamino, haloalkylamino, cycloalkyl, halocycloalkyl, heterocyclyl, aryl, heteroaryl, hydroxyl, halogen, cyano, nitro, amino, aminoalkyl, carboxyl, acylamino, sulfonamido, or spiroalkyl.

The term "alkynyl" used in the present invention refers to a straight or branched hydrocarbon group containing 1 to 8 carbon atoms and at least one C=C triple bond, including but not limited to acetenyl, 1-propinyl, 2-propinyl, 1-butynyl, 2-butynyl, 3-butynyl, 1-pentynyl, 2-pentynyl, 3-pentynyl, 4-pentynyl, 3-methyl-1-butynyl, 4-methyl-1-butynyl, 2-methyl-3-butynyl, 1-methyl-4-butynyl, 1-hexynyl, 2-hexynyl, 3-hexynyl, 4-hexynyl, 1-methyl-2-pentynyl, 1-methyl-3-pentynyl, 1-methyl-4-pentynyl, 2-methyl-3-pentynyl, 2,2-dimethyl-4-pentynyl, 1-heptynyl, 2-heptynyl, 3-heptynyl, 4-heptynyl, 5-heptynyl, 2-methyl-3-hexynyl, 3-methyl-1-hexynyl, 3,3-dimethyl-1-hexynyl, 4-methyl-1-hexynyl, etc. Alkynyl can be substituted by one or more substituents, and when there are ploysubstitutions, the substituents can be the same or different; the substituents are independently D, alkyl, haloalkyl, alkoxy, haloalkoxy, hydroxyalkyl, halohydroxyalkyl, alkylamino, haloalkylamino, cycloalkyl, halocycloalkyl, heterocyclyl, aryl, heteroaryl, hydroxyl, halogen, cyano, nitro, amino, aminoalkyl, carboxyl, acylamino, sulfonamido, or spiroalkyl.

The terms "halogen" and "halogenated" used in the present invention refer to fluorine, chlorine, bromine, and iodine, preferably fluorine, chlorine and bromine.

The term "alkoxy" used in the present invention refers to alkyl-O-, where alkyl is defined as above. Examples of "alkoxy" used in the present invention include but are not limited to methoxy, ethoxy, n-propyloxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, etc. "Alkoxy" also includes substituted alkoxy, and the substituent thereof can be D, halogen, oxo, amino, hydroxyl, cyano, nitro, carboxyl, acylamino, sulfonamido, spiroalkyl, C₁-C₈ alkyl, C₁-C₈ hydroxyalkyl, C₁-C₈ alkoxy, C₁-C₈ alkylamino, halogenated C₁-C₈ alkyl, halogenated C₁-C₈ hydroxyalkyl, halogenated C₁-C₈ alkoxy, halogenated C₁-C₈ alkylamino, C₃-C₁₂ cycloalkyl, C₃-C₁₂ heterocyclyl, C₆-C₁₂ aryl, or C₅-C₁₄ heteroaryl.

The term "hydroxyalkyl" used in the present invention refers to -alkyl-OH, where alkyl is defined as above. Examples of "hydroxyalkyl" used in the present invention include but are not limited to hydroxymethyl, hydroxyethyl, hydroxypropyl, hydroxyisopropyl, hydroxybutyl, hydroxypentyl, etc. "Hydroxyalkyl" also includes substituted hydroxyalkyl, and the substituent thereof can be D, halogen, oxo, amino, hydroxyl, cyano, nitro, carboxyl, acylamino, sulfonamido, spiroalkyl, C₁-C₈ alkyl, C₁-C₈ hydroxyalkyl, C₁-C₈ alkoxy, C₁-C₈ alkylamino, halogenated C₁-C₈ alkyl, halogenated C₁-C₈ hydroxyalkyl, halogenated C₁-C₈ alkoxy, halogenated C₁-C₈ alkylamino, C₃-C₁₂ cycloalkyl, C₃-C₁₂ heterocyclyl, C₆-C₁₂ aryl, or C₅-C₁₄ heteroaryl.

The term "aminoalkyl" used in the present invention refers to NH₂-alkyl-, where alkyl is defined as above. Examples of "aminoalkyl" used in the present invention include but are not limited to aminomethyl, aminoethyl, aminopropyl, aminoisopropyl, aminobutyl, aminopentyl, etc. "Aminoalkyl" also includes substituted aminoalkyl, and the substituent thereof can be D, halogen, oxo, amino, hydroxyl, cyano, nitro, carboxyl, acylamino, sulfonamido, spiroalkyl, C₁-C₈ alkyl, C₁-C₈ hydroxyalkyl, C₁-C₈ alkoxy, C₁-C₈ alkylamino, halogenated Ci-Cs alkyl, halogenated C₁-C₈ hydroxyalkyl, halogenated C₁-C₈ alkoxy, halogenated C₁-C₈ alkylamino, C₃-C₁₂ cycloalkyl, C₃-C₁₂ heterocyclyl, C₆-C₁₂ aryl, or C₅-C₁₄ heteroaryl, which can be substituted on alkyl or N.

The term "alkylamino" used in the present invention refers to alkyl-NH-, where alkyl is defined as above. Examples of "alkylamino" used in the present invention include but are not limited to methylamino, ethylamino, propylamino, isopropanolamino, butylamino, pentalamino, etc. "Alkylamino" also includes substituted alkylamino, and the substituent thereof can be D, halogen, oxo, amino, hydroxyl, cyano, nitro, carboxyl, acylamino, sulfonamido, spiroalkyl, C₁-C₈ alkyl, C₁-C₈ hydroxyalkyl, C₁-C₈ alkoxy, C₁-C₈ alkylamino, halogenated C₁-C₈ alkyl, halogenated C₁-C₈ hydroxyalkyl, halogenated C₁-C₈ alkoxy, halogenated C₁-C₈ alkylamino, C₃-C₁₂ cycloalkyl, C₃-C₁₂ heterocyclyl, C₆-C₁₂ aryl, or C₅-C₁₄ heteroaryl, which can be substituted on alkyl or N.

The term "cycloalkyl" used in the present invention refers to a single- or multi-ring non-aromatic monovalent hydrocarbon group with 3 to 12 carbon atoms (two single rings form a connection or bridged ring or spiral ring or fused ring with chemical bonds), and may have one or more chemical bonds as double or triple bonds. "Cycloalkyl" includes but is not limited to cyclopropyl, cyclobutyl, cyclopentyl, cyclohexyl, cycloheptyl, cyclooctyl, cyclononyl, cyclodecyl, a octahydroindene group, a decahydronaphthalene group, bicyclic[1.1.0]butyl, bicyclic[2.1.0]pentyl, bicyclic[2.2.0]hexyl, bicyclic[3.2.0]heptyl, bicyclic[4.2.0]octyl, bicyclic[1.1.1]pentyl, bicyclic[2.1.1]hexyl, bicyclic[3.1.1]heptyl, bicyclic[2.2.1]heptyl, bicyclic[4.1.1]octyl, bicyclic[3.2.1]octyl, bicyclic[5.1.1]nonyl, bicyclic[4.2.1]nonyl, bicyclic[4.3.1]nonyl, bicyclic[3.2.2]nonyl, bicyclic[5.2.1]decyl, bicyclic[4.2.2]decyl, spirocyclic[2.2]pentyl, spirocyclic[2.3]hexyl, spirocyclic[2.4]heptyl, spirocyclic[2.5]octyl, spirocyclic[2.6]nonyl, spirocyclic[3.5]nonyl, spirocyclic[3.4]octyl, spirocyclic[3.3]heptyl, spirocyclic[4.5]decyl, spirocyclic 4.4]nonyl, etc. Cycloalkyl can be substituted by one or more substituents, and when there are ploysubstitutions, the substituents can be the same or different; the substituents are independently D (deuterium), oxo, halogen, cyano, nitro, hydroxyl, amino, aminoalkyl, alkenyl, alkynyl, carboxyl, a carboxylate group, acyl, acylamino, methylsulfonyl, alkylacylamino, C₁-C₈ alkyl, C₁-C₈ alkoxy, C₁-C₈ hydroxyalkyl, C₁-C₈ alkylamino, halogenated C₁-C₈ alkyl, halogenated C₁-C₈ alkoxy, halogenated C₁-C₈ hydroxyalkyl, halogenated C₁-C₈ alkylamino, C₃-C₁₂ cycloalkyl, halogenated C₃-C₁₂ cycloalkyl, cycloalkenyl, cycloalkynyl, alkoxycarbonyl, alkylthio, alkylsulfonyl, hydroxyalkylacylamino, sulfonamido, spiroalkyl, C₆-C₁₂ aryl, C₅-C₁₄ heteroaryl, or C₃-C₁₂ heterocyclyl.

The term "cycloalkenyl" used in the present invention refers to a single- or multi-ring non-aromatic monovalent hydrocarbon group with 3 to 12 carbon atoms and at least one C=C double bond (two single rings form a connection or bridged ring or spiral ring or fused ring with chemical bonds), including but not limited to cyclopropenyl, cyclobutenyl, cyclopentenyl, cyclohexenyl, cycloheptenyl, cycloalkenyl, spiro[2.2]pent-1-enyl, spiro[2.2]penta-1,4-dienyl, spiro[2.3]hex-1-enyl, spiro[2.3]hexa-1,4-dienyl, spiro[3.3]hept-1-enyl, spiro[3.3]hepta-1,5-dienyl, spiro[3.4]oct-1-enyl, spiro[3.4]octo-1,6-dienyl, spiro[3.4]oct-5-enyl, spiro[3.4]oct-6-enyl, bicyclic[2.1.1]hex-1-enyl, bicyclic[2.1.1]hex-2-enyl, bicyclic[3.1.1]hept-1-enyl, bicyclic[3.1.1]hept-2-enyl, bicyclic[2.2.1]hept-1-enyl, bicyclic[2.2.1]hept-2-enyl, etc. Cycloalkene and cycloalkenyl can be substituted by one or more substituents, and when there are ploysubstitutions, the substituents can be the same or different; the substituents are independently D (deuterium), oxo, halogen, cyano, nitro, hydroxyl, aminoalkyl, alkenyl, alkynyl, carboxyl, a carboxylate group, acyl, acylamino, methylsulfonyl, alkylacylamino, C₁-C₈ alkyl, C₁-C₈ alkoxy, C₁-C₈ hydroxyalkyl, C₁-C₈ alkylamino, halogenated C₁-C₈ alkyl, halogenated C₁-C₈ alkoxy, halogenated C₁-C₈ hydroxyalkyl, halogenated C₁-C₈ alkylamino, C₃-C₁₂ cycloalkyl, halogenated C₃-C₁₂ cycloalkyl, cycloalkenyl, cycloalkynyl, alkoxycarbonyl, alkylthio, alkylsulfonyl, hydroxyalkylacylamino, sulfonamido, spiroalkyl, C₆-C₁₂ aryl, C₅-C₁₂ heteroaryl, or C₃-C₁₂ heterocyclyl.

The term "heterocyclyl" used in the present invention refers to a single- or multi-ring non-aromatic cyclic group with 3 to 12 ring atoms (two single rings are connected or bridged or spiro or fused by chemical bonds), has one or more heteroatoms selected from N, O and S, and may have one or more chemical bonds as double or triple bonds. Heterocyclyl includes but is not limited to pyranyl, piperidyl, piperazinyl, morpholinyl, a dioxane group, oxocyclopropyl, oxocyclobutyl, oxocyclohexyl, oxocycloheptyl, oxacyclooctyl, azacyclopropyl, azacyclobutyl, azacyclopentyl, azacycloheptyl, azacyclooctyl, thiacyclobutyl, thiacyclopropyl, azacyclooctyl, a oxazepine group, a diazepine group, thiazepine, dihydrofuranyl, dihydrothienyl, dihydropyranyl, tetrahydrofuranyl, tetrahydrothienyl, tetrahydropyranyl, tetrahydrothiapyranyl, tetrahydrothiazolyl, tetrahydroimidazolyl, hexahydropyridazinyl, hexahydropyrimidinyl, 1-azaspiro[2.2]pentyl, 1-azaspiro[2.3]hexyl, 4-azaspiro[2.3]hexyl, 5-azaspiro[2.3]hexyl, 2-azaspiro[3.3]heptyl, 2,6-diazaspiro[3.3]heptyl, 2-oxa-6-azaspiro[3.3]heptyl, 1-azaspiro[2.5]octyl, 2-azaspiro[3.4]octyl, 6-azaspiro[3.4]octyl, 2,6-diazaspiro[3.4]octyl, 2-azaspiro[3.5]nonyl, 6-azaspiro[3.5]nonyl, 7-azaspiro[3.5]nonyl, 2,7-diazaspiro[3.5]nonyl, 2-oxa-7-azaspiro[3.5]nonyl, 1-azaspiro[4.4]nonyl, 2-azaspiro[4.4]nonyl, 8-azaspiro[4.5]decyl, 2,8-diazaspiro[4.5]decyl, 1-oxaspiro[2.2]pentyl, 1-oxaspiro[2.3]hexyl, 4-oxaspiro[2.3]hexyl, 5-oxaspiro[2.3]hexyl, 2-oxaspiro[3.3]heptyl, 1-oxaspiro[2.5]octyl, 2-oxaspiro[3.4]octyl, 6-oxaspiro[3.4]octyl, 2-oxaspiro[3.5]nonyl, 6-oxaspiro[3.5]nonyl, 7-oxaspiro[3.5]nonyl, 1-oxaspiro[4.4]nonyl, 2-oxaspiro[4.4]nonyl, 8-oxaspiro[4.5]decyl, decahydroquinolyl, decahydroisoquinolyl, 2-azabicyclo[1.1.1]pentyl, 2-oxabicyclo[1.1.1]pentyl, azabicyclo[2.1.1]hexyl, oxabicyclo[2.1.1]hexyl, azabicyclo[3.1.1]heptyl, oxabicyclo[3.1.1]heptyl, azabicyclo[2.2.1]heptyl, azabicyclo[4.1.1]octyl, azabicyclo[3.2.1]octyl, azabicyclo[3.2.1]octyl, etc. Heterocyclyl can be substituted by one or more substituents, and when there are ploysubstitutions, the substituents can be the same or different; the substituents are independently D (deuterium), oxo, halogen, cyano, nitro, hydroxyl, amino, aminoalkyl, alkenyl, alkynyl, carboxyl, a carboxylate group, acyl, acylamino, methylsulfonyl, alkylacylamino, Ci-Cs alkyl, C₁-Cs alkoxy, C₁-C₈ hydroxyalkyl, C₁-C₈ alkylamino, halogenated Ci-Cs alkyl, halogenated C₁-C₈ alkoxy, halogenated C₁-C₈ hydroxyalkyl, halogenated C₁-C₈ alkylamino, C₃-C₁₂ cycloalkyl, halogenated C₃-C₁₂ cycloalkyl, alkoxycarbonyl, alkylthio, alkylsulfonyl, hydroxyalkylacylamino, sulfonamido, spiroalkyl, C₆-C₁₂ aryl, C₅-C₁₄ heteroaryl, or C₃-C₁₂ heterocyclyl.

The term "aryl" used in the present invention refers to an all-carbon single-ring or fused multi-ring aromatic ring group with 6 to 12 carbon atoms (when multiple rings are fused, one of the fused rings can be partially saturated), including but not limited to a benzene ring, a naphthalene ring, an anthracene ring, an indene ring, a dihydroindene group (indanyl), a dihydronaphthalene ring, a tetrahydronaphthalene ring, etc. Aryl can be unsubstituted or substituted, can be monosubstituted (such as ortho-, meta-, para-substituted), and can also be bisubstituted or trisubstituted, etc. When there are ploysubstitutions, the substituents can be the same or different; the substituents are independently D (deuterium), halogen, cyano, nitro, hydroxyl, amino, aminoalkyl, alkenyl, alkynyl, carboxyl, acyl, acylamino, alkylacylamino, C₁-C₈ alkyl, C₁-C₈ alkoxy, C₁-C₈ hydroxyalkyl, C₁-C₈ alkylamino, halogenated C₁-C₈ alkyl, halogenated C₁-C₈ alkoxy, halogenated C₁-C₈ hydroxyalkyl, halogenated C₁-C₈ alkylamino, C₃-C₁₂ cycloalkyl, halogenated C₃-C₁₂ cycloalkyl, alkoxycarbonyl, alkylthio, alkylsulfonyl, hydroxyalkylacylamino, sulfonamido, spiroalkyl, C₆-C₁₂ aryl, C₅-C₁₄ heteroaryl, or C₃-C₁₂ heterocyclyl.

The term "heteroaryl" used in the present invention refers to a single-ring or fused multi-ring aromatic ring group with 5 to 14 ring atoms (when multiple rings are fused, one of the fused rings can be partially saturated), equivalent to one or more carbons in the aforementioned "aryl" being substituted by heteroatoms such as N, O, S, etc. Heteroaromatic rings can be single rings or double rings formed by the fusion of two rings. Heteroaryl includes but is not limited to pyridyl, pyrimidinyl, pyrazinyl, pyridazinyl, triazinyl, isoxazolyl, isothiazolyl, pyrazolyl, thiazolyl, thienyl, furyl, triazolyl, oxazolyl, imidazolyl, indazolyl, indolizinyl, indolyl, indolinyl, isoindolinyl, quinolyl, isoquinolyl, quinoxalinyl, quinazolinyl, dihydroquinolyl, tetrahydroquinolyl, dihydroisoquinolyl, tetrahydroisoquinolyl, cinnolinyl, pteridyl, purinyl, benzimidazolyl, benzofuryl, benzothiophenyl, benzothiazolyl, benzotriazolyl, benzotriazinyl, benzoxadiazolyl, benzoxazolyl, benzoisoxazolyl, imidazopyridyl, imidazothiazolyl, pyrrolopyridyl, thienopyrrolyl, thienothienyl, thienopyridyl, thienopyrimidinyl, pyrazolopyrimidinyl, pyrrolopyrrolyl, pyrrolopyrimidinyl, pyrrolopyridazinyl, pyrrolopyrazinyl, imidazopyrimidinyl, imidazopyrazinyl, imidazopyridazinyl, pyrazolopyridyl, pyrazolopyridazinyl, pyrazolopyrazinyl, pyrimidopyridyl, pyrimidopyrazinyl, pyrimidopyridazinyl, pyrimidopyrimidinyl, pyridinopyridyl, pyridinopyrazinyl, pyridinopyridazinyl, pyridazinopyridazinyl, pyridazinopyrazinyl, pyrazinopyrazinyl, etc.
Heteroaryl can be unsubstituted or monosubstituted or polysubstituted, and when there are ploysubstitutions, the substituents can be the same or different; the substituents are independently D (deuterium), halogen, cyano, nitro, amino, aminoalkyl, hydroxyl, carboxyl, a carboxylate group, acyl, acylamino, alkylacylamino, alkoxycarbonyl, alkylthio, alkylsulfonyl, hydroxyalkylacylamino, sulfonamido, spiroalkyl, C₁-C₈ alkyl, C₁-C₈ hydroxyalkyl, C₁-C₈ alkoxy, C₁-C₈ alkylamino, halogenated C₁-C₈ alkyl, halogenated C₁-C₈ hydroxyalkyl, halogenated C₁-C₈ alkoxy, halogenated C₁-C₈ alkylamino, C₃-C₁₂ cycloalkyl, halogenated C₃-C₁₂ cycloalkyl, C₃-C₁₂ heterocyclyl, C₆-C₁₂ aryl, or C₅-C₁₄ heteroaryl.

The terms "spiral ring" and "spirocyclyl" used in the present invention refer to a multi-ring structure in which at least 2 rings share a common atom (usually C atom). In this multi-ring structure, one or more chemical bonds may be double or triple bonds, and one or more heteroatoms may exist. Spirocyclyl includes but is not limited to cyclobutylspiroazacyclobutyl, cyclobutylspiroazacyclopentyl, cyclobutylspiroazacyclohexyl, cyclopentylspiroazacyclobutyl, cyclopentylspiroazacyclopentyl, cyclopentylspiroazacyclohexyl, cyclohexylspiroazacyclobutyl, cyclohexylspiroazacyclopentyl, cyclohexylspiroazacyclohexyl, azacyclobutylspiroazacyclobutyl, azacyclobutylspiroazacyclopentyl, azacyclobutylspiroazacyclohexyl, azacyclopentylspiroazacyclobutyl, azacyclopentylspiroazacyclopentyl, azacyclopentylspiroazacyclohexyl, azacyclohexylspiroazacyclobutyl, azacyclohexylspiroazacyclopentyl, azacyclohexylspiroazacyclohexyl, cyclobutylspiropiperidine, cyclopentylspiropiperidine, cyclohexylspiropiperidine, azacyclobutylspiropiperidine, azacyclopentylspiropiperidine, azacyclohexylspiropiperidine, etc. The spirocyclyl can be unsubstituted or monosubstituted or polysubstituted, and when there are ploysubstitutions, the substituents can be the same or different; the substituents are independently D (deuterium), oxo, halogen, cyano, nitro, hydroxyl, amino, aminoalkyl, alkenyl, alkynyl, carboxyl, a carboxylate group, acyl, acylamino, methylsulfonyl, alkylacylamino, C₁-C₈ alkyl, C₁-C₈ alkoxy, C₁-C₈ hydroxyalkyl, Ci-Cs alkylamino, halogenated C₁-C₈ alkyl, halogenated C₁-C₈ alkoxy, halogenated C₁-C₈ hydroxyalkyl, halogenated C₁-C₈ alkylamino, C₃-C₁₂ cycloalkyl, halogenated C₃-C₁₂ cycloalkyl, cycloalkenyl, cycloalkynyl, alkoxycarbonyl, alkylthio, alkylsulfonyl, hydroxyalkylacylamino, sulfonamido, spiroalkyl, C₆-C₁₂ aryl, C₅-C₁₄ heteroaryl, or C₃-C₁₂ heterocyclyl.

The term "bridged ring group" used in the present invention refers to a multi-ring structure in which at least 2 rings share two or more atoms. In this multi-ring structure, one or more chemical bonds may be double or triple bonds, and one or more heteroatoms may exist. The bridged ring group can be unsubstituted or monosubstituted or polysubstituted, and when there are ploysubstitutions, the substituents can be the same or different; the substituents are independently D (deuterium), oxo, halogen, cyano, nitro, hydroxyl, amino, aminoalkyl, alkenyl, alkynyl, carboxyl, a carboxylate group, acyl, acylamino, methylsulfonyl, alkylacylamino, C₁-C₈ alkyl, C₁-Cs alkoxy, C₁-C₈ hydroxyalkyl, C₁-C₈ alkylamino, halogenated C₁-C₈ alkyl, halogenated C₁-C₈ alkoxy, halogenated C₁-C₈ hydroxyalkyl, halogenated C₁-C₈ alkylamino, C₃-C₁₂ cycloalkyl, halogenated C₃-C₁₂ cycloalkyl, cycloalkenyl, cycloalkynyl, alkoxycarbonyl, alkylthio, alkylsulfonyl, hydroxyalkylacylamino, sulfonamido, spiroalkyl, C₆-C₁₂ aryl, C₅-C₁₄ heteroaryl, or C₃-C₁₂ heterocyclyl.

The term "fused bicyclic group" used in the present invention refers to a bicyclic group composed of 5 to 14 carbon atoms, where the two rings are connected by chemical bonds. In this bicyclic structure, one or more chemical bonds may be double or triple bonds, and one or more heteroatoms may exist, including but not limited to N, O, S, etc. The "fused bicyclic group" can be unsubstituted or monosubstituted or polysubstituted, and when there are ploysubstitutions, the substituents can be the same or different; the substituents are independently D (deuterium), oxo, halogen, cyano, nitro, hydroxyl, amino, aminoalkyl, alkenyl, alkynyl, carboxyl, a carboxylate group, acyl, acylamino, methylsulfonyl, alkylacylamino, C₁-C₈ alkyl, C₁-C₈ alkoxy, C₁-C₈ hydroxyalkyl, Ci-Cs alkylamino, halogenated C₁-C₈ alkyl, halogenated C₁-C₈ alkoxy, halogenated C₁-C₈ hydroxyalkyl, halogenated C₁-C₈ alkylamino, C₃-C₁₂ cycloalkyl, halogenated C₃-C₁₂ cycloalkyl, cycloalkenyl, cycloalkynyl, alkoxycarbonyl, alkylthio, alkylsulfonyl, hydroxyalkylacylamino, sulfonamido, spiroalkyl, C₆-C₁₂ aryl, C₅-C₁₄ heteroaryl, or C₃-C₁₂ heterocyclyl.

The terms "alkylcarbonyl" used in the present invention refer to alkyl-C(O)-, where alkyl is defined as above. "Alkylcarbonyl" also includes substituted alkylcarbonyl, and the substituent thereof can be D, halogen, oxo, amino, hydroxyl, cyano, nitro, carboxyl, acylamino, sulfonamido, spiroalkyl, C₁-C₈ alkyl, C₁-C₈ hydroxyalkyl, C₁-C₈ alkoxy, C₁-C₈ alkylamino, halogenated C₁-C₈ alkyl, halogenated C₁-C₈ hydroxyalkyl, halogenated C₁-C₈ alkoxy, halogenated C₁-C₈ alkylamino, C₃-C₁₂ cycloalkyl, C₃-C₁₂ heterocyclyl, C₆-C₁₂ aryl, or C₅-C₁₄ heteroaryl. "C(O)" represents C=O.

The term "alkoxycarbonyl" used in the present invention refers to alkyl-O-C(O)-, where alkyl is defined as above. "Alkoxycarbonyl" also includes substituted alkoxycarbonyl, and the substituent thereof can be D, halogen, oxo, amino, hydroxyl, cyano, nitro, carboxyl, acylamino, sulfonamido, spiroalkyl, C₁-C₈ alkyl, C₁-C₈ hydroxyalkyl, C₁-C₈ alkoxy, C₁-C₈ alkylamino, halogenated C₁-C₈ alkyl, halogenated C₁-C₈ hydroxyalkyl, halogenated C₁-C₈ alkoxy, halogenated C₁-C₈ alkylamino, C₃-C₁₂ cycloalkyl, C₃-C₁₂ heterocyclyl, C₆-C₁₂ aryl, or C₅-C₁₄ heteroaryl. "C(O)" represents C=O.

The term "alkylthio" used in the present invention refers to alkyl-S-, where alkyl is defined as above. Alkylthio includes but is not limited to methylthio, ethylthio, propylthio, butylthio, etc.

The term "alkanoyl" used in the present invention refers to alkyl-C(O)-, where alkyl is defined as above.

The term "alkylsulfonyl" used in the present invention refers to alkyl-S(O)₂-, where alkyl is defined as above.

The term "haloalkyl" used in the present invention refers to a linear or branched alkyl group substituted by a halogen (preferably fluorine, chlorine, bromine, iodine), where "alkyl" is defined as above. Examples of "haloalkyl" used in the present invention include but are not limited to fluoromethyl, difluoromethyl, trifluoromethyl, chloromethyl, dichloromethyl, trichloromethyl, 2,2,2-trifluoroethyl, 2-fluoroethyl, 2-chloroethyl, tetrafluoroethyl, pentafluoroethyl, fluoropropyl, difluoropropyl, trifluoropropyl, chloropropyl, fluorobutyl, etc. "Haloalkyl" can be substituted by a halogen once or multiple times.

The term "halohydroxyalkyl" used in the present invention refers to a hydroxyalkyl group substituted by a halogen (preferably fluorine, chlorine, bromine, iodine), where hydroxyalkyl is defined as above. "Halohydroxyalkyl" can be substituted by a halogen once or multiple times.

The term "haloalkoxy" used in the present invention refers to an alkoxygroup substituted by a halogen (preferably fluorine, chlorine, bromine, iodine), where alkoxy is defined above. "Haloalkoxy" can be substituted by a halogen once or multiple times.

The term "haloalkylamino" used in the present invention refers to an alkylamino group substituted by a halogen (preferably fluorine, chlorine, bromine, iodine), where alkylamino is defined above. "Haloalkylamino" can be substituted by a halogen once or multiple times.

The term "pharmaceutically acceptable salt" used in the present invention refers to a salt suitable for use as a drug formed by the compound of the present invention and an acid or base. Pharmaceutically acceptable salts include inorganic and organic salts. A preferred type of salts is a salt formed by the compound of the present invention and an acid. Acids suitable for forming salts include but are not limited to inorganic acids such as hydrochloric acid, hydrobromic acid, hydrofluoric acid, hydroiodic acid, sulfuric acid, nitric acid, phosphoric acid, and carbonic acid, and organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, p-toluenesulfonic acid, ethanesulfonic acid, benzenesulfonic acid, camphorsulfonic acid, 2-O-(beta-D-glucopyranosyl)ascorbic acid, isonicotinic acid, salicylic acid, ascorbic acid, gentisic acid, gluconic acid, pyruvic acid, naphthalene sulfonic acid, stearic acid, phenylacetic acid, p-aminobenzenesulfonic acid, hydroxyethanesulfonic acid, dihydroxynaphthalic acid, and tannic acid; and acidic amino acids such as aspartic acid and glutamic acid. A preferred type of salts is a salt formed by the compound of the present invention and a base. Bases suitable for forming salts include but are not limited to inorganic bases such as sodium hydroxide, potassium hydroxide, sodium carbonate, sodium bicarbonate, and sodium phosphate, and organic bases such as ammonium hydroxide, triethylamine, diethylamine, piperazine, guanidine, and diethanolamine.

The second objective of the present invention is to provide a pharmaceutical composition including one or more of the compound according to any one of the aforementioned technical solutions. The pharmaceutical composition described in the present invention can be one or more of the compounds according to any one of the aforementioned technical solutions combined with other compounds, or one or more of the compounds according to any one of the aforementioned technical solutions.

In another aspect, the present invention provides uses of compounds described using general formulas I to VII disclosed herein in preparation of a drug for treating diseases, disorders or conditions that benefit from inhibiting BCL-2 activity, either alone or in combination with other drugs.

In another aspect, the compound or pharmaceutically acceptable salt of the present invention can be used alone or in combination with other therapeutic agents.

In another aspect, when the compound of the present invention is used in combination with other therapeutic agents, the administration route may be the same as other drugs, or due to different physical and chemical properties, the administration route may be different. Therefore, the compound described herein can be administered simultaneously, sequentially, or separately with another therapeutic agent.

In another aspect, the present invention provides a compound or pharmaceutically acceptable salt described using general formulas I to VII disclosed herein, which are expected to be effective when used in combination with one or more of the following drugs: alkylating agents, angiogenesis inhibitors, antibodies, antimetabolites, antimitotic agents, antiproliferative agents, antiviral agents, Aurora kinase inhibitors, other promoter (e.g. Bcl-xL, Bcl-w and Bfl-1) inhibitors of cell apoptosis, death receptor pathway activators, Bcr-Abl kinase inhibitors, antibodies to BiTEs (bispecific T cell engagers), antibody-drug conjugates, biological response modifiers, cyclin-dependent kinase inhibitors, cell cycle inhibitors, cyclooxygenase-2 inhibitors, DVDs, leukaemia viral oncogene homologous gene (ErbB2) receptor inhibitors, growth factor inhibitors, heat shock protein (HSP)-90 inhibitors, histone deacetylase (HDAC) inhibitors, hormonotherapy, immune preparations, inhibitors of apoptotic proteins (IAPs), embedded antibiotics, kinase inhibitors, kinesin inhibitors, JAK2 inhibitors, rapamycin inhibitors targeting mammals, microRNAs, mitogen-activated extracellular signal-regulated kinase inhibitors, multivalent binding proteins, non-steroidal anti-inflammatory drugs (NSAIDs), poly-ADP (adenosine diphosphate)-ribose polymerase (PARP) inhibitors, platinum chemotherapeutic drugs, polo-like kinase (Plk) inhibitors, phosphoinositide 3-kinase (PI3K) inhibitors, BTK inhibitors, proteasome inhibitors, purine analogues, pyrimidine analogues, receptor tyrosine kinase inhibitors, retinoid/deltoid plant alkaloids, small interfering RNAs (siRNAs), topoisomerase inhibitors, ubiquitin ligase inhibitors, and the like.

Specifically, the diseases, disorders or conditions include but are not limited to infectious diseases, immune diseases, inflammatory diseases, or cell proliferation abnormalities.

Specifically, the infectious diseases, immune diseases, inflammatory diseases include but are not limited to asthma, diseases caused by neutrophil chemotaxis (e.g. myocardial infarction and reperfusion injury in stroke and inflammatory arthritis), infectious shock, T cell-mediated diseases, immunosuppression-related diseases (e.g. prevention of organ transplant rejection, graft-versus-host disease, lupus erythematosus, multiple sclerosis, and rheumatoid arthritis), pancreatitis, vasculogenesis- or angiogenesis-related diseases (e.g. acute and chronic inflammatory diseases such as rheumatoid arthritis and inflammatory bowel disease, and skin diseases such as psoriasis, eczema and scleroderma); chronic obstructive pulmonary disease (COPD) and other diseases.

Specifically, the cell proliferation abnormalities include cancerous proliferative diseases and non-cancerous proliferative diseases, including but not limited to hematological diseases and/or solid tumors, where the hematological tumor is preferably one or more of acute myeloid leukemia, acute lymphocytic leukemia, chronic myeloid leukemia, myelofibrosis, myelodysplastic syndrome, diffuse large B-cell lymphoma, follicular lymphoma, chronic lymphocytic leukemia, small lymphocytic lymphoma, mantle cell lymphoma, Waldenstrom's macroglobulinemia, multiple myeloma, and T-cell lymphoma; and the solid tumor is preferably one or more of osteosarcoma, skin cancer, breast cancer, kidney cancer, prostate cancer, colorectal cancer, thyroid cancer, ovarian cancer, pancreatic cancer, glioma, neuroblastoma, epidermoid carcinoma, hemangioma, lung cancer or gastric cancer, restenosis and benign prostatic hypertrophy (BPH), and melanoma.

The compound of the present invention can be administered in the form of a pharmaceutical composition and can be administered through any conventional route; and the compound of the present invention can form pharmaceutical preparations in solid, semi-solid, liquid or gas forms, such as tablets, capsules, injections, suspensions, lotions, gel, ointments, creams, suppositories, and inhalants.

The compound described in the present invention can be a pharmaceutical combination of a compound in a free base or a pharmaceutically acceptable salt form with at least one pharmaceutically acceptable carrier or diluent, and can be manufactured in a conventional manner through mixing, granulation, coating, dissolution, or freeze-drying processes.

Through experiments, it has been confirmed that the compound of the present invention has strong BCL-2BAK blocking activity and in vitro anti-tumor cell proliferation inhibitory activity. It can be used to treat infectious diseases, immune diseases, inflammatory diseases, or cell proliferation abnormalities that benefit from the inhibition of anti-apoptotic protein BCL-2, either alone or in combination with other drugs.

Through experiments, it has been confirmed that the compound of the present invention has strong BCL2 (G101V) inhibitory activity.

Through experiments, it has been confirmed that the compound of the present invention has strong BCL2 (D103Y) inhibitory activity.

Through experiments, it has been confirmed that the compound of the present invention has significant proliferative inhibitory activity on RS4;11 cells.

Through experiments, it has been confirmed that the compound of the present invention has no significant toxicity to platelets.

Through experiments, it has been confirmed that the compound of the present invention has no inhibitory effect on CYP3A4.

Through experiments, it has been confirmed that the compound of the present invention has good pharmacokinetic properties.

Through experiments, it has been confirmed that the compound of the present invention has efficient in vivo anti-tumor effects.

### DESCRIPTION OF THE EMBODIMENTS

### Intermediate 1: (5)-2-(2-isopropylphenyl)pyrrolidine

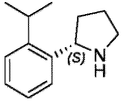

### Synthesis step 1: (S)-tert-butyl 2-(2-isopropylphenyl)pyrrolidine-1-carboxylate (intermediate 1-1)

(*S*)-tert-butyl2-(2-bromophenyl)pyrrolidine-1-carboxylate (4.5 g, 13.85 mmol) and isopropylboronic acid (3.1 g, 35.22 mmol) were dissolved in a mixed solvent of 15 mL of 1,4-Dioxane and 3 mL of water. Potassium carbonate (6 g, 43.40 mmol) and Pd(dppf)₂Cl₂ (0.9 g) were added under nitrogen protection, and heated until reflux reaction overnight. After the reaction was completed, the temperature was dropped to room temperature and EA was added. The organic phase was washed with water/saturated salt water, separated, dried and filtered, and concentrated under reduced pressure. The resulting crude product was purified by silica gel column chromatography to obtain 3.4 g of (*S*)-tert-butyl 2-(2-isopropylphenyl)pyrrolidine-1-carboxylate, with a yield of 85%. ESI-MS(M+H)⁺=290.2.

### Synthesis step 2: (S)-2-(2-isopropylphenyl)pyrrolidine (intermediate 1)

Intermediate 1-1 (3 g, 10.40 mmol) was dissolved in 18 mL of dichloromethane, and 9 mL of TFA was added and stirred for reaction overnight at room temperature. After the reaction was completed, concentration was perfomred under reduced pressure to obtain 1.92 g of the colorless oily product of intermediate 1, with a yield of 97%. ESI-MS(M+H)⁺=190.2.

### Intermediate 2: 4-((dimethylamino)methyl)-3-methoxybenzaldehyde

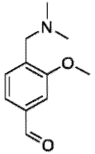

### Synthesis step 1: 1-(4-bromo-2-methoxyphenyl)-N,N-dimethylmethylamine (intermediate 2-1)

4-bromo-2-methoxybenzaldehyde (3 g, 13.95 mmol) was dissolved in 50 mL of DCM, 2 mol/L of tetrahydrofuran solution of dimethylamine (8.4 mL, 16.74 mmol) was added and stirred at room temperature for 30 min, and then STAB (3.85 g, 18.14 mmol) was added and reacted at room temperature for 4 h. The reaction was completed, and a saturated sodium bicarbonate aqueous solution was added for extraction. The DCM phase was separated and concentrated under reduced pressure. The resulting crude product was purified by column chromatography to obtain 1.8 g of 1-(4-bromo-2-methoxyphenyl)-N,N-dimethylmethylamine, with a yield of 53%. ESI-MS(M+H)⁺=244.0.

### Synthesis step 2: 4-((dimethylamino)methyl)-3-methoxybenzaldehyde (intermediate 2)

Intermediate 2-1 (1.8 g, 7.39 mmol) was dissolved in 30 mL of anhydrous THF, the system was cooled to -70°C, 2.5 mol/L of hexane solution of n-butyl lithium (3.6 mL, 8.87 mmol) was slowly added and reacted at -70°C for 1 h, then anhydrous DMF (1 mL, 14.78 mmol) was added for further 1-h reaction at the same temperature, followed by changing to room temperature, the reaction was quenched with water, EA was added for extraction (100 ml*2), the organic phase was separated and merged, dried with anhydrous sodium sulfate, and filtered and concentrated, and the resulting crude product was purifed by column chromatography to obtain 871 mg of the product of intermediate 2, with a yield of 61%. ESI-MS(M+H)⁺=194.1.

### Intermediate 3: 3-methoxy-4-(pyrrolidin-1-ylmethyl)benzaldehyde

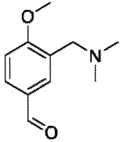

Referring to the synthesis route and method of intermediate 2, the 4-bromo-2-methoxybenzaldehyde in synthesis step 1 was replaced with 5-bromo-2-methoxybenzaldehyde to synthesize intermediate 3. ESI-MS(M+H)⁺=194.1.

Referring to the synthesis route and method of intermediates 2-3, the following intermediate structures were synthesized:

| Interm ediate number | Intermediate structure | LC-MS (ESI-MS) *m*/*z* [M+H]⁺ |
|---|---|---|
| 4 | 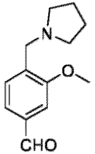 | 220.1 |
| 5 | 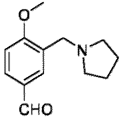 | 220.1 |
| 6 | 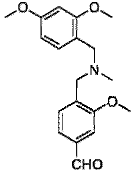 | 330.2 |
| 7 | 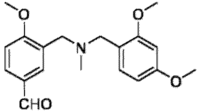 | 330.2 |

### Intermediate 8: 4-(((2,4-dimethoxybenzyl)(methyl)amino)methyl)benzaldehyde

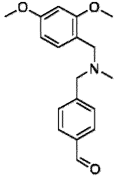

### Synthesis step 1: N-(4-(diethoxymethyl)benzyl)-1-(2,4-dimethoxyphenyl)-N-methylmethylamine (intermediate 8-1)

4-(diethoxymethyl)benzaldehyde (500 mg, 2.4 mmol) was disolved in 20 mL of DCM, and 1-(2,4-dimethoxyphenyl)-N-methylmethylamine (522 mg, 2.88 mmol) and STAB (1.5 g, 7.2 mmol) were added sequentially and reacted at room temperature for 10 h. The TLC monitoring reaction was completed, water was added for extraction and liquid separation, the DCM phase was separated and dried with anhydrous sodium sulfate, and the resulting crude product was purified by column chromatography to obtain 600 mg of N-(4-(diethyloxymethyl)benzyl)-1-(2,4-dimethoxyphenyl)-N-methylmethylamine, with a yield of 67%. ESI-MS(M+H)⁺=374.2.

### Synthesis step 2: 4-(((2,4-dimethoxybenzyl)(methyl)amino)methyl)benzaldehyde (intermediate 8)

Intermediate 8-1 (600 mg, 1.6 mmol) was dissolved in 10 mL of THF, 10 mL of 2 N HCl aqueous solution was added and stirred at room temperature for 1 h, then THF was concentrated, EA and water were added for extraction, the organic phase was separated and dried with anhydrous sodium sulfate, and the resulting crude product was purified by column chromatography to obtain 426.3 mg of the product of intermediate 8, with a yield of 89%. ESI-MS(M+H)⁺=300.2.

### Intermediate 9: 3-(2-isopropylphenyl)-1-(4-methoxybenzyl)piperazine

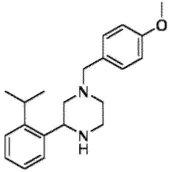

### Synthesis step 1: (2-isopropylphenyl)boronic acid (intermediate 9-1)

1-bromo-2-isopropylbenzene (20 g, 0.10 mol) was dissolved in 200 mL of THF and stirred at -80°C for 10 min, then n-BuLi (44 mL, 0.11 mol) was added dropwise, and after adding, the temperature was kept at -80°C for 1 h. 50 mL of THF dissolved with triisopropyl borate was added to the system, the temperature was still kept at -80°C for 1 h and then changed to 20°C for stirring and reaction for 1 h, and at the same time, a HCl aqueous solution (150 mL,0.15 mol, 1 N) was added dropwise, and stirring was continued for 1 h. After the reaction was completed, EA was added for extraction, and the organic phase was separated, dried with anhydrous sodium sulfate, filtered, and concentrated under reduced pressure to obtain crude (2-isopropylphenyl) boric acid, which was directly used for the next reaction. ESI-MS(M+H)⁺ = 165.1.

### Synthesis step 2: 2-(2-isopropylphenyl)pyrazine (intermediate 9-2)

150 mL of dioxane and 150 mL of water were added to intermediate 9-1 (16.5 g, 0.10 mol), stirred and dissolved, then sequentially added 2-chloropyrazine (13.80 g, 0.12 mol), Pd(PPh₃)₄ (1.74 g, 1.50 mmol) and Na₂CO₃ (21.20 g, 0.20 mol) to the system, and reacted at 90°C for 12 h. After the reaction was completed, the temperature was cooled to room temperature, the system was passed through diatomaceous earth, EA was added for extraction, and the organic phase was separated, dried and filtered, and concentrated under reduced pressure. The resulting crude product was purified by column chromatography to obtain 13 g of colorless oily product 2-(2-isopropylphenyl)pyrazine, with a yield of 65%. ESI-MS(M+H)⁺= 199.1.

### Synthesis step 3: 2-(2-isopropylphenyl)piperazine (intermediate 9-3)

150 mL of anhydrous ethanol and 10 mL of glacial acetic acid were added to intermediate 9-2 (10.2 g, 51.55 mmol), stirred and dissolved, and then Pd/C (5 g) was added to the reaction solution. Afterwards, the system was placed in a hydrogen (4 atm) environment at 60°C for 10 h. After the reaction was complete, the diatomaceous earth was filtered and removed, and methanol (300 mL) and water (100 mL) were used for washing, respectively. Reduced pressure concentration was used to remove most of the methanol. EA was used for extraction, and the aqueous phase was separated. A 10% NaOH aqueous solution was used to adjust the pH to 13-14. Then DCM was used for extraction, and the organic phase was separated, dried and filtered, and concentrated under reduced pressure. The resulting crude product was purified by column chromatography to obtain 9.66 g of product 2-(2-isopropylphenyl)piperazine, with a yield of 91%. ESI-MS(M+H)⁺= 205.2.

### Synthesis step 4: 3-(2-isopropylphenyl)-1-(4-methoxybenzyl)piperazine (intermediate 9)

Intermediate 9-3 (2.00 g, 9.80 mmol) and 4-methoxybenzaldehyde (1.20 mL, 9.80 mmol) were dissolved in 40 mL of DCM, and STAB (6.20 g, 29.40 mmol) was added and reacted at room temperature for 3 h. After the reaction was complete, DCM was added for extraction, and the organic phase was separated, dried and filtered, and concentrated under reduced pressure. The resulting crude product was separated and purified by column chromatography to obtain 1.7 g of the solid product of intermediate 9, with a yield of 53%. ESI-MS(M+H)⁺= 325.2.

### Intermediate 10: (R)-3-(2-isopropylphenyl)-1-(4-methoxybenzyl)piperazine

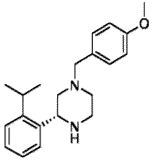

### Synthesis step 1: (R)-2-(2-isopropylphenyl)piperazine (intermediate 10-1)

Intermediate 9-3 (2 g, 9.8 mmol) was dissolved in a mixed solvent of 650 mL MeOH/EA (V/V=1:10), N-acetyl-D-leucine (17 g, 98 mmol) was added for reflux reaction for 1 h until the solution was completely dissolved, and the system was allowed to cool naturally to room temperature and stirred for reaction for 16 h. The reaction was complete, filtration was performed, and the filter cake was rinsed with ethyl acetate before being concentrated at 45°C for 1 h under reduced pressure to obtain approximately 15 g of crude product. The crude product was extracted three times (100 mL*3) using a DCM/1 N NaOH aqueous solution (V/V=3:2), and the organic phase was merged, and dried, filtered and concentrated with anhydrous sodium sulfate to obtain 5.3 g of (R)-2-(2-isopropylphenyl)piperazine, with a yield of 26.5%. ESI-MS(M+H)⁺= 205.2.¹H NMR (400 MHz, Chloroform-*d* δ 7.55 (dd, *J* = 7.6, 1.6 Hz, 1H), 7.30 - 7.10 (m, 3H), 4.06 (dd, *J* = 10.1, 2.7 Hz, 1H), 3.31 (hept, *J* = 6.9 Hz, 1H), 3.16 - 3.07 (m, 1H), 3.04 - 2.83 (m, 4H), 2.69 (dd, *J* = 12.1, 10.0 Hz, 1H), 1.23 (dd, *J* = 6.9, 1.9 Hz, 6H).

### Synthesis step 2: (R)-3-(2-isopropylphenyl)-1-(4-methoxybenzyl)piperazine (intermediate 10)

Intermediate 10-1 (5 g, 24.5 mmol) was dissolved in 50 mL of DCM, 4-methoxybenzaldehyde (3.33 g, 24.5 mmol) was added and stirred at 0°C for 30 min, then STAB (7.8 g, 36.7 mmol) was added and stirred evenly for reaction, and the reaction was continued at 0°C for 16 h. After the reaction was completed, DCM and water extraction were added for extraction and liquid separation, and the organic phase was concentrated under reduced pressure. The resulting crude product was purified by column chromatography to obtain 6.9 g of the solid product of intermediate 10, with a yield of 87%.ESI-MS(M+H)⁺= 325.2. ¹H NMR (400 MHz, Chloroform-*d*) δ 7.53 (dd, *J* = 7.5, 1.3 Hz, 1H), 7.28 - 7.19 (m, 4H), 7.15 (ddd, *J* = 7.7, 6.5, 2.2 Hz, 1H), 6.87 - 6.80 (m, 2H), 4.33 (dd, *J* = 10.5, 2.7 Hz, 1H), 3.77 (s, 3H), 3.57 (d, *J* = 1.8 Hz, 2H), 3.24 - 3.06 (m, 3H), 2.91 (td, *J* = 12.2, 11.2, 2.3 Hz, 2H), 2.44 - 2.33 (m, 1H), 2.23 (dd, *J* = 11.8, 10.5 Hz, 1H), 1.22 (d, *J* = 6.7 Hz, 3H), 1.12 (d, *J* = 6.8 Hz, 3H).

### Intermediate 11: 3-(2-isopropylphenyl)-1-(tetrahydro-2H-pyran-4-yl)piperazine

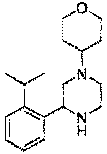

Referring to the synthesis route and method of intermediate 9, 4-methoxybenzaldehyde in synthesis step 4 was replaced with tetrahydro-4H-pyran-4-one to synthesize intermediate 11.ESI-MS(M+H)⁺= 289.2.

### Intermediate 12: (R)-3-(2-isopropylphenyl)-1-((6-methoxypyrid-3-yl)methyl)piperazine

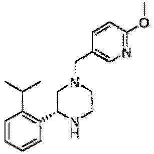

Referring to the synthesis route and method of intermediate 10, 4-methoxybenzaldehyde in synthesis step 2 was replaced with 6-methoxynicotinaldehyde to synthesize intermediate 12.ESI-MS(M+H)⁺= 326.2.

Referring to the synthesis route and method of intermediates 9-12, the following intermediate structures were synthesized:

| Inter media te numb er | Intermediate structure | LC-MS (ESI-MS) *m*/*z* [M+H]⁺ | Inter medi ate num ber | Intermediate structure | LC-MS (ESI-MS) *m*/*z* [M+H]⁺ |
|---|---|---|---|---|---|
| 13 | 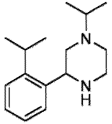 | 247.2 | 34 | 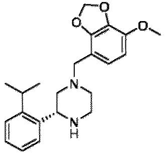 | 369.2 |
| 14 | 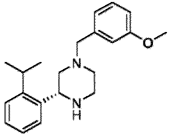 | 325.2 | 35 | 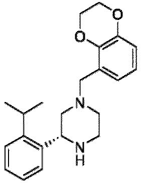 | 353.2 |
| 15 | 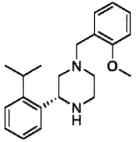 | 325.2 | 36 | 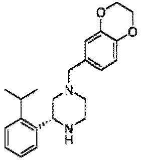 | 353.2 |
| 16 | 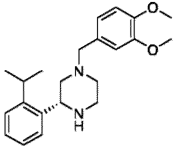 | 355.2 | 37 | 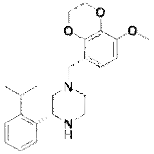 | 383.2 |
| 17 | 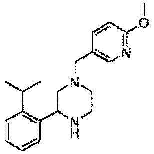 | 326.2 | 38 | 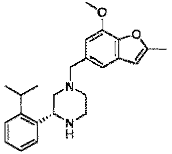 | 379.2 |
| 18 | 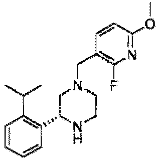 | 344.2 | 39 | 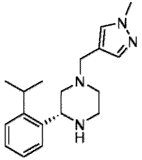 | 299.2 |
| 19 | 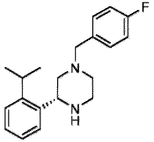 | 313.2 | 40 | 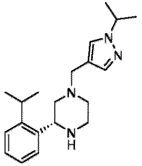 | 327.2 |
| 20 | 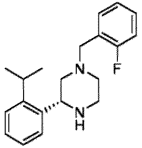 | 313.2 | 41 | 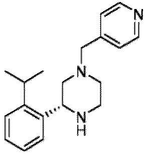 | 296.2 |
| 21 | 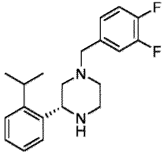 | 331.2 | 42 | 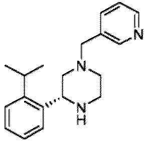 | 296.2 |
| 22 | 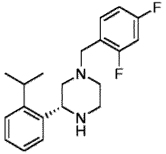 | 331.2 | 43 | 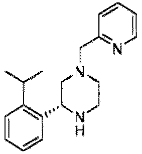 | 296.2 |
| 23 | 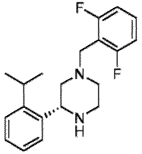 | 331.2 | 44 | 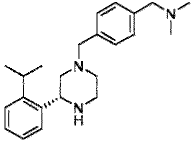 | 352.3 |
| 24 | 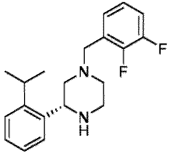 | 331.2 | 45 | 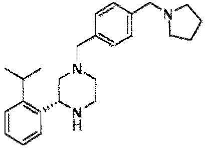 | 378.3 |
| 25 | 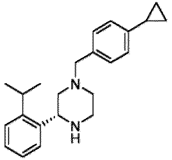 | 335.2 | 46 | 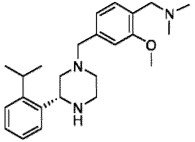 | 382.3 |
| 26 | 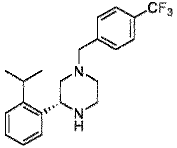 | 363.2 | 47 | 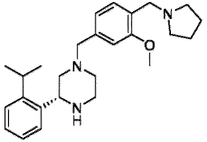 | 408.3 |
| 27 | 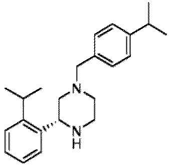 | 337.3 | 48 | 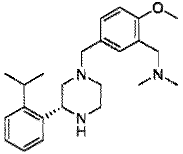 | 382.3 |
| 28 | 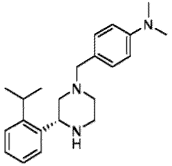 | 338.3 | 49 | 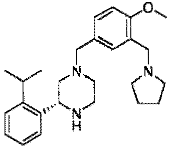 | 408.3 |
| 29 | 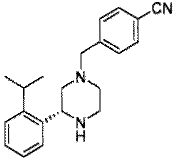 | 320.2 | 50 | 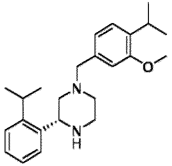 | 367.3 |
| 30 | 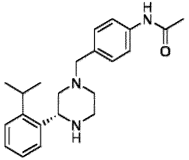 | 352.2 | 51 | 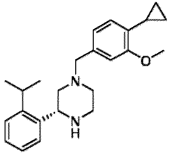 | 365.3 |
| 31 | 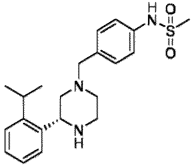 | 388.2 | 52 | 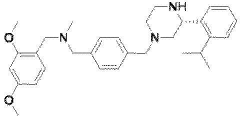 | 488.3 |
| 32 | 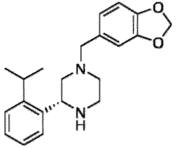 | 339.2 | 53 | 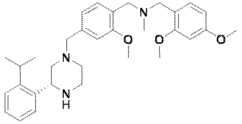 | 518.3 |
| 33 | 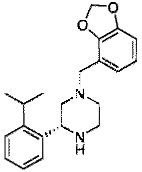 | 339.2 | 54 | 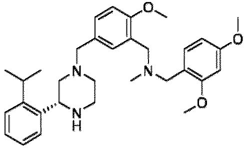 | 518.3 |

### Intermediate 55: methyl 2-((1H-pyrrolo[2,3-b]pyrid-5-yl)oxy)-4-(2-oxo-7-azaspiro [3.5] non-7-yl)benzoate

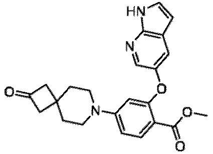

### Synthesis step 1: 7-azaspiro[3.5]nonan-2-one trifluoroacetate (intermediate 55-1)

Tert-butyl 2-oxo-7-azaspiro[3.5]nonane-7-carboxylate (5 g, 20.6 mmol) was dissolved in 40 mL of DCM, and TFA (14 mL, 206 mmol) was added and stirred for reaction at 25°C for 2 h. The reaction was complete, and the system was concentrated to remove the solvent and TFA, without further purification, and can be directly used for the next reaction.

### Synthesis step 2: methyl 2-fluoro-4-(2-oxo-7-azaspiro[3.5]non-7-yl)benzoate (intermediate 55-2)

Methyl 2,4-difluorobenzoate (3.5 g, 20.6 mmol) was dissolved in 100 mL of DMF, and intermediate 55-1 (5.2 g, 20.6 mmol) and sodium carbonate (6.5 g, 61.8 mmol) were sequentially added and reacted at 80°C for 10 h. TLC showed that the reaction was complete. The system was concentrated to remove DMF, and EA and water were added for extraction. The organic phase was separated and merged, dried with anhydrous sodium sulfate, and concentrated under reduced pressure. The resulting crude product was purified by column chromatography to obtain 4.7 g of methyl 2-fluoro-4-(2-oxo-7-azaspiro[3.5]non-7-yl)benzoate, with a yield of 78%. ESI-MS(M+H)⁺= 292.1.

### Synthesis step 3: methyl 2-((1H-pyrrolo[2,3-b]pyrid-5-yl)oxy)-4-(2-oxo-7-azaspiro[3.5]non-7-yl)benzoate (intermediate 55)

Intermediate 55-2 (4.7 g, 16.17 mmol) and 1H-pyrrolo[2,3-b]pyripropan-5-ol (2.17 g, 16.17 mmol) were dissolved in 100 mL of diethylene glycol diethyl ether, and potassium phosphate (6.9 g, 32.34 mmol) was added and sealed for reaction at 130°C for 8 h. TLC showed that the reaction was complete, the oil pump was used for drying, the crude product was purified by column chromatography, and PE was used for pulping, resulting in 1.3 g of the solid product of intermediate 55, with a yield of 20%. ESI-MS(M+H)⁺= 406.2.

### Intermediate 56: methyl 2-((1H-pyrrolo[2,3-b]pyrid-5-yl)oxy)-5-fluoro-4-(2-oxo-7-azaspiro [3.5] non-7-yl)benzoate

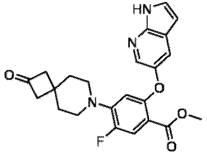

Referring to the synthesis route and method of intermediate 55, methyl 2,4-difluorobenzoate in synthesis step 2 was replaced with methyl 2,4,5-trifluorobenzoate to synthesize intermediate 56.ESI-MS(M+H)⁺= 424.2. ¹H NMR (400 MHz, Chloroform-*d*) δ 10.09 (s, 1H), 8.12 (dd, *J* = 2.6, 0.4 Hz, 1H), 7.65 (d, *J* = 13.5 Hz, 1H), 7.49 (dd, *J* = 2.5, 0.6 Hz, 1H), 7.36 (dd, *J* = 3.5, 2.4 Hz, 1H), 6.45 - 6.38 (m, 2H), 3.79 (s, 3H), 3.09 - 3.00 (m, 4H), 2.79 (s, 4H), 1.87 - 1.78 (m, 4H).

### Intermediate 57: methyl 2-((3-fluoro-1H-pyrrolo[2,3-b]pyrid-5-yl)oxy)-4-(2-oxo-7-azaspiro [3.5] non-7-yl)benzoate

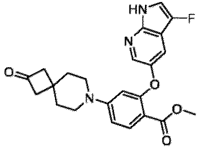

### Synthesis step 1: 5-bromo-3-fluoro-1H-pyrrolo[2,3-b]pyridine (intermediate 57-1)

5-bromo1H-pyrrolo[2,3-b]pyridine (20 g, 101.50 mmol) was dissolved in a solution of 400 mL acetonitrile and 100 mL acetic acid, 1-chloromethyl-4-fluoro-1,4-diazabicyclic[2.2.2]octanedi(tetrafluoroborate) salt (54 g, 152.25 mmol) was added and reacted at 90°C for 16 h. TLC showed that the reaction was complete. The system was concentrated to remove acetonitrile and acetic acid, and the residue was dissolved in EA. Then a saturated sodium bicarbonate aqueous solution was slowly added for neutralization, adjusting the pH of the aqueous phase to 8-9. The EA phase was separated and merged, and dried and concentrated. The resulting crude product underwent column chromatography to obtain 5.2 g of product 5-bromo-3-fluoro-1H-pyrrolo[2,3-b]pyridine, with a yield of 24%. ESI-MS(M+H)⁺=215.0.

### Synthesis step 2: 3-fluoro-5-methoxy-1H-pyrrolo[2,3-b]pyridine (intermediate 57-2)

Intermediate 57-1 (5.2 g, 24.18 mmol) was dissolved in 100 mL of anhydrous DMF, and cuprous bromide (6.9 g, 48.36 mmol) and sodium methoxide (3.9 g, 72.54 mmol) were added at room temperature, and then heated up to 130°C for reaction for 3 h. TLC showed that the reaction was complete, cooled to room temperature, and slowly quenched with ice water. EA (60 mL*3) was added for extraction, and the organic phase was separated and merged, and dried and filtered with anhydrous sodium sulfate. The resulting crude product was purified by column chromatography to obtain 1.4 g of 3-fluoro-5-methoxy-1H-pyrrolo[2,3-b]pyridine, with a yield of 36%. ESI-MS(M+H)⁺=167.1.

### Synthesis step 3: 3-fluoro-1H-pyrrolo[2,3-b]pyripropan-5-ol (intermediate 57-3)

Intermediate 57-2 (1.4 g, 8.43 mmol) was dissolved in 30 mL of DCM, the system was cooled to -20°C, a DCM solution of boron tribromide (6.4 g, 25.29 mmol) was added dropwise slowly, internal temperature was maintained between -20°C and -10°C, and the temperature was continued to be kept at -20°C for 1 h after dropwise addition. After the reaction was completed, the reaction solution was slowly poured into ice water, a saturated sodium bicarbonate aqueous solution was added to neutralize the pH to 7-9, EA was added for extraction, and the organic phase was separated, dried with anhydrous sodium sulfate and filtered. The resulting crude product was purified by column chromatography to obtain 743 mg of 3-fluoro-1H-pyrrolo[2,3-b]pyripropan-5-ol, with a yield of 58%. ESI-MS(M+H)⁺=153.0.

### Synthesis step 4: methyl 2-(3-fluoro-1H-pyrrolo[2,3-b]pyrid-5-yl)oxy)-4-(2-oxo-7-azaspiro[3.5]non-7-yl)benzoate (intermediate 57)

Referring to the synthesis route and method of intermediate 55, 1H-pyrrolo[2,3-b]pyripropan-5-ol in synthesis step 3 was replaced with intermediate 57-3 to synthesize the target intermediate 57. ESI-MS(M+H)⁺= 424.2.

Referring to the synthesis route and method of intermediates 55-57, the following intermediate structures were synthesized:

| Interm ediate number | Intermediate structure | LC-MS (ESI-MS) *m*/*z* [M+H]⁺ |
|---|---|---|
| 58 | 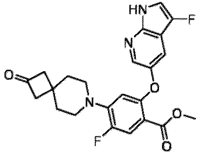 | 442.2 |
| 59 | 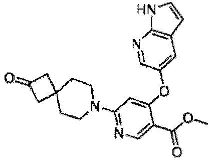 | 407.2 |
| 60 | 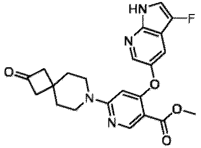 | 425.2 |
| 118 | 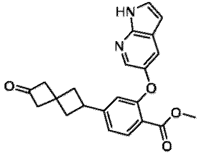 | 377.1 |
| 119 | 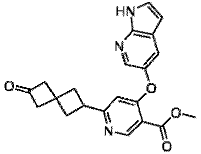 | 378.1 |
| 120 | 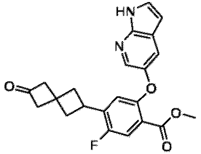 | 395.1 |

### Intermediate 61: methyl 2-(3,4-dihydro-2H-pyrrolo[3',2':5,6]pyridino[2,3-b][1,4]oxazacyclohept-1(7H)-yl)-4-(2-oxo-7-azaspiro[3.5]non-7-yl)benzoate

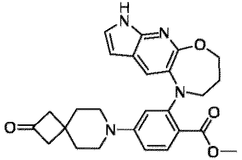

### Synthesis step 1: 5-bromo-6-fluoro-1-((2-(trimethylsilyl)ethoxy)methyl)-1H-pyrrolo[2,3-b]pyridine (intermediate 61-1)

5-bromo-6-fluoro-1H-pyrrolo[2,3-b]pyridine (10 g, 46.50 mmol) was dissolved in 200 mL of DMF, 60% NaH (846.4 mg, 51.16 mmol) was added and stirred at room temperature for 30 min, then SEMCl (10.08 g, 60.45 mmol) was added, and the reaction was continued at room temperature for 3 h. After the reaction was completed, water and EA were added for extraction, and the EA phase was separated, dried and filtered, and concentrated under reduced pressure. The resulting crude product was purified by column chromatography to obtain 13 g of 5-bromo-6-fluoro-1-((2-(trimethylsilyl)ethoxy)methyl)-1H-pyrrolo[2,3-b]pyridine, with a yield of 80%. ESI-MS(M+H)⁺= 345.0.

### Synthesis step 2: N-(3-((5-bromo-1-((2-(trimethylsilyl)ethoxy)methyl)-1H-pyrrolo[2,3-b]pyrid-6-yl)oxy)propyl)-4-methylbenzenesulfonamide (intermediate 61-2)

N-(3-hydroxypropyl)-4-methylbenzenesulfonamide (10.62 g, 46.32 mmol) was dissolved in 400 mL of THF and cooled to 0°C, 60% NaH (2.3 g, 57.90 mmol) was added in batches and reacted for 1 h, and then intermediate 61-1 (8 g, 23.16 mmol) was added and reacted overnight at room temperature. After the reaction was completed, an ammonium chloride saturated solution was added to quench the reaction. EA and water were added for extraction, and the EA phase was separated, dried and filtered, and concentrated under reduced pressure. The resulting crude product was purified by column chromatography to obtain 5.8 g of product N-(3-((5-bromo-1-((2-(trimethylsilyl)ethoxy)methyl)-1H-pyrrolo[2,3 -b]pyrid-6-yl)oxy)propyl)-4-methylbenzenesulfonamide, with a yield of 45%. ESI-MS(M+H)⁺=554.1.

### Synthesis step 3: 1-tosyl-7-((2-(trimethylsilyl)ethoxy)methyl)-1,3,4,7-tetrahydro-2H-pyrrolo[3',2':5,6]pyridino[2,3-b][1,4] oxazacycloheptane (intermediate 61-3)

Intermediate 61-2 (5.04 g, 9.1 mmol) was dissolved in 100 mL of DMSO, 2-pyridinecarboxylic acid (900 mg, 7.3 mmol), cuprous iodide (2.1 g, 10.92 mmol) and potassium carbonate (3.8 g, 27.3 mmol) were added sequentially and reacted overnight at 140°C. After the reaction was completed, EA and water were added for extraction. The EA phase was separated, dried with anhydrous sodium sulfate, filtered, and concentrated under reduced pressure. The resulting crude product was purified by column chromatography to obtain 2.1 g of product 1-tosyl-7-((2-(trimethylsilyl)ethoxy)methyl)-1,3,4,7-tetrahydro-2H-pyrrolo[3',2':5,6]pyridino[2,3-b][1,4] oxazacycloheptane, with a yield of 48.6%. ESI-MS(M+H)⁺= 474.2.

### Synthesis step 4: 7-((2-(trimethylsilyl)ethoxy)methyl)-1,3,4,7-tetrahydro-2H-pyrrolo[3',2':5,6]pyridino[2,3-b][1,4] oxazacycloheptane(intermediate 61-4)

Intermediate 61-3 (1.9 g, 4 mmol) was dissolved in 100 mL of anhydrous methanol, magnesium powder (2.9 g, 120 mmol) was added, and after reflux for 1 h, and a saturated ammonium chloride aqueous solution was added to quench the reaction. EA and water were added for extraction, and the EA phase was separated, dried with anhydrous sodium sulfate, filtered, and concentrated under reduced pressure. The resulting crude product obtained was separated and purified by column chromatography to obtain 1.1 g of product 7-((2-(trimethylsilyl)ethoxy)methyl)-1,3,4,7-tetrahydro-2H-pyrrolo[3',2':5,6]pyridino[2,3-b][1,4] oxazacycloheptane, with a yield of 86%. ESI-MS(M+H)⁺= 320.2.

Synthesis step 5: methyl 4-(2-oxo-7-azaspiro[3.5]non-7-yl)-2-(7-((2-(trimethylsilyl)ethoxy)methyl)-3,4-dihydro-2H-pyrrolo[3',2':5,6]pyridino[2,3-b][1,4]oxazacyclohept-1(7H)-yl)benzoate (intermediate 61-5)

Intermediate 55-2 (669 mg, 2.3 mmol) was dissolved in 70 mL of toluene, intermediate 61-4 (500 mg, 1.57 mmol), cesium carbonate (1.53 g, 4.7 mmol), XantPhos (700 mg, 0.80 mmol), and Pd(dba)₂ (144 mg, 0.16 mmol) were added sequentially, and the system was filled with nitrogen for protection and reacted overnight at 110°C. After the reaction was completed, the system was cooled to room temperature, and EA and water were added for extraction. The EA phase was separated, dried with anhydrous sodium sulfate, and filtered and concentrated. The resulting crude product was separated and purified by column chromatography to obtain 471 mg of methyl 4-(2-oxo-7-azaspiro[3.5]non-7-yl)-2-(7-((2-(trimethylsilyl)ethoxy)methyl)-3,4-dihydro-2H-pyrrolo[3',2':5,6]pyridino[2,3-b][1,4]oxazacyclohept-1(7H)-yl)benzoate, with a yield of 51%. ESI-MS(M+H)⁺= 591.3.

### Synthesis step 6: methyl 2-(3,4-dihydro-2H-pyrrolo[3',2':5,6]pyridino[2,3-b][1,4]oxazacyclohept-1(7H)-yl)-4-(2-oxo-7-azaspiro[3.5]non-7-yl)benzoate (intermediate 61)

Intermediate 61-5 (471 mg, 0.80 mmol) was dissolved in 20 mL of THF, 30 mL of TBAF solution (1 mol/L THF solution) was added, and after heating and refluxing overnight, the system was cooled to room temperature. EA and water were added for extraction, and the EA phase was separated, dried with anhydrous sodium sulfate, and filtered and concentrated. The resulting crude product was purified by column chromatography to obtain 326 mg of the solid product of intermediate 61, with a yield of 89%. ESI-MS(M+H)⁺= 461.2.

Referring to the synthesis route and method of intermediate 61, the following intermediate structures were synthesized:

| Interm ediate number | Intermediate structure | LC-MS (ESI-MS) *m*/*z* [M+H]⁺ |
|---|---|---|
| 62 | 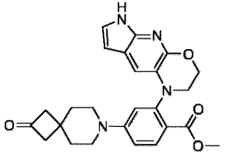 | 447.2 |
| 63 | 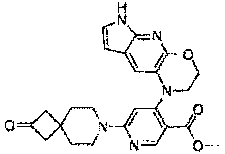 | 448.2 |
| 121 | 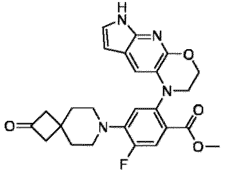 | 465.2 |

### Intermediate 64: 4-(((4-fluorotetrahydro-2H-pyran-4-yl)methyl)amino)-3-nitrobenzenesulfonamide

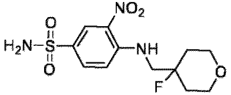

(4-fluorotetrahydro-2H-pyran-4-yl)methylamine (0.5 g, 5 mmol) was dissplved in 15 mL of THF, and 4-fluoro-3-nitrobenzenesulfonamide and triethylamine (4 g, 40 mmol) were added (1.1 g, 5 mmol) and reacted at room temperature for 8 hours. TLC showed that the reaction was complete. The reaction solution was concentrated, DCM/H₂O was added for extraction, and the organic phase was separated, dried with anhydrous sodium sulfate, and filtered and concentrated. The resulting crude product was separated and purified by column chromatography to obtain 1.23 g of the solid product of intermediate 64, with a yield of 74%.ESI-MS(M+H)⁺= 334.1.

### Intermediate 65: 4-((((1r, 4r)-4-hydroxy-4-methylcyclohexyl)methyl)amino)-3-nitrobenzenesulfonamide

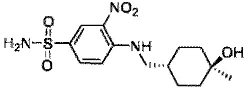

Referring to the synthesis route and method of intermediate 64, (4-fluorotetrahydro-2H-pyran-4-yl)methylamine was replaced with (1r, 4r)-4-(aminomethyl)-1-methylcyclohexan-1-ol to synthesize intermediate 65.ESI-MS(M+H)⁺= 344.1.

### Intermediate 66: 4-((4-fluoro-1-(oxocycl-3-yl)piperid-4-yl)methyl)amino)-3-nitrobenzenesulfonamide

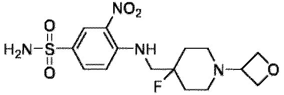

### Synthesis step 1: tert-butyl 4-fluoro-4-(((2-nitro-4-aminosulfonylphenyl)amino)methyl)piperidine-1-carboxylate (intermediate 66-1)

Referring to the synthesis route and method of intermediate 64, (4-fluorotetrahydro-2H-pyran-4-yl)methylamine was replaced with tert-butyl 4-(aminomethyl)-4-fluoropiperidine-1-carboxylate to synthesize tert-butyl 4-fluoro-4-(((2-nitro-4-aminosulfonylphenyl)amino)methyl)piperidine-1-carboxylate. ESI-MS(M+H)⁺=433.1.

### Synthesis step 2: 4-((4-fluoro-1-(oxocycl-3-yl)piperid-4-yl)methyl)amino)-3-nitrobenzenesulfonamide (intermediate 66)

Intermediate 66-1 (2 g, 4.62 mmol) was dissolved in 30 mL of DCM, and 15 mL of TFA was added and reacted at room temperature for 4 h. TLC showed that the reaction was complete. The reaction solution was directly spin dried to obtain 1.96 g of yellow viscous liquid. The obtained yellow liquid product was dissolved in 20 mL of DCM, 3-oxocyclobutanone (475 mg, 6.58 mmol) was added and stirred at room temperature for 30 min, STAB (1.39 g, 6.58 mmol) was added, and the reaction was continued at room temperature for 2 h. TLC showed that the reaction was complete. DCM and water were added for extraction, and the organic phase was separated, dried and filtered, and concentrated under reduced pressure. The resulting crude product was purified by column chromatography to obtain 1.61 g of the yellow solid product of intermediate 66, with a yield of 63%. ESI-MS(M+H)⁺=389.1.

Referring to the synthesis route and method of intermediates 64-66, the following intermediate structures were synthesized:

| Interm ediate number | Intermediate structure | LC-MS (ESI-MS) *m*/*z* [M+H]⁺ |
|---|---|---|
| 67 | 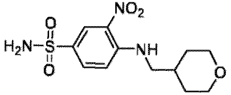 | 316.1 |
| 68 | 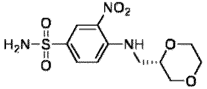 | 318.1 |
| 69 | 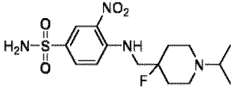 | 375.1 |

### Intermediate 70: 6-nitro-7-(((tetrahydro-2H-pyran-4-yl)methyl)amino)-1H-benzo [d] imidazole-4-sulfonamide

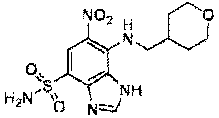

### Synthesis step 1: 4,7-dibromo-1H-benzo[d]imidazole (intermediate 70-1)

3,6-dibromobenzene-1,2-diamine (4.5 g, 16.92 mmol) was dissolved in 100 mL of THF, trimethyl formate (2.70 g, 25.38 mmol) was added and stirred for 5 min, then p-toluenesulfonic acid (291 mg, 1.69 mmol) was added, and the system was placed at room temperature and reacted for 1 h. TLC showed that the reaction was complete, suction filtration was performed, and the solid was washed with the mother liquor and then sent to a 60°C oven for drying to obtain 4.3 g of yellow solid product 4,7-dibromo-1H-benzo[d]imidazole, with a yield of 93%. ESI-MS(M+H)⁺= 274.9.

### Synthesis step 2: 4,7-dibromo-6-nitro-1H-benzo[d]imidazole (intermediate 70-2)

At 0°C, the intermediate 70-1 (4.30 g, 15.58 mmol) was dissolved in 45 mL of concentrated sulfuric acid and stirred for 5 min, and HNO₃ (830 mL, 18.70 mmol) was added dropwise slowly. The system temperature was controlled between 0 and 10°C, and stirring was continued at 0°C for 1 h after dropwise addition. After the reaction was completed, the reaction solution was slowly poured into ice ammonia water for quenching. After quenching, the pH was around 8, suction filtration was performed, and the solid was washed with water and then sent to a 60°C oven for drying to obtain 4 g of yellow solid product of 4,7-dibromo-6-nitro-1H-benzo[d]imidazole, with a yield of 80%. ESI-MS(M+H)⁺= 319.9.

### Synthesis step 3: 4-bromo-6-nitro-N-((tetrahydro-2H-pyran-4-yl)methyl)-1H-benzo[d]imidazole-7-amine (intermediate 70-3)

The intermediate 70-2 (3 g, 9.35 mmol) was dissolved in 20 mL of dioxane, then (tetrahydro-2H-pyran-4-yl)methylamine (1.62 g, 14.03 mmol) and K₂CO₃ (3.9 g, 28.05 mmol) were added sequentially and sealed for reaction at 120°C for 48 h. TLC showed that the reaction was complete, the system was concentrated to remove dioxane, water and EA were added for extraction, and the organic phase was separated, dried with anhydrous sodium sulfate, and filtered and concentrated. The resulting crude product was purified by column chromatography to obtain 2.9 g of yellow solid product 4-bromo-6-nitro-N-(tetrahydro-2H-pyran-4-yl)methyl)-1H-benzo[d]imidazole-7-amine, with a yield of 88%.ESI-MS(M+H)⁺=355.0.

### Synthesis step 4: 4-(benzylthio)-6-nitro-N-((tetrahydro-2H-pyran-4-yl)methyl)-1H-benzo[d]imidazole-7-amine (intermediate 70-4)

The intermediate 70-3 (2.9 g, 8.23 mmol) was dissolved in 30 mL of toluene, benzyl mercaptan (2.04 g, 16.46 mmol), Xantphos (952 mg, 1.65 mmol), Pd₂(dba)₃ (754 mg, 0.823 mmol), and DIPEA (3.2 g, 24.69 mmol) were added sequentially and sealed for reaction at 110°C for 16 h. LCMS showed that the reaction was complete, water and EA were added for extraction, and the organic phase was separated, dried with anhydrous sodium sulfate, and filtered and concentrated. The resultig crude product was purified by column chromatography to obtain 1.26 g of yellow solid product 4-(benzylthio)-6-nitro-N-((tetrahydro-2H-pyran-4-yl)methyl)-1H-benzo[d]imidazole-7-amine, with a yield of 38.5%.ESI-MS(M+H)⁺= 399.1.

### Synthesis step 5: 6-nitro-7-(((tetrahydro-2H-pyran-4-yl)methyl)amino)-1H-benzo[d]imidazole-4-sulfonamide (intermediate 70)

Intermediates 70-4 (1.26 g, 3.17 mmol), MeCN (14 mL), H₂O (1.4 mL), and AcOH (0.7 mL) were added to a single necked bottle sequentially. The reaction system was lowered to 0°C, NCS (1.27 g, 9.51 mmol) was added in batches, stirred at 0°C for 1 h, and then transferred to room temperature for further reaction for 1 h. Then the reaction solution was added dropwise to an ice 25% ammonia solution (22.5 ml, 158.45 mmol), and stirring was continued at room temperature for 1 h. After the reaction was complete, water was added for dilution, and the aqueous phase was extracted with an ethyl acetate solution of 10% ethanol. The organic phase was separated, dried with anhydrous sodium sulfate, and filtered and concentrated. The resulting crude product was purified by column chromatography to obtain 338 mg of the yellow solid product of intermediate 70, with a yield of 30%.ESI-MS(M+H)⁺=356.1.

### Intermediate 71: 7-(((4-fluoro-1-(oxocyclobut-3-yl)piperid-4-yl)methyl)amino)-6-nitro-1H-benzo[d]imidazole-4-sulfonamide

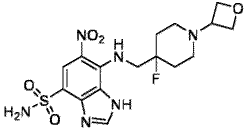

### Synthesis step 1: tert-butyl 4-fluoro-4-(((6-nitro-4-aminosuₗfonyl-1H-benzo[d]imidazol-7-yl)amino)methyl)piperidine-1-carboxylate (intermediate 71-1)

Referring to the synthesis route and method of intermediate 70, the (tetrahydro-2H-pyran-4-yl)methylamine in synthesis step 3 was replaced with tert-butyl 4-(aminomethyl)-4-fluoropiperidine-1-carboxylate to synthesize tert-butyl 4-fluoro-4-(((6-nitro-4-aminosulfonyl-1H-benzo[d]imidazol-7-yl)amino)methyl)piperidine-1-carboxylate. ESI-MS(M+H)⁺=473.2.

### Synthesis step 2: 7-(((4-fluoro-1-(oxocyclobut-3-yl)piperid-4-yl)methyl)amino)-6-nitro-1H-benzo[d]imidazole-4-sulfonamide (intermediate 71)

Intermediate 71-1 (190 mg, 0.40 mmol) was dissolved in 10 mL of DCM, and 2 mL of trifluoroacetic acid was added and reacted at room temperature for 6 h. TLC showed that the reaction was complete. The reaction solution was concentrated to obtain a yellow oily substance. The above product was dissolved in 10 mL of DCM, 3-oxocyclobutanone (44 mg, 0.60 mmol) was added and stirred at room temperature for 30 min, then STAB (128 mg, 0.60 mmol) was added, and the reaction was continued at room temperature for 2 h. TLC showed that the reaction was complete. DCM and water were added for extraction, and the organic phase was separated, dried with anhydrous sodium sulfate, and filtered and concentrated. The resulting crude product was purified by column chromatography to obtain 145 mg of the yellow solid product of intermediate 71, with a yield of 85%.ESI-MS(M+H)⁺= 429.1.

Referring to the synthesis route and method of intermediates 70-71, the following intermediate structures were synthesized:

| Interm ediate number | Intermediate structure | LC-MS (ESI-MS) *m*/*z* [M+H]⁺ |
|---|---|---|
| 72 | 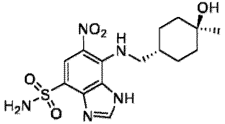 | 384.1 |
| 73 | 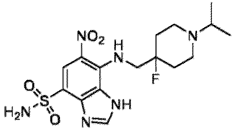 | 415.1 |
| 74 | 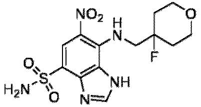 | 374.1 |
| 75 | 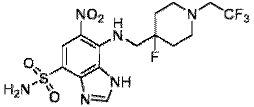 | 455.1 |
| 76 | 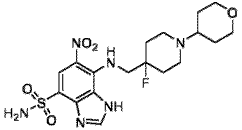 | 457.2 |
| 77 | 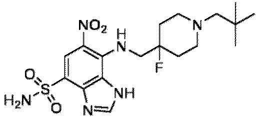 | 443.2 |
| 78 | 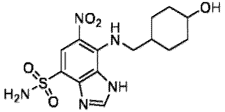 | 370.1 |
| 79 | 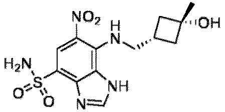 | 356.1 |
| 80 | 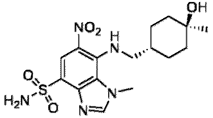 | 398.1 |
| 81 | 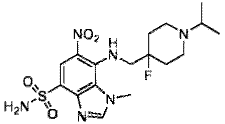 | 429.2 |
| 82 | 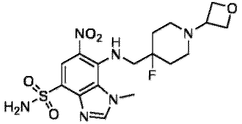 | 443.1 |
| 83 | 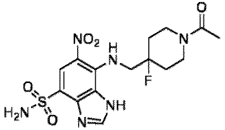 | 415.1 |
| 84 | 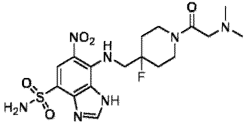 | 458.2 |
| 85 | 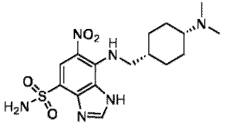 | 397.2 |

### Intermediate 86: 6-nitro-7-(((tetrahydro-2H-pyran-4-yl)methyl)amino)benzo[d][1,3]dioxolane-4-sulfonamide

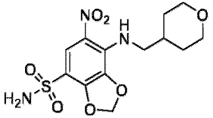

### Synthesis step 1: 2-bromo-6-methoxy-3-nitrophenol (intermediate 86-1)

2-methoxy-5-nitrophenol (100g, 591.2 mmol) was dissolved in 500 mL of acetic acid, 30 mL of Br₂ was added dropwise at room temperature and reacted at room temperature for 14 h. After the reaction was completed, saturated sodium sulfite was added to quench the reaction. The solid was precipitated with water and filtered, the filter cake was rinsed with water, and drying under reduced pressure was performed at 50°C to obtain 99 g of crude brown solid product 2-bromo-6-methoxy-3-nitrophenol, with a yield of 67.51%.ESI-MS(M+H)⁺=247.9.

### Synthesis step 2: 3-bromo-4-nitrobenzene-1,2-diol (intermediate 86-2)

Intermediate 86-1 (99 g, 399.15 mmol) was dissolved in 1000 mL of dichloromethane, an anhydrous AlCl₃ solid (243 g, 1.82 mol) was added and heated up to 40°C for reflux reaction for 13 h. After the reaction was completed, the reaction solution was poured into ice water, ethyl acetate was added for extraction, and the organic phase was separated and concentrated. The resulting crude product was separated by column chromatography to obtain 82 g of product 3-bromo-4-nitrobenzene-1,2-diol, with a yield of 87.8%.ESI-MS(M+H)⁺=233.9.

### Synthesis step 3: 4-bromo-5-nitrobenzo[d][1,3]dioxolane (intermediate 86-3)

Intermediate 86-2 (10 g, 42.74 mmol) was dissolved in 100 mL DMAC, and bromochloromethane (3.32 mL, 51.20 mmol) and cesium carbonate (27.86 g, 85.48 mmol) were added and reacted at 110°C for 16 h. After the reaction was completed, the reaction solution was cooled to room temperature, and ethyl acetate (500ml*3) and water were added for extraction. The organic phase was separated and merged, and dried and concentrated. The resulting crude product was purified by column chromatography to obtain 7.3 g of4-bromo-5-nitrobenzo[d][1,3]dioxolane, with a yield of 69.4%.ESI-MS(M+H)⁺=245.9.

### Synthesis step 4: 5-nitro-N-((tetrahydro-2H-pyran-4-yl)methyl)benzo[d][1,3]dioxolane-4-amine (intermediate 86-4)

Intermediate 86-3 (2.5 g, 10.2 mmol) was dissolved in 35 mL of tetrahydrofuran, (tetrahydro-2H-pyran-4-yl)methylamine (3.5 g, 30.6 mmol), BINAP (635 mg, 1.02 mmol), Pd₂(dba)₃ (934 mg, 1.02 mmol), and cesium carbonate (6.6 g, 20.4 mmol) were added sequentially for reflux reaction at room temperature for 16 h. After the reaction was completed, the reaction solution was passed through diatomaceous earth, concentrated in tetrahydrofuran, and extracted with ethyl acetate and water. The organic phase was separated, and dried and concentrated. The resulting crude product was purified by column chromatography to obtain 650 mg of product 5-nitro-N-(tetrahydro-2H-pyran-4-yl)methyl)benzo[d][1,3]dioxolane-4-amine, with a yield of 22.7%.ESI-MS(M+H)⁺=281.1.

### Synthesis step 5: 7-bromo-5-nitro-N-((tetrahydro-2H-pyran-4-yl)methyl)benzo[d][1,3]dioxolane-4-amine (intermediate 86-5)

Intermediate 86-4 (650 mg, 2.32 mmol) was dissolved in 7 mL of anhydrous DMF, NBS (413 mg, 2.32 mmol) was slowly added and reacted at room temperature for 4 h. After the reaction was completed, ethyl acetate (20 mL*3) and water were added for extraction. The organic phase was separated and merged, dried with anhydrous sodium sulfate, and filtered and concentrated. The resulting crude product was purified by column chromatography to obtain 300 mg of product 7-bromo-5-nitro-N-((tetrahydro-2H-pyran-4-yl)methyl)benzo[d][1,3]dioxolane-4-amine, with a yield of 36%.ESI-MS(M+H)⁺=359.0.

### Synthesis step 6: 6-nitro-7-(((tetrahydro-2H-pyran-4-yl)methyl)amino)benzo[d][1,3]dioxolane-4-sulfonamide (intermediate 86)

Referring to the synthesis route and method of synthesis steps 4 and 5 in the synthesis of intermediate 70, intermediate 70-3 in synthesis step 4 was replaced with intermediate 86-5 to synthesize 80 mg of yellow solid of intermediate 86.ESI-MS(M+H)⁺=360.1.

### Intermediate 87: 7-((((1r, 4r)-4-hydroxy-4-methylcyclohexyl)methyl)amino)-6-nitrobenzo[d][1,3]dioxolane-4-sulfonamide

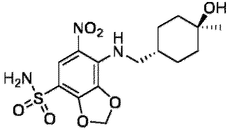

Referring to the synthesis route and method of intermediate 86, (tetrahydro-2H-pyran-4-yl)methylamine in synthesis step 4 was replaced with (1r, 4r)-4-(aminomethyl)-1-methylcyclohexan-1-ol to synthesize intermediate 87.ESI-MS(M+H)⁺= 388.1.

### Intermediate 88: 5,8-dibromo-6-nitro-2,3-dihydrobenzo[b][1,4]dioxin

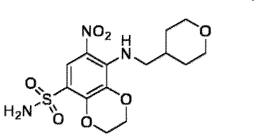

Referring to the synthesis route and method of intermediate 86, bromochloromethane in synthesis step 3 was replaced with 1-bromo-2-chloroethane to synthesize intermediate 88. ESI-MS(M+H)⁺=374.1.

Referring to the synthesis route and method of intermediates 86-88, the following intermediate structures were synthesized:

| Interm ediate number | Intermediate structure | LC-MS (ESI-MS) *m*/*z* [M+H]⁺ |
|---|---|---|
| 89 | 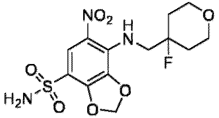 | 378.1 |
| 90 | 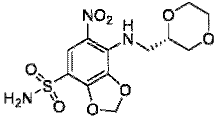 | 362.1 |
| 91 | 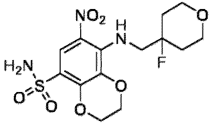 | 392.1 |
| 92 | 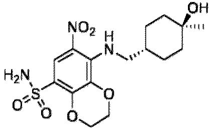 | 402.1 |
| 93 | 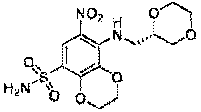 | 376.1 |
| 94 | 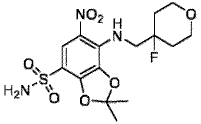 | 406.1 |
| 95 | 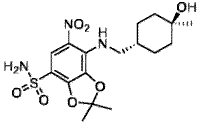 | 416.1 |
| 96 | 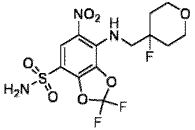 | 414.1 |
| 97 | 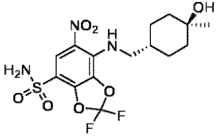 | 424.1 |

### Intermediate 98: 7-(((4-fluorotetrahydro-2H-pyran-4-yl)methyl)amino)-6-nitrobenzo[d]oxazole-4-sulfonamide

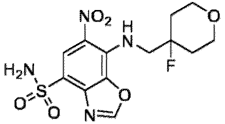

### Synthesis step 1: 7-bromobenzo[d]oxazole-4-amine (intermediate 98-1)

Benzo[*d*]oxazole-4-amine (2 g, 14.92 mmol) was dissolved in 20 mL of DMF and cooled to 0°C, and NBS (2.65 g, 14.92 mmol) was added for selective bromination. The reaction was completed after 1 hour, water and ethyl acetate were added for extraction, and the organic phase was separated, dried with anhydrous sodium sulfate, and filtered and concentrated. The resulting crude product was purified by column chromatography to obtain 2.2 g of product 7-bromobenzo[d]oxazole-4-amine, with a yield of 71%.ESI-MS(M+H)⁺= 213.0.

### Synthesis step 2: 7-bromobenzo[d]oxazole-4-sulfonyl chloride (intermediate 98-2)

Intermediate 98-1 (1.1 g, 5.29 mmol) was suspended in 11 mL of a 6 N sulfuric acid solution, the system was cooled to 0°C, sodium nitrite (474 mg, 6.88 mmol) was added, the suspended substance was gradually dissolved and reacted at 0°C for 1 h, then 20 mL of a saturated sulfur dioxide acetic acid solution was added, and then the system was transferred to room temperature for reaction for 2 h. After the reaction was completed, the reaction solution was poured into ice water, and ethyl acetate was add for stirring and extraction at the same time. The organic phase was separated, dried with anhydrous sodium sulfate, and filtered and concentrated. The resulting crude product was purified by column chromatography to obtain 639 mg of product 7-bromobenzo[d]oxazole-4-sulfonyl chloride, with a yield of 41%.ESI-MS(M+H)⁺= 295.9.

### Synthesis step 3: 7-bromo-6-nitrobenzo[d]oxazole-4-sulfonamide (intermediate 98-3)

Intermediate 98-2 (639 mg, 2.16 mmol) was added to 10 mL of concentrated sulfuric acid at 0°C and stirred, and then 0.2 mL of 68% concentrated nitric acid was added, the temperature was elevated to 80°C for reaction for 1 h. Afterwards, the reaction solution was poured into 50 mL of ice ammonia solution and stirring was continued for 1 h. The reaction was completed, water was added for dilution, and ethyl acetate (100 mL*2) was added for extraction. The organic phase was separated and merged, and dried and concentrated. The resulting crude product was purified by column chromatography to obtain 332 mg of product 7-bromo-6-nitrobenzo[d]oxazole-4-sulfonamide, with a yield of 48%.ESI-MS(M+H)⁺= 321.9.

### Synthesis step 4: 7-(((4-fluorotetrahydro-2H-pyran-4-yl)methyl)amino)-6-nitrobenzo[d]oxazole-4-sulfonamide (intermediate 98)

Intermediate 98-3 (332 mg, 1.03 mmol) was dissolved in 10 mL of 1,4-dioxane solution, and potassium carbonate (285 mg, 2.06 mmol) and (4-fluorotetrahydro-2H-pyran-4-yl)methylamine (179 mg, 1.34 mmol) were added sequentially and sealed for reaction at 120°C for 9 h. After the reaction was completed, the system was cooled to room temperature, and ethyl acetate (30 mL*2) and water were added for extraction. The organic phase was separated and merged, and dried and concentrated. The resulting crude product was separated and purified by column chromatography to obtain 300 mg of the solid product of intermediate 98, with a yield of 78%.ESI-MS(M+H)⁺= 375.1.

### Intermediate 99: 7-(((4-fluorotetrahydro-2H-pyran-4-yl)methyl)amino)-6-nitrobenzo[d]thiazole-4-sulfonamide

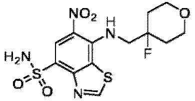

Referring to the synthesis route and method of intermediate 98, benzo[d]oxazole-4-amine in synthesis step 1 was replaced with benzo[d]thiazole-4-amine to synthesize intermediate 99. ESI-MS(M+H)⁺=391.0.

### Intermediate 100: 7-(((4-fluorotetrahydro-2H-pyran-4-yl)methyl)amino)-6-nitrobenzofuran-4-sulfonamide

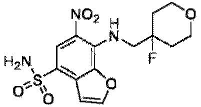

Referring to the synthesis route and method of intermediate 98, benzo[*d*]oxazole-4-amine in synthesis step 1 was replaced with benzofuran-4-amine to synthesize intermediate 100. ESI-MS(M+H)⁺=374.1.

### Intermediate 101: (S)-7-(((1,4-dioxan-2-yl)methyl)amino)-6-nitrobenzo[d]oxazole-4-sulfonamide

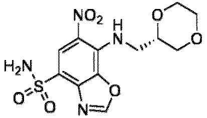

Referring to the synthesis route and method of intermediate 98, (4-fluorotetrahydro-2H-pyran-4-yl)methylamine in synthesis step 4 was replaced with (S)-(1,4-dioxan-2-yl)methylamine to synthesize intermediate 101. ESI-MS(M+H)⁺=359.1.

Referring to the synthesis route and method of intermediates 98-101, the following intermediate structures were synthesized:

| Interm ediate number | Intermediate structure | LC-MS (ESI-MS) *m*/*z* [M+H]⁺ |
|---|---|---|
| 102 | 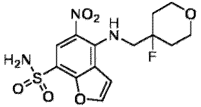 | 374.1 |
| 103 | 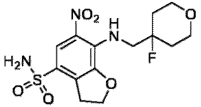 | 376.1 |
| 104 | 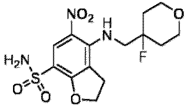 | 376.1 |
| 105 | 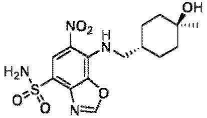 | 385.1 |
| 106 | 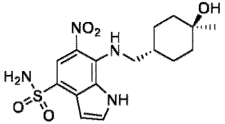 | 383.1 |
| 107 | 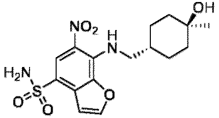 | 384.1 |
| 108 | 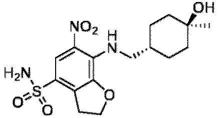 | 386.1 |
| 109 | 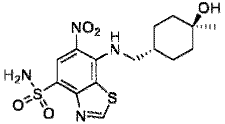 | 401.1 |
| 110 | 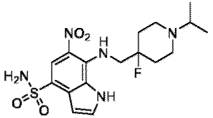 | 414.2 |
| 111 | 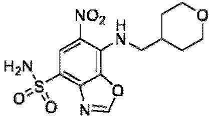 | 357.1 |
| 112 | 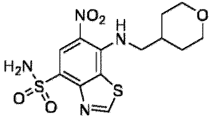 | 373.1 |
| 113 | 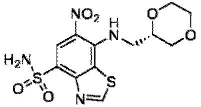 | 375.0 |
| 114 | 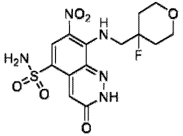 | 402.1 |
| 115 | 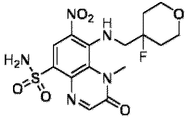 | 416.1 |
| 116 | 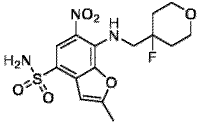 | 388.1 |
| 117 | 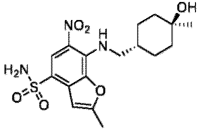 | 398.1 |

### Embodiment 1: 2-((1H-pyrrolo[2,3-b]pyrid-5-yl)oxy)-N-((7-((((1r,4r)-4-hydroxy-4-methylcyclohexyl)methyl)amino)-6-nitro-1H-benzo[d]imidazol-4-yl)sulfonyl)-4-(2((S)-2-(2-isopropylphenyl)pyrrolidin-1-yl)-7-azaspiro[3.5]non-7-yl)benzamide (001)

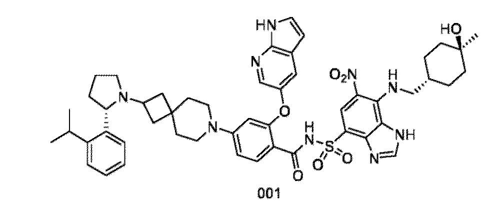

### Synthesis step 1: methyl ((S)-2-((1H-pyrrolo[2,3-b]pyrid-5-yl)oxy)-4-(2-(2-(2-isopropylphenyl)pyrrolidin-1-yl)-7-azaspiro[3.5]non-7-yl)benzoate (001-1)

Intermediate 55 (772 mg, 1.902 mmol) and intermediate 1 (300 mg, 1.585 mmol) were dissolved in 30 mL of DCM, stirred and slowly added wth STAB (1007 mg, 4.755 mmol), and reacted at room temperature for 3 h. LCMS showed that the reaction was complete, water was added for quenching, and DCM and water were added for exaction. The organic phase was separated and merged, and concentrated under reduced pressure. The resulting crude product was purified by column chromatography to obtain 587 mg of product methyl (S)-2-((1H-pyrrolo[2,3-b]pyrid-5-yl)oxy)-4-(2-(2-(2-isopropylphenyl)pyrrolidin-1-yl)-7-azaspiro[3.5]non-7-yl)benzoate, with a yield of 64%. ESI-MS(M+H)⁺=579.3.

### Synthesis step 2: (S)-2-((1H-pyrrolo[2,3-b]pyrid-5-yl)oxy)-4-(2-(2-(2-isopropylphenyl)pyrrolidin-1-yl)-7-azaspiro[3.5]non-7-yl)benzoic acid (001-2)

Compound 001-1 (587 mg, 1.014 mmol) was dissolved in 6 mL of THF, and 6 mL of 3 N NaOH and 6 mL of MeOH were added sequentially and reacted at room temperature for 2 h. LCMS showed that the reaction was complete, 2 N HCl was added to adjust the pH to 6-7, the system was concentrated to remove methanol and tetrahydrofuran, 2-methyltetrahydrofuran and water were added for extraction. The organic phase was separated and merged, and dried and concentrated. The resulting crude product was beaten with MTBE, filtered, and dried at 60°C for 3 h to obtain 418 mg of product (S)-2-((1H-pyrrolo[2,3-b]pyrid-5-yl)oxy)-4-(2-(2-(2-isopropylphenyl)pyrrolidin-1-yl)-7-azaspiro[3.5]non-7-yl)benzoic acid, with a yield of 73%. ESI-MS(M+H)⁺=565.3.

### Synthesis step 3: 2-((1H-pyrrolo[2,3-b]pyrid-5-yl)oxy)-N-((7-((((1r,4r)-4-hydroxy-4-methylcyclohexyl)methyl)amino)-6-nitro-1H-benzo[d]imidazol-4-yl)sulfonyl)-4-(2-((S)-2-(2-isopropylphenyl)pyrrolidin-1-yl)-7-azaspiro[3.5]non-7-yl)benzamide (001)

Intermediate 72 (228 mg, 0.595 mmol) was added to reaction flask 1, 15 mL of DCM was added to form a suspension, and then DMAP (145 mg, 1.19 mmol) and EDCI (171 mg, 0.893 mmol) were added and stirred at room temperature for 5 min. Compound 001-2 (336 mg, 0.595 mmol) was added to reaction flask 2, 15 mL of DCM was added, and then triethylamine (151 mg, 1.49 mmol) was added and stirred at room temperature for 5 min. The reaction solution from reaction flask 2 was slowly added to reaction flask 1, lasting for 1 hour. After the addition was complete, the reaction was continued at room temperature for 14 h. After the reaction was completed, water and DCM were added for extraction, and the DCM phase was separated and concentrated. The resulting crude product was purified by column chromatography to obtain 385 mg of the yellow solid product of compound 001, with a yield of 56%.ESI-MS(M+H)⁺=930.4.

### Embodiment 2: (S)-2-((1H-pyrrolo[2,3-b]pyrid-5-yl)oxy)-N-((7-((((4-fluoro-1-(oxocyclobut-3-yl)piperid-4-yl)methyl)amino)-6-nitro-1H-benzo[d]imidazol-4-yl)sulfonyl)-4-(2-(2-(2-isopropylphenyl)pyrrolidin-1-yl)-7-azaspiro[3.5]non-7-yl)benzamide (002)

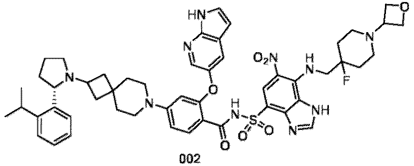

Referring to the synthesis route and method of embodiment 1, intermediate 72 in synthesis step 3 was replaced with intermediate 71 to synthesize the target compound 002. ESI-MS(M+H)⁺=975.4.¹H NMR (400 MHz, Chloroform-*d* δ 11.26 (s, 1H), 9.53 (t, *J* = 6.2 Hz, 1H), 9.26 (s, 1H), 8.75 (s, 1H), 8.13 (d, *J* = 2.6 Hz, 1H), 7.97 (s, 1H), 7.84 (d, *J* = 9.2 Hz, 1H), 7.66 (d, *J* = 2.5 Hz, 1H), 7.47 - 7.41 (m, 1H), 7.20 (s, 3H), 6.54 - 6.46 (m, 2H), 5.92 (d, *J* = 2.2 Hz, 1H), 4.80 (d, *J* = 6.3 Hz, 1H), 4.75 (d, *J* = 6.3 Hz, 1H), 4.66 (t, *J* = 6.5 Hz, 2H), 4.58 (t, *J* = 6.2 Hz, 2H), 3.50 - 3.48 (m, 1H), 3.21 (s, 2H), 2.95 - 2.82 (m, 5H), 2.58 - 2.47 (m, 3H), 2.19 - 2.10 (m, 4H), 2.04 - 1.89 (m, 4H), 1.52 - 1.36 (m, 6H), 1.24 - 1.21 (m, 5H), 1.19 - 1.08 (m, 6H).

### Embodiment 3: 2-(3,4-dihydro-2H-pyrrolo[3',2':5,6]pyridino[2,3-b][1,4]oxazacyclohept-1(7H)-yl)-N-((7-((((1r,4r)-4-hydroxy-4-methylcyclohexyl)methyl)amino)-6-nitro-1H-benzo[d]imidazol-4-yl)sulfonyl)-4-(2-((S)-2-(2-isopropylphenyl)pyrrolidin-1-yl)-7-azaspiro[3.5]non-7-yl)benzamide (085)

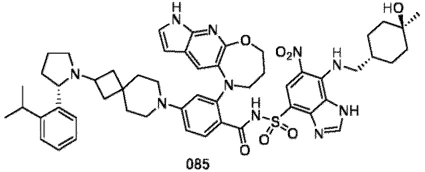

Referring to the synthesis route and method of embodiment 1, intermediate 55 in synthesis step 1 was replaced with intermediate 61 to synthesize the target compound 085. ESI-MS(M+H)⁺=985.5.

### Embodiment 4: (R)-2-((1H-pyrrolo[2,3-b]pyrid-5-yl)oxy)-4-(2-(2-(2-isopropylphenyl)-4-(4-methoxybenzyl)piperazin-1-yl)-7-azaspiro[3.5]non-7-yl)-N-((6-nitro-7-(((tetrahydro-2H-pyran-4-yl)methyl)amino)benzo[d][1,3]dioxocyclopent-4-yl)sulfonyl)benzamide (145)

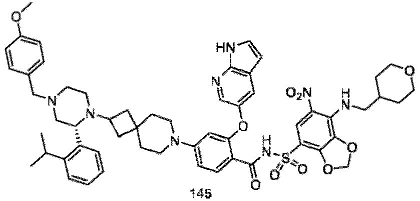

Referring to the synthesis route and method of embodiment 1, intermediate 1 in synthesis step 1 was replaced with intermediate 10, and intermediate 72 in synthesis step 3 was replaced with intermediate 86 to synthesize the target compound 145. ESI-MS(M+H)⁺=1041.4.¹H NMR (400 MHz, Chloroform-*d*) δ 9.22 (s, 1H), 8.63 (s, 1H), 8.14 (t, *J* = 6.4 Hz, 2H), 7.90 (d, *J* = 9.1 Hz, 1H), 7.66 (d, *J* = 2.3 Hz, 1H), 7.43 (t, *J* = 2.9 Hz, 2H), 7.18 (d, *J* = 6.1 Hz, 2H), 7.08 (d, *J* = 7.4 Hz, 1H), 6.80 (d, *J* = 8.1 Hz, 2H), 6.52 (q, *J* = 3.4 Hz, 2H), 5.96 (s, 2H), 3.98 (dd, *J* = 11.6, 4.4 Hz, 2H), 3.76 - 2.62 (m, 4H), 3.54 - 3.27 (m, 9H), 3.01 - 2.82 (m, 9H), 2.64 (s, 1H), 2.22 - 2.09 (m, 3H), 2.03 - 1.96 (m, 1H), 1.18 - 1.13 (m, 5H), 1.10 - 0.85 (m, 12H).

### Embodiment 5: (R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-methoxybenzyl)piperazin-1-yl)-7-azaspiro[3.5]non-7-yl)benzamide (152)

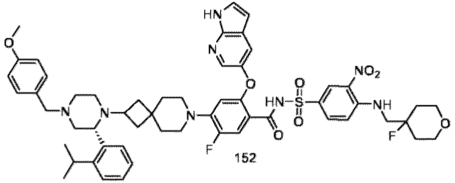

Referring to the synthesis route and method of embodiment 1, intermediate 1 in synthesis step 1 was replaced with intermediate 10, intermediate 55 in synthesis step 1 was replaced with intermediate 56, and intermediate 72 in synthesis step 3 was replaced with intermediate 64 to synthesize the target compound 152. ESI-MS(M+H)⁺=1033.4.¹H NMR (400 MHz, Chloroform-*d*) δ 9.45 (s, 1H), 8.87 (d, *J* = 2.3 Hz, 1H), 8.66 (t, *J* = 5.8 Hz, 1H), 8.14 (dd, *J* = 8.8, 2.4 Hz, 2H), 7.68 - 7.58 (m, 2H), 7.52 - 7.37 (m, 2H), 7.23 - 7.14 (m, 4H), 7.10 (d, *J* = 7.2 Hz, 1H), 6.97 (d, *J* = 9.3 Hz, 1H), 6.84 - 6.78 (m, 2H), 6.52 (dd, *J* = 3.5, 1.9 Hz, 1H), 6.03 (d, *J* = 7.0 Hz, 1H), 3.93 - 3.83 (m, 2H), 3.77 (s, 4H), 3.74 (d, *J* = 2.4 Hz, 1H), 3.71 (d, *J* = 2.4 Hz, 1H), 3.56 - 3.53 (m, 1H), 3.42 - 3.38 (m, 3H), 3.30 - 3.28 (m, 1H), 3.02 - 2.62 (m, 6H), 2.25 - 2.18 (m, 4H), 1.98 - 1.57 (m, 4H), 1.39 - 1.31 (m, 4H), 1.28 - 1.23 (m, 2H), 1.18 - 1.13 (m, 3H), 1.10 - 1.01 (m, 6H).

### Example 6: (R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-((methylamino)methyl)benzyl)piperazin-1-yl)-7-azaspiro [3.5] non-7-yl)benzamide (243)

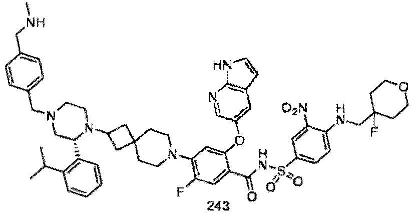

### Synthesis step 1: (R)-2-((1H-pyrrolo[2,3-b]pyrid-5-yl)oxy)-4-(2-(4-(4-(((2,4-dimethoxybenzyl)(methyl)amino)methyl)benzyl)-2-(2-isopropylphenyl)piperazin-1-yl)-7-azaspiro [3.5] non-7-yl)-5-fluoro-N-((4-(((4-fluorotetrahydro-2H-pyran-4-yl)methyl)amino)-3-nitrophenyl)sulfonyl)benzamide (243-1)

Referring to the synthesis route and method of embodiment 1, intermediate 55 in synthesis step 1 was replaced with intermediate 56, intermediate 1 in synthesis step 1 was replaced with intermediate 52, and intermediate 72 in synthesis step 3 was replaced with intermediate 64 to synthesize product (R)-2-((1H-pyrrolo[2,3-b]pyrid-5-yl)oxy)-4-(2-(4-(4-(((2,4-dimethoxybenzyl)(methyl)amino)methyl)benzyl)-2-(2-isopropylphenyl)piperazin-1-yl)-7-azaspiro[3.5]non-7-yl)-5-fluoro-N-((4-(((4-fluorotetrahydro-2H-pyran-4-yl)methyl)amino)-3-nitrophenyl)sulfonyl)benzamide. ESI-MS(M+H)⁺=1196.5.

### Synthesis step 2: (R)-2-((1H-pyrrolo[2,3-b]pyrid-5-yl)oxy)-5-fluoro-N-((4-(((4-fluorotetrahydro-2H-pyran-4-yl)methyl)amino)-3-nitrophenyl)sulfonyl)-4-(2-(2-(2-isopropylphenyl)-4-(4-((methylamino)methyl)benzyl)piperazin-1-yl)-7-azaspiro[3.5]non-7-yl)benzamide (243)

Compound 243-1 (408 mg, 0.35 mmol) was dissolved in 20 mL of DCM and 10 mL of TFA was added for reflux reaction for 18 h. After the reaction was completed, the system was concentrated to remove the reaction liquid, a saturated sodium bicarbonate aqueous solution was added to neutralize the pH to 8-9, dichloromethane was added for extraction, and the organic phase was separated and concentrated. The resulting crude product was purified by column chromatography to obtain 125 mg of the solid product of compound 243, with a yield of 34%. ESI-MS(M+H)⁺=1046.5.

Referring to the synthesis route and method of embodiments 1-6, the following target compounds were synthesized:

| Emb odi ment | Compound structural formula | Characterization data |
|---|---|---|
| 7 | 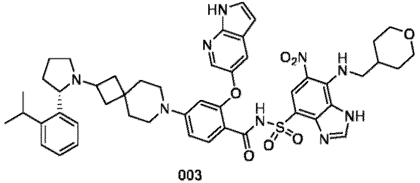 | ESI-MS(M+H)⁺=902.4 |
| 8 | 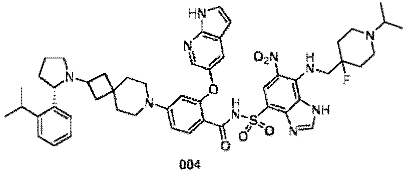 | ESI-MS(M+H)⁺=961.4 |
| 9 | 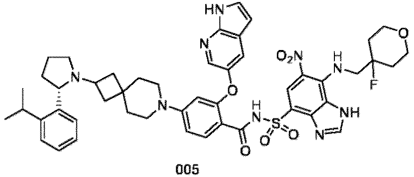 | ESI-MS(M+H)⁺=920.4 |
| 10 | 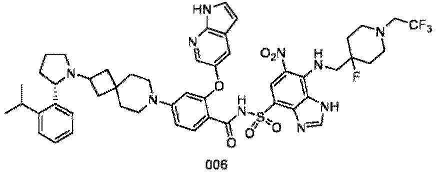 | ESI-MS(M+H)⁺=1001.4 |
| 11 | 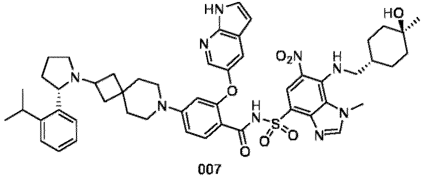 | ESI-MS(M+H)⁺=944.4 |
| 12 | 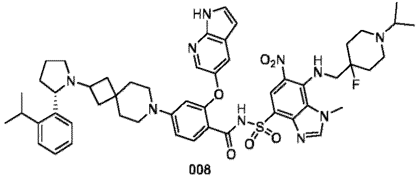 | ESI-MS(M+H)⁺=975.5 |
| 13 | 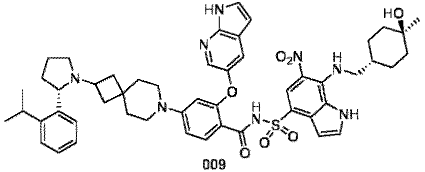 | ESI-MS(M+H)⁺=929.4 |
| 14 | 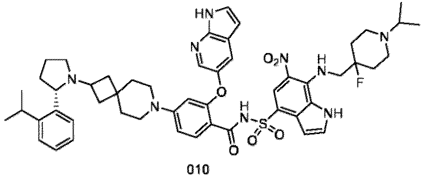 | ESI-MS(M+H)⁺=960.5 |
| 15 | 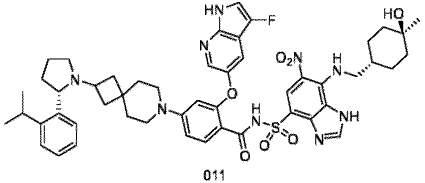 | ESI-MS(M+H)⁺=948.4 |
| 16 | 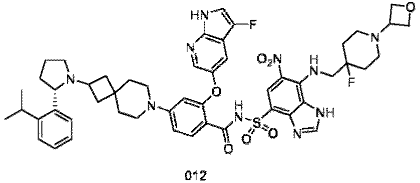 | ESI-MS(M+H)⁺=993.4 |
| 17 | 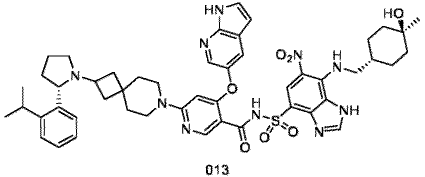 | ESI-MS(M+H)⁺=931.4 |
| 18 | 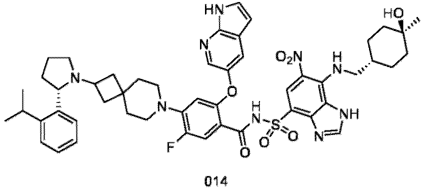 | ESI-MS(M+H)⁺=948.4 |
| 19 | 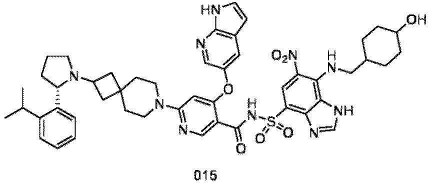 | ESI-MS(M+H)⁺=917.4 |
| 20 | 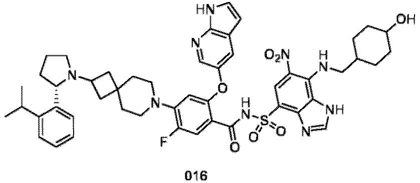 | ESI-MS(M+H)⁺=934.4 |
| 21 | 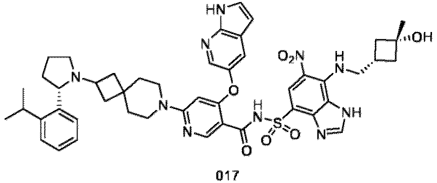 | ESI-MS(M+H)⁺=903.4 |
| 22 | 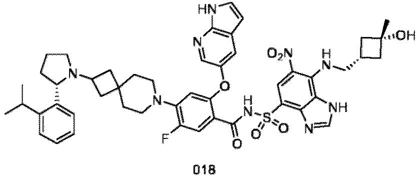 | ESI-MS(M+H)⁺=920.4 |
| 23 | 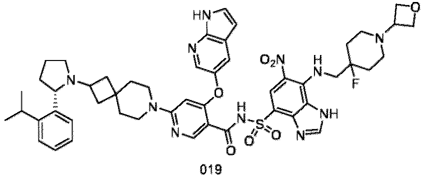 | ESI-MS(M+H)⁺=976.4 |
| | | ¹H NMR (400 MHz, Chloroform-*d*) δ 11.23 (s, 1H), 9.55 (s, 1H), 9.14 (s, 1H), 8.74 (d, *J* = 32.8 Hz, 2H), 8.16 (s, 1H), 8.02 (s, 1H), 7.73 (s, 1H), 7.55 (s, 1H), 7.46 (s, 1H), 7.17 (d, *J* = 10.2 Hz, 1H), 6.57 (s, 1H), 5.51 (s, 1H), 5.34 (s, 1H), 4.86 - 4.74 (m, 2H), 4.67 (t, *J* = 6.5 Hz, 3H), 4.58 - 4.31 (m, 4H), 3.56 - 3.48 (m, 4H), 3.42 - 3.13 (m, 4H), 3.02 (s, 1H), 2.84 (s, 2H), 2.59 (s, 1H), 2.37 - 2.34 (m, 2H), 2.16 - 2.13 (m, 2H), 2.05 - 1.94 (m, 4H), 1.40 - 1.31 (m, 4H), 1.18 - 1.01 (m, 9H). |
| 24 | 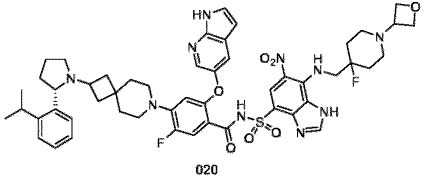 | ESI-MS(M+H)⁺=993.4 |
| 25 | 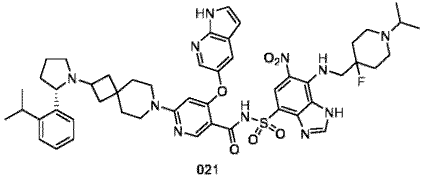 | ESI-MS(M+H)⁺=962.4 |
| 26 | 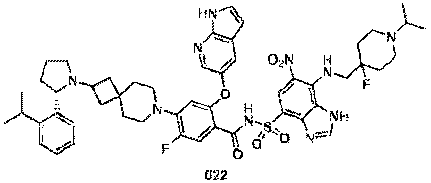 | ESI-MS(M+H)⁺=979.4 |
| 27 | 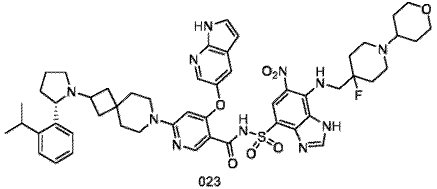 | ESI-MS(M+H)⁺=1004.5 |
| 28 | 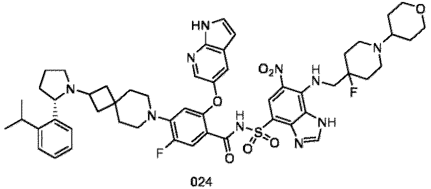 | ESI-MS(M+H)⁺=1021.4 |
| 29 | 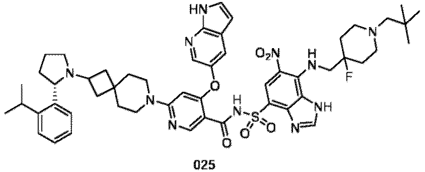 | ESI-MS(M+H)⁺=990.5 |
| 30 | 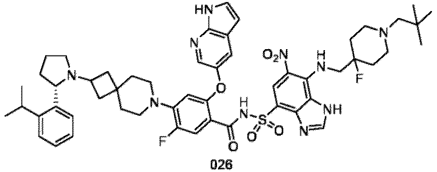 | ESI-MS(M+H)⁺=1007.5 |
| 31 | 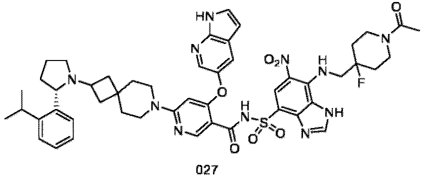 | ESI-MS(M+H)⁺=962.4 |
| 32 | 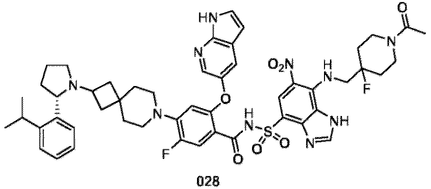 | ESI-MS(M+H)⁺=979.4 |
| 33 | 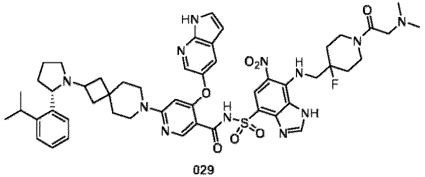 | ESI-MS(M+H)⁺=1005.4 |
| 34 | 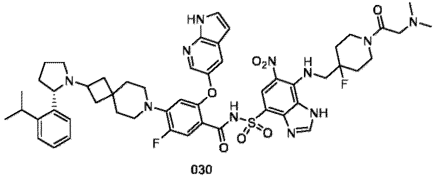 | ESI-MS(M+H)⁺=1022.4 |
| 35 | 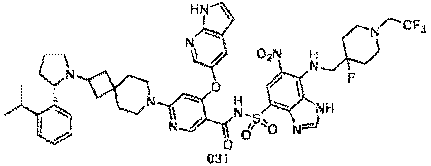 | ESI-MS(M+H)⁺=1002.4 |
| 36 | 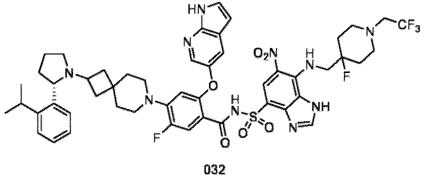 | ESI-MS(M+H)⁺=1019.4 |
| 37 | 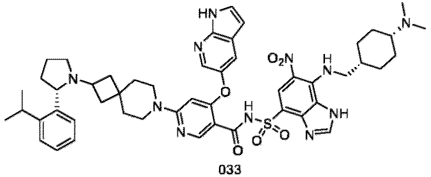 | ESI-MS(M+H)⁺=944.5 |
| 38 | 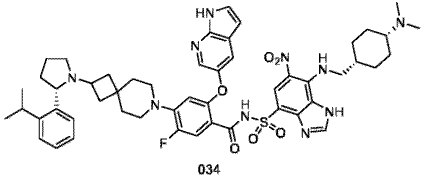 | ESI-MS(M+H)⁺=961.4 |
| 39 | 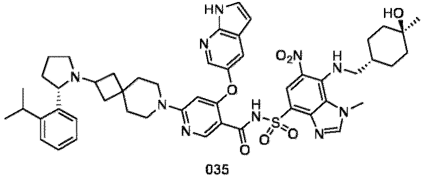 | ESI-MS(M+H)⁺=945.4 |
| 40 | 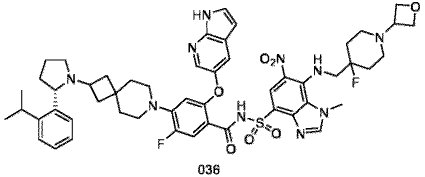 | ESI-MS(M+H)⁺=1007.4 |
| 41 | 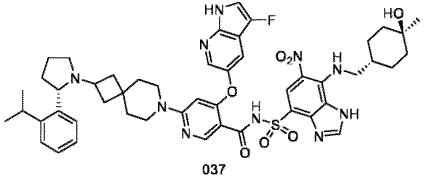 | ESI-MS(M+H)⁺=949.4 |
| 42 | 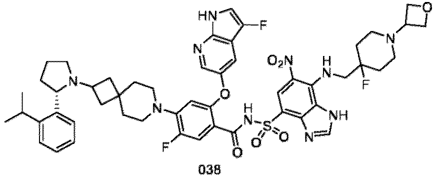 | ESI-MS(M+H)⁺=1011.4 |
| 43 | 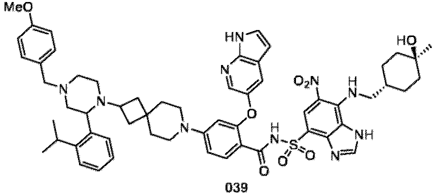 | ESI-MS(M+H)⁺=1065.5 |
| 44 | 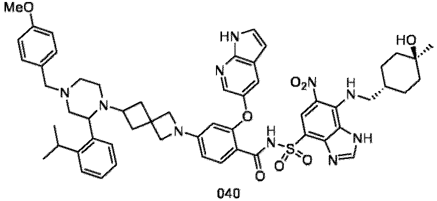 | ESI-MS(M+H)⁺=1037.5 |
| 45 | 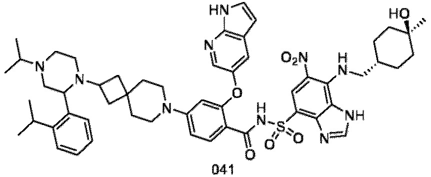 | ESI-MS(M+H)⁺=987.5 |
| 46 | 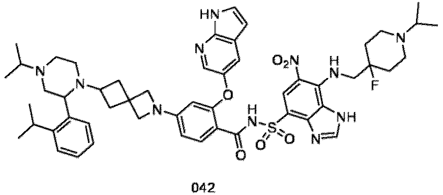 | ESI-MS(M+H)⁺=990.5 |
| 47 | 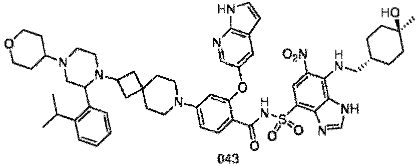 | ESI-MS(M+H)⁺=1029.5 |
| 48 | 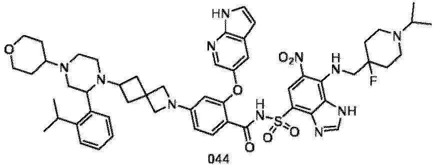 | ESI-MS(M+H)⁺=1032.5 |
| 49 | 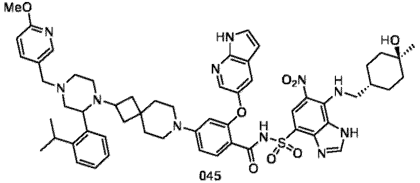 | ESI-MS(M+H)⁺=1066.5 |
| 50 | 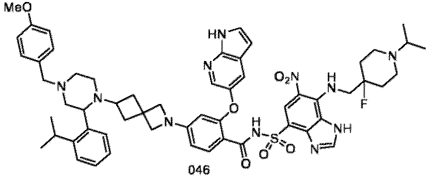 | ESI-MS(M+H)⁺=1068.5 |
| 51 | 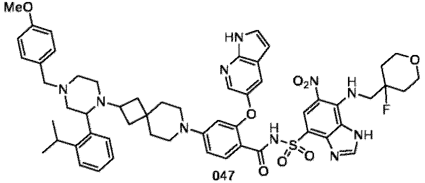 | ESI-MS(M+H)⁺=1055.5 |
| 52 | 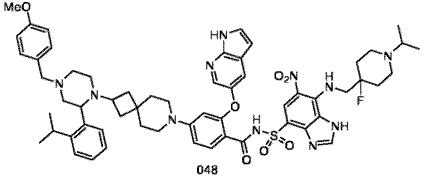 | ESI-MS(M+H)⁺=1096.5 |
| 53 | 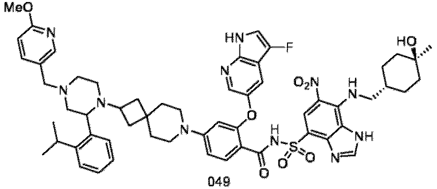 | ESI-MS(M+H)⁺=1084.5 |
| 54 | 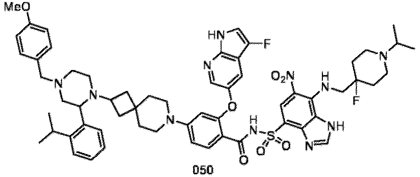 | ESI-MS(M+H)⁺=1114.5 |
| 55 | 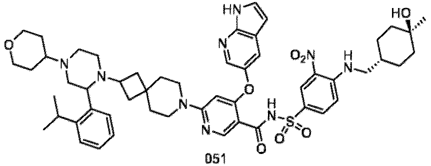 | ESI-MS(M+H)⁺=990.5 |
| 56 | 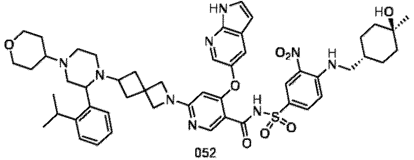 | ESI-MS(M+H)⁺=962.5 |
| 57 | 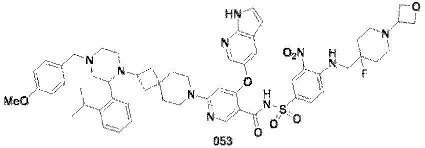 | ESI-MS(M+H)⁺=1071.5 |
| | | ¹H NMR (400 MHz, Chloroform-*d*) δ 9.51 (s, 1H), 8.89 (d, *J* = 2.3 Hz, 1H), 8.73 (s, 1H), 8.67 (t, *J* = 5.7 Hz, 1H), 8.20 (dd, *J* = 9.2, 2.3 Hz, 1H), 8.14 (d, *J* = 2.5 Hz, 1H), 7.71 (d, *J* = 2.5 Hz, 1H), 7.46 (dd, *J* = 3.6, 2.5 Hz, 2H), 7.20 -7.13 (m, 4H), 7.07 (d, *J* = 7.1 Hz, 1H), 6.99 (d, *J* = 9.3 Hz, 1H), 6.83 - 6.76 (m, 2H), 6.55 (dd, *J* = 3.5, 1.9 Hz, 1H), 5.48 (s, 1H), 4.68 - 4.62 (m, 2H), 4.59 -4.51 (m, 2H), 3.75 (s, 4H), 3.63 (s, 1H), 3.59 - 3.39 (m, 6H), 3.19 - 3.11 (m, 2H), 2.91 - 2.90 (m, 2H), 2.61 - 2.58 (m, 3H), 2.33 - 2.11 (m, 4H), 2.09 - 1.96 (m, 4H), 1.78 - 1.53 (m, 4H), 1.38 - 1.22 (m, 4H), 1.17 - 1.11 (m, 4H), 1.09 - 1.01 (m, 5H). |
| 58 | 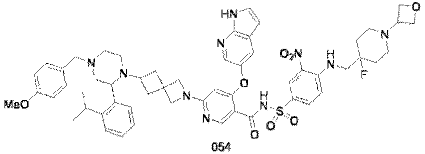 | ESI-MS(M+H)⁺=1043.5 |
| 59 | 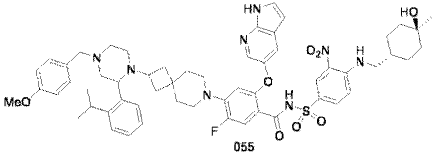 | ESI-MS(M+H)⁺=1043.5 |
| 60 | 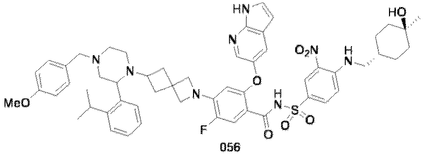 | ESI-MS(M+H)⁺=1015.4 |
| 61 | 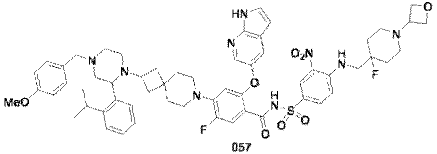 | ESI-MS(M+H)⁺=1088.5 |
| 62 | 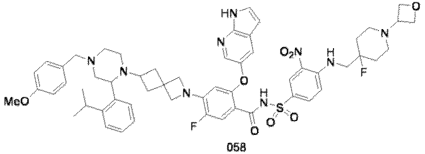 | ESI-MS(M+H)⁺=1060.4 |
| 63 | 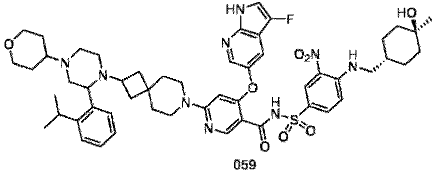 | ESI-MS(M+H)⁺=1008.5 |
| 64 | 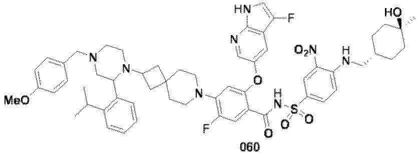 | ESI-MS(M+H)⁺=1061.5 |
| 65 | 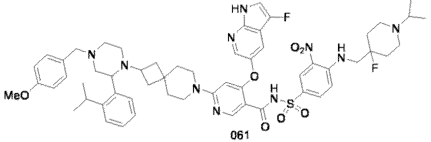 | ESI-MS(M+H)⁺=1075.5 |
| 66 | 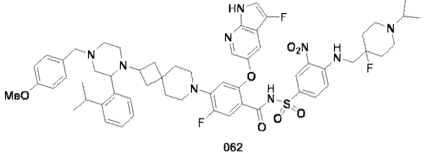 | ESI-MS(M+H)⁺=1092.5 |
| 67 | 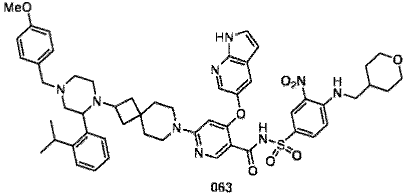 | ESI-MS(M+H)⁺=998.5 |
| | | ¹H NMR (400 MHz, Chloroform-*d* δ 9.41 (s, 1H), 8.89 (d, *J* = 2.3 Hz, 1H), 8.74 (s, 1H), 8.53 (t, *J* = 5.5 Hz, 1H), 8.21 (dd, *J* = 9.2, 2.3 Hz, 1H), 8.15 (d, *J* = 2.5 Hz, 1H), 7.71 (d, *J* = 2.5 Hz, 1H), 7.50 - 7.38 (m, 2H), 7.21 -7.12 (m, 4H), 7.06 (t, *J* = 7.3 Hz, 1H), 6.94 (d, *J* = 9.3 Hz, 1H), 6.83 - 6.76 (m, 2H), 6.56 (dd, *J* = 3.5, 1.9 Hz, 1H), 5.49 (s, 1H), 4.06 - 3.97 (m, 2H), 3.75 (s, 3H), 3.61 (s, 1H), 3.50 - 3.36 (m, 4H), 3.34 - 3.09 (m, 4H), 2.98 - 2.78 (m, 3H), 2.63 - 2.61 (m, 1H), 2.31 - 2.06 (m, 3H), 1.97 - 1.92 (m, 2H), 1.78 - 1.56 (m, 5H), 1.49 - 1.35 (m, 3H), 1.29 - 1.23 (m, 3H), 1.17 - 1.11 (m, 4H), 1.09 - 1.01 (m, 5H). |
| 68 | 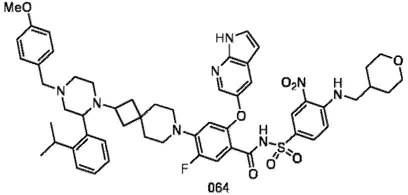 | ESI-MS(M+H)⁺=1015.4 |
| 69 | 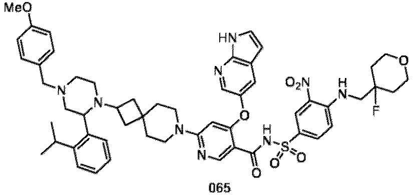 | ESI-MS(M+H)⁺=1016.4 |
| 70 | 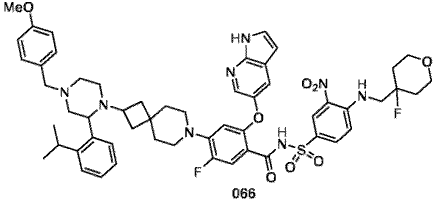 | ESI-MS(M+H)⁺=1033.4 |
| 71 | 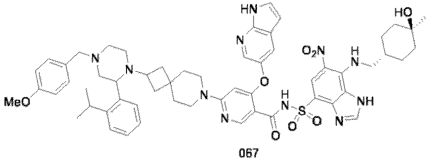 | ESI-MS(M+H)⁺=1066.5 |
| 72 | 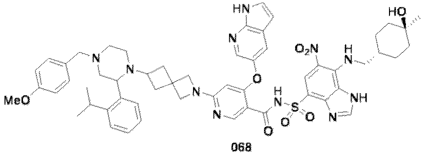 | ESI-MS(M+H)⁺=1038.5 |
| 73 | 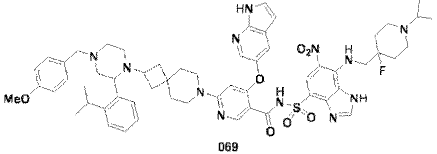 | ESI-MS(M+H)⁺=1097.5 |
| 74 | 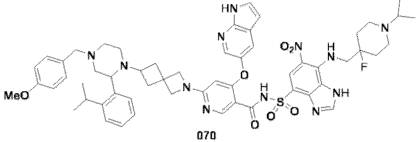 | ESI-MS(M+H)⁺=1069.5 |
| 75 | 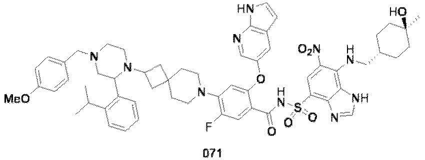 | ESI-MS(M+H)⁺=1083.5 |
| 76 | 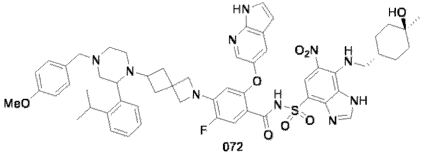 | ESI-MS(M+H)⁺=1055.5 |
| 77 | 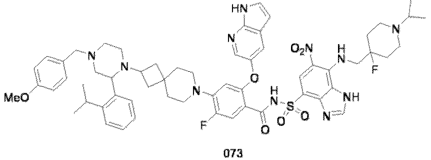 | ESI-MS(M+H)⁺=1114.5 |
| 78 | 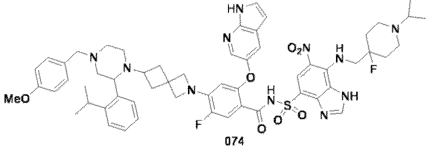 | ESI-MS(M+H)⁺=1086.5 |
| 79 | 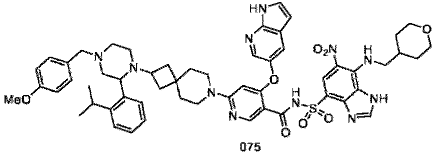 | ESI-MS(M+H)⁺=1038.5 |
| 80 | 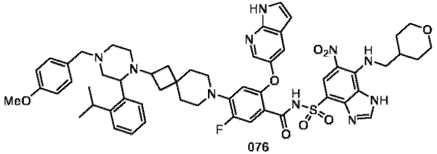 | ESI-MS(M+H)⁺=1055.5 |
| 81 | 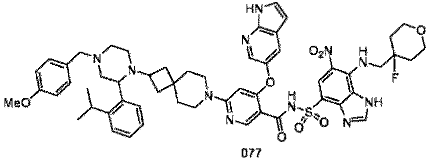 | ESI-MS(M+H)⁺=1056.4 |
| 82 | 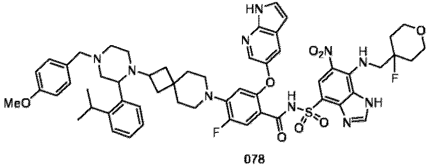 | ESI-MS(M+H)⁺=1073.4 |
| 83 | 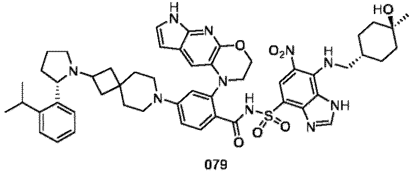 | ESI-MS(M+H)⁺=971.5 |
| 84 | 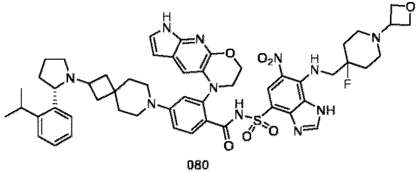 | ESI-MS(M+H)⁺=1016.5 |
| 85 | 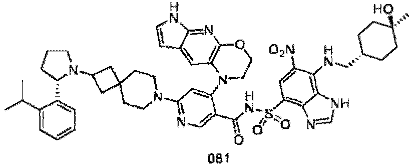 | ESI-MS(M+H)⁺=972.4 |
| 86 | 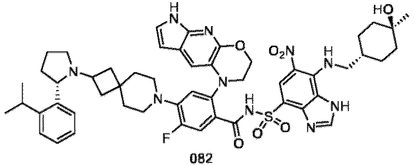 | ESI-MS(M+H)⁺=989.4 |
| 87 | 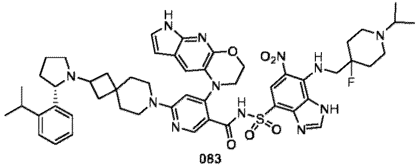 | ESI-MS(M+H)⁺=1003.5 |
| 88 | 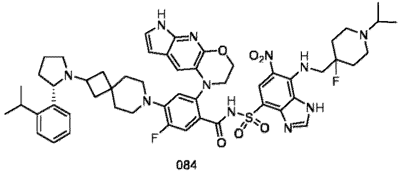 | ESI-MS(M+H)⁺=1020.5 |
| 89 | 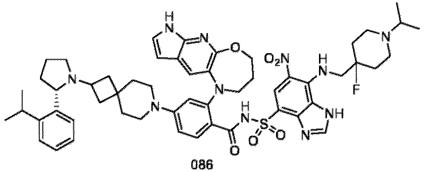 | ESI-MS(M+H)⁺=1016.5 |
| 90 | 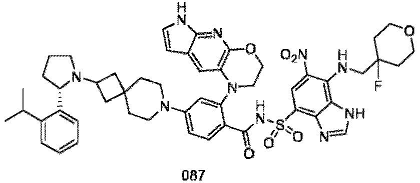 | ESI-MS(M+H)⁺=961.4 |
| 91 | 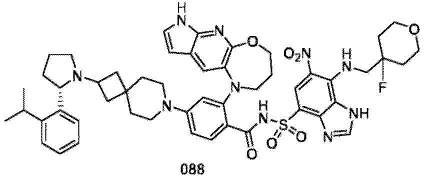 | ESI-MS(M+H)⁺=975.4 |
| 92 | 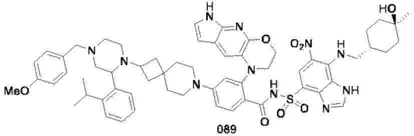 | ESI-MS(M+H)⁺=1106.5 |
| 93 | 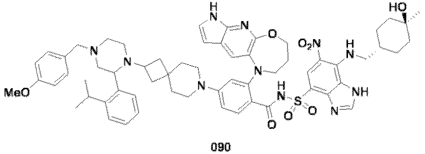 | ESI-MS(M+H)⁺=1120.5 |
| 94 | 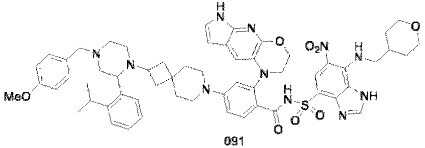 | ESI-MS(M+H)⁺=1078.5 |
| 95 | 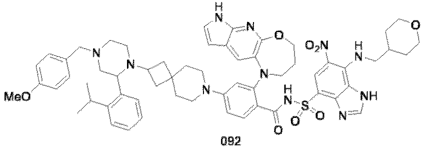 | ESI-MS(M+H)⁺=1092.5 |
| 96 | 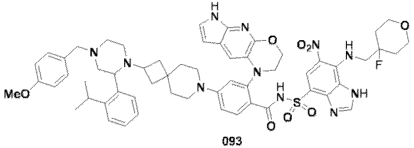 | ESI-MS(M+H)⁺=1096.5 |
| 97 | 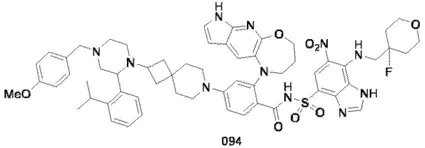 | ESI-MS(M+H)⁺=1110.5 |
| 98 | 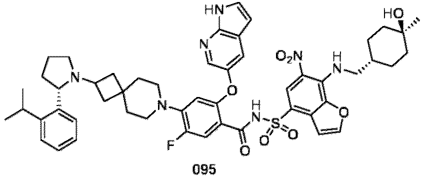 | ESI-MS(M+H)⁺=948.4 |
| 99 | 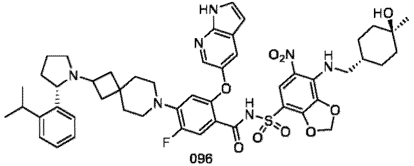 | ESI-MS(M+H)⁺=952.4 |
| 100 | 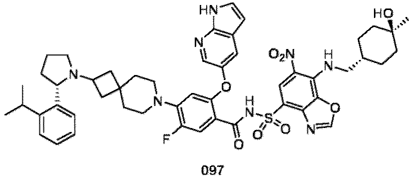 | ESI-MS(M+H)⁺=949.4 |
| 101 | 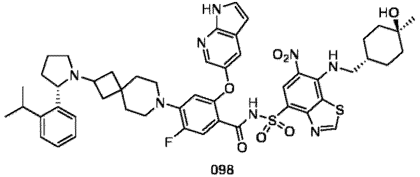 | ESI-MS(M+H)⁺=965.4 |
| 102 | 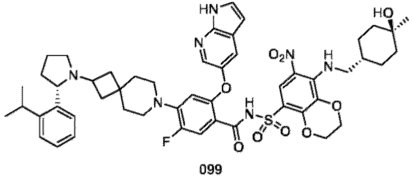 | ESI-MS(M+H)⁺=966.4 |
| 103 | 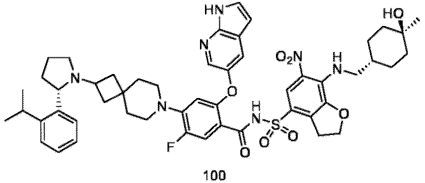 | ESI-MS(M+H)⁺=950.4 |
| 104 | 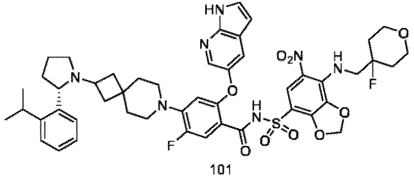 | ESI-MS(M+H)⁺=942.4 |
| 105 | 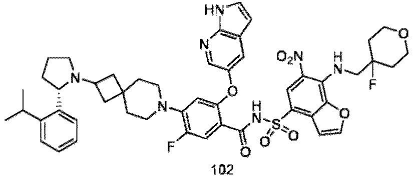 | ESI-MS(M+H)⁺=938.4 |
| 106 | 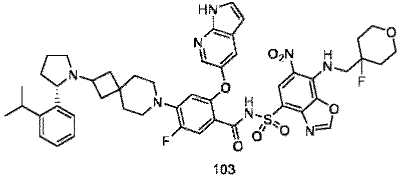 | ESI-MS(M+H)⁺=939.4 |
| 107 | 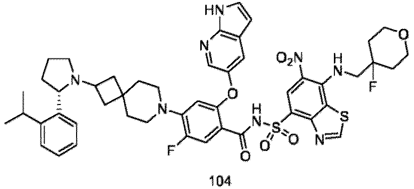 | ESI-MS(M+H)⁺=955.3 |
| 108 | 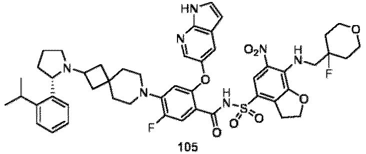 | ESI-MS(M+H)⁺=940.4 |
| 109 | 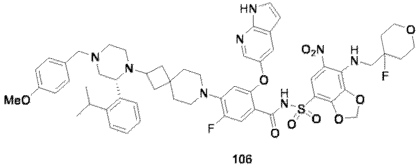 | ESI-MS(M+H)⁺=1077.4 |
| 110 | 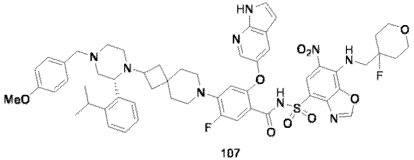 | ESI-MS(M+H)⁺=1074.4 |
| 111 | 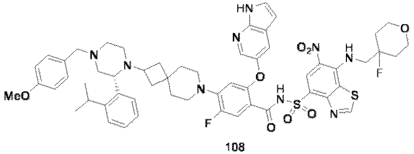 | ESI-MS(M+H)⁺=1090.4 |
| 112 | 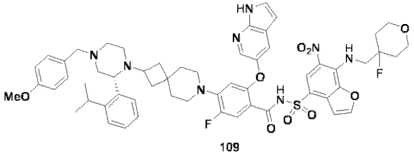 | ESI-MS(M+H)⁺=1073.4 |
| 113 | 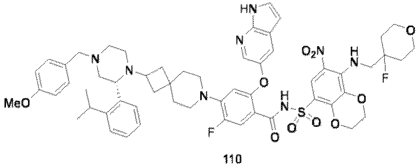 | ESI-MS(M+H)⁺=1091.4 |
| 114 | 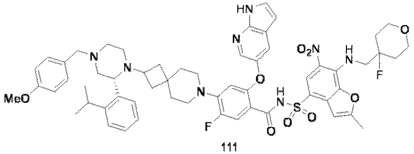 | ESI-MS(M+H)⁺=1087.5 |
| 115 | 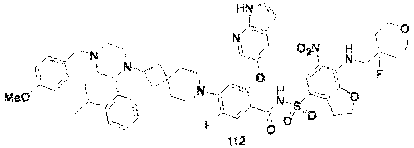 | ESI-MS(M+H)⁺=1075.5 |
| 116 | 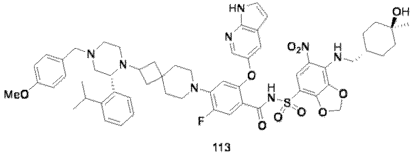 | ESI-MS(M+H)⁺=1087.5 |
| 117 | 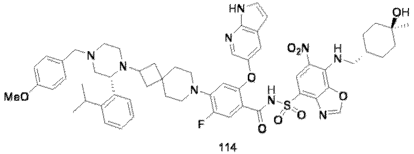 | ESI-MS(M+H)⁺=1084.5 |
| 118 | 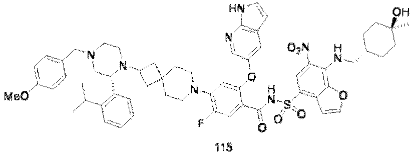 | ESI-MS(M+H)⁺=1083.5 |
| 119 | 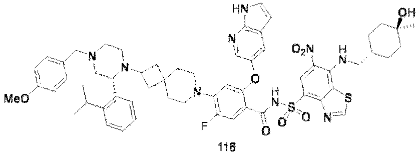 | ESI-MS(M+H)⁺=1100.5 |
| 120 | 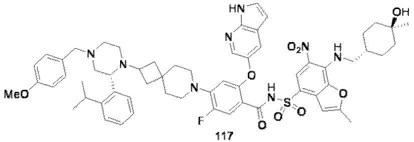 | ESI-MS(M+H)⁺=1097.5 |
| 121 | 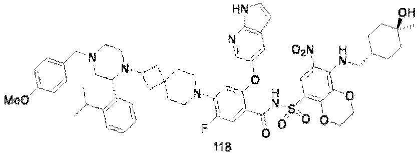 | ESI-MS(M+H)⁺=1101.5 |
| 122 | 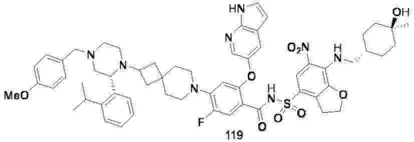 | ESI-MS(M+H)⁺=1085.5 |
| 123 | 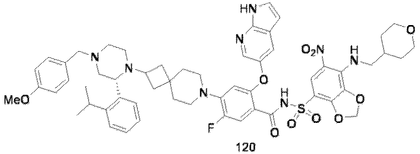 | ESI-MS(M+H)⁺=1059.4 |
| | | ¹H NMR (400 MHz, Chloroform-*d*) δ 9.04 (s, 1H), 8.61 (s, 1H), 8.19 - 8.08 (m, 2H), 7.67 - 7.60 (m, 2H), 7.46 - 7.40 (m, 2H), 6.81 - 6.78 (m, 2H), 6.54 - 6.50 (m, 1H), 6.05 (d, *J* = 7.0 Hz, 1H), 5.94 (s, 2H), 3.98 - 3.90 (m, 2H), 3.76 - 3.60 (m, 4H), 3.49 - 3.44 (m, 6H), 3.36 - 3.18 (m, 4H), 2.95 - 2.79 (m, 4H), 2.76 - 2.73 (m, 3H), 2.64 - 2.24 (s, 4H), 1.20 - 1.13 (m, 6H), 1.11 - 1.02 (m, 6H), 0.97 - 0.83 (m, 9H). |
| 124 | 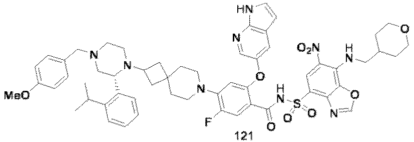 | ESI-MS(M+H)⁺=1056.4 |
| 125 | 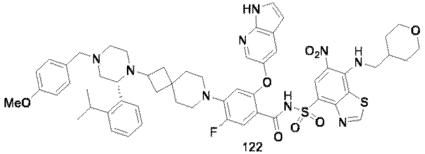 | ESI-MS(M+H)⁺=1072.4 |
| 126 | 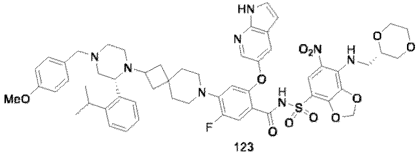 | ESI-MS(M+H)⁺=1061.4 |
| 127 | 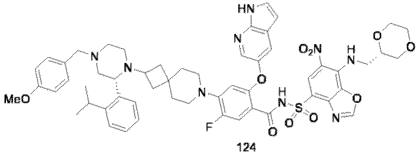 | ESI-MS(M+H)⁺=1058.4 |
| 128 | 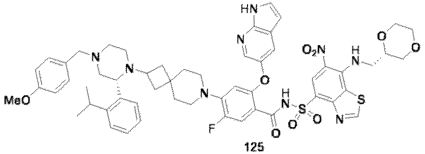 | ESI-MS(M+H)⁺=1074.4 |
| 129 | 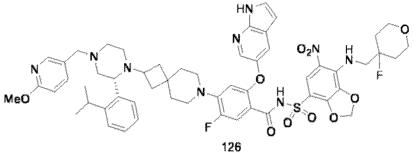 | ESI-MS(M+H)⁺=1078.4 |
| 130 | 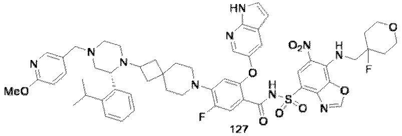 | ESI-MS(M+H)⁺=1075.4 |
| 131 | 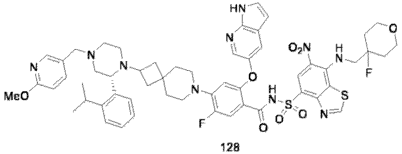 | ESI-MS(M+H)⁺=1091.4 |
| 132 | 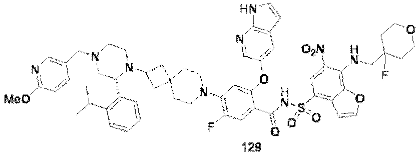 | ESI-MS(M+H)⁺=1074.4 |
| 133 | 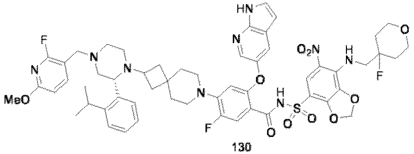 | ESI-MS(M+H)⁺=1096.4 |
| 134 | 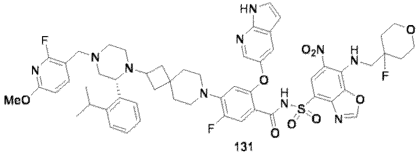 | ESI-MS(M+H)⁺=1093.4 |
| 135 | 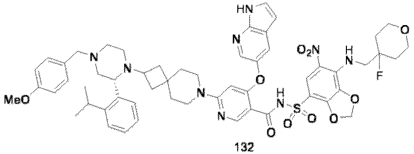 | ESI-MS(M+H)⁺=1060.4 |
| 136 | 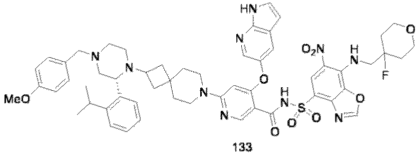 | ESI-MS(M+H)⁺=1057.4 |
| 137 | 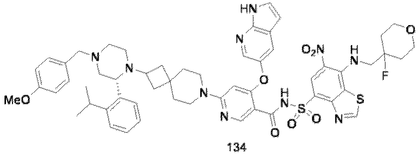 | ESI-MS(M+H)⁺=1073.4 |
| 138 | 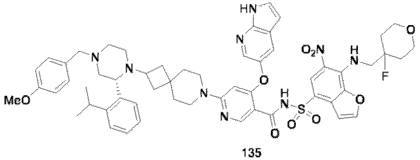 | ESI-MS(M+H)⁺=1056.4 |
| 139 | 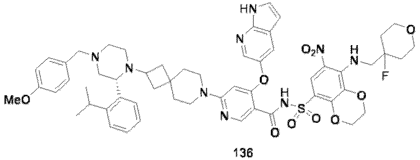 | ESI-MS(M+H)⁺=1074.5 |
| 140 | 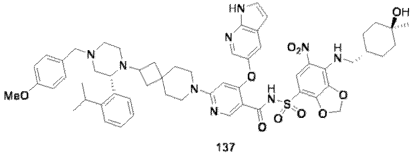 | ESI-MS(M+H)⁺=1070.5 |
| 141 | 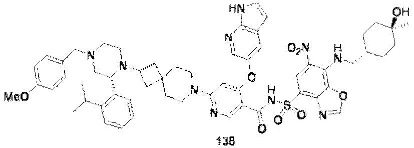 | ESI-MS(M+H)⁺=1067.5 |
| 142 | 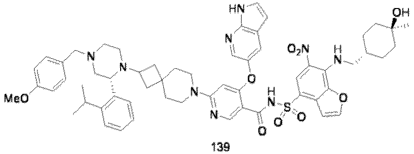 | ESI-MS(M+H)⁺=1066.5 |
| 143 | 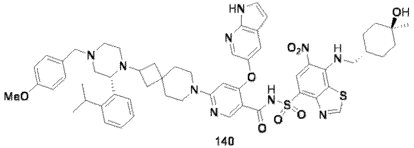 | ESI-MS(M+H)⁺=1083.5 |
| 144 | 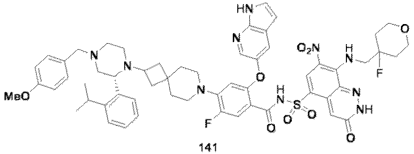 | ESI-MS(M+H)⁺=1101.4 |
| 145 | 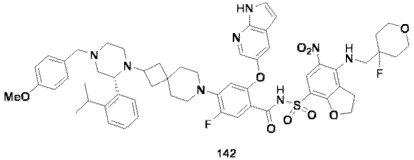 | ESI-MS(M+H)⁺=1075.4 |
| 146 | 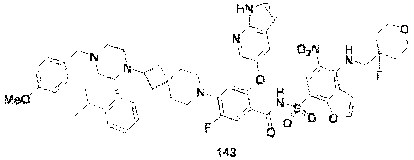 | ESI-MS(M+H)⁺=1073.4 |
| 147 | 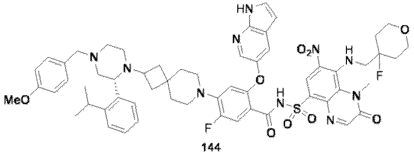 | ESI-MS(M+H)⁺=1115.5 |
| 148 | 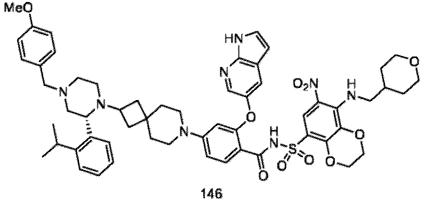 | ESI-MS(M+H)⁺=1055.5 |
| | | ¹H NMR (400 MHz, Chloroform-*d*) δ 9.49 (t, *J* = 2.3 Hz, 1H), 8.68 (s, 1H), 8.52 (t, *J* = 5.8 Hz, 1H), 8.12 (d, *J* = 2.6 Hz, 1H), 7.91 (d, *J* = 9.2 Hz, 1H), 7.65 (d, *J* = 2.5 Hz, 1H), 7.44 (dd, *J* = 3.6, 2.5 Hz, 2H), 7.19 -7.16 (m, 3H), 7.07 (td, *J* = 7.2, 6.5, 2.0 Hz, 1H), 6.79 (d, *J* = 8.7 Hz, 2H), 6.50 (dd, *J* = 3.5, 2.0 Hz, 2H), 5.97 (d, *J* = 2.3 Hz, 1H), 4.08 - 4.01 (m, 4H), 3.96 - 3.89 (m, 2H), 3.76 - 3.69 (m, 3H), 3.55 - 3.50 (m, 3H), 3.48 - 3.29 (m, 5H), 3.02 - 2.79 (m, 8H), 2.63 (s, 1H), 2.29 - 2.18 (m, 2H), 2.13 - 2.11 (m, 1H), 1.65 - 1.41 (m, 4H), 1.38 - 1.28 (m, 7H), 1.18 -1.10 (m, 8H). |
| 0149 | 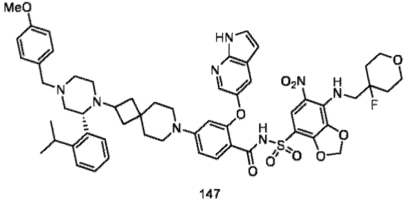 | ESI-MS(M+H)⁺=1059.4 |
| 150 | 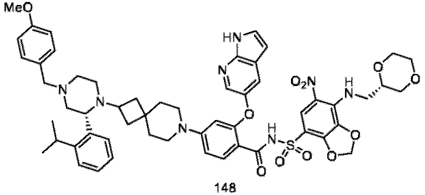 | ESI-MS(M+H)⁺=1043.4 |
| 151 | 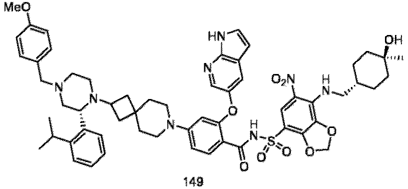 | ESI-MS(M+H)⁺=1069.5 |
| 152 | 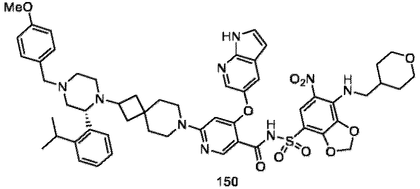 | ESI-MS(M+H)⁺=1042.4 |
| 153 | 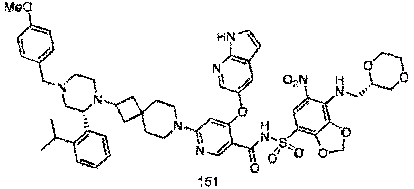 | ESI-MS(M+H)⁺=1044.4 |
| 154 | 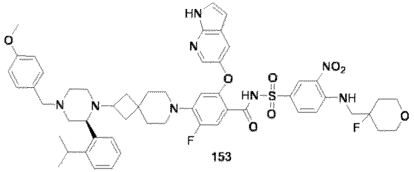 | ESI-MS(M+H)⁺=1033.4 |
| 155 | 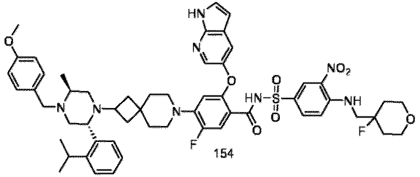 | ESI-MS(M+H)⁺=1047.5 |
| 156 | 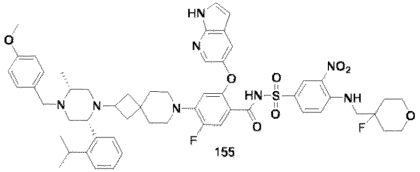 | ESI-MS(M+H)⁺=1047.5 |
| 157 | 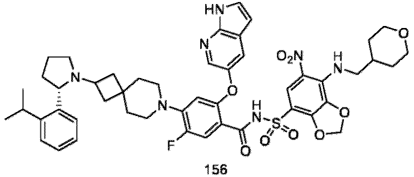 | ESI-MS(M+H)⁺=924.4 |
| 158 | 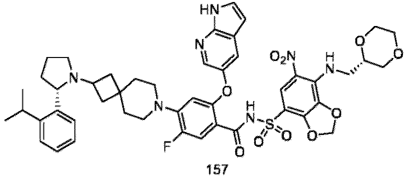 | ESI-MS(M+H)⁺=926.3 |
| 159 | 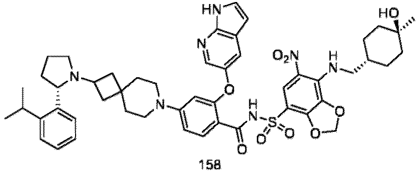 | ESI-MS(M+H)⁺=934.4 |
| 160 | 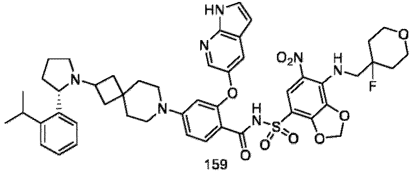 | ESI-MS(M+H)⁺=924.4 |
| 161 | 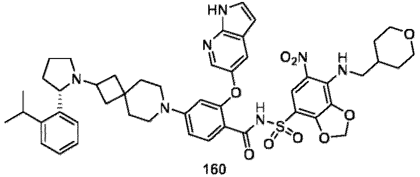 | ESI-MS(M+H)⁺=906.4 |
| 162 | 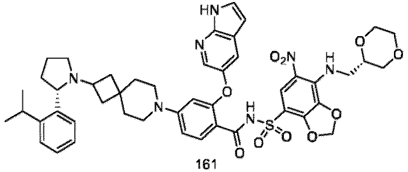 | ESI-MS(M+H)⁺=908.4 |
| 163 | 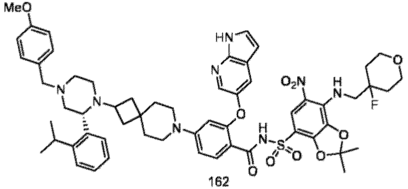 | ESI-MS(M+H)⁺=1087.5 |
| 164 | 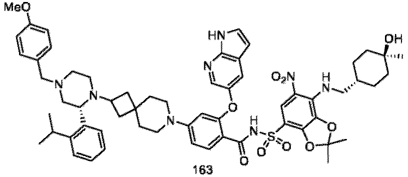 | ESI-MS(M+H)⁺=1097.5 |
| 165 | 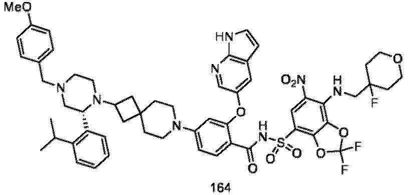 | ESI-MS(M+H)⁺=1095.4 |
| 166 | 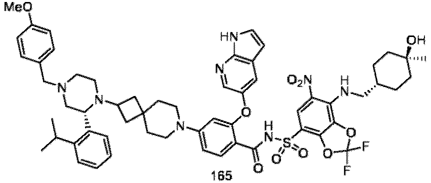 | ESI-MS(M+H)⁺=1105.5 |
| 167 | 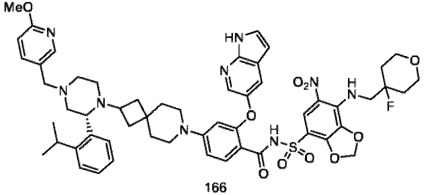 | ESI-MS(M+H)⁺=1060.4 |
| 168 | 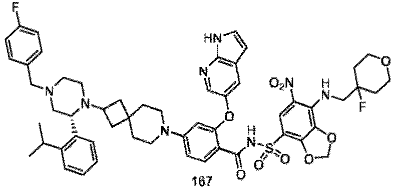 | ESI-MS(M+H)⁺=1047.4 |
| 169 | 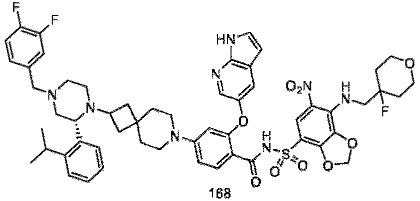 | ESI-MS(M+H)⁺=1065.4 |
| 170 | 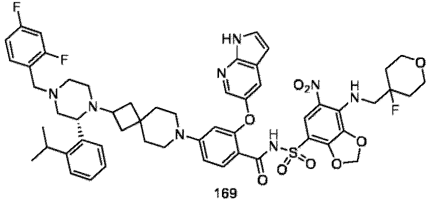 | ESI-MS(M+H)⁺=1065.4 |
| 171 | 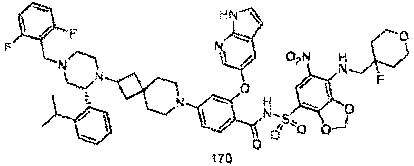 | ESI-MS(M+H)⁺=1065.4 |
| 172 | 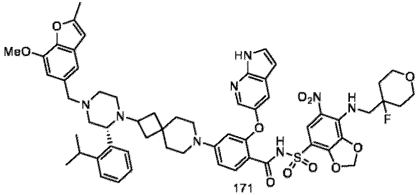 | ESI-MS(M+H)⁺=1113.4 |
| 173 | 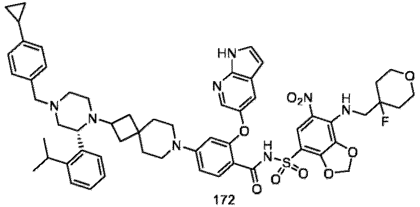 | ESI-MS(M+H)⁺=1069.5 |
| 174 | 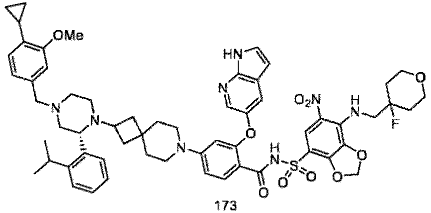 | ESI-MS(M+H)⁺=1099.5 |
| 175 | 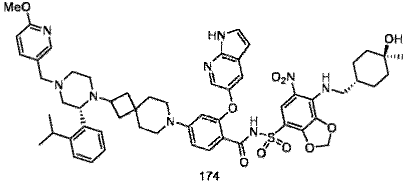 | ESI-MS(M+H)⁺=1070.5 |
| 176 | 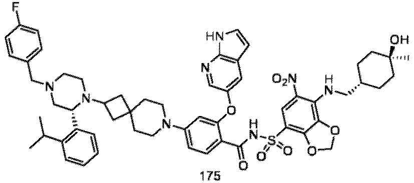 | ESI-MS(M+H)⁺=1057.5 |
| 177 | 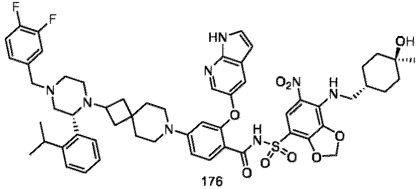 | ESI-MS(M+H)⁺=1075.4 |
| 178 | 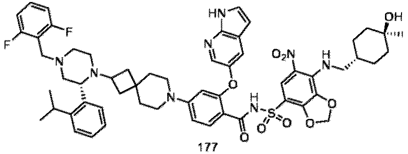 | ESI-MS(M+H)⁺=1075.4 |
| 179 | 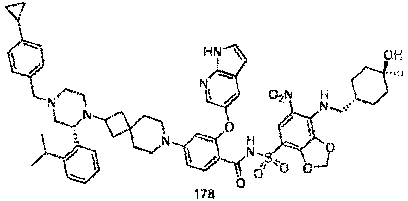 | ESI-MS(M+H)⁺=1079.5 |
| 180 | 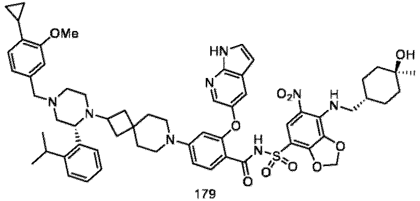 | ESI-MS(M+H)⁺=1109.5 |
| 181 | 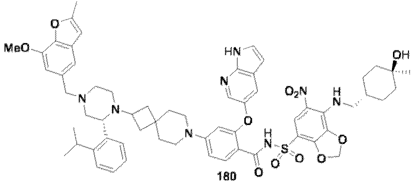 | ESI-MS(M+H)⁺=1123.5 |
| 182 | 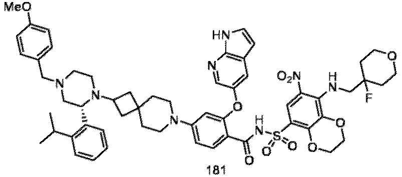 | ESI-MS(M+H)⁺=1073.5 |
| 183 | 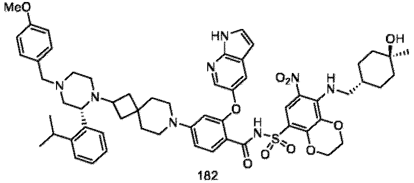 | ESI-MS(M+H)⁺=1083.5 |
| 184 | 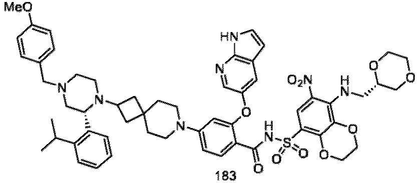 | ESI-MS(M+H)⁺=1057.4 |
| 185 | 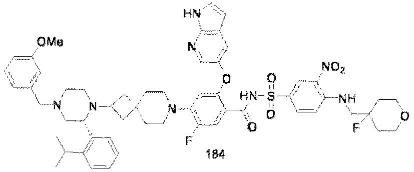 | ESI-MS(M+H)⁺=1033.4 |
| 186 | 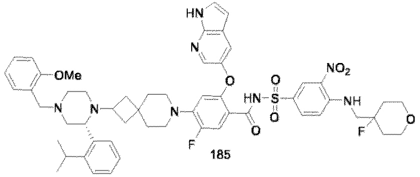 | ESI-MS(M+H)⁺=1033.4 |
| 187 | 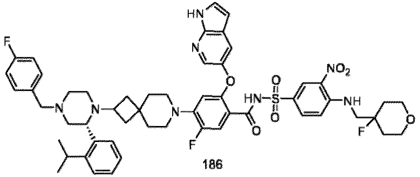 | ESI-MS(M+H)⁺=1021.4 |
| 188 | 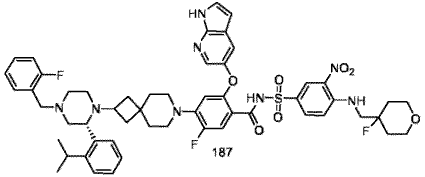 | ESI-MS(M+H)⁺=1021.4 |
| 189 | 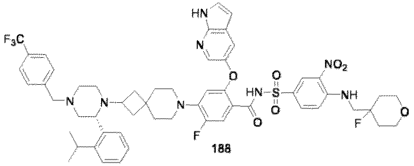 | ESI-MS(M+H)⁺=1071.4 |
| 190 | 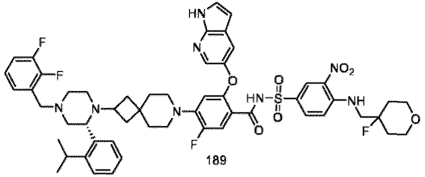 | ESI-MS(M+H)⁺=1039.4 |
| | | ¹H NMR (400 MHz, Chloroform-*d*) δ 9.37 (s, 1H), 8.86 (d, *J* = 2.2 Hz, 1H), 8.66 (s, 1H), 8.14 (td, *J* = 5.2, 4.4, 2.2 Hz, 2H), 7.68 - 7.61 (m, 2H), 7.44 (dd, *J* = 3.5, 2.5 Hz, 2H), 7.20 - 7.17 (m, 2H), 7.09 (q, *J* = 3.9, 3.4 Hz, 2H), 6.96 (d, *J* = 9.2 Hz, 2H), 6.52 (dd, *J* = 3.5, 1.9 Hz, 1H), 6.01 (d, *J* = 7.0 Hz, 1H), 3.86 - 3.75 (m, 4H), 3.64 - 3.49 (m, 6H), 2.91 - 2.78 (m, 5H), 2.73 - 2.69 (m, 2H), 2.63 - 2.25 (d, *J* = 11.4 Hz, 6H), 1.96 - 1.82 (m, 6H), 1.41 - 1.18 (m, 6H), 1.10 - 0.95 (m, 5H). |
| 191 | 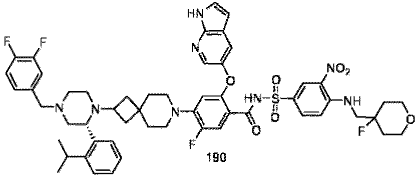 | ESI-MS(M+H)⁺=1039.4 |
| | | ¹H NMR (400 MHz, Chloroform-*d*) δ 9.31 (s, 1H), 8.86 (d, *J* = 2.3 Hz, 1H), 8.66 (s, 1H), 8.14 (dd, *J* = 8.7, 2.4 Hz, 2H), 7.68 - 7.61 (m, 2H), 7.44 (dd, *J* = 3.5, 2.5 Hz, 2H), 7.22 - 7.11 (m, 4H), 6.96 (d, *J* = 9.3 Hz, 2H), 6.52 (dd, *J* = 3.6, 2.0 Hz, 1H), 6.02 (d, *J* = 7.0 Hz, 1H), 3.90 - 3.85 (m, 2H), 3.75 (dd, *J* = 11.7, 2.4 Hz, 2H), 3.55 (d, *J* = 5.8 Hz, 2H), 3.50 (d, *J* = 5.8 Hz, 1H), 3.42 (s, 2H), 2.93 (d, *J* = 9.8 Hz, 1H), 2.81 - 2.61 (m, 6H), 2.57 - 2.25 (m, 3H), 1.95 - 1.83 (m, 4H), 1.38 - 1.23 (m, 8H), 1.19 -1.08 (m, 9H). |
| 192 | 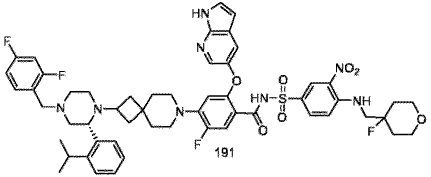 | ESI-MS(M+H)⁺=1039.4 |
| 193 | 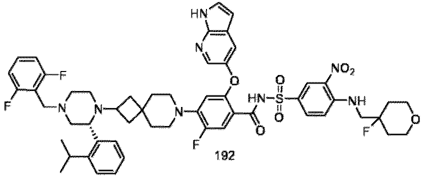 | ESI-MS(M+H)⁺=1039.4 |
| 194 | 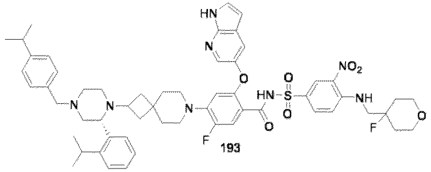 | ESI-MS(M+H)⁺=1045.5 |
| 195 | 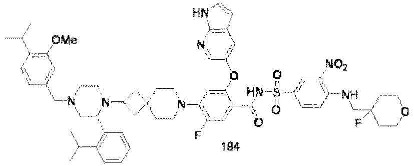 | ESI-MS(M+H)⁺=1075.5 |
| 196 | 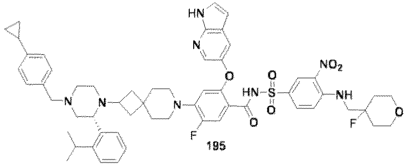 | ESI-MS(M+H)⁺=1043.5 |
| 197 | 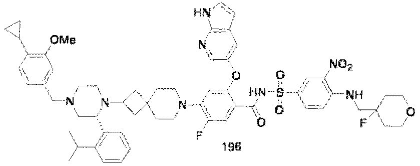 | ESI-MS(M+H)⁺=1073.5 |
| 198 | 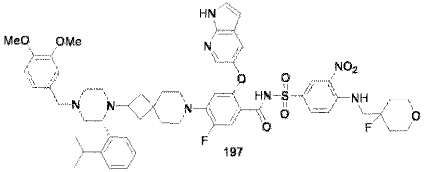 | ESI-MS(M+H)⁺=1063.4 |
| | | ¹H NMR (400 MHz, Chloroform-*d*) δ 9.32 (s, 1H), 8.85 (d, *J* = 2.3 Hz, 1H), 8.64 (d, *J* = 5.9 Hz, 1H), 8.13 (dd, *J* = 8.2, 2.5 Hz, 2H), 7.67 - 7.60 (m, 2H), 7.43 (dd, *J* = 3.5, 2.5 Hz, 2H), 7.17 (dd, *J* = 5.7, 1.7 Hz, 2H), 7.08 (d, *J* = 7.4 Hz, 1H), 6.87 (s, 1H), 6.77 - 6.74 (m, 2H), 6.51 (dd, *J* = 3.5, 2.0 Hz, 1H), 6.02 (d, *J* = 7.0 Hz, 1H), 3.90 - 3.81 (m, 11H), 3.73 - 3.65 (m, 3H), 3.57 - 3.45 (m, 5H), 2.77 - 2.40 (m, 5H), 2.30 - 2.20 (m, 2H), 1.95 - 1.81 (m, 6H), 1.37 - 1.29 (m, 2H), 1.24 - 1.18 (m, 7H), 1.15 - 1.02 (m, 5H). |
| 199 | 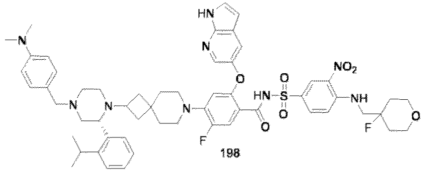 | ESI-MS(M+H)⁺=1046.5 |
| 200 | 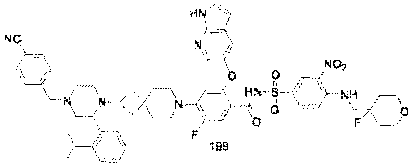 | ESI-MS(M+H)⁺=1028.4 |
| 201 | 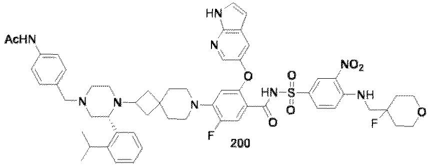 | ESI-MS(M+H)⁺=1060.4 |
| 202 | 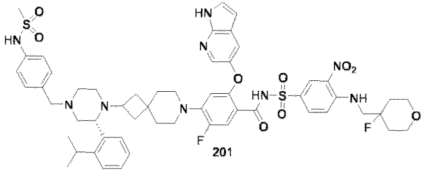 | ESI-MS(M+H)⁺=1096.4 |
| 203 | 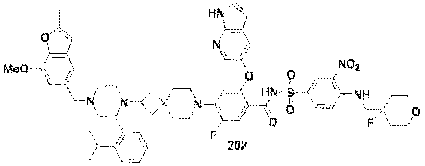 | ESI-MS(M+H)⁺=1087.4 |
| 204 | 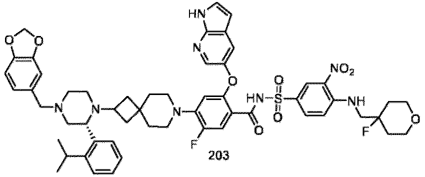 | ESI-MS(M+H)⁺=1047.4 |
| 205 | 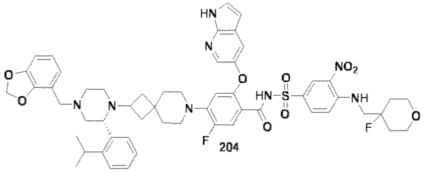 | ESI-MS(M+H)⁺=1047.4 |
| 206 | 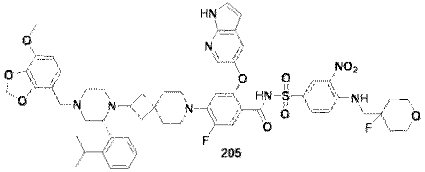 | ESI-MS(M+H)⁺=1077.4 |
| 207 | 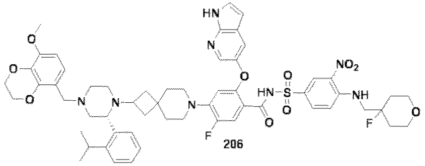 | ESI-MS(M+H)⁺=1091.4 |
| 208 | 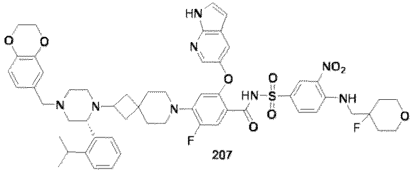 | ESI-MS(M+H)⁺=1061.4 |
| 209 | 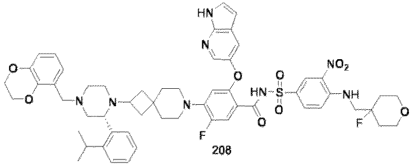 | ESI-MS(M+H)⁺=1061.4 |
| 210 | 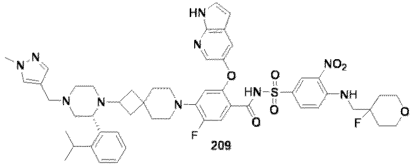 | ESI-MS(M+H)⁺=1007.4 |
| | | ¹H NMR (400 MHz, Chloroform-*d*) δ 9.38 (s, 1H), 8.85 (d, *J* = 2.2 Hz, 1H), 8.65 (t, *J* = 5.9 Hz, 1H), 8.12 (dd, *J* = 9.1, 2.3 Hz, 2H), 7.67 - 7.60 (m, 2H), 7.47 - 7.38 (m, 2H), 7.35 (s, 1H), 7.23 - 7.17 (m, 2H), 7.09 (d, *J* = 7.4 Hz, 1H), 6.95 (d, *J* = 9.3 Hz, 1H), 6.51 (d, *J* = 3.5 Hz, 1H), 6.02 (d, *J* = 7.0 Hz, 1H), 3.88 - 3.82 (m, 6H), 3.73 - 3.53 (m, 6H), 3.45 - 3.30 (m, 2H), 2.95 - 2.76 (m, 7H), 2.27 - 2.10 (m, 3H), 1.98 - 1.80 (m, 6H), 1.24 - 1.14 (m, 13H). |
| 211 | 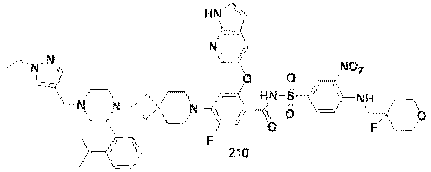 | ESI-MS(M+H)⁺=1035.5 |
| 212 | 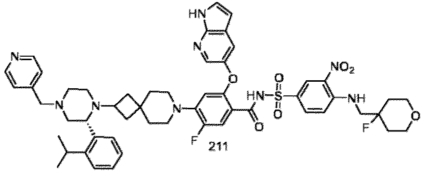 | ESI-MS(M+H)⁺=1004.4 |
| 213 | 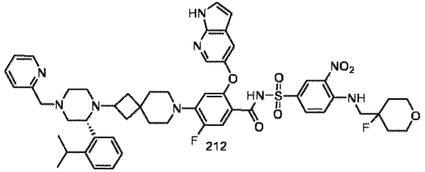 | ESI-MS(M+H)⁺=1004.4 |
| 214 | 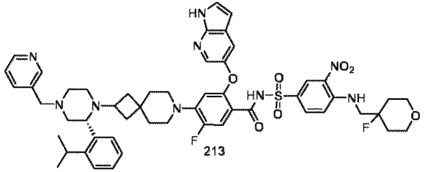 | ESI-MS(M+H)⁺=1004.4 |
| 215 | 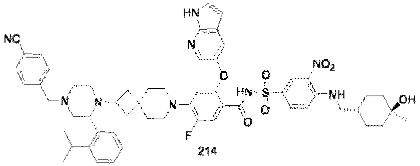 | ESI-MS(M+H)⁺=1038.5 |
| 216 | 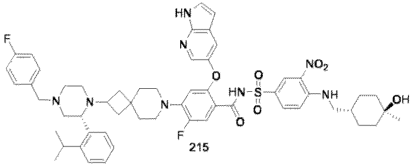 | ESI-MS(M+H)⁺=1031.5 |
| 217 | 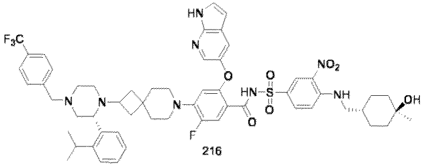 | ESI-MS(M+H)⁺=1081.5 |
| 218 | 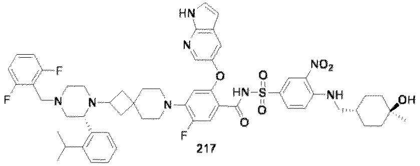 | ESI-MS(M+H)⁺=1049.4 |
| 219 | 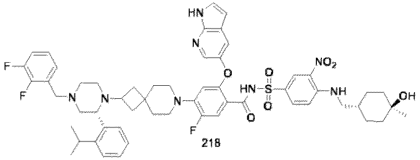 | ESI-MS(M+H)⁺=1049.4 |
| 220 | 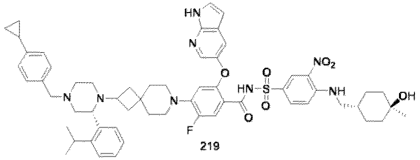 | ESI-MS(M+H)⁺=1053.5 |
| 221 | 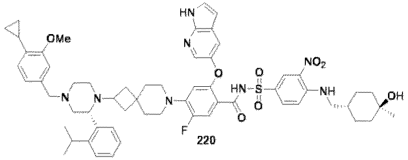 | ESI-MS(M+H)⁺=1083.5 |
| 222 | 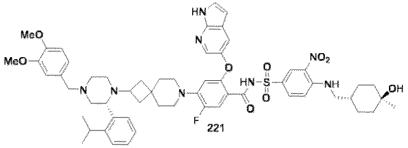 | ESI-MS(M+H)⁺=1073.5 |
| 223 | 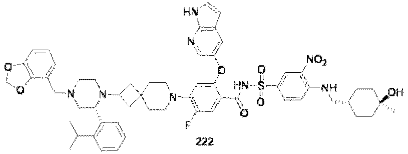 | ESI-MS(M+H)⁺=1057.5 |
| 224 | 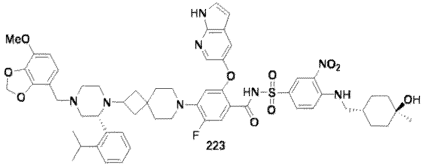 | ESI-MS(M+H)⁺=1087.5 |
| 225 | 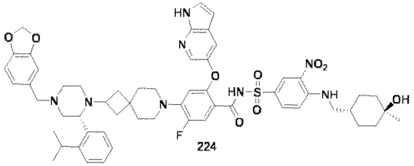 | ESI-MS(M+H)⁺=1057.5 |
| 226 | 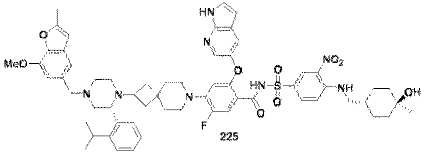 | ESI-MS(M+H)⁺=1097.5 |
| 227 | 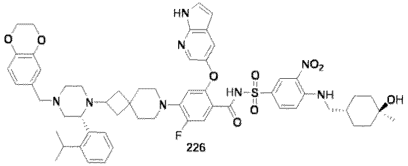 | ESI-MS(M+H)⁺=1071.5 |
| 228 | 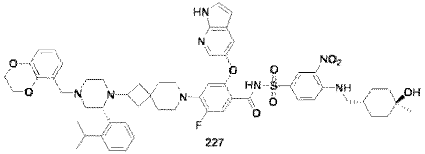 | ESI-MS(M+H)⁺=1071.5 |
| 229 | 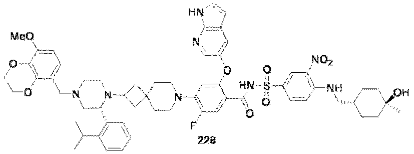 | ESI-MS(M+H)⁺=1101.5 |
| 230 | 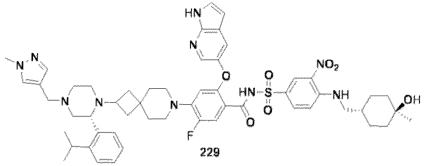 | ESI-MS(M+H)⁺=1017.5 |
| 231 | 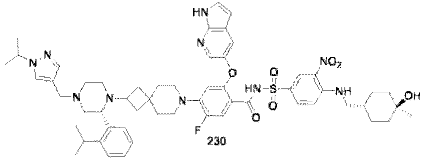 | ESI-MS(M+H)⁺=1045.5 |
| 232 | 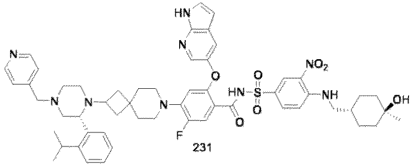 | ESI-MS(M+H)⁺=1014.5 |
| 233 | 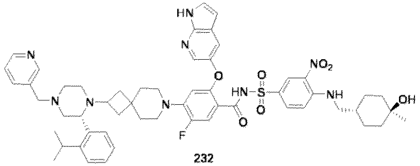 | ESI-MS(M+H)⁺=1014.5 |
| 234 | 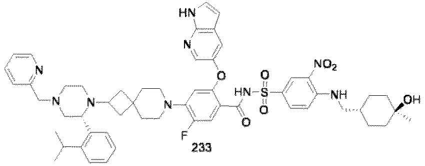 | ESI-MS(M+H)⁺=1014.5 |
| 235 | 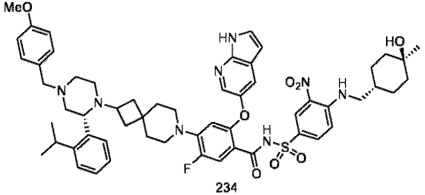 | ESI-MS(M+H)⁺=1043.5 |
| | | ¹H NMR (400 MHz, Chloroform-*d*) δ 9.77 (s, 1H), 8.74 (d, *J* = 2.2 Hz, 1H), 8.41 (t, *J* = 5.4 Hz, 1H), 8.18 - 8.11 (m, 2H), 8.07 (d, *J* = 2.6 Hz, 1H), 8.02 (dd, *J* = 9.1, 2.3 Hz, 1H), 7.67 (d, *J* = 13.8 Hz, 1H), 7.46 (d, *J* = 2.6 Hz, 2H), 7.37 (dd, *J* = 3.5, 2.0 Hz, 1H), 7.18 (s, 1H), 7.13 - 7.05 (m, 1H), 6.85 - 6.73 (m, 2H), 6.51 - 6.44 (m, 1H), 6.40 (dd, *J* = 3.4, 1.5 Hz, 1H), 6.18 (d, *J* = 7.1 Hz, 1H), 3.77 (s, 4H), 3.62 (s, 1H), 3.51-3.40 (m, 3H), 3.32 (s, 1H), 3.20 (t, *J* = 6.1 Hz, 3H), 3.03 - 2.97 (m, 2H), 2.94 - 2.90 (m, 3H), 2.85 - 2.78 (m, 3H), 2.75 (s, 1H), 2.64 - 2.58 (m, 2H), 2.31 - 2.19 (m, 3H), 2.15 - 2.12 (s, 1H), 1.84 - 1.81 (m, 2H), 1.79 - 1.60 (m, 2H), 1.56 - 1.36 (m, 2H), 1.26-1.21 (m, 4H), 1.19 - 1.13 (m, 4H), 1.11 - 1.02 (m, 7H). |
| 236 | 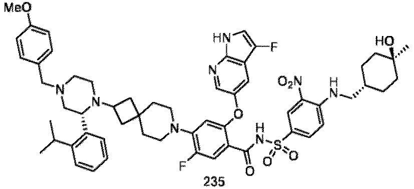 | ESI-MS(M+H)⁺=1061.5 |
| 237 | 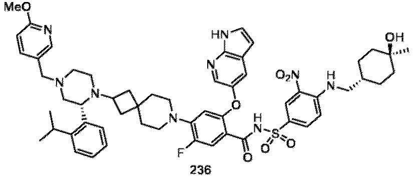 | ESI-MS(M+H)⁺=1044.5 |
| 238 | 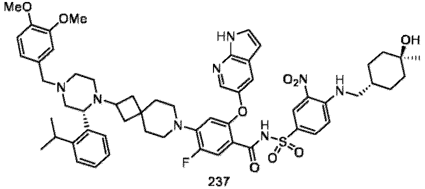 | ESI-MS(M+H)⁺=1073.5 |
| 239 | 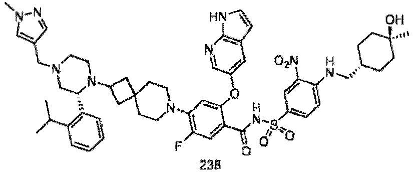 | ESI-MS(M+H)⁺=1017.5 |
| 240 | 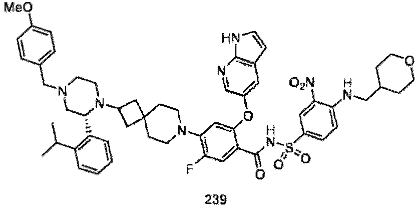 | ESI-MS(M+H)⁺=1015.4 |
| | | ¹H NMR (400 MHz, Chloroform-*d*) δ 9.32 (s, 1H), 8.84 (d, *J* = 2.3 Hz, 1H), 8.51 (t, *J* = 5.4 Hz, 1H), 8.12 (dd, *J* = 9.5, 2.4 Hz, 2H), 7.67 - 7.60 (m, 2H), 7.44 (t, *J* = 3.0 Hz, 2H), 7.21 - 7.12 (m, 4H), 7.08 (d, *J* = 7.3 Hz, 1H), 6.89 (d, *J* = 9.3 Hz, 1H), 6.82 - 6.76 (m, 2H), 6.51 (dd, *J* = 3.5, 1.9 Hz, 1H), 6.02 (d, *J* = 7.0 Hz, 1H), 4.02 - 3.99 (m, 2H), 3.76 - 3.68 (m, 4H), 3.63 (s, 1H), 3.50 - 3.33 (m, 2H), 3.35 - 3.17 (m, 4H), 3.03 - 2.57 (m, 3H), 2.31-2.08 (m, 2H), 2.05 - 1.87 (m, 4H), 1.79 - 1.54 (m, 6H), 1.49 - 1.29 (m, 4H), 1.17 - 1.11 (m, 5H), 1.09 - 1.01 (m, 6H). |
| 241 | 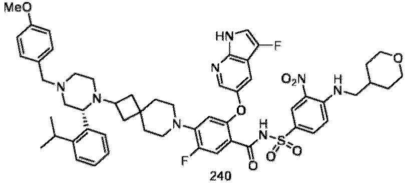 | ESI-MS(M+H)⁺=1033.4 |
| 242 | 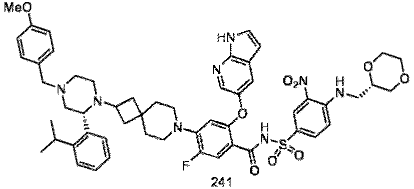 | ESI-MS(M+H)⁺=1017.4 |
| | | ¹H NMR (400 MHz, Chloroform-*d*) δ 9.52 (s, 1H), 8.82 (d, *J* = 2.3 Hz, 1H), 8.58 (q, *J* = 8.8, 7.0 Hz, 1H), 8.15 - 8.07 (m, 2H), 7.69 - 7.56 (m, 2H), 7.42 (t, *J* = 3.0 Hz, 2H), 7.20 - 7.16 (m, 4H), 7.08 (d, *J* = 7.2 Hz, 1H), 6.85 - 6.78 (m, 3H), 6.54 - 6.45 (m, 1H), 6.03 (d, *J* = 7.0 Hz, 1H), 3.85 - 3.78 (m, 3H), 3.76 - 3.68 (m, 5H), 3.64 - 3.58 (m, 2H), 3.51 - 3.44 (m, 3H), 3.34 - 3.18 (m, 3H), 2.89 - 2.85 (m, 3H), 2.83 - 2.70 (m, 5H), 2.27 - 2.09 (m, 2H), 1.69 - 1.59 (m, 2H), 1.38 - 1.29 (m, 4H), 1.24 - 1.19 (m, 2H), 1.17 - 1.09 (m, 7H). |
| 243 | 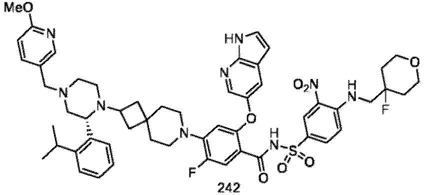 | ESI-MS(M+H)⁺=1034.4 |
| | | ¹H NMR (400 MHz, Chloroform-*d*) δ 9.35 (s, 1H), 8.85 (d, *J* = 2.2 Hz, 1H), 8.65 (t, *J* = 5.8 Hz, 1H), 8.13 (dt, *J* = 5.9, 2.7 Hz, 2H), 7.98 (d, *J* = 2.3 Hz, 1H), 7.68 - 7.60 (m, 2H), 7.56 - 7.50 (m, 1H), 7.43 (dd, *J* = 3.5, 2.4 Hz, 2H), 7.21 - 7.15 (m, 2H), 7.08 (s, 1H), 6.96 (d, *J* = 9.3 Hz, 1H), 6.66 (dd, *J* = 8.5, 0.7 Hz, 1H), 6.51 (dd, *J* = 3.5, 2.0 Hz, 1H), 6.01 (d, *J* = 7.0 Hz, 1H), 3.90 - 3.84 (m, 6H), 3.78 - 3.67 (m, 3H), 3.53 - 3.45 (m, 3H), 3.42 - 3.30 (m, 2H), 2.87 - 2.77 (m, 4H), 2.73 - 2.65 (m, 2H), 2.60 - 1.82 (m, 7H), 1.42 - 1.34 (m, 4H), 1.27 - 1.23 (m, 5H), 1.17 - 1.09 (m, 6H). |
| 244 | 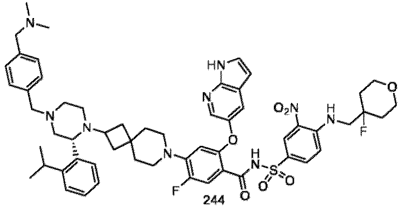 | ESI-MS(M+H)⁺=1060.5 |
| 245 | 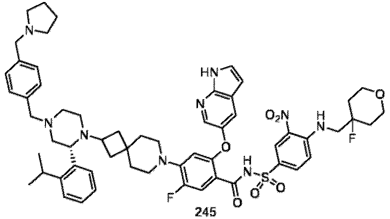 | ESI-MS(M+H)⁺=1086.5 |
| 246 | 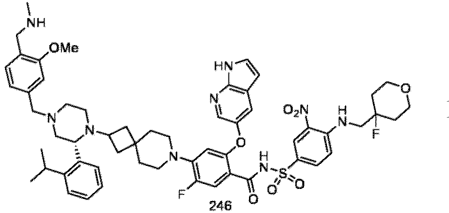 | ESI-MS(M+H)⁺=1076.5 |
| 247 | 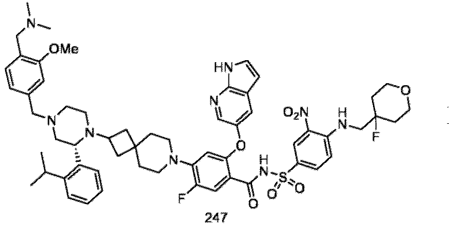 | ESI-MS(M+H)⁺=1090.5 |
| 248 | 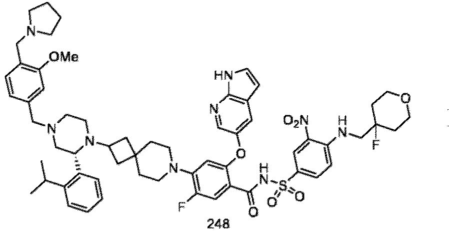 | ESI-MS(M+H)⁺=1116.5 |
| 249 | 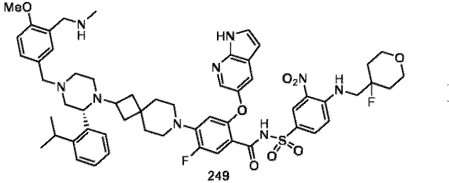 | ESI-MS(M+H)⁺=1076.5 |
| 250 | 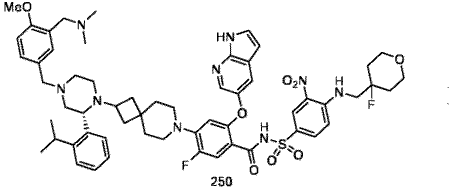 | ESI-MS(M+H)⁺=1090.5 |
| 251 | 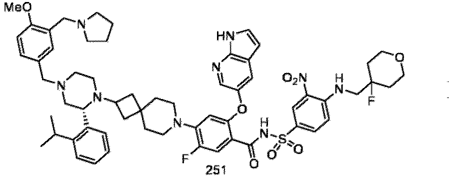 | ESI-MS(M+H)⁺=1116.5 |
| 252 | 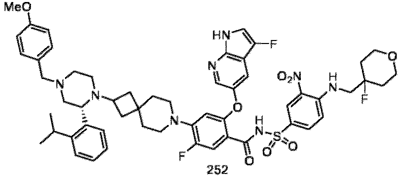 | ESI-MS(M+H)⁺=1051.4 |
| 253 | 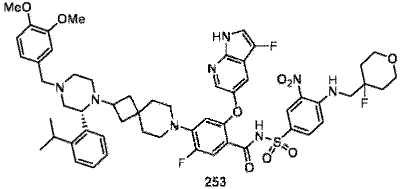 | ESI-MS(M+H)⁺=1081.4 |
| 254 | 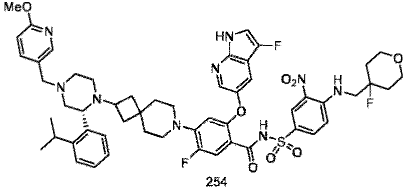 | ESI-MS(M+H)⁺=1052.4 |
| 255 | 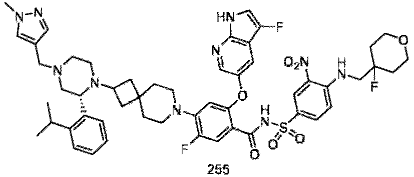 | ESI-MS(M+H)⁺=1025.4 |
| 256 | 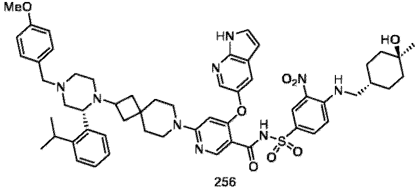 | ESI-MS(M+H)⁺=1026.5 |
| | | ¹H NMR (400 MHz, Chloroform-d) δ 9.01 (s, 1H), 8.88 (s, 1H), 8.74 (s, 1H), 8.53 (s, 1H), 8.20 (d, J = 9.0 Hz, 1H), 8.15 (s, 1H), 7.72 (s, 1H), 7.45 (s, 2H), 7.18 -7.11 (m, 6H), 7.07 (s, 2H), 6.93 (d, J = 9.3 Hz, 1H), 6.80 (d, J = 8.1 Hz, 3H), 6.57 (s, 1H), 5.48 (s, 1H), 5.34 (s, 2H), 3.76 - 3.68 (m, 4H), 3.46 - 3.39 (m, 3H), 3.31 - 3.13 (m, 4H), 2.88 - 2.79 (m, 4H), 2.64 - 2.61 (m, 1H), 2.21 - 2.11 (m, 4H), 2.00 - 1.91 (m, 4H), 1.86 - 1.81 (m, 3H), 1.74 - 1.61 (m, 3H), 1.29 - 1.20 (m, 3H), 1.10-1.01 (m, 8H). |
| 257 | 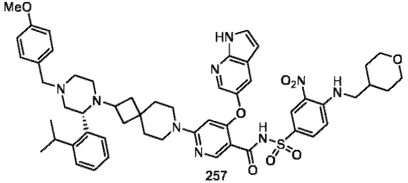 | ESI-MS(M+H)⁺=998.5 |
| | | ¹H NMR (400 MHz, Chloroform-*d*) δ 9.34 (s, 1H), 8.89 (d, *J* = 2.3 Hz, 1H), 8.74 (s, 1H), 8.53 (t, *J* = 5.4 Hz, 1H), 8.21 (dd, *J* = 9.2, 2.3 Hz, 1H), 8.15 (d, *J* = 2.6 Hz, 1H), 7.72 (d, *J* = 2.6 Hz, 1H), 7.50 - 7.37 (m, 3H), 7.17 (d, *J* = 7.5 Hz, 3H), 7.07 (d, *J* = 7.2 Hz, 1H), 6.94 (d, *J* = 9.3 Hz, 1H), 6.79 (d, *J* = 8.2 Hz, 3H), 6.56 (t, *J* = 2.8 Hz, 1H), 5.49 (s, 1H), 5.34 (t, *J* = 4.8 Hz, 1H), 4.02 (dd, *J* = 11.6, 4.4 Hz, 3H), 3.76 (s, 2H), 3.62 (d, *J* = 14.2 Hz, 2H), 3.50 - 3.36 (m, 4H), 3.35 -3.19 (m, 2H), 3.16 (s, 3H), 2.99 - 2.78 (m, 3H), 2.63 - 2.52 (m, 2H), 2.26 - 2.17 (m, 2H), 1.99 - 1.87 (m, 5H), 1.73 - 1.66 (m, 2H), 1.62 - 1.50 (m, 3H), 1.17 - 1.11 (m, 2H), 1.09 - 1.02 (m, 6H). |
| 258 | 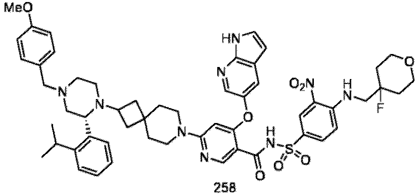 | ESI-MS(M+H)⁺=1016.4 |
| | | ¹H NMR (400 MHz, Chloroform-*d*) δ 9.52 (s, 1H), 8.90 (d, *J* = 2.3 Hz, 1H), 8.74 (s, 1H), 8.68 (t, *J* = 5.7 Hz, 1H), 8.21 (dd, *J* = 9.1, 2.6 Hz, 1H), 8.15 (d, *J* = 2.5 Hz, 1H), 7.72 (d, *J* = 2.5 Hz, 1H), 7.47 (dd, *J* = 3.6, 2.5 Hz, 2H), 7.22 - 7.13 (m, 3H), 7.08 (t, *J* = 7.2 Hz, 1H), 7.00 (d, *J* = 9.3 Hz, 1H), 6.84 - 6.76 (m, 2H), 6.56 (s, 1H), 3.76 - 3.66 (m, 4H), 3.59 - 3.41 (m, 6H), 3.20 - 2.98 (m, 6H), 2.88 - 2.71 (m, 3H), 2.62 - 2.55 (m, 4H), 2.35 - 1.95 (m, 4H), 1.91 - 1.52 (m, 4H), 1.39 - 1.21 (m, 4H), 1.18 - 1.13 (m, 4H), 1.10 - 1.01 (m, 5H). |
| 259 | 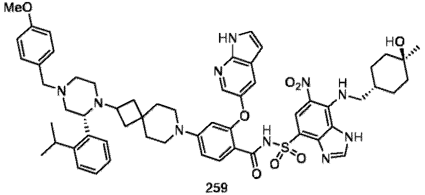 | ESI-MS(M+H)⁺=1065.5 |
| | | ¹H NMR (400 MHz, DMSO-*d*₆) δ 11.64 (s, 1H), 9.35 (t, *J* = 6.2 Hz, 1H), 8.42 (s, 1H), 8.13 (s, 1H), 7.98 (s, 1H), 7.61 - 7.34 (m, 2H), 7.30 - 7.02 (m, 2H), 6.87 (d, *J* = 8.1 Hz, 3H), 6.59 (d, *J* = 9.1 Hz, 1H), 6.34 (s, 1H), 6.10 (s, 1H), 4.20 (dd, *J* = 17.2, 10.7 Hz, 4H), 3.71 - 3.65 (m, 2H), 3.59 - 3.51 (m, 2H), 2.89 - 2.86 (m, 2H), 2.67 - 2.63 (m, 2H), 2.50 - 2.44 (m, 2H), 1.71 - 1.67 (m, 3H), 1.53 - 1.46 (m, 2H), 1.40 - 1.27 (m, 4H), 1.23 - 1.19 (m, 2H), 1.17 - 1.13 (m, 8H), 1.08 - 1.04 (m, 8H), 1.02 - 0.93 (m, 10H). |
| 260 | 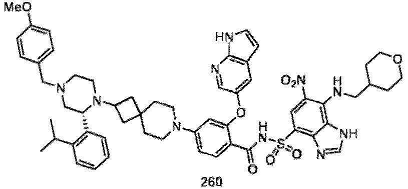 | ESI-MS(M+H)⁺=1037.5 |
| | | ¹H NMR (400 MHz, Chloroform-*d*) δ 11.42 (s, 1H), 10.01 (s, 1H), 9.46 (t, *J* = 6.1 Hz, 1H), 8.75 (s, 1H), 8.12 (d, *J* = 2.6 Hz, 1H), 7.97 (s, 1H), 7.82 (d, *J* = 9.2 Hz, 1H), 7.61 (d, *J* = 2.6 Hz, 1H), 7.50 - 7.35 (m, 2H), 7.17 (tt, *J* = 5.5, 2.3 Hz, 3H), 7.11 - 7.00 (m, 1H), 6.83 - 6.73 (m, 2H), 6.53 - 6.42 (m, 2H), 5.88 (d, *J* = 2.3 Hz, 1H), 4.24 - 4.13 (m, 2H), 4.00 - 3.91 (m, 2H), 3.75 (s, 2H), 3.62 (s, 1H), 3.52 - 3.34 (m, 5H), 3.29 (s, 1H), 3.04 - 2.75 (m, 4H), 2.64 - 2.51 (m, 2H), 2.19 - 212 (m, 4H), 1.99 - 1.92 (m, 2H), 1.84 - 1.75 (m, 2H), 1.45 - 1.39 (m, 3H), 1.36 - 1.20 (m, 3H), 1.16 - 1.11 (m, 4H), 1.08 - 1.01 (m, 6H). |
| 261 | 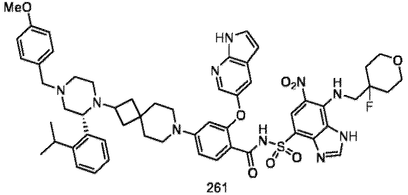 | ESI-MS(M+H)⁺=1055.5 |
| | | ¹H NMR (400 MHz, DMSO-*d*₆) δ 11.62 (s, 1H), 9.35 (t, *J* = 6.3 Hz, 1H), 8.43 (s, 1H), 8.15 (s, 1H), 7.96 (d, *J* = 2.5 Hz, 1H), 7.57 (d, *J* = 8.8 Hz, 1H), 7.46 - 7.42 (m, 2H), 7.23 (d, *J* = 8.4 Hz, 4H), 7.13 (s, 1H), 6.87 (d, J= 8.2 Hz, 2H), 6.59 (dd, *J* = 9.0, 2.3 Hz, 1H), 6.36 - 6.28 (m, 1H), 6.11 (d, *J* = 2.3 Hz, 1H), 4.77 (dd, *J* = 21.7, 6.3 Hz, 2H), 3.71 - 3.59 (m, 9H), 3.53 - 3.38 (m, 5H), 2.88 - 7.05 (m, 6H), 2.69 - 2.67 (m, 1H), 1.91 - 1.68 (m, 5H), 1.61 - 1.59 (m, 1H), 1.30 - 1.13 (m, 10H), 1.04 - 0.96 (m, 4H). |
| 262 | 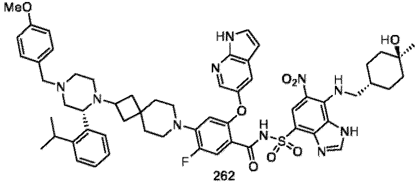 | ESI-MS(M+H)⁺=1083.5 |
| | | ¹H NMR (400 MHz, DMSO-*d*₆) δ 11.55 (s, 1H), 9.31 (t, *J* = 6.3 Hz, 1H), 8.30 (s, 1H), 8.04 (s, 1H), 7.89 (s, 1H), 7.43 (d, *J* = 12.9 Hz, 5H), 7.35 - 7.19 (m, 8H), 7.16 (s, 2H), 6.89 (d, *J* = 8.0 Hz, 3H), 6.28 (d, *J* = 7.3 Hz, 3H), 4.24 (s, 2H), 4.17 (t, *J* = 6.4 Hz, 3H), 3.71 - 3.58 (m, 5H), 3.04 - 2.91 (m, 5H), 2.67 - 2.48 (m, 4H), 1.67 - 1.58 (m, 4H), 1.53 - 1.49 (m, 2H), 1.33 - 1.21 (m, 4H), 1.16 - 1.10 (m, 4H), 1.06 - 1.01 (m, 6H). |
| 263 | 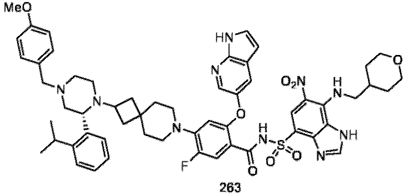 | ESI-MS(M+H)⁺=1055.5 |
| | | ¹H NMR (400 MHz, Chloroform-*d*) δ 11.22 (s, 1H), 9.55 (s, 1H), 9.46 (t, *J* = 6.1 Hz, 1H), 8.72 (s, 1H), 8.11 (d, *J* = 2.6 Hz, 1H), 7.95 (s, 1H), 7.66 - 7.52 (m, 3H), 7.51 - 7.35 (m, 2H), 7.22-7.13 (m, 2H), 7.08 (s, 1H), 6.86 - 6.75 (m, 3H), 6.47 (dd, *J* = 3.5, 1.9 Hz, 1H), 6.01 (d, *J* = 7.0 Hz, 1H), 4.24 (t, *J* = 6.5 Hz, 2H), 4.05 - 3.95 (m, 2H), 3.75 - 3.71 (m, 2H), 3.52 (s, 1H), 3.27 - 3.13 (m, 2H), 2.95 - 2.90 (m, 1H), 2.86 - 2.78 (m, 2H), 2.72 - 2.68 (m, 2H), 2.32 - 2.30 (m, 2H), 2.27 - 213 (m, 3H), 2.02 - 1.91 (m, 2H), 1.65 - 1.61 (m, 2H), 1.51 - 1.39 (m, 3H), 1.35 - 1.33 (m, 2H), 1.29 - 1.19 (m, 4H), 1.15 - 1.11 (m, 4H), 1.08 - 1.02 (m, 6H). |
| 264 | 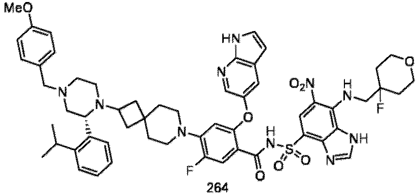 | ESI-MS(M+H)⁺=1073.4 |
| | | ¹H NMR (400 MHz, DMSO-*d*₆) δ 11.54 (s, 1H), 9.31 (t, *J* = 6.3 Hz, 1H), 8.32 (s, 1H), 8.08 (s, 1H), 7.91 -7.83 (m, 1H), 7.42 (s, 3H), 7.28 (s, 5H), 6.91 (d, *J* = 8.1 Hz, 2H), 6.33 - 6.22 (m, 2H), 4.75 (dd, *J* = 21.9, 6.3 Hz, 2H), 3.71 - 3.62 (m, 8H), 3.57 - 3.47 (m, 4H), 2.67 - 2.58 (m, 6H), 1.89 - 1.69 (m, 6H), 1.38 - 1.29 (m, 5H), 1.21 - 1.13 (m, 7H), 1.06 - 0.94 (m, 5H). |
| 265 | 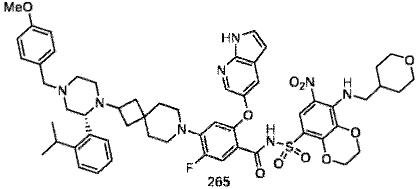 | ESI-MS(M+H)⁺=1073.5 |
| | | ¹H NMR (400 MHz, Chloroform-*d*) δ 9.77 (s, 1H), 8.65 (s, 1H), 8.52 (s, 1H), 8.10 (d, *J* = 2.6 Hz, 1H), 7.69 - 7.58 (m, 2H), 7.49 - 7.39 (m, 2H), 7.18 - 7.15 (m, 3H), 7.07 (d, *J* = 7.5 Hz, 1H), 6.79 (d, *J* = 8.2 Hz, 2H), 6.53 - 6.47 (s, 1H), 6.08 (d, *J* = 7.0 Hz, 1H), 4.09 (d, *J* = 4.5 Hz, 2H), 4.04 - 3.95 (m, 4H), 3.75 - 3.69 (m, 3H), 3.55 - 3.48 (m, 2H), 3.45 - 3.40 (m, 2H), 3.37 - 3.21 (m, 3H), 2.84 - 2.80 (m, 4H), 2.76 - 2.70 (m, 2H), 2.65 (s, 1H), 2.28 - 2.21 (m, 2H), 2.15 - 2.01 (m, 1H), 1.70 - 1.60 (m, 4H), 1.42 - 1.32 (m, 6H), 1.30 - 1.22 (m, 5H), 1.17 - 1.09 (m, 7H). |
| 266 | 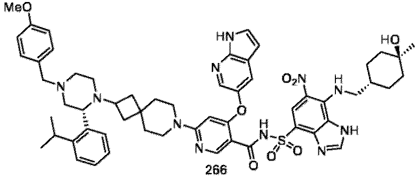 | ESI-MS(M+H)⁺=1066.5 |
| | | ¹H NMR (400 MHz, DMSO-*d*₆) δ 11.74 (s, 1H), 9.34 (d, *J* = 6.4 Hz, 1H), 8.40 (s, 2H), 8.05 (d, *J* = 52.4 Hz, 2H), 7.75 - 7.33 (m, 3H), 7.34 - 7.01 (m, 7H), 6.89 (d, *J* = 8.1 Hz, 3H), 6.40 (s, 1H), 5.67 (s, 1H), 4.20 (dd, *J* = 13.3, 7.1 Hz, 4H), 3.71-3.68 (m, 6H), 3.07 - 2.91 (m, 4H), 1.62 - 1.51 (m, 10H), 1.38 - 1.26 (m, 5H), 1.17 -1.10 (m, 4H), 1.08 -1.01 (m, 11H). |
| 267 | 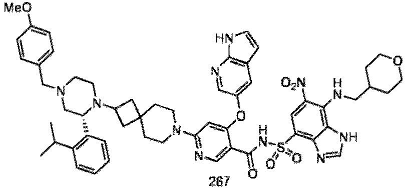 | ESI-MS(M+H)⁺=1038.5 |
| | | ¹H NMR (400 MHz, Chloroform-*d*) δ 11.20 (s, 1H), 9.49 (t, *J* = 6.2 Hz, 1H), 9.19 (s, 1H), 8.77 (s, 1H), 8.68 (s, 1H), 8.15 (d, *J* = 2.5 Hz, 1H), 8.01 (s, 1H), 7.71 (d, *J* = 2.5 Hz, 1H), 7.50 - 7.37 (m, 2H), 7.21 - 7.11 (m, 2H), 7.07 (d, *J* = 7.3 Hz, 1H), 6.83 - 6.76 (m, 2H), 6.55 (dd, *J* = 3.5, 2.0 Hz, 1H), 5.48 (s, 1H), 5.34 (t, *J* = 4.7 Hz, 2H), 4.26 (t, *J* = 6.5 Hz, 2H), 4.00 (dt, *J* = 11.6, 2.9 Hz, 2H), 3.76 (s, 4H), 3.61 (s, 1H), 3.50 - 3.35 (m, 2H), 3.20 - 3.09 (m, 3H), 2.97 - 2.76 (m, 4H), 2.63 (d, *J* = 11.6 Hz, 1H), 2.27 - 2.16 (m, 2H), 2.05 - 1.94 (m, 2H), 1.77 - 1.70 (m, 3H), 1.63 - 1.59 (m, 2H), 1.51 - 1.40 (m, 2H), 1.29 - 1.21 (m, 3H), 1.17 - 1.11 (m, 3H), 1.09 - 1.01 (m, 6H). |
| 268 | 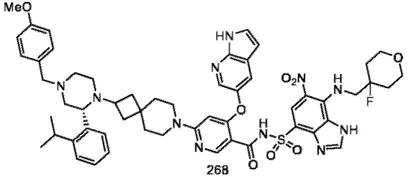 | ESI-MS(M+H)⁺=1056.4 |
| | | ¹H NMR (400 MHz, DMSO-*d*₆) δ 11.73 (s, 1H), 9.34 (s, 1H), 8.41 (s, 2H), 8.15 (s, 1H), 7.97 (s, 1H), 7.51 (t, *J* = 38.8 Hz, 4H), 7.14 (s, 2H), 6.89 (d, *J* = 8.0 Hz, 3H), 6.39 (s, 1H), 5.68 (s, 1H), 4.77 (dd, *J* = 21.8, 6.3 Hz, 2H), 3.72 - 3.63 (m, 9H), 3.57 - 3.47 (m, 6H), 3.11 - 3.02 (m, 7H), 1.78 - 1.63 (m, 6H), 1.18 - 1.09 (m, 7H), 1.05 - 0.93 (m, 6H). |

### Embodiment 269 Molecular level activity test of anti-apoptotic protein BCL2

The detection of the binding ability between anti-apoptotic protein BCL2 and pro-apoptotic protein Bim was performed using Homogeneous Time-Resolved Fluorescence technology. The reaction of this method occurred in a white shallow 384-well plate, with a total reaction volume of 10 µL. Specifically, it included 2 µL of the compound to be tested (2% DMSO), 4 µL His-tagged recombinant protein, and 4 µL Biotin-tagged BIM protein peptide. After 1 hour of reaction, 5 µL of each Anti-His and streptavidin-tagged XI,665 antibody diluents diluted with a detection buffer were added. After incubating at room temperature for 4 hours, the values were read using the Envision multifunctional microplate reader to detect the effect of the compound to be tested on the binding ability of BCL2 to Bim protein peptides. The Envision parameter settings were exciting light at 320 nm, and emitting light at 615 nm and 665 nm. The binding ability of anti-apoptotic proteins to Bim protein peptides was indirectly reflected by the signal ratio of 665 nm and 615 nm. In the reaction, background wells without BCL2 and active wells for full binding of recombinant proteins without compounds and Bim protein peptides were set up.

The IC₅₀ value of the compounds inhibiting the binding ability of anti-apoptotic proteins to Bim protein peptides was calculated using Graphpad Prism 7.00 software through the formula Y=100/(1+10^((LogIC50-X)*HillSlope)).

**Table 1 Inhibitory activity of the compounds of the present invention on BCL2**

| **Compo und number** | **BCL2 nM** | **Compo und number** | **BCL2 nM** | **Compo und number** | **BCL2 nM** | **Compo und number** | **BCL2 nM** |
|---|---|---|---|---|---|---|---|
| 001 | + | 068 | + | 135 | + | 202 | + |
| 002 | + | 069 | + | 136 | + | 203 | + |
| 003 | + | 070 | + | 137 | + | 204 | + |
| 004 | + | 071 | + | 138 | + | 205 | + |
| 005 | + | 072 | + | 139 | + | 206 | + |
| 006 | + | 073 | + | 140 | + | 207 | + |
| 007 | + | 074 | + | 141 | + | 208 | + |
| 008 | + | 075 | + | 142 | + | 209 | + |
| 009 | + | 076 | + | 143 | + | 210 | + |
| 010 | + | 077 | + | 144 | + | 211 | + |
| 011 | + | 078 | + | 145 | + | 212 | + |
| 012 | + | 079 | + | 146 | + | 213 | + |
| 013 | + | 080 | + | 147 | + | 214 | + |
| 014 | + | 081 | + | 148 | + | 215 | + |
| 015 | + | 082 | + | 149 | + | 216 | + |
| 016 | + | 083 | + | 150 | + | 217 | + |
| 017 | + | 084 | + | 151 | + | 218 | + |
| 018 | + | 085 | + | 152 | + | 219 | + |
| 019 | + | 086 | + | 153 | + | 220 | + |
| 020 | + | 087 | + | 154 | + | 221 | + |
| 021 | + | 088 | + | 155 | + | 222 | + |
| 022 | + | 089 | + | 156 | + | 223 | + |
| 023 | + | 090 | + | 157 | + | 224 | + |
| 024 | + | 091 | + | 158 | + | 225 | + |
| 025 | + | 092 | + | 159 | + | 226 | + |
| 026 | + | 093 | + | 160 | + | 227 | + |
| 027 | + | 094 | + | 161 | + | 228 | + |
| 028 | + | 095 | + | 162 | + | 229 | + |
| 029 | + | 096 | + | 163 | + | 230 | + |
| 030 | + | 097 | + | 164 | + | 231 | + |
| 031 | + | 098 | + | 165 | + | 232 | + |
| 032 | + | 099 | + | 166 | + | 233 | + |
| 033 | + | 100 | + | 167 | + | 234 | + |
| 034 | + | 101 | + | 168 | + | 235 | + |
| 035 | + | 102 | + | 169 | + | 236 | + |
| 036 | + | 103 | + | 170 | + | 237 | + |
| 037 | + | 104 | + | 171 | + | 238 | + |
| 038 | + | 105 | + | 172 | + | 239 | + |
| 039 | + | 106 | + | 173 | + | 240 | + |
| 040 | + | 107 | + | 174 | + | 241 | + |
| 041 | + | 108 | + | 175 | + | 242 | + |
| 042 | + | 109 | + | 176 | + | 243 | + |
| 043 | + | 110 | + | 177 | + | 244 | + |
| 044 | + | 111 | + | 178 | + | 245 | + |
| 045 | + | 112 | + | 179 | + | 246 | + |
| 046 | + | 113 | + | 180 | + | 247 | + |
| 047 | + | 114 | + | 181 | + | 248 | + |
| 048 | + | 115 | + | 182 | + | 249 | + |
| 049 | + | 116 | + | 183 | + | 250 | + |
| 050 | + | 117 | + | 184 | + | 251 | + |
| 051 | + | 118 | + | 185 | + | 252 | + |
| 052 | + | 119 | + | 186 | + | 253 | + |
| 053 | + | 120 | + | 187 | + | 254 | + |
| 054 | + | 121 | + | 188 | + | 255 | + |
| 055 | + | 122 | + | 189 | + | 256 | + |
| 056 | + | 123 | + | 190 | + | 257 | + |
| 057 | + | 124 | + | 191 | + | 258 | + |
| 058 | + | 125 | + | 192 | + | 259 | + |
| 059 | + | 126 | + | 193 | + | 260 | + |
| 060 | + | 127 | + | 194 | + | 261 | + |
| 061 | + | 128 | + | 195 | + | 262 | + |
| 062 | + | 129 | + | 196 | + | 263 | + |
| 063 | + | 130 | + | 197 | + | 264 | + |
| 064 | + | 131 | + | 198 | + | 265 | + |
| 065 | + | 132 | + | 199 | + | 266 | + |
| 066 | + | 133 | + | 200 | + | 267 | + |
| 067 | + | 134 | + | 201 | + | 268 | + |

| | | | | | | | |
|---|---|---|---|---|---|---|---|
| +: IC₅₀ < 10nM; ++: 10nM<IC₅₀<100nM; +++:100nM<IC₅₀<1000nM; ++++: 1000nM<IC₅₀ | | | | | | | |

The results in Table 1 indicated that the compounds of the present invention had strong BCL2 inhibitory activity.

### Embodiment 270 Molecular level activity test of anti-apoptotic protein BCL2 (G101V)

The detection of the binding ability between anti-apoptotic protein BCL2 (G101V) and pro-apoptotic protein Bim was performed using Homogeneous Time-Resolved Fluorescence technology. The reaction of this method occurred in a white shallow 384-well plate, with a total reaction volume of 10 µL. Specifically, it included 2 µL of the compound to be tested (2% DMSO), 4 µL His-tagged recombinant protein, and 4 µL Biotin-tagged BIM protein peptide. After 1 hour of reaction, 5 µL of each Anti-His and streptavidin-tagged XI,665 antibody diluents diluted with a detection buffer were added. After incubating at room temperature for 4 hours, the values were read using the Envision multifunctional microplate reader to detect the effect of the compound to be tested on the binding ability of BCL2 (G101V) to BIM protein peptides. The Envision parameter settings were exciting light at 320 nm, and emitting light at 615 nm and 665 nm. The binding ability of anti-apoptotic proteins to Bim protein peptides was indirectly reflected by the signal ratio of 665 nm and 615 nm. In the reaction, background wells without BCL2 and active wells for full binding of recombinant proteins without compounds and Bim protein peptides were set up.

The IC₅₀ value of the compounds inhibiting the binding ability of anti-apoptotic proteins to Bim protein peptides was calculated using Graphpad Prism 7.00 software through the formula Y=100/(1+10^((LogIC₅₀-X)*HillSlope)).

**Table 2 Inhibitory activity of the compounds of the present invention on BCL2 (G101V)**

| Compound number | BCL-2 (G101V) (nM) | Compound number | BCL-2 (G101V) (nM) |
|---|---|---|---|
| 002 | + | 242 | + |
| 019 | + | 243 | + |
| 053 | + | 244 | + |
| 058 | + | 245 | + |
| 062 | + | 246 | + |
| 063 | + | 247 | + |
| 120 | + | 248 | + |
| 145 | + | 249 | + |
| 146 | + | 250 | + |
| 152 | + | 251 | + |
| 184 | + | 252 | + |
| 185 | + | 253 | + |
| 186 | + | 254 | + |
| 189 | + | 255 | + |
| 190 | + | 256 | + |
| 196 | + | 257 | + |
| 197 | + | 258 | + |
| 203 | + | 259 | + |
| 209 | + | 260 | + |
| 234 | + | 261 | + |
| 235 | + | 262 | + |
| 236 | + | 263 | + |
| 237 | + | 264 | + |
| 238 | + | 265 | + |
| 239 | + | 266 | + |
| 240 | + | 267 | + |
| 241 | + | 268 | + |

| | | | |
|---|---|---|---|
| +: IC₅₀ < 10nM; ++: 10nM<IC₅₀<100nM; +++:100nM<IC₅₀<1000nM; ++++: 1000nM<IC₅₀ | | | |

The results in Table 2 indicated that the compounds of the present invention had strong BCL2 (G101V) inhibitory activity.

### Embodiment 271 Molecular level activity test of BCL2 (D103Y)

The detection of the binding ability between anti-apoptotic protein BCL2 (D103Y) and pro-apoptotic protein Bim was performed using Homogeneous Time-Resolved Fluorescence technology. The reaction of this method occurred in a white shallow 384-well plate, with a total reaction volume of 10 µL. Specifically, it included 2 µL of the compound to be tested (2% DMSO), 4 µL His-tagged recombinant protein, and 4 µL Biotin-tagged BIM protein peptide. After 1 hour of reaction, 5 µL of each Anti-His and streptavidin-tagged XI,665 antibody diluents diluted with a detection buffer were added. After incubating at room temperature for 4 hours, the values were read using the Envision multifunctional microplate reader to detect the effect of the compound to be tested on the binding ability of BCL2 (D103Y) to BIM protein peptides. The Envision parameter settings were exciting light at 320 nm, and emitting light at 615 nm and 665 nm. The binding ability of anti-apoptotic proteins to Bim protein peptides was indirectly reflected by the signal ratio of 665 nm and 615 nm. In the reaction, background wells without BCL2 and active wells for full binding of recombinant proteins without compounds and Bim protein peptides were set up.

The IC₅₀ value of the compounds inhibiting the binding ability of anti-apoptotic proteins to Bim protein peptides was calculated using Graphpad Prism 7.00 software through the formula Y=100/(1+10^((LogIC50-X)*HillSlope)).

**Table 3 Inhibitory activity of the compounds of the present invention on BCL2 (D103Y)**

| Compound number | BCL-2 (D103Y) (nM) | Compound number | BCL-2 (D103Y) (nM) |
|---|---|---|---|
| 002 | + | 246 | + |
| 019 | + | 247 | + |
| 053 | + | 248 | + |
| 063 | + | 249 | + |
| 120 | + | 250 | + |
| 145 | + | 251 | + |
| 146 | + | 252 | + |
| 152 | + | 253 | + |
| 189 | + | 254 | + |
| 190 | + | 255 | + |
| 197 | + | 256 | + |
| 209 | + | 257 | + |
| 234 | + | 258 | + |
| 235 | + | 259 | + |
| 236 | + | 260 | + |
| 237 | + | 261 | + |
| 238 | + | 262 | + |
| 240 | + | 263 | + |
| 241 | + | 264 | + |
| 242 | + | 265 | + |
| 243 | + | 266 | + |
| 244 | + | 267 | + |
| 245 | + | 268 | + |

| | | | |
|---|---|---|---|
| +: IC₅₀ < 10nM; ++: 10nM<IC₅₀<100nM; +++:100nM<IC₅₀<1000nM; ++++: 1000nM<IC₅₀ | | | |

The results in Table 3 indicated that the compounds of the present invention had strong BCL2 (D103Y) inhibitory activity.

### Embodiment 272 Tumor proliferation inhibitory activity test

The cell proliferation inhibitory ability of the compounds on RS4;11 was tested using the MTS method. MTS is a tetrazolium blue salt compound that can be reduced by various dehydrogenases in live cell mitochondria to their respective colored methylzan products. Its color depth is highly correlated with the number of live cells in certain sensitive cell lines within a certain range. RS4;11, RS4;11(BCL-2-G101V) and MOLT-4 tumor cells were cultivated separately and collected, 180 µL of cell suspension was added to each well in a 96-well cell plate, 20 µL of DMSO with a final concentration of 0.2% was added to the control cell wells, 20 µL of gradient diluted compounds were added to cell wells, and the treated cells were incubated in a 5% CO₂ incubator at 37°C for 3 days. After the incubation was completed, the reaction solution was prepared according to the MTS reagent kit, 20 µL was added per well and incubated in a 5% CO₂ incubator at 37°C for 3-4 hours. The absorbance value at 490 nm was read using an enzyme-linked immunosorbent assay (ELISA) plate, and the absorbance value at 690 nm was used as the background value, with OD₄₉₀nm-OD₆₉₀nm as the final raw data. The formula for calculating the inhibition ratio of compounds was as follows: inhibition ratio=(OD_{DMSO}-OD_{Compound})/(OD_{DMSO}-OD_{blank})×100%.

The proliferation inhibition IC₅₀ of the compounds was fitted by GraphPad Prism software and calculated using the formula Y=100/(1+10^((LogIC50-X)*HillSlope)).

**Table 4 Proliferation inhibitory activity of the compounds of the present invention on RS4;11**

| Compound number | RS4;11 | Compound number | RS4;11 |
|---|---|---|---|
| 002 | ++ | 246 | ++ |
| 019 | ++ | 247 | ++ |
| 053 | + | 248 | ++ |
| 063 | + | 249 | ++ |
| 120 | ++ | 250 | ++ |
| 145 | + | 251 | ++ |
| 146 | ++ | 252 | ++ |
| 152 | + | 253 | ++ |
| 189 | + | 254 | ++ |
| 190 | + | 255 | ++ |
| 197 | + | 256 | + |
| 209 | + | 257 | + |
| 234 | + | 258 | + |
| 235 | ++ | 259 | + |
| 236 | ++ | 260 | + |
| 237 | ++ | 261 | + |
| 238 | ++ | 262 | ++ |
| 239 | + | 263 | ++ |
| 240 | ++ | 264 | ++ |
| 241 | + | 265 | ++ |
| 242 | + | 266 | + |
| 243 | ++ | 267 | ++ |
| 244 | ++ | 268 | + |
| 245 | ++ | | |

| | | | |
|---|---|---|---|
| +: IC₅₀ < 0.5 nM; ++: 0.5 nM<IC₅₀<1nM; +++:1nM<IC₅₀<100nM; ++++: 100nM<IC₅₀ | | | |

The results in Table 4 indicated that the compounds of the present invention had significant proliferation inhibitory activity on RS4;11 cells.

### Embodiment 273 Experimental scheme for the BCL-XL enzyme molecular level activity test and platelet viability detection

### (1) BCL-XL enzyme molecular level activity test

In a white shallow 384-well plate, a total reaction volume was 10 µL. Specifically, it included 2 µL of the compound to be tested (2% DMSO), 4 µL His-tagged recombinant protein, and 4 µL Biotin-tagged BIM protein peptide. After 1 hour of reaction, 5 µL of each Anti-His and streptavidin-tagged XI,665 antibody diluents diluted with a detection buffer were added. After incubating at room temperature for 4 hours, the values were read using the Envision multifunctional microplate reader to detect the effect of the compound to be tested on the binding ability of BCL-XL to BIM protein peptides. The Envision parameter settings were exciting light at 320 nm, and emitting light at 615 nm and 665 nm. The binding ability of anti-apoptotic proteins to Bim protein peptides was indirectly reflected by the signal ratio of 665 nm and 615 nm. In the reaction, background wells without BCL2 and active wells for full binding of recombinant proteins without compounds and Bim protein peptides were set up.

The IC₅₀ value of the compounds inhibiting the binding ability of anti-apoptotic proteins to Bim protein peptides was calculated using Graphpad Prism 7.00 software through the formula Y=100/(1+10^((LogIC₅₀-X)*HillSlope)).

### (2) Platelet viability test

After 10 mL of platelet-rich plasma was added to 30 mL of ACD-A solution and uniformly mixed, centrifugation was performed at 1200 g, and the supernatant was discarded; 10 mL of ACD-A solution was added, resuspended, uniformly mixed, and centrifuged at 1200 g for 10 min, and the supernatant was discarded. 5 mL of HEPES-Tyrode's buffer containing 1 µM PGE1 and 0.2 U/mL apyrase was added, slightly inverted, and then centrifuged at 800 g for 5 min, and the supernatant was discarded. 10 mL of HEPES-Tyrode's buffer containing 1 µM PGE1 and 0.2 U/mL apyrase was added, resuspended, and centrifuged at 1000 g for 5 min, and the supernatant was discarded. 2 mL of HEPES-Tyrode's buffer containing 1 µM PGE1+10% FBS-resuspended platelets was added, 200 µL was taken into a centrifuge tube and diluted to 2 mL. Then it was diluted to 1, 0.8, 0.6, 0.4, 0.2, 0.1, 0.08, 0.06, 0.04, 0.02, 0.01 times, the volume was complemented to 100 µL, and 50 µL of CTG reagent was added to detect cell viability. The maximum valid dilution with a fluorescence value not exceeding 108 was selected for dilution, 90 µL/well was connected to a 96-well cell plate for chemiluminescence detection. A compound was prepared with HEPES-Tyrode's buffer containing 1 µM of PGE1+10% FBS, and added to the cells, which was gently beaten evenly and detected for cell viability by adding 50 µL CTG after 24 h.

The IC₅₀ value of the compound inhibiting platelet viability was calculated using Graphpad Prism 7.00 software through the formula Y=100/(1+10^((LogIC₅₀-X)*HillSlope)).

**Table 5 Inhibitory activity of the compounds of the present invention on BCL-XL**

| Number | BCL-XL | Number | BCL-XL | Number | BCL-XL |
|---|---|---|---|---|---|
| 002 | >500 nM | 197 | >1 µM | 243 | >500 nM |
| 019 | >1 µM | 203 | >500 nM | 244 | >500 nM |
| 058 | >1 µM | 204 | >500 nM | 245 | >500 nM |
| 062 | >1 µM | 205 | >500 nM | 246 | >500 nM |
| 063 | >1 µM | 207 | >500 nM | 247 | >500 nM |
| 066 | >500 nM | 209 | 1.9 µM | 248 | >500 nM |
| 120 | 1.6 µM | 213 | >500 nM | 249 | >500 nM |
| 145 | >1 µM | 216 | >500 nM | 250 | >500 nM |
| 146 | 2.7 µM | 217 | >500 nM | 251 | >500 nM |
| 152 | >1 µM | 224 | >500 nM | 252 | >500 nM |
| 184 | >500 nM | 226 | >500 nM | 253 | >500 nM |
| 185 | >500 nM | 227 | >500 nM | 254 | >500 nM |
| 186 | >500 nM | 234 | >500 nM | 255 | >500 nM |
| 187 | >500 nM | 236 | >500 nM | 260 | >500 nM |
| 188 | >500 nM | 237 | >500 nM | 262 | >1 µM |
| 189 | 1.9 µM | 238 | >500 nM | 263 | 1.3 µM |
| 190 | >500 nM | 239 | >500 nM | 265 | >20 µM |
| 193 | >500 nM | 240 | >500 nM | 267 | >500 nM |
| 195 | >500 nM | 241 | >500 nM | 268 | >500 nM |
| 196 | >500 nM | 242 | >500 nM | Compound a | 233 nM |

**Table 6 Results of the compounds of the present invention on human platelet viability**

| Compound number | Human platelet IC₅₀ |
|---|---|
| 152 | >5 µM |
| 190 | >5 µM |
| 197 | >5 µM |
| 209 | >5 µM |
| 234 | >5 µM |
| 239 | >5 µM |
| 241 | >5 µM |
| 242 | >5 µM |
| 247 | >5 µM |
| 252 | >5 µM |
| Compound a | 1050 nM |

| | |
|---|---|
| Note: Compound a was selected from WO2021208963 | |

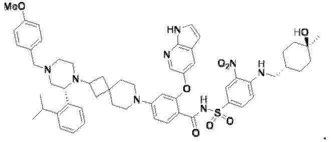

The results in Tables 5 and 6 indicated that the compounds of the present invention had no inhibitory activity on BCL-XL and had no toxic effect on human platelets.

### Embodiment 274 CYP3A4 enzyme inhibitory activity

The inhibitory activity of compounds on human CYP3A4 enzyme was detected using a VIVID^{™} CYP3A4 GREEN SCRNING KIT reagent kit. The Vivid^{™} CYP450 screening kit includes a Vivid^{™} substrate, Vivid^{™} fluorescence standard, reaction buffer, Vivid^{™} regeneration system, NADP+ and CYP450 BACULOSOMES^{™} Plus reagent. Operations were performed following the instructions, and a blank control group was set up without adding any compounds.

The IC₅₀ value of the compounds inhibiting human CYP3A4 enzyme was calculated using Graphpad Prism 7.00 software through the formula Y=100/(1+10^((LogIC50-X)*HillSlope)).

**Table 7 Results of the inhibitory effect of the compounds of the present invention on CYP3A4 enzyme**

| Compound number | CYP3A4 (IC₅₀) | Compound number | CYP3A4 (IC₅₀) |
|---|---|---|---|
| 002 | >20 µM | 256 | >20 µM |
| 053 | >20 µM | 257 | >20 µM |
| 063 | >20 µM | 258 | >20 µM |
| 152 | >20 µM | 259 | >20 µM |
| 189 | >20 µM | 261 | >20 µM |
| 190 | >20 µM | 262 | >20 µM |
| 197 | >20 µM | 263 | >20 µM |
| 209 | >20 µM | 264 | >20 µM |
| 234 | >20 µM | 266 | >20 µM |
| 239 | >20 µM | 268 | >20 µM |
| 242 | >20 µM | Compound a | 2.55 µM |

The results in Table 7 indicated that the compounds of the present invention had no inhibitory effect on CYP3A4 enzyme.

### Embodiment 275: Rat absorption test

Experimental method: SD rats were used as experimental animals and administered 10 mg/kg by gavage. The blood collection time points for gavage administration were 0, 0.5, 1, 2, 3, 4, 6, 8, and 24 hours. 3 mL of whole blood was collected, and after centrifugation, 1 mL of plasma was taken for LC-MS analysis.

**Table 8 Results of the compounds of the present invention on the rat absorption test**

| Compound number | AUC (h*ng/mL) |
|---|---|
| 152 | 3326 |
| 234 | 5631 |
| 256 | 1799 |
| 261 | 1212 |
| Compound b | 527 |

| | |
|---|---|
| Note: Compound b was selected from the compound number 142 of CN114478520A. | |

The results in Table 8 indicated that the compounds of the present invention had good pharmacokinetic properties in SD rats, supporting oral administration routes.

### Embodiment 276 Canine absorption test

Experimental method: Beagle dogs were used as experimental animals and administered 5 mg/kg by gavage. The blood collection time points for gavage administration were 0, 0.5, 1, 2, 3, 4, 6, 8, and 24 hours. 3 mL of whole blood was collected, and after centrifugation, plasma was taken for LC-MS analysis.

**Table 9 Results of the compounds of the present invention on the canine absorption test**

| Compound number | DNAUC |
|---|---|
| 152 | 13685 |
| 234 | 9103 |
| Compound a | 3790 |

| | |
|---|---|
| Note: DNAUC represented AUC (h*ng/mL)/dose (mg/kg). | |

The results in Table 9 indicated that the compounds of the present invention had good pharmacokinetic properties in beagle dogs, supporting oral administration routes.

### Embodiment 277 In vivo anti-tumor efficacy

Tumor models were established by subcutaneous inoculation of RS4;11 cells into NOD/SCID mice. When the tumors grew to about 100 mm³, the mice were divided into groups and administered as follows: The control group was given a corresponding volume of CMC-Na solution by gavage; the experimental group was given 5 mg/kg of the test compound by gavage; the weight and tumor size changes of the mice were weighed twice a week, and after 8 days, the efficacy of the compounds on RS4;11 mice transplanted tumors was investigated.

The relative tumor proliferation rate T/C% = T_{RTC}/C_{RTV}×100% (T_{RTV}: average RTV of the treatment group; C_{RTV}: average RTV of the negative control group). Based on the results of tumor measurements, the relative tumor volume (RTV) was calculated using the formula RTV=Vₜ/V₀, where V₀ was the tumor volume measured during group division (i.e. D₀), Vt was the tumor volume at a certain measurement, and T_{RTV} and C_{RTV} used the data of the same day.

**Table 10 In vivo anti-tumor activity of the compounds of the present invention**

| Compound number | T/C % |
|---|---|
| 152 (5mg/kg) | 0.35 |
| 234 (5mg/kg) | 1.26 |
| 261 (15 mg/kg) | 5.35 |
| 266 (5mg/kg) | 15.38 |
| Compound b (15 mg/kg) | 14.87 |

| | |
|---|---|
| Note: Compound b was selected from the compound number 142 of CN114478520A. | |

The results in Table 10 indicated that the compounds of the present invention had a significant anti-tumor effect, and there were no abnormalities in the animals during the experiments.

## Claims

1. A compound, having a structure of general formula (I): 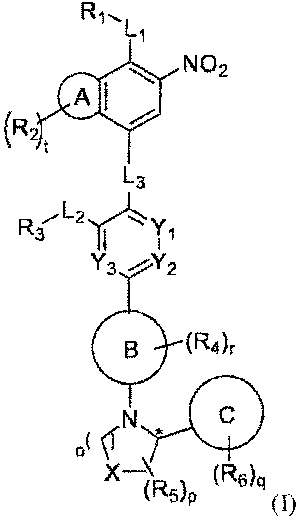
or a stereoisomer or stereoisomer mixture or pharmaceutically acceptable salt thereof,
wherein a C marked with * is of an R configuration or S configuration;
a ring A is selected from absent, 3-10 membered cycloalkyl, 4-10 membered cycloalkenyl, 5-10 membered cycloalkynyl, 4-10 membered heterocyclyl, a 6-10 membered aromatic ring, or a 5-10 membered heteroaromatic ring; and the heterocyclyl and heteroaromatic ring contain 1 to 4 heteroatoms selected from N, O and S;
R₂ is selected from hydrogen, deuterium, -C₁-C₈ alkyl, -C₂-C₈ alkenyl, -C₂-C₈ alkynyl, -C₁-C₈ alkoxy, cycloalkyl, 4-10 membered heterocyclyl, aryl, heteroaryl, halogen, hydroxyl, amino, oxo, cyano, nitro, -OR_{g}, -SR_{g}, -S(O)R_{g}, -SO₂R_{g}, -C(O)R_{g}, -C(O)OR_{g}, -C(O)NRₕRᵢ, -OC(O)R_{g}, -NRₕRᵢ, -NRₕC(O)Rᵢ, -NRₕC(O)NRᵢRₕ, -NRₕC(O)OR_{g}, -NRₕS(O)NRᵢRₕ, -NRₕSO₂NRᵢRₕ, -P(O)RₕRᵢ, halogenated C₁-C₈ alkyl, halogenated C₁-C₈ alkoxy, halogenated C₁-C₈ cycloalkyl, hydroxyl-substituted C₁-C₈ alkyl, or hydroxyl-substituted C₁-C₈ alkoxy; and 2 R₂ can form a 3-6 membered spiral ring when on the same atom and can form a 3-8 membered parallel ring when on adjacent atoms;
Y₁, Y₂ and Y₃ are each independently selected from CR₇ or N;
R₇ is selected from H, halogen, C₁-C₈ alkyl, halogenated C₁-C₈ alkyl, cycloalkyl, or alkoxy;
L₂ is selected from a chemical bond, -NRₐ-, -O-, -S-, -C(O)-, -C(O)NRₐ-, or -NRₐC(O)-;
L₃ is selected from -C(O)NR₈SO₂-;
R₈ is selected from H, C₁-C₈ alkyl, -RₚOC(O)R_{q}, -RₚOC(O)OR_{q}, -RₚOC(O)NR_{q}Rₛ, or - RₚCOOR_{q};
Rₚ, R_{q} and Rₛ are each independently selected from H, C₁-C₈ alkyl, 3-10 membered cycloalkyl, 4-10 membered heterocyclyl, aryl, or 5-10 membered heteroaryl; and the alkyl, cycloalkyl, heterocyclyl, aryl, and heteroaryl can be further substituted by C₁-C₈ alkyl, 3-10 membered cycloalkyl, 4-10 membered heterocyclyl, aryl, and 5-10 membered heteroaryl;
R₃ is selected from 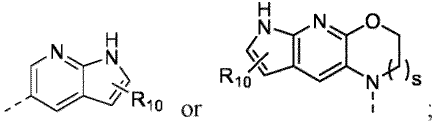
R₁₀ is selected from hydrogen, deuterium, alkyl, alkenyl, alkynyl, cycloalkyl, cycloalkenyl, heterocyclyl, aryl, heteroaryl, halogen, nitro, oxo, cyano, or haloalkyl, wherein R₁₀ can be substituted on a carbon or nitrogen atom;
a ring B is selected from 5-12 membered spiroheterocyclyl;
a ring C is selected from cycloalkyl, cycloalkenyl, a bridged ring group, heterocyclyl, or aryl or heteroaryl;
X is selected from CR_{b}R_{c} or NR_{b};
o is selected from 0, 1, 2, 3, or 4;
p is selected from 0, 1, 2, or 3;
q is selected from 0, 1, 2, or 3;
r is selected from 0, 1, 2, or 3;
s is selected from 0, 1, 2, or 3;
t is selected from 0, 1, 2, or 3;
Rₐ, R_{b} and R_{c} are each independently selected from H, deuterium, halogen, C₁-C₈ alkyl, alkoxy, cycloalkyl, heterocyclyl, heteroaryl, or aryl; and the alkyl, cycloalkyl, heteroaryl, and aryl can be further substituted by one or more R_{d} substituents;
R_{d} is selected from H, deuterium, halogen, C₁-C₈ alkyl, alkoxy, oxo, amino, hydroxyl, cyano, alkenyl, alkynyl, cycloalkyl, heterocyclyl, aryl, heteroaryl, or a fused bicyclic group; and the alkyl, alkenyl, alkynyl, cycloalkyl, heterocyclyl, heteroaryl, aryl, and fused bicyclic group can be further substituted by one or more Rₑ substituents, and 2 Rₑ can form a 3-6 membered spiral ring when on the same atom and can form a 3-8 membered parallel ring when on adjacent atoms;
Rₑ is selected from H, deuterium, halogen, C₁-C₈ alkyl, C₁-C₈ alkoxy, oxo, amino, hydroxyl, cyano, alkenyl, alkynyl, cycloalkyl, heterocyclyl, haloalkyl, haloalkoxy, methylsulfonyl, aryl, or heteroaryl; the alkyl, alkoxy, alkenyl, alkynyl, cycloalkyl, heterocyclyl, heteroaryl, and aryl can be further substituted by one or more Rₘ; and 2 Rₘ can form a 3-6 membered spiral ring when on the same atom and can form a 3-8 membered parallel ring when on adjacent atoms;
R₄ is selected from H, halogen, C₁-C₈ alkyl, halogenated C₁-C₈ alkyl, cycloalkyl, or alkoxy;
R₅ is selected from H, deuterium, halogen, C₁-C₈ alkyl, haloalkyl, alkoxy, cycloalkyl, heterocyclyl, heteroaryl, or aryl;
R₆ is selected from H, deuterium, halogen, C₁-C₈ alkyl, haloalkyl, alkoxy, cycloalkyl, heterocyclyl, heteroaryl, or aryl;
L₁ is selected from a chemical bond, NR_{f} or O;
R_{f} is selected from H, C₁-C₈ alkyl, haloalkyl, cycloalkyl, heterocyclyl, heteroaryl, or aryl;
R₁ is selected from hydrogen, deuterium, alkyl, a bridged ring group, spirocyclyl, alkenyl, alkynyl, cycloalkyl, cycloalkenyl, heterocyclyl, aryl or heteroaryl, halogen, nitro, oxo, cyano, - OR_{g}, -SR_{g}, alkyl-R_{g}, NH(CH)R_{g}, -C(O)R_{g}, -S(O)R_{g}, -SO₂R_{g}, -C(O)OR_{g}, -OC(O)R_{g}, -NRₕRᵢ, C(O)N(Rₕ)Rᵢ, -N(Rₕ)C(O)Rᵢ, -NRₕC(O)NRᵢRₕ, -NRₕC(O)OR_{g}, -NRₕS(O)NRᵢRₕ, -NRₕSO₂NRᵢRₕ, or -P(O)RₕRᵢ, and the alkyl, bridged ring group, spirocyclyl, alkenyl, alkynyl, cycloalkyl, cycloalkenyl, heterocyclyl, and aryl or heteroaryl can be further substituted by one or more Rⱼ;
R_{g}, Rₕ, Rᵢ and Rⱼ are selected from hydrogen, deuterium, C₁-C₈ alkyl, spirocyclyl, alkenyl, alkynyl, halogen, cyano, amino, nitro, hydroxyl, oxo, carboxyl, amide, alkoxy, haloalkyl, hydroxyalkyl, aminoalkyl, alkylcarbonyl, alkoxycarbonyl, alkylamino, halohydroxyalkyl, haloalkylamino, cycloalkyl, cycloalkenyl, a bridged ring group, heterocyclyl, or aryl or heteroaryl, and the alkyl, spirocyclyl, alkenyl, alkynyl, alkoxy, hydroxyalkyl, aminoalkyl, alkylcarbonyl, alkoxycarbonyl, alkylamino, cycloalkyl, cycloalkenyl, bridged ring group, heterocyclyl, and aryl or heteroaryl can be further substituted by one or more Rₘ;
Rₘ is selected from hydrogen, deuterium, C₁-C₈ alkyl, halogen, cyano, amino, nitro, hydroxyl, oxo, alkoxy, haloalkyl, hydroxyalkyl, aminoalkyl, alkylcarbonyl, alkoxycarbonyl, alkylamino, halohydroxyalkyl, haloalkylamino, cycloalkyl, or heterocyclyl, and the alkyl, cycloalkyl and heterocyclyl can be further substituted by one or more Rᵣ;
Rᵣ is selected from hydrogen, deuterium, C₁-C₈ alkyl, halogen, cyano, amino, hydroxyl, oxo, alkoxy, hydroxyalkyl, aminoalkyl, alkylcarbonyl, heterocyclyl, alkylamino, alkoxycarbonyl, halohydroxyalkyl, haloalkylamino, haloalkyl, cycloalkyl, spirocyclyl, alkenyl, alkynyl, nitro, carboxyl, amide, cycloalkenyl, a bridged ring, or aryl or heteroaryl; and
when the ring A is selected from absent,
X is selected from NR_{b}, and at least one of Y₁/Y₂/Y₃ is selected from N or CR₇, and R₇ is not H.

2. The compound according to claim 1, having a structure of general formula (I'): 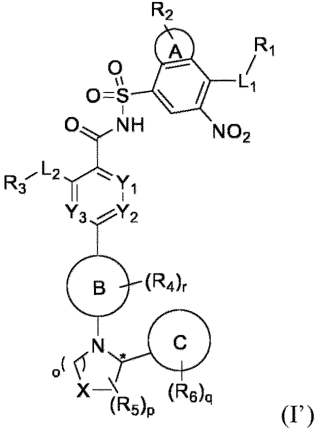
or a stereoisomer or stereoisomer mixture or pharmaceutically acceptable salt thereof,
wherein a C marked with * is of an R configuration or S configuration;
the ring A is selected from absent, 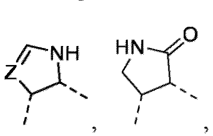 a 6-10 membered heterocyclic ring, a 6-10 membered aromatic ring, or a 6-10 membered heteroaromatic ring;
Z is selected from N or CH;
R₂ is selected from hydrogen, deuterium, C₁-C₈ alkyl, halogen, or halogenated C₁-C₈ alkyl;
Y₁, Y₂ and Y₃ are each independently selected from CR₇ or N;
R₇ is selected from H, halogen, C₁-C₈ alkyl, halogenated C₁-C₈ alkyl, cycloalkyl, or alkoxy; when the ring A is selected from absent, and when two of Y₁, Y₂ and Y₃ are CH,
the other Y₁/Y₂/Y₃ is N or CR₇, and R₇ is not H;
R₃ is selected from 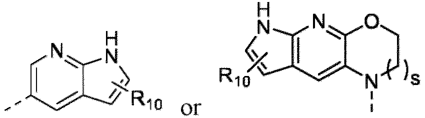
R₁₀ is selected from hydrogen, deuterium, alkyl, alkenyl, alkynyl, cycloalkyl, cycloalkenyl, heterocyclyl, aryl, heteroaryl, halogen, nitro, oxo, cyano, or haloalkyl; and R₁₀ can be substituted on a carbon or nitrogen atom;
the ring B is selected from 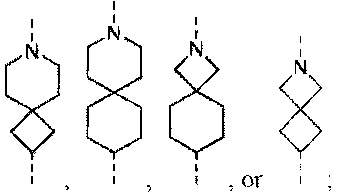
o is selected from 0, 1, 2, 3, or 4;
p is selected from 0, 1, 2, or 3;
q is selected from 0, 1, 2, or 3;
r is selected from 0, 1, 2, or 3;
s is selected from 0, 1, 2, or 3;
L₂ is selected from a chemical bond, NRₐ or O;
X is selected from CR_{b}R_{c} or NR_{b};
Rₐ, R_{b} and R_{c} are each independently selected from H, deuterium, halogen, C₁-C₈ alkyl, alkoxy, cycloalkyl, heterocyclyl, heteroaryl, or aryl; and the alkyl, cycloalkyl, heteroaryl, and aryl can be further substituted by one or more R_{d} substituents;
R_{d} is selected from H, deuterium, halogen, C₁-C₈ alkyl, alkoxy, oxo, amino, hydroxyl, cyano, alkenyl, alkynyl, cycloalkyl, heterocyclyl, aryl, heteroaryl, or a fused bicyclic group; and the alkyl, alkenyl, alkynyl, cycloalkyl, heterocyclyl, heteroaryl, aryl, and fused bicyclic group can be further substituted by one or more Rₑ substituents, and 2 Rₑ can form a 3-6 membered spiral ring when on the same carbon and can form a 3-8 membered parallel ring when on adjacent carbons;
Rₑ is selected from H, deuterium, halogen, C₁-C₈ alkyl, C₁-C₈ alkoxy, oxo, amino, hydroxyl, cyano, alkenyl, alkynyl, cycloalkyl, heterocyclyl, haloalkyl, haloalkoxy, or methylsulfonyl;
R₄ is selected from H, halogen, C₁-C₈ alkyl, halogenated C₁-C₈ alkyl, cycloalkyl, or alkoxy;
R₅ is selected from H, deuterium, halogen, C₁-C₈ alkyl, haloalkyl, alkoxy, cycloalkyl, heterocyclyl, heteroaryl, or aryl;
R₆ is selected from H, deuterium, halogen, C₁-C₈ alkyl, haloalkyl, alkoxy, cycloalkyl, heterocyclyl, heteroaryl, or aryl;
the ring C is selected from cycloalkyl, cycloalkenyl, a bridged ring group, heterocyclyl, or aryl or heteroaryl;
L₁ is selected from NR_{f} or O;
R_{f} is selected from H, C₁-C₈ alkyl, haloalkyl, cycloalkyl, heterocyclyl, heteroaryl, or aryl;
R₁ is selected from hydrogen, deuterium, alkyl, a bridged ring group, spirocyclyl, alkenyl, alkynyl, cycloalkyl, cycloalkenyl, heterocyclyl, aryl or heteroaryl, halogen, nitro, oxo, cyano, OR_{g}, SR_{g}, alkyl-R_{g}, NH(CH) R_{g}, C(O) R_{g}, S(O) R_{g}, SO₂R_{g}, C(O)OR_{g}, OC(O)R_{g}, NRₕRᵢ, C(O)N(Rₕ)Rᵢ, N(Rₕ)C(O)Rᵢ, or -P(O)RₕRᵢ, and the alkyl, bridged ring group, spirocyclyl, alkenyl, alkynyl, cycloalkyl, cycloalkenyl, heterocyclyl, and aryl or heteroaryl can be further substituted by one or more Rⱼ;
R_{g}, Rₕ, Rᵢ and Rⱼ are selected from hydrogen, deuterium, C₁-C₈ alkyl, spirocyclyl, alkenyl, alkynyl, halogen, cyano, amino, nitro, hydroxyl, oxo, carboxyl, amide, alkoxy, haloalkyl, hydroxyalkyl, aminoalkyl, alkylcarbonyl, alkoxycarbonyl, alkylamino, halohydroxyalkyl, haloalkylamino, cycloalkyl, cycloalkenyl, a bridged ring group, heterocyclyl, or aryl or heteroaryl, and the alkyl, spirocyclyl, alkenyl, alkynyl, alkoxy, hydroxyalkyl, aminoalkyl, alkylcarbonyl, alkoxycarbonyl, alkylamino, cycloalkyl, cycloalkenyl, bridged ring group, heterocyclyl, and aryl or heteroaryl can be further substituted by one or more Rₘ;
Rₘ is selected from hydrogen, deuterium, C₁-C₈ alkyl, halogen, cyano, amino, nitro, hydroxyl, oxo, alkoxy, haloalkyl, hydroxyalkyl, aminoalkyl, alkylcarbonyl, alkoxycarbonyl, alkylamino, halohydroxyalkyl, haloalkylamino, cycloalkyl, or heterocyclyl, and the alkyl, cycloalkyl and heterocyclyl can be further substituted by one or more Rᵣ;
Rᵣ is selected from hydrogen, deuterium, C₁-C₈ alkyl, halogen, cyano, amino, hydroxyl, oxo, alkoxy, hydroxyalkyl, aminoalkyl, alkylcarbonyl, heterocyclyl, alkylamino, alkoxycarbonyl, halohydroxyalkyl, haloalkylamino, haloalkyl, cycloalkyl, spirocyclyl, alkenyl, alkynyl, nitro, carboxyl, amide, cycloalkenyl, a bridged ring, or aryl or heteroaryl.

3. The compound according to claim 2, having a structure of general formulas IIa and lib: 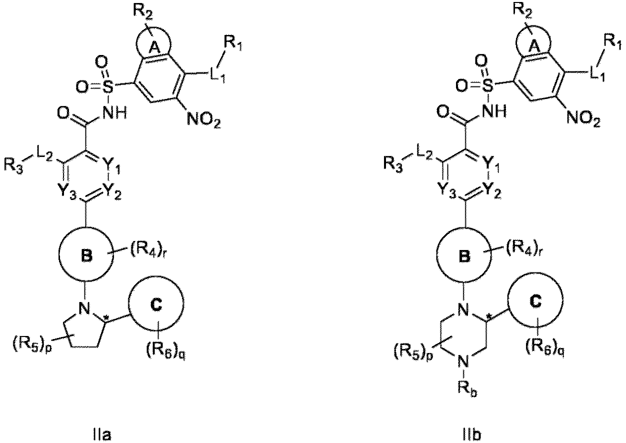 or a stereoisomer or stereoisomer mixture or pharmaceutically acceptable salt thereof.

4. The compound according to claim 3, having a structure of general formulas IIIa and IIIb: 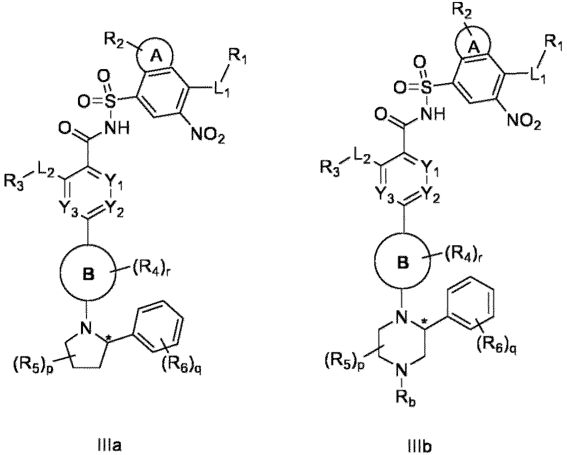 or a stereoisomer or stereoisomer mixture or pharmaceutically acceptable salt thereof.

5. The compound according to claim 1, wherein the ring A is selected from absent, or

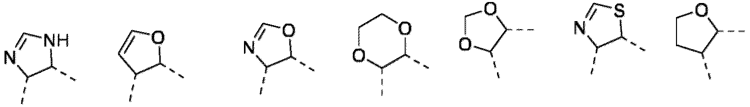

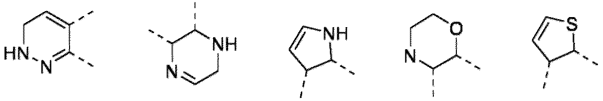

6. The compound according to claim 4, having a structure of general formulas IVa, IVb and IVc: 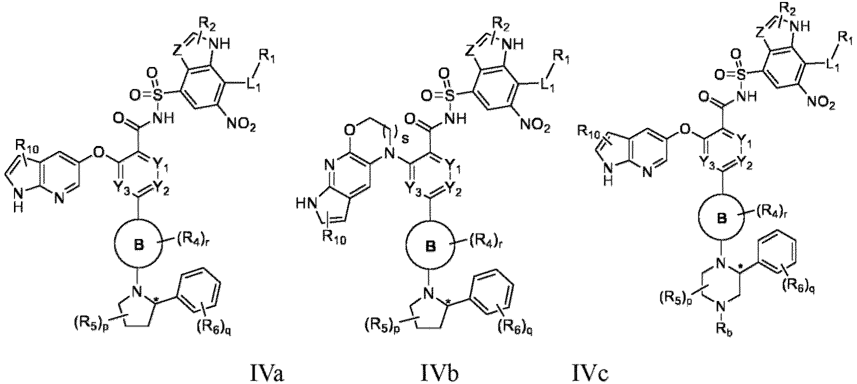
or a stereoisomer or stereoisomer mixture or pharmaceutically acceptable salt thereof,
wherein R₂ is selected from hydrogen, deuterium, C₁-C₈ alkyl, halogen, or halogenated C₁-C₈ alkyl; and R₂ can be substituted on carbon or nitrogen.

7. The compound according to claim 6, having a structure of general formulas Va-Vf: 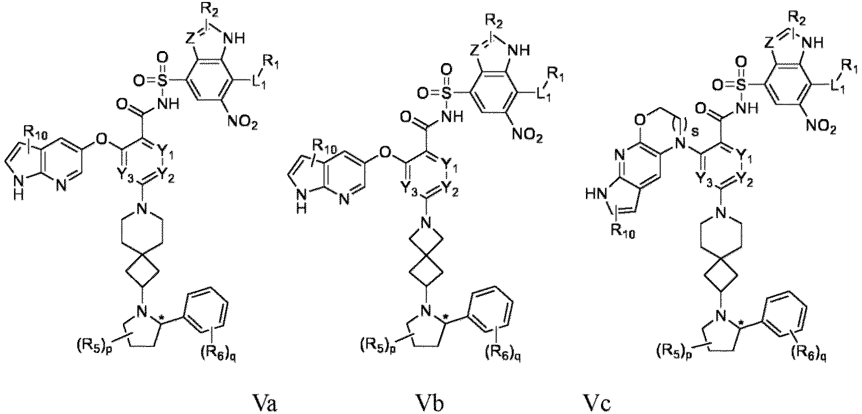 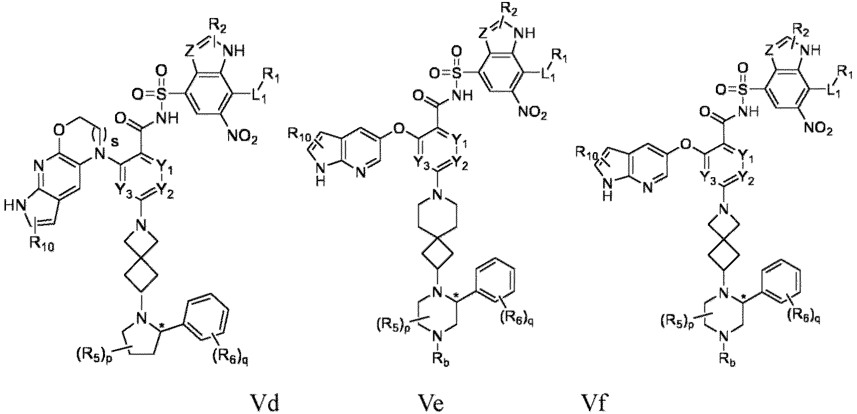
or a stereoisomer or stereoisomer mixture or pharmaceutically acceptable salt thereof,
wherein R₁₀ is selected from hydrogen, deuterium, alkyl, cycloalkyl, halogen, nitro, cyano, or haloalkyl;
wherein R₂ is selected from hydrogen, deuterium, C₁-C₈ alkyl, halogen, or halogenated C₁-C₈ alkyl; and R₂ can be substituted on carbon or nitrogen.

8. The compound according to claim 4, having a structure of general formulas VIa and VIb: 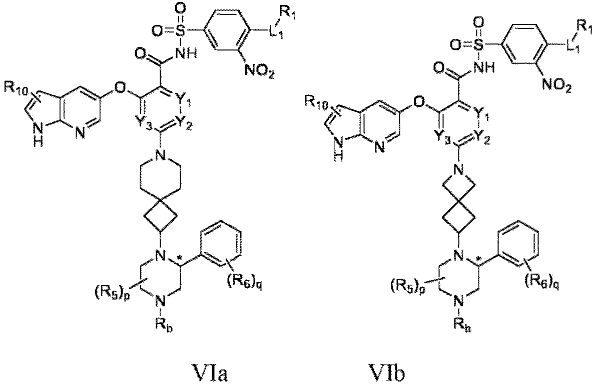
or a stereoisomer or stereoisomer mixture or pharmaceutically acceptable salt thereof,
wherein when two of Y₁, Y₂ and Y₃ are CH, the other Y₁/Y₂/Y₃ is N or CR₇; and
R₇ is selected from halogen, C₁-C₈ alkyl, halogenated C₁-C₈ alkyl, cycloalkyl, or alkoxy.

9. The compound according to claim 1, 2 or 8, wherein R₁ is selected from 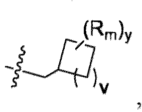 and any carbon atom on its ring can be substituted by one or more N or O;
Y is selected from 0, 1, 2, 3, or 4; and
V is selected from 0, 1, 2, or 3.

10. The compound according to claim 1, wherein
the ring A is selected from a 4-membered heterocyclic ring, a 5-membered heterocyclic ring or 5-membered heteroaryl; L₃ is selected from -C(O)NHSO₂-; the ring C is selected from aryl or heteroaryl; further preferably, the ring C is selected from a benzene ring; and the ring B is selected from 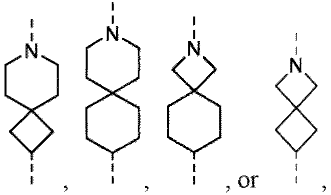 wherein nitrogen is connected to a ring containing Y₁/Y₂/Y₃.

11. The compound according to claim 10, wherein X is selected from CR_{b}R_{c};
R_{b} and R_{c} are each independently selected from H, deuterium, halogen, C₁-C₈ alkyl, C₁-C₈ alkoxy, cycloalkyl, heterocyclyl, heteroaryl, or aryl;
o is selected from 0, 1 or 2; and
L₂ is selected from a chemical bond, -NH- or -O-.

12. The compound according to claim 10, having a structure of general formula (VII): 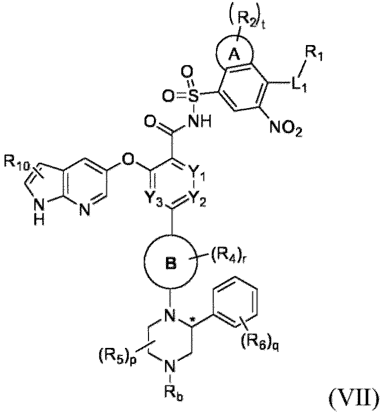
or a stereoisomer or stereoisomer mixture or pharmaceutically acceptable salt thereof, wherein
Y₁, Y₂ and Y₃ are each independently selected from CR₇ or N; and
R₇ is selected from H, halogen, C₁-C₈ alkyl, halogenated C₁-C₈ alkyl, cycloalkyl, or alkoxy.

13. The compound according to claim 1, which is selected from following compounds: 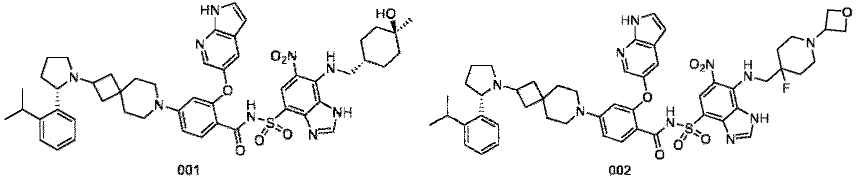 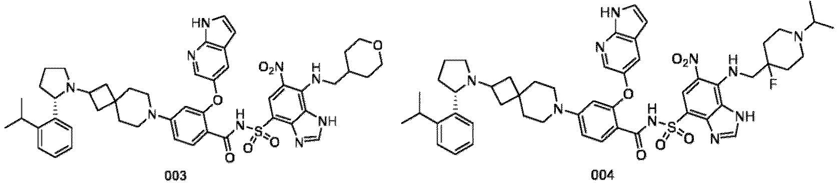 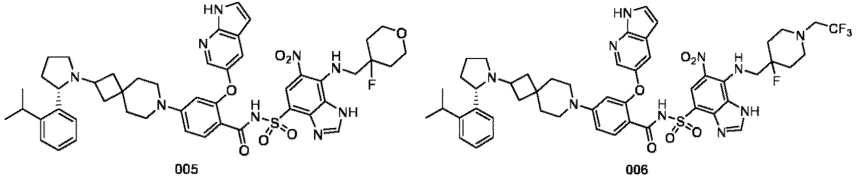 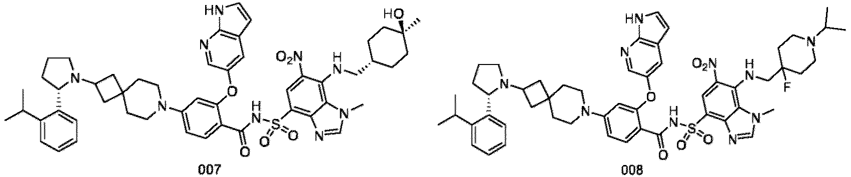 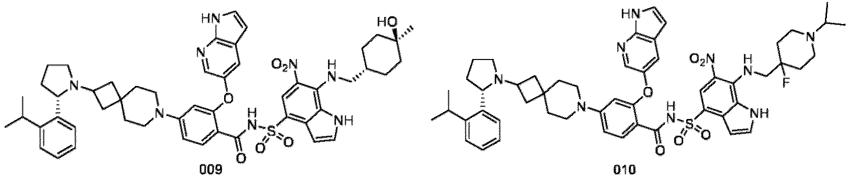 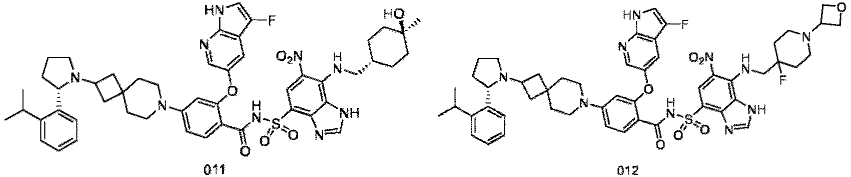 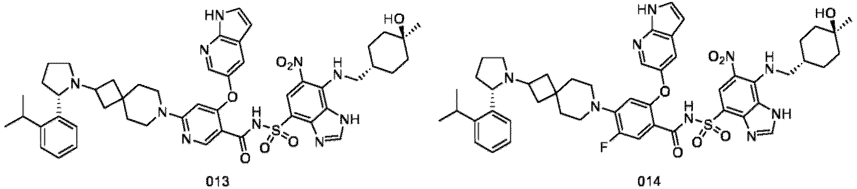 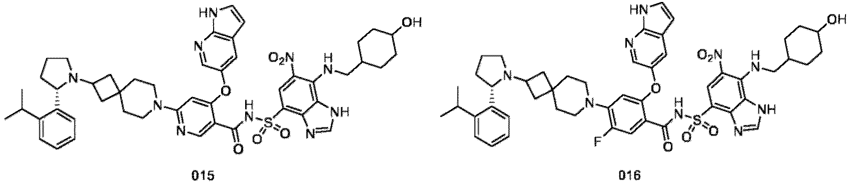 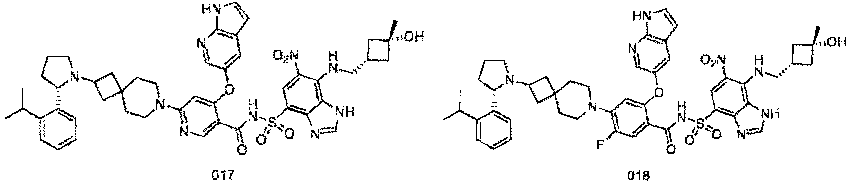 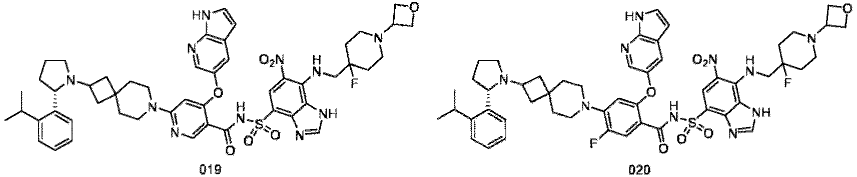 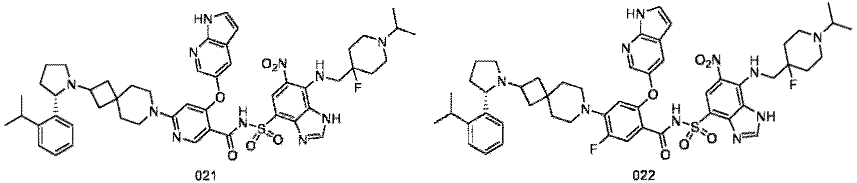 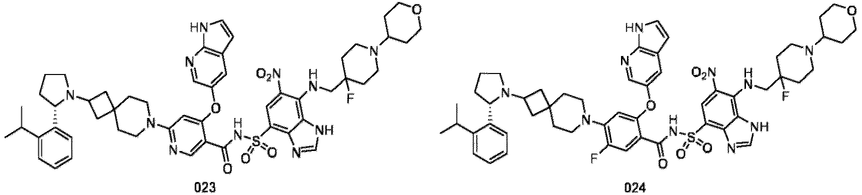 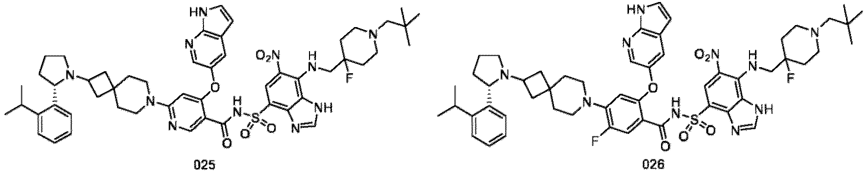 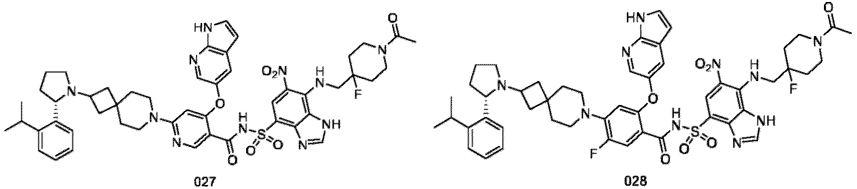 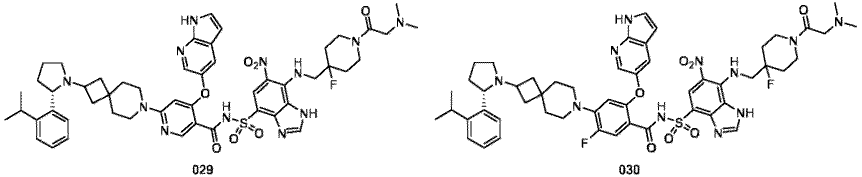 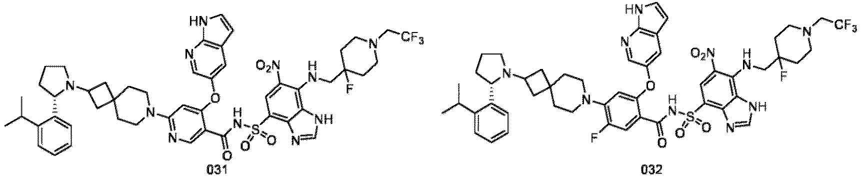 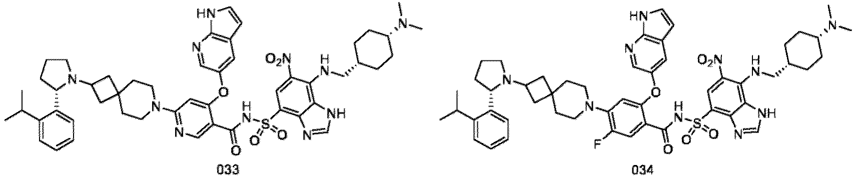 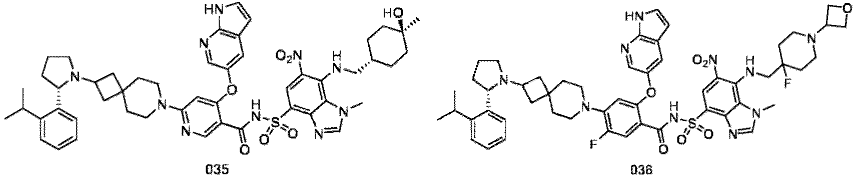 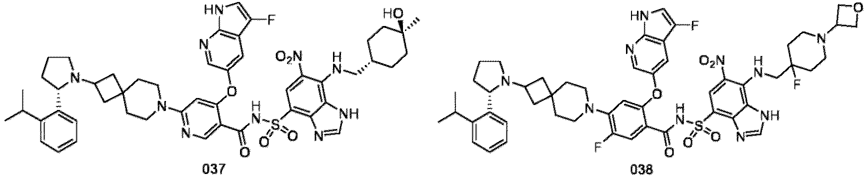 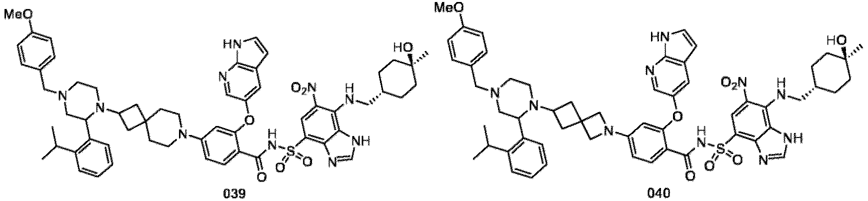 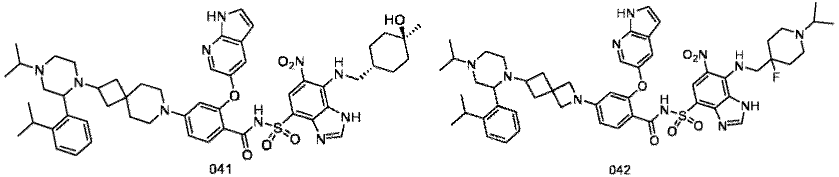 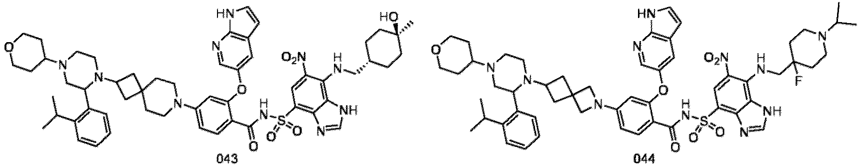 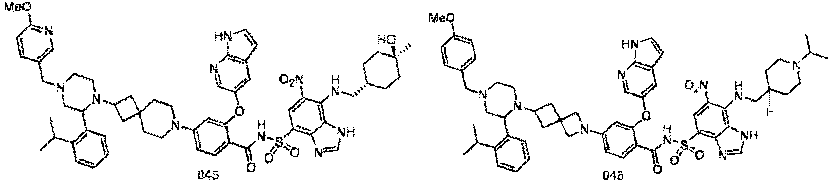 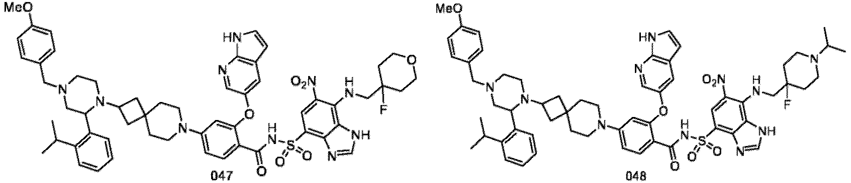 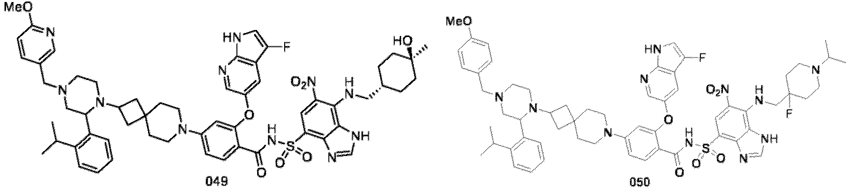 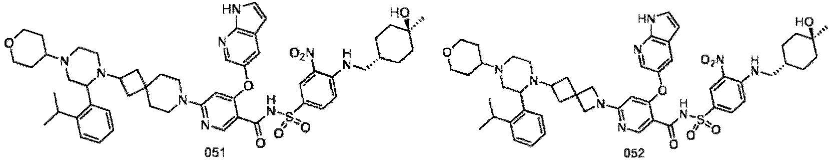 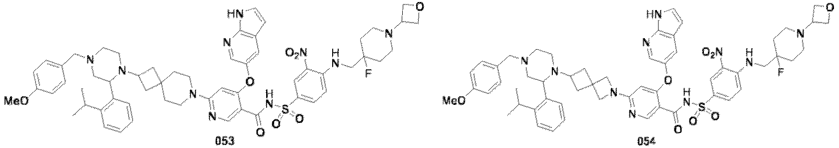 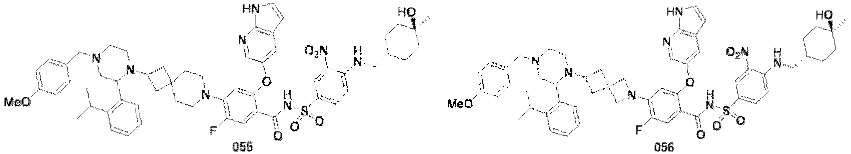 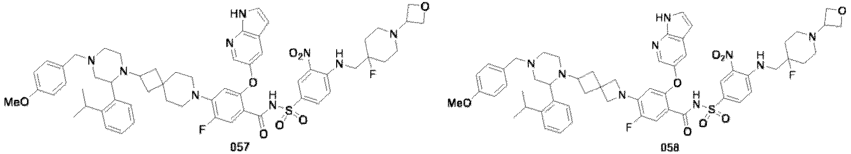 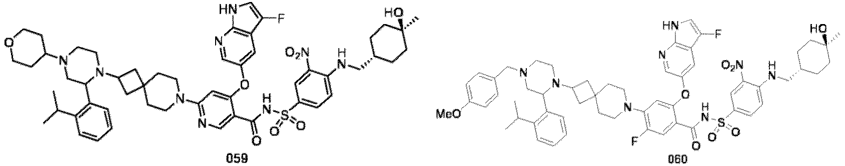 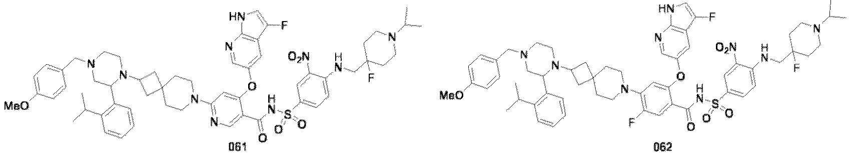 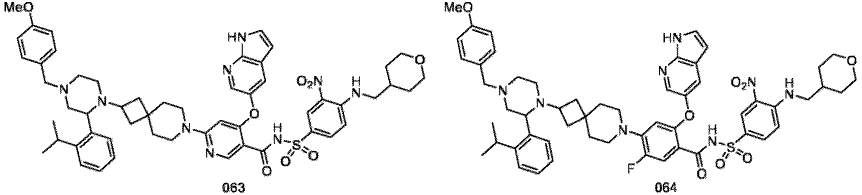 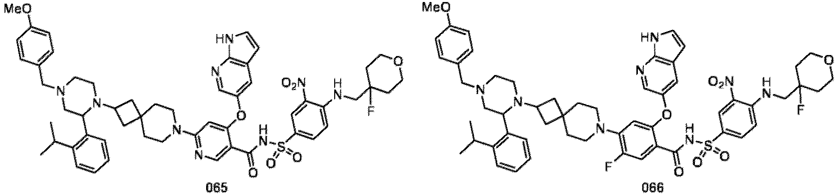 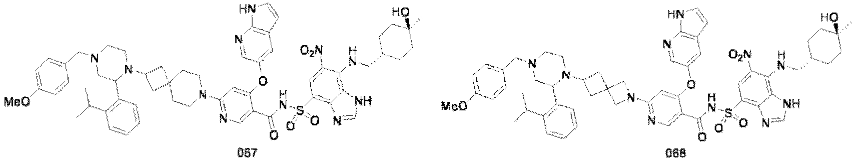 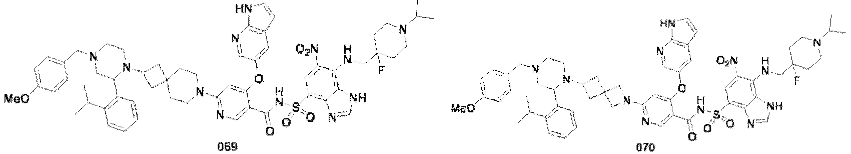 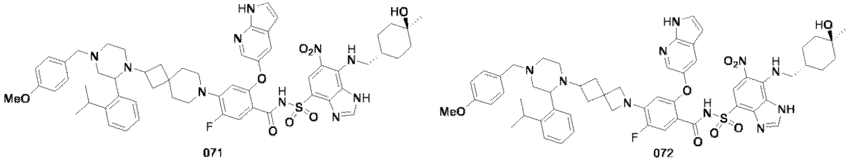 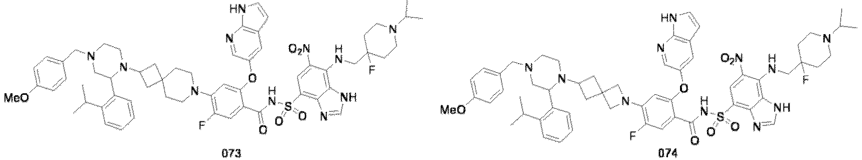 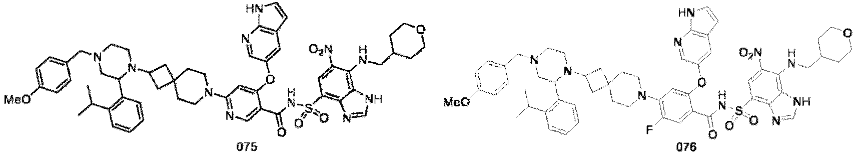 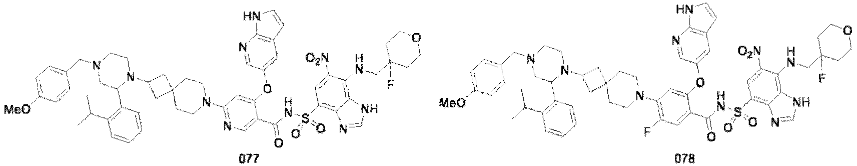 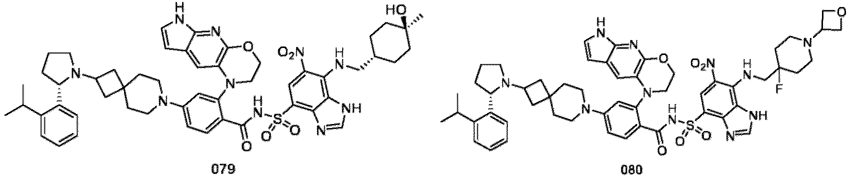 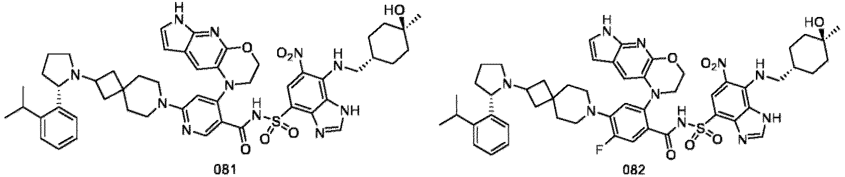 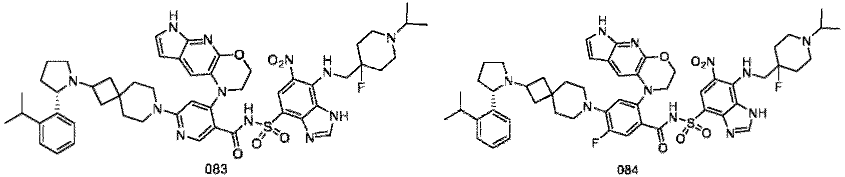 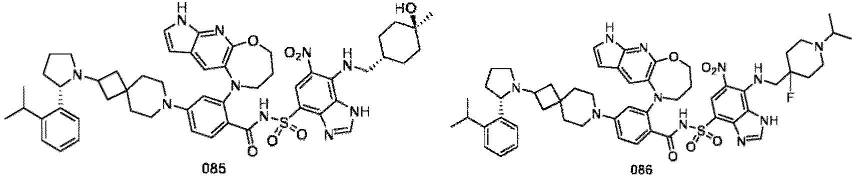 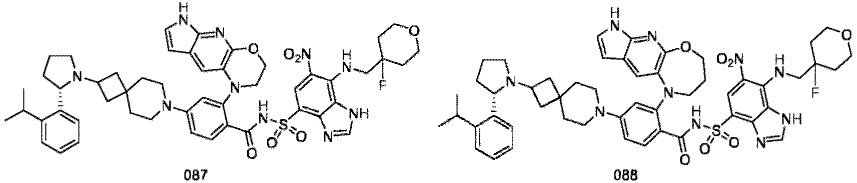 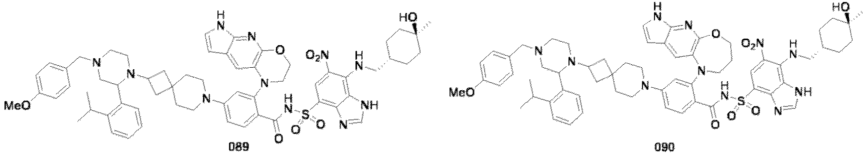 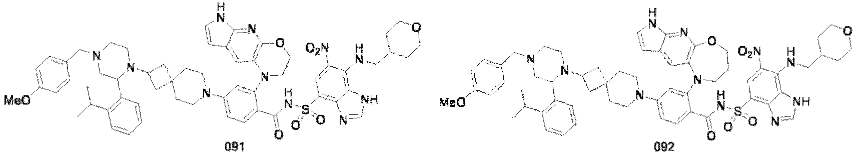 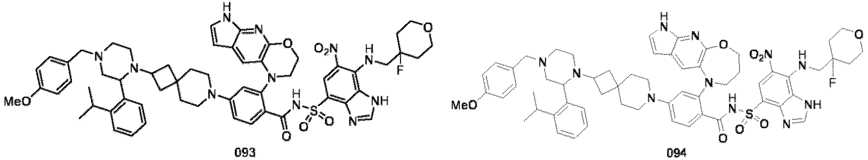 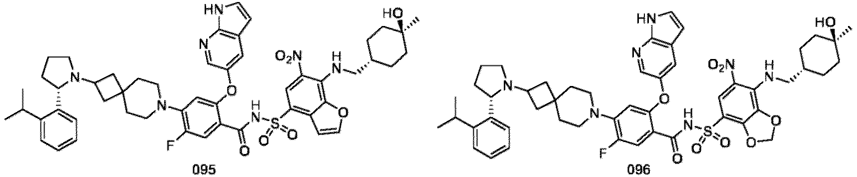 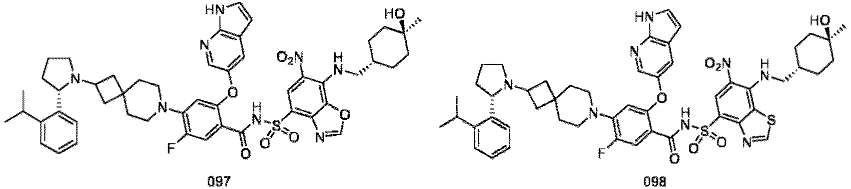 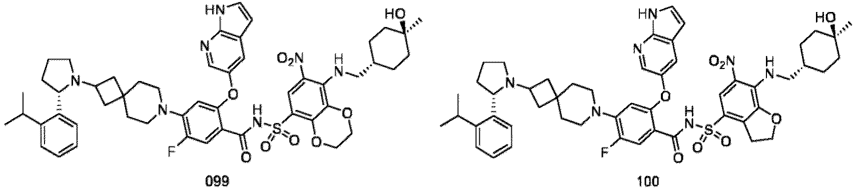 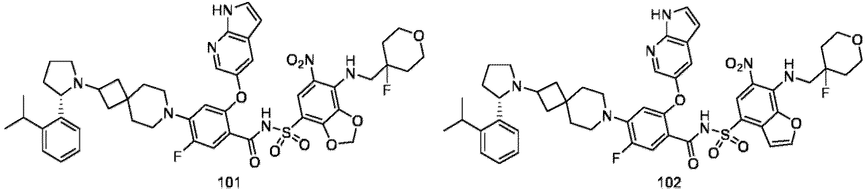 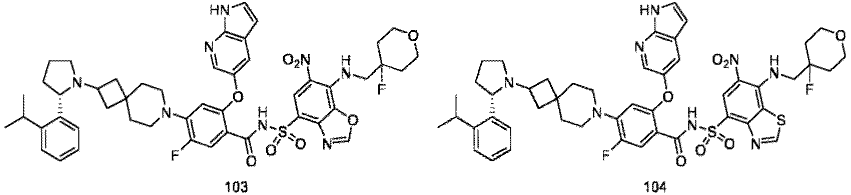 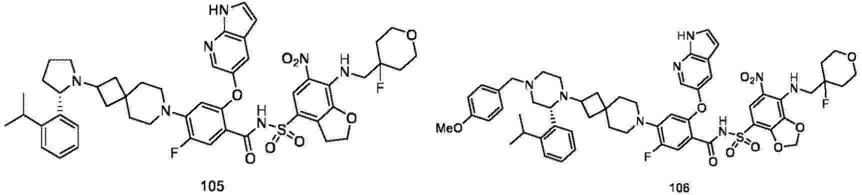 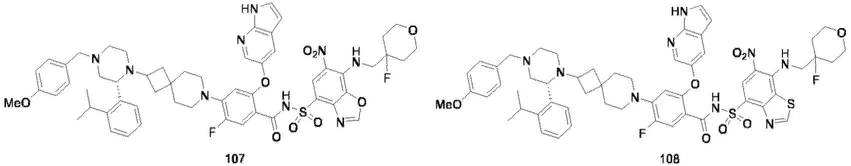 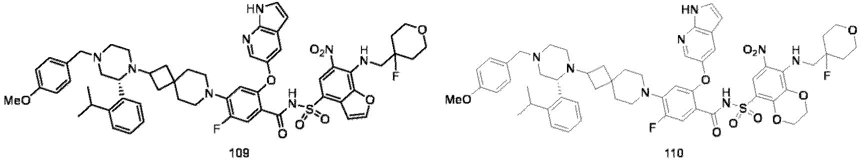 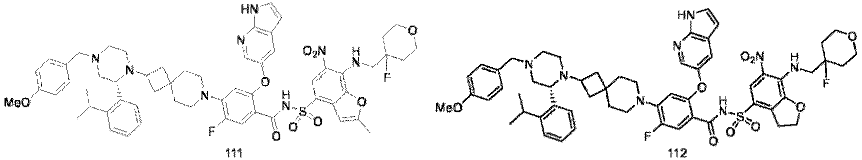 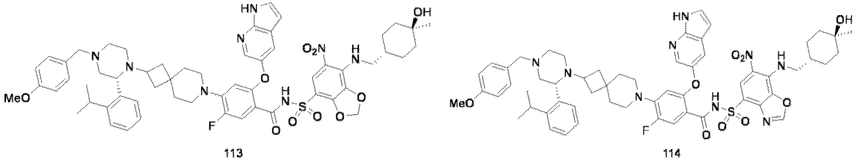 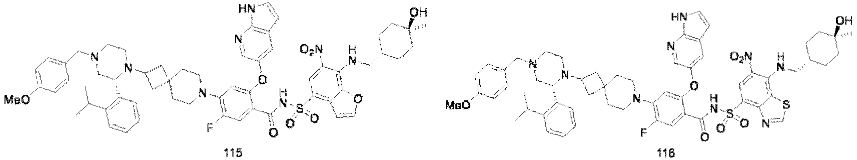 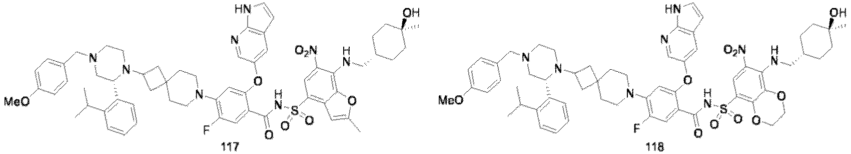 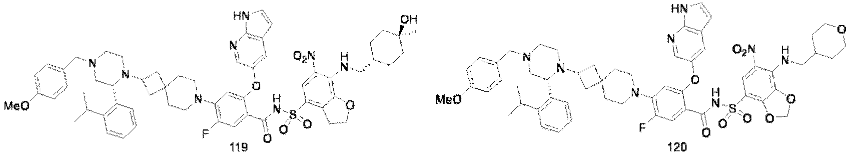 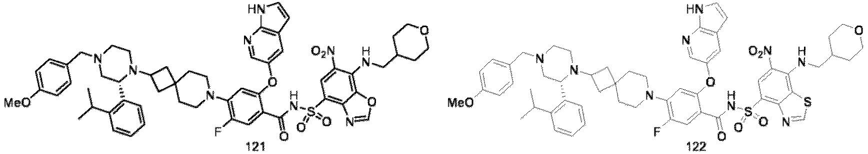 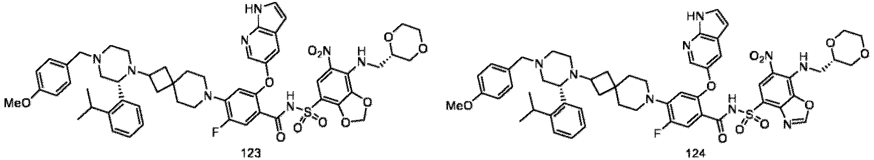 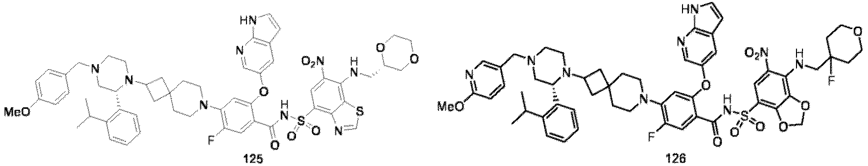 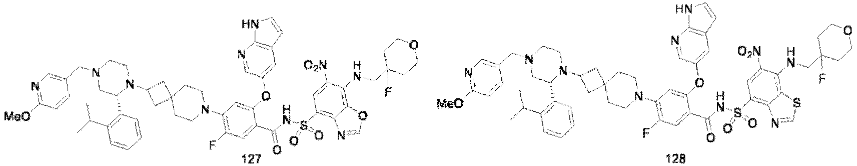 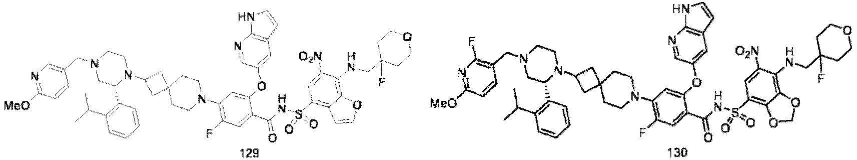 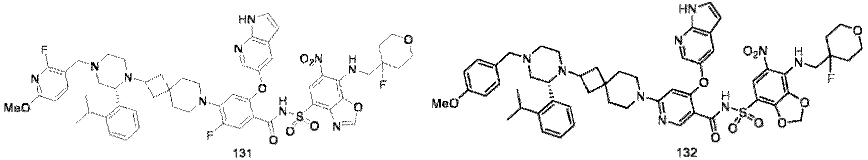 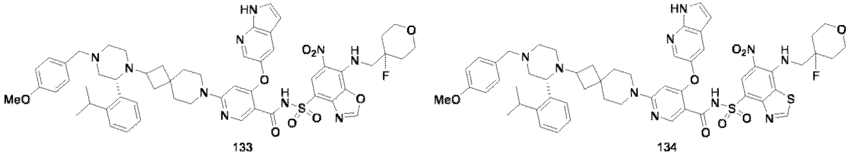 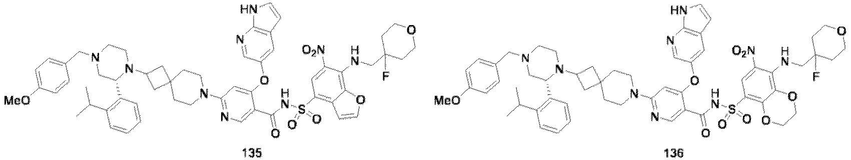 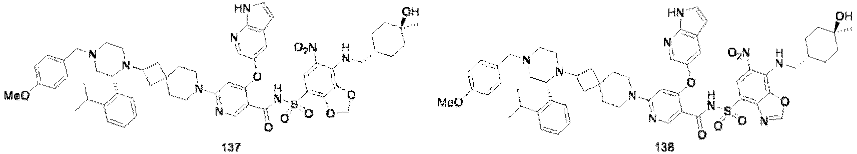 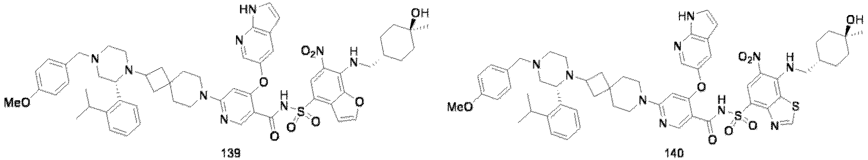 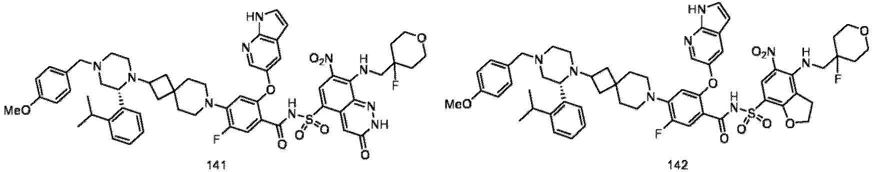 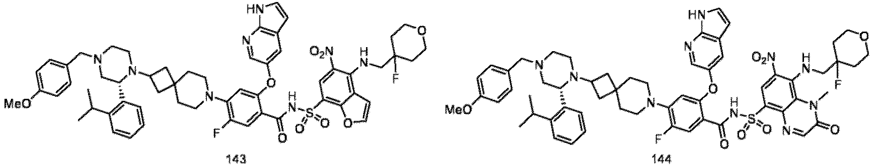 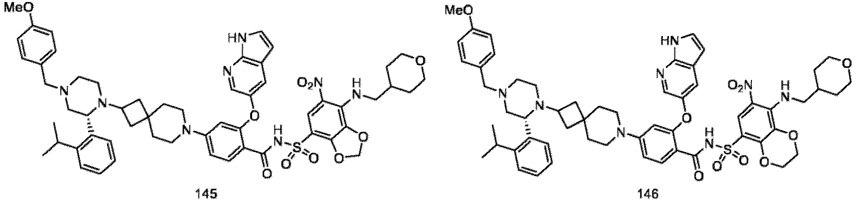 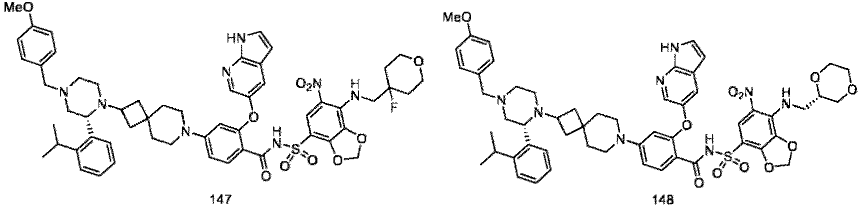 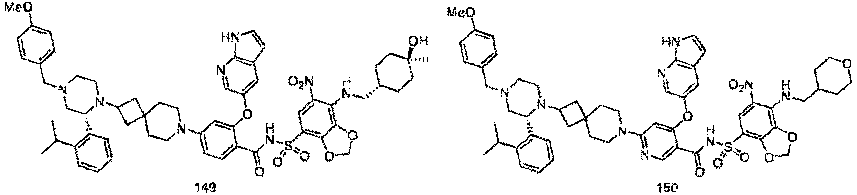 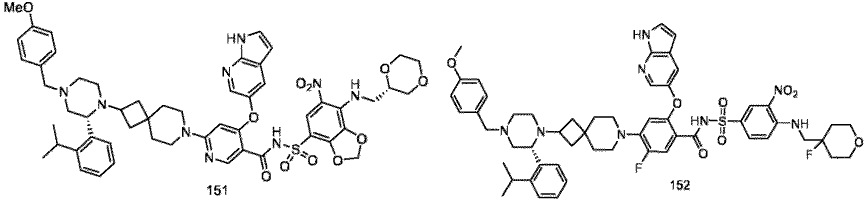 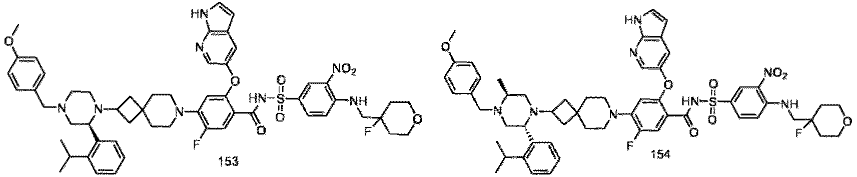 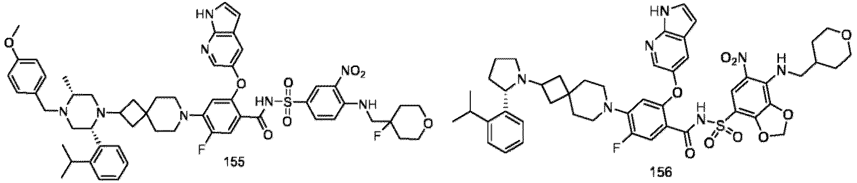 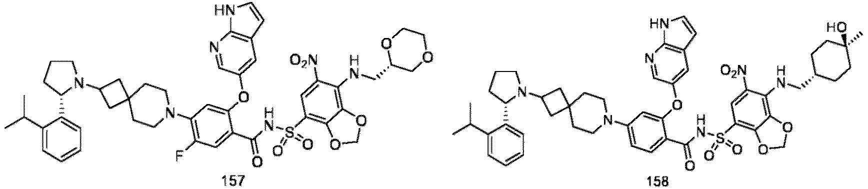 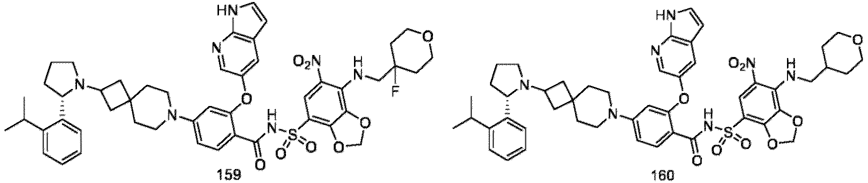 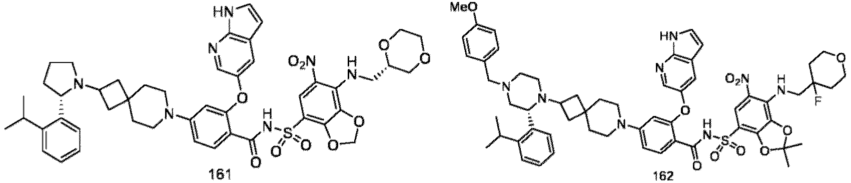 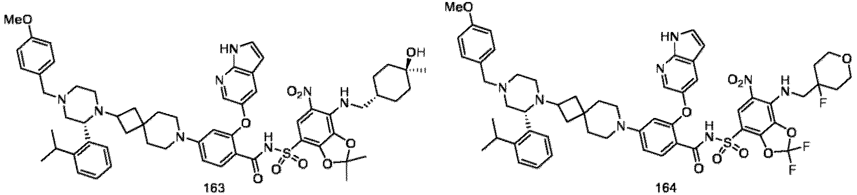 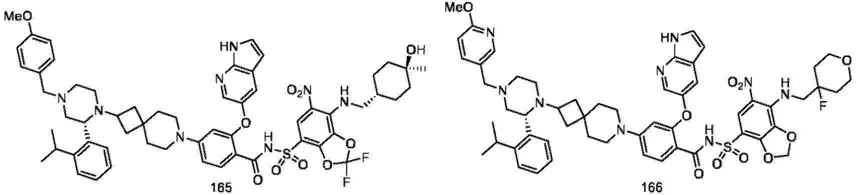 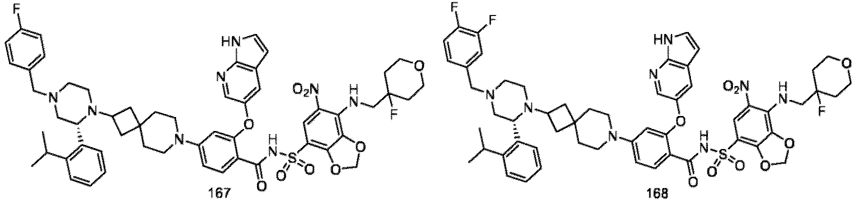 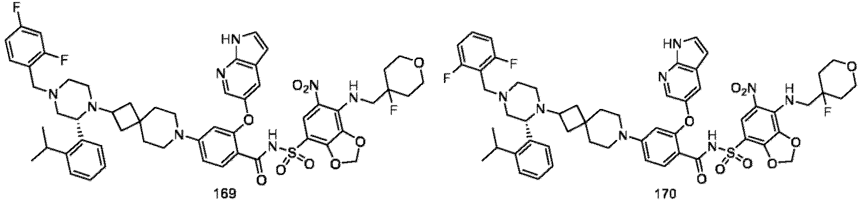 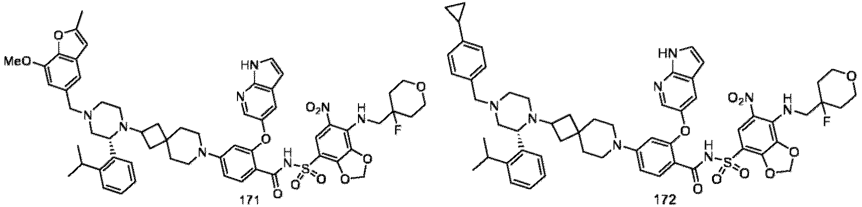 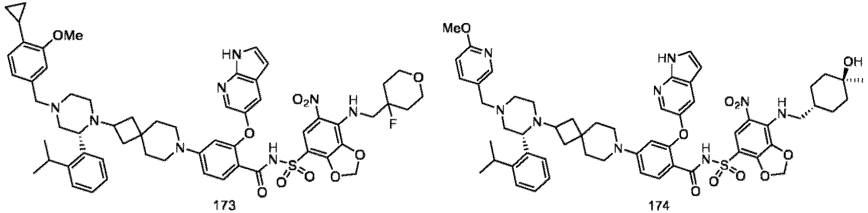 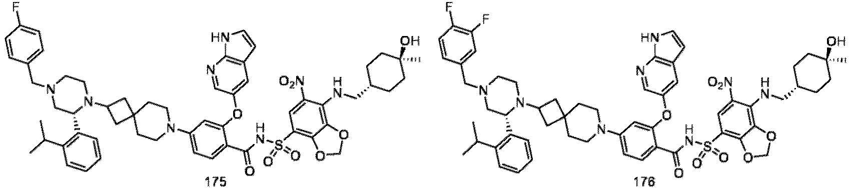 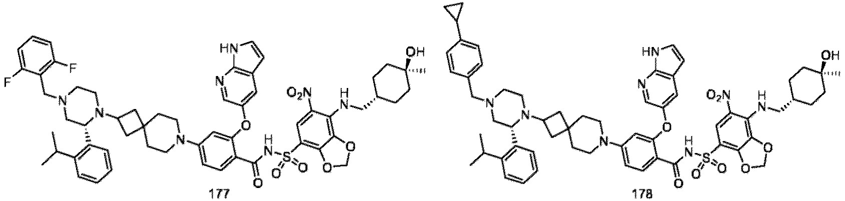 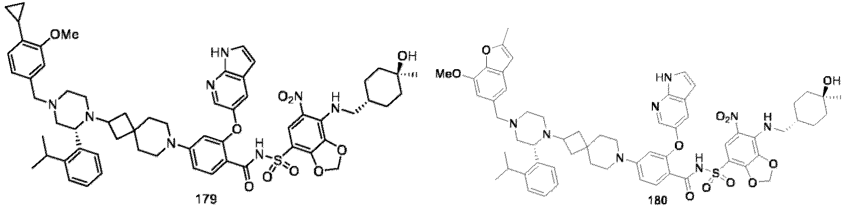 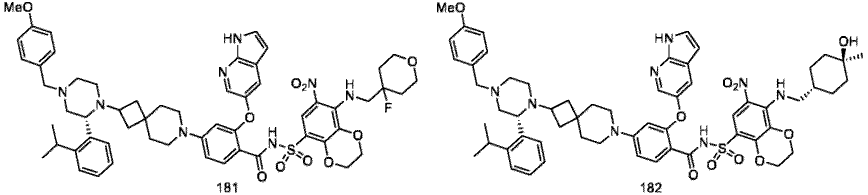 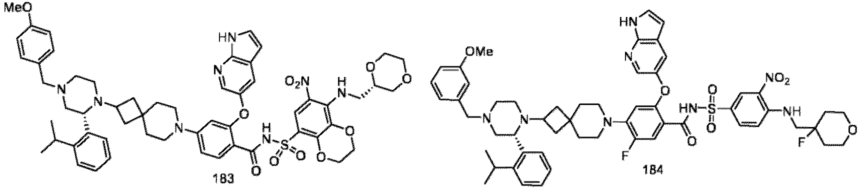 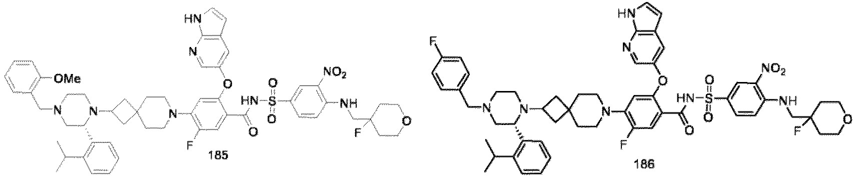 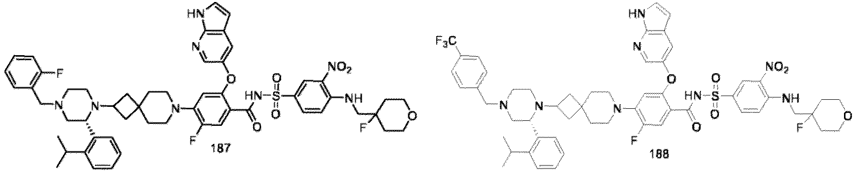 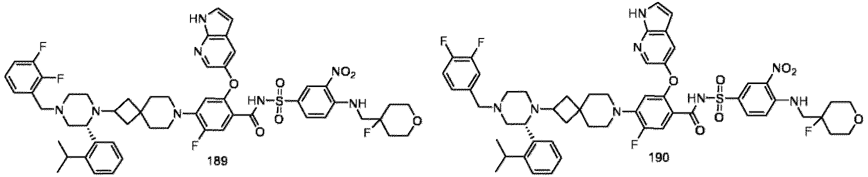 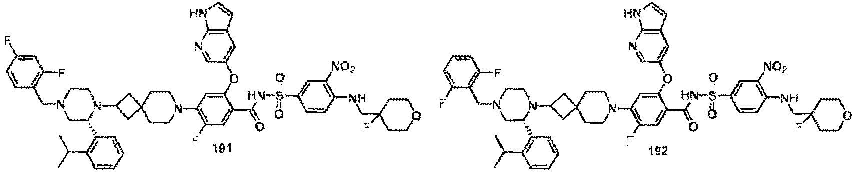 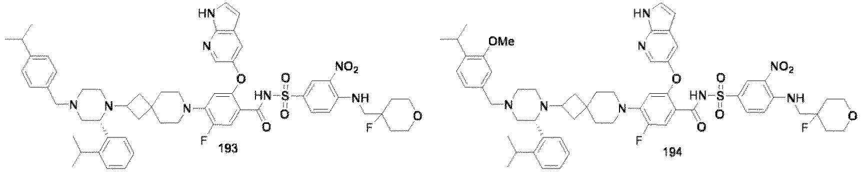 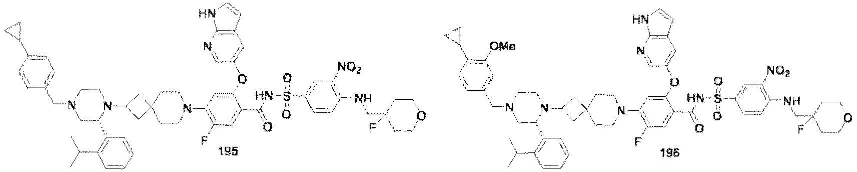 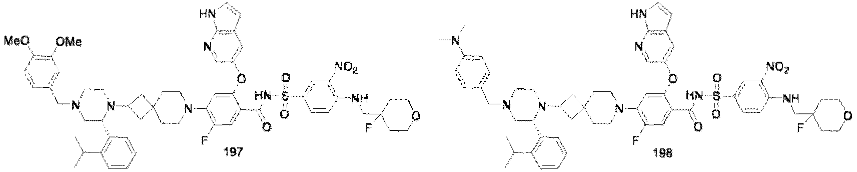 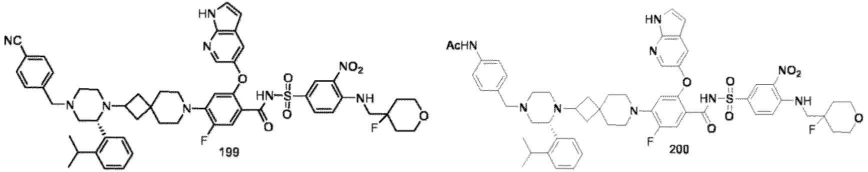 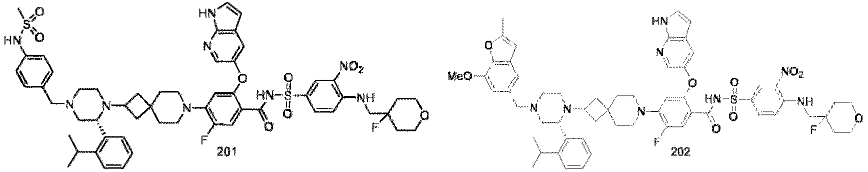 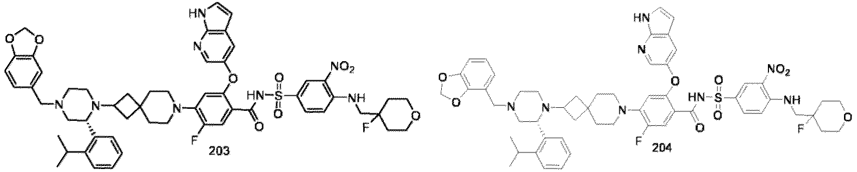 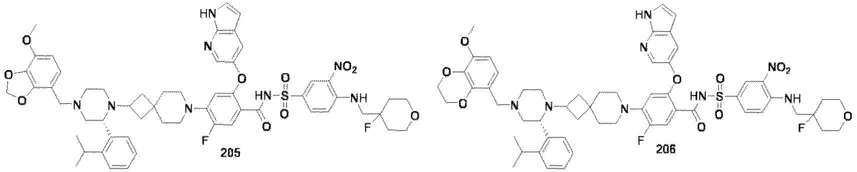 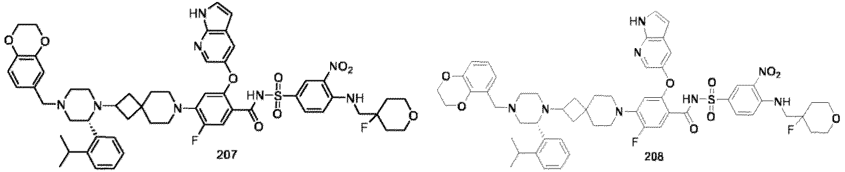 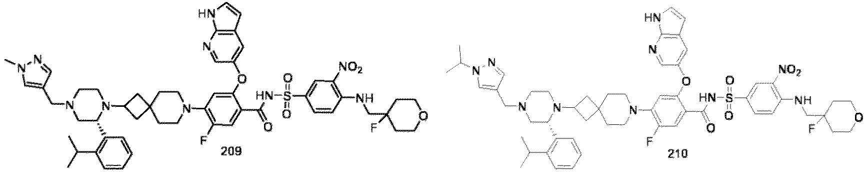 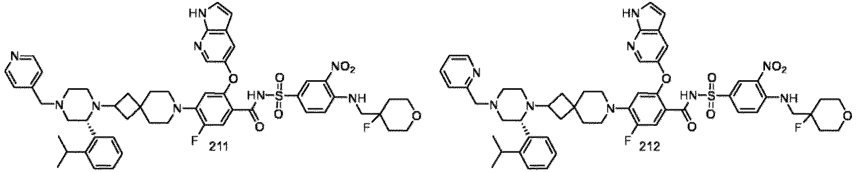 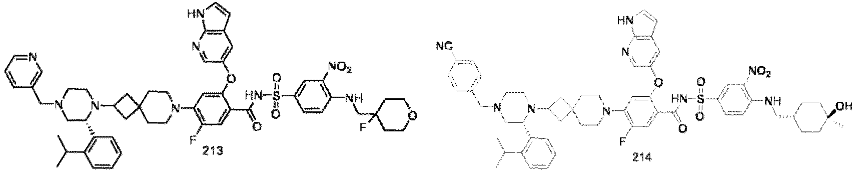 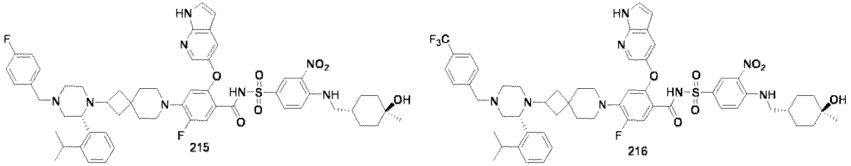 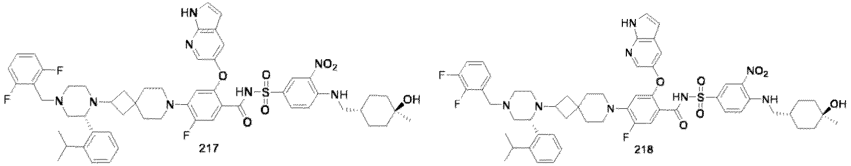 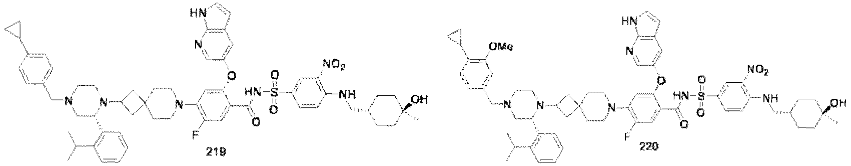 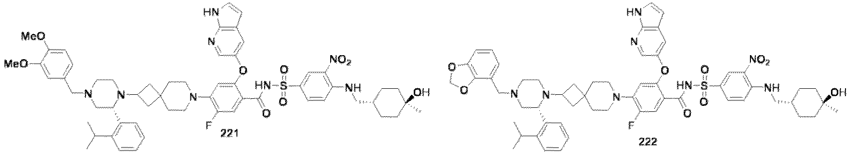 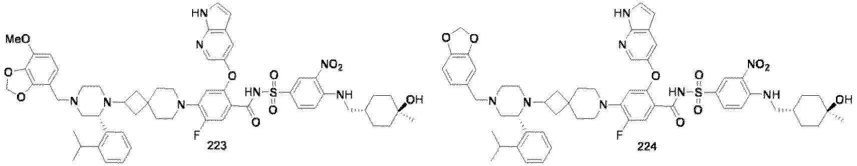 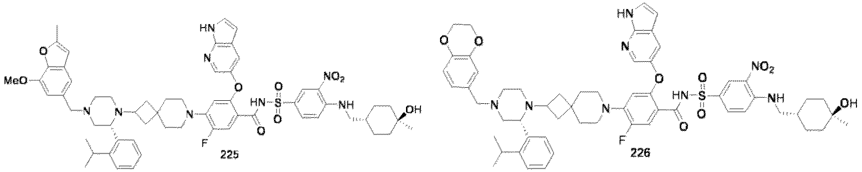 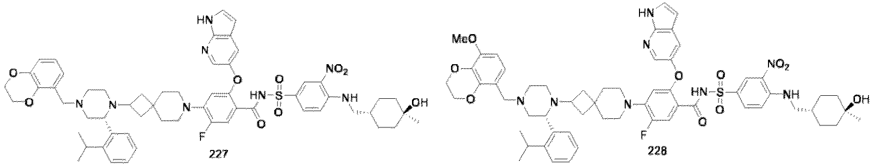 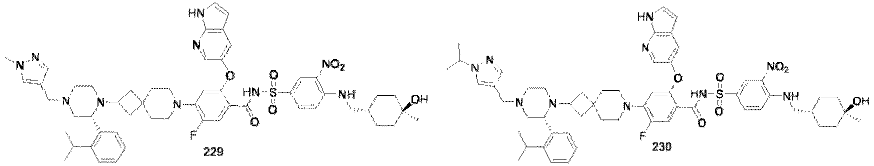 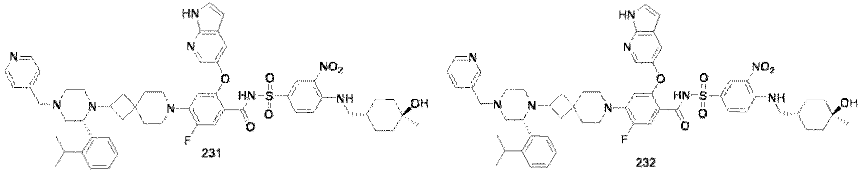 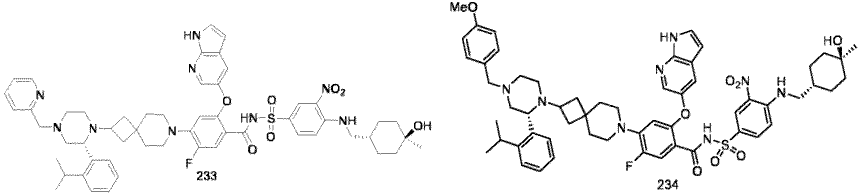 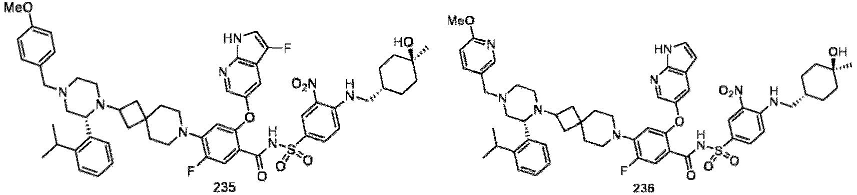 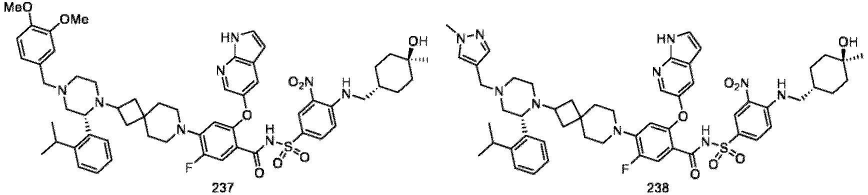 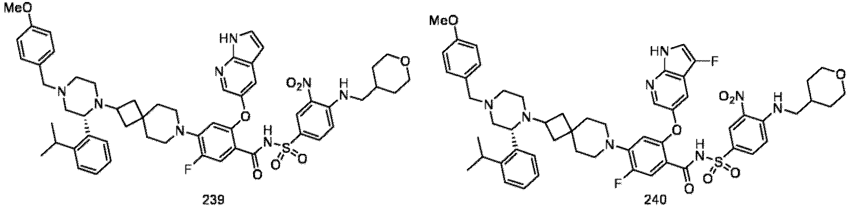 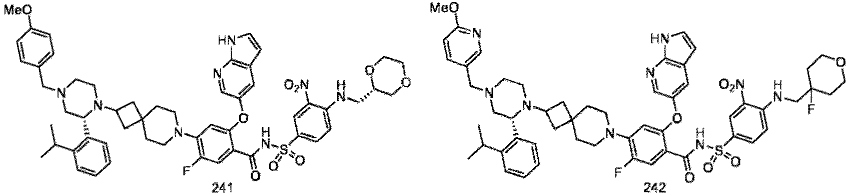 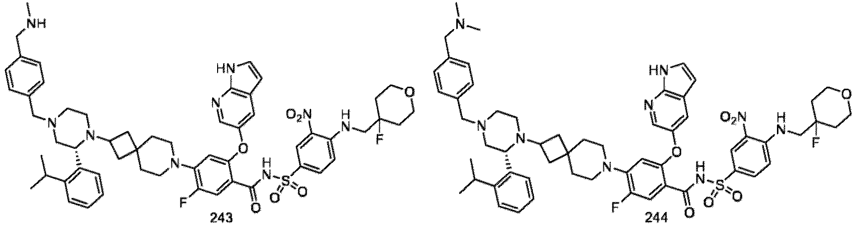 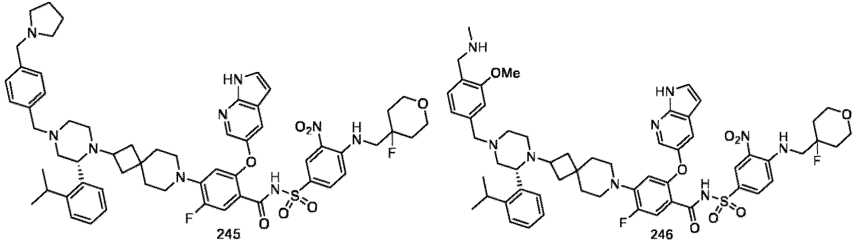 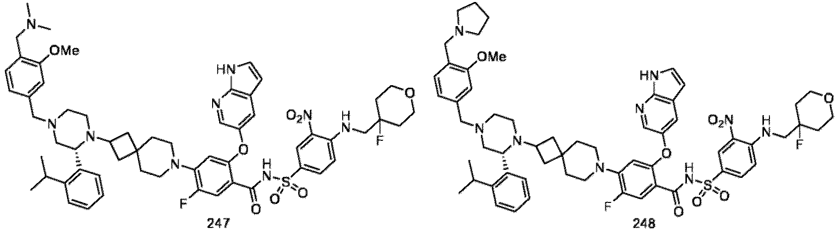 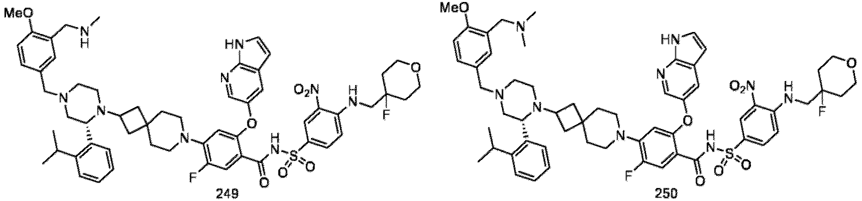 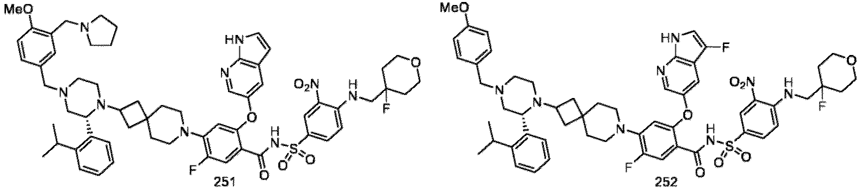 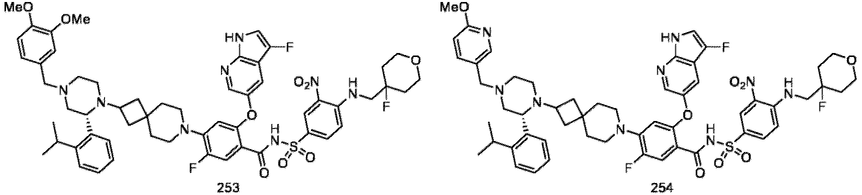 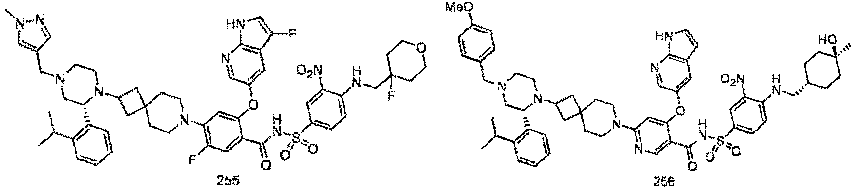 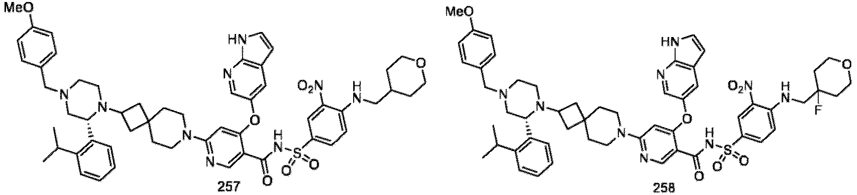 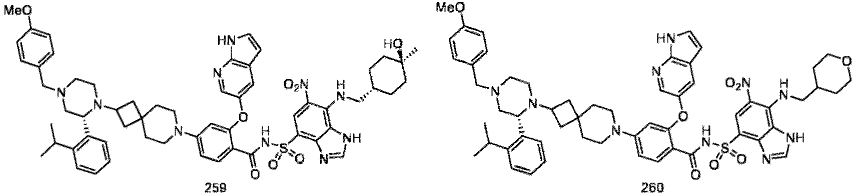 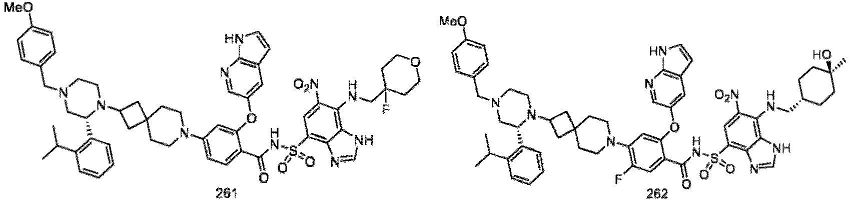 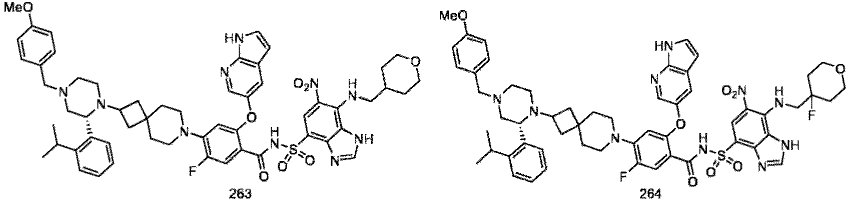 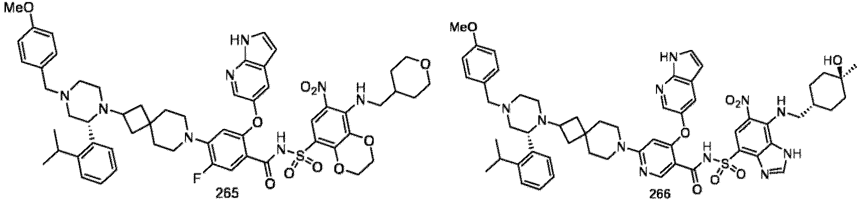 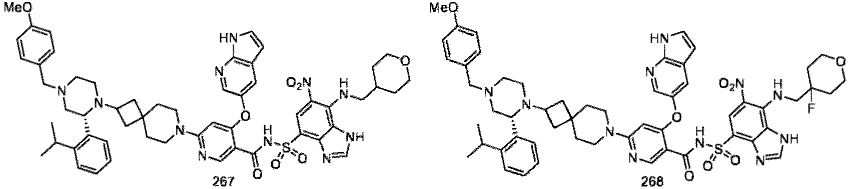 or a stereoisomer or stereoisomer mixture or pharmaceutically acceptable salt thereof.

14. A pharmaceutical composition, comprising one or more of the compound according to any one of claims 1-13.

15. A use of the compound according to any one of claims 1-14 in preparation of a drug for treating a disease, disorder or condition that benefits from inhibition of BCL-2 activity, either alone or in combination with other drugs.

16. The use according to claim 15, wherein the other drugs are selected from alkylating agents, angiogenesis inhibitors, antibodies, antimetabolites, antimitotic agents, antiproliferative agents, antiviral agents, Aurora kinase inhibitors, other promoter inhibitors of cell apoptosis, death receptor pathway activators, Bcr-Abl kinase inhibitors, antibodies to bispecific T cell engagers, antibody-drug conjugates, biological response modifiers, cyclin-dependent kinase inhibitors, cell cycle inhibitors, cyclooxygenase-2 inhibitors, DVDs, leukaemia viral oncogene homologous gene homolog 2 receptor inhibitors, growth factor inhibitors, heat shock protein inhibitors, histone acetylase inhibitors, hormonotherapy, immune preparations, inhibitors of apoptotic protein inhibitors, embedded antibiotics, kinase inhibitors, kinesin inhibitors, JAK2 inhibitors, rapamycin inhibitors targeting mammals, microRNAs, mitogen-activated extracellular signal-regulated kinase inhibitors, multivalent binding proteins, non-steroidal anti-inflammatory drugs, poly-ADP (adenosine diphosphate)-ribose polymerase inhibitors, platinum chemotherapeutic drugs, polo-like kinase inhibitors, phosphoinositide 3-kinase inhibitors, BTK inhibitors, immune checkpoint inhibitors, proteasome inhibitors, purine analogues, pyrimidine analogues, receptor tyrosine kinase inhibitors, retinoid/deltoid plant alkaloids, small interfering RNAs, topoisomerase inhibitors, ubiquitin ligase inhibitors, and the like.

17. The use according to claim 15, wherein the disease, disorder or condition is selected from infectious diseases, immune diseases, inflammatory diseases, and cell proliferation abnormalities.

18. The use according to claim 17, wherein the disease is a hematological disease and/or solid tumor, wherein the hematological disease is preferably one or more of acute myeloid leukemia, acute lymphocytic leukemia, chronic myeloid leukemia, myelofibrosis, myelodysplastic syndrome, diffuse large B-cell lymphoma, follicular lymphoma, chronic lymphocytic leukemia, small lymphocytic lymphoma, mantle cell lymphoma, Waldenstrom's macroglobulinemia, multiple myeloma, and T-cell lymphoma; and the solid tumor is preferably one or more of osteosarcoma, skin cancer, breast cancer, kidney cancer, prostate cancer, colorectal cancer, thyroid cancer, ovarian cancer, pancreatic cancer, glioma, neuroblastoma, epidermoid carcinoma, hemangioma, lung cancer or gastric cancer, restenosis and benign prostatic hypertrophy, and melanoma.
